(19) Europäisches Patentamt
European Patent Office
Office européen des brevets

(11) EP 0 668 931 B1

(12) **EUROPEAN PATENT SPECIFICATION**

(45) Date of publication and mention of the grant of the patent:
**04.01.2006 Bulletin 2006/01**

(21) Application number: **93924904.1**

(22) Date of filing: **28.09.1993**

(51) Int Cl.:
*C12Q 1/68* (2006.01) *C12P 19/34* (2006.01)
*C07H 21/02* (2006.01) *C07H 21/04* (2006.01)

(86) International application number:
**PCT/US1993/009296**

(87) International publication number:
**WO 1994/008050 (14.04.1994 Gazette 1994/09)**

(54) **NUCLEIC ACID LIGANDS AND METHODS FOR PRODUCING THE SAME**

NUKLEINSÄURELIGANDEN UND IHRE HERSTELLUNGSMETHODEN

LIGANDS D'ACIDE NUCLEIQUE ET PROCEDES DE PRODUCTION

(84) Designated Contracting States:
**AT BE CH DE DK ES FR GB GR IE IT LI LU MC NL PT SE**

(30) Priority: **29.09.1992 US 953694**
**21.10.1992 US 964624**
**06.11.1992 US 973333**
**22.04.1993 US 61691**

(43) Date of publication of application:
**30.08.1995 Bulletin 1995/35**

(60) Divisional application:
**05023827.8**

(73) Proprietor: **GILEAD SCIENCES, INC.**
**Foster City,**
**California 94404 (US)**

(72) Inventors:
- **GOLD, Larry M.**
  **Boulder, CO 80303 (US)**
- **TUERK, Craig**
  **Boulder, CO 80303 (US)**
- **TASSET, Diane**
  **Boulder, CO 80301 (US)**
- **JANJIC, Nebojsa**
  **Lafayette, CO 80026 (US)**

(74) Representative: **Armitage, Ian Michael et al**
**Mewburn Ellis LLP**
**York House**
**23 Kingsway**
**London WC2B 6HP (GB)**

(56) References cited:
**WO-A-91/19813**
**WO-A-92/14843**
**WO-A-93/04204**

- **FAMULOK M ET AL: "IN VITRO SELECTION OF SPECIFIC LIGAND-BINDING NUCLEIC ACIDS" ANGEWANDTE CHEMIE, vol. 31, no. 4, 1992, pages 979-988, XP000617744**
- **BARTEL D.P. RT AL.,: "HIV-1 rev regulation involves recognition of non-watson-crick base pairs in viral RNA" CELL, vol. 67, - 1 November 1991 pages 529-536, XP002063385**
- **Science, Vol. 249, issued 03 August 1990, CRAIG TUERK and LARRY GOLD, "Systematic Evolution of Ligands by Exponential Enrichment: RNA Ligands to Bacteriophage T4 DNA Polymerase", pages 505-510, see entire document.**
- **Nature, Vol. 355, issued 06 February 1992, LOUIS C. BOCK et al., "Selection of Single-Stranded DNA Molecules that Bind and Inhibit Human Thrombin", pages 564-566, see Abstract and Figure 1 at page 565.**
- **Nucleic Acids Research, Vol. 18, No. 11, issued 1990, HANS-JURGEN THIESEN and CHRISTIAN BACH, "Target Detection Assay (TDA): a Versatile Procedure to Determine DNA Binding Sites as Demonstrated on SP1", pages 3203-3209, see Abstract and Figure 1 at page 3204.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 668 931 B1

SEQUENCE LISTING

(1) GENERAL INFORMATION:

(i) APPLICANT:

Larry Gold
Craig Tuerk
Diane Tasset
Nebojsa Janjic

(ii) TITLE OF INVENTION: Nucleic Acid Ligands and Methods for Producing the Same

(iii) NUMBER OF SEQUENCES: 159

(iv) CORRESPONDENCE ADDRESS:

(A) ADDRESSEE: Beaton & Swanson, P.C.
(B) STREET: 4582 South Ulster Street Parkway, Suite # 403
(C) CITY: Denver
(D) STATE: Colorado
(E) COUNTRY: USA
(F) ZIP: 80237

(v) COMPUTER READABLE FORM:

(A) MEDIUM TYPE: Diskette, 3.50 inch, 800 Kb storage
(B) COMPUTER: IBM
(C) OPERATING SYSTEM: MS-DOS
(D) SOFTWARE: WordPerfect 5.1

(vi) CURRENT APPLICATION DATA:

(A) APPLICATION NUMBER:
(B) FILING DATE:
(C) CLASSIFICATION:

(vii)PRIOR APPLICATION DATA:

(A) APPLICATION NUMBER: 07/714,131
(B) FILING DATE: 10-JUNE-1991

(vii)PRIOR APPLICATION DATA:

(A) APPLICATION NUMBER: 07/536,428
(B) FILING DATE: 11-JUNE-1990

(vii)PRIOR APPLICATION DATA:

(A) APPLICATION NUMBER: 08/061,691
(B) FILING DATE: 22-APRIL-1993

(vii)PRIOR APPLICATION DATA:

(A) APPLICATION NUMBER: 07/973,333
(B) FILING DATE: 06-NOVEMBER-1992

(vii)PRIOR APPLICATION DATA:

(A) APPLICATION NUMBER: 07/964,624
(B) FILING DATE: 21-OCTOBER-1992

(vii)PRIOR APPLICATION DATA:

(A) APPLICATION NUMBER: 07/953,694
(B) FILING DATE: 29-SEPTEMBER-1992

(viii)ATTORNEY/AGENT INFORMATION:

(A) NAME: Barry J. Swanson
(B) REGISTRATION NUMBER: 33,215
(C) REFERENCE/DOCKET NUMBER: NEX03/PCT

(ix) TELECOMMUNICATION INFORMATION:

(A) TELEPHONE: (303) 850-9900
(B) TELEFAX: (303) 850-9401

(2) INFORMATION FOR SEQ ID NO:1:

(i) SEQUENCE CHARACTERISTICS:

(A) LENGTH: 15 base pairs
(B) TYPE: nucleic acid
(C) STRANDEDNESS: single
(D) TOPOLOGY: linear

(xi) SEQUENCE DESCRIPTION: SEQ ID NO:1:

```
GGTTGGTGTG GTTGG                                                    15
```

(3) INFORMATION FOR SEQ ID NO:2:

(i) SEQUENCE CHARACTERISTICS:

(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:2:

```
AAAAAUCCGA AGUGCAACGG GAAAAUGCAC U                                  31
```

(4) INFORMATION FOR SEQ ID NO:3:

(i) SEQUENCE CHARACTERISTICS:

(A) LENGTH: 85 base pairs
(B) TYPE: nucleic acid
(C) STRANDEDNESS: single
(D) TOPOLOGY: linear

(xi) SEQUENCE DESCRIPTION: SEQ ID NO:3:

CCCGGATCCT CTTTACCTCT GTGTGAGATA CAGAGTCCAC AAACGTGTTC TCAATGCACC 60
CGGTCGGAAG GCCATCAATA GTCCC 85

(5) INFORMATION FOR SEQ ID NO:4:

(i) SEQUENCE CHARACTERISTICS:

(A) LENGTH: 49 base pairs
(B) TYPE: nucleic acid
(C) STRANDEDNESS: single
(D) TOPOLOGY: linear

(xi) SEQUENCE DESCRIPTION: SEQ ID NO:4:

CCGAAGCTTA ATACGACTCA CTATAGGGAC TATTGATGGC CTTCCGACC 49

(6) INFORMATION FOR SEQ ID NO:5:

(i) SEQUENCE CHARACTERISTICS:

(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:5:

CCCGGATCCT CTTTACCTCT GTGTG 25

(7) INFORMATION FOR SEQ ID NO:6:

(i) SEQUENCE CHARACTERISTICS:

(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:6:

UUCCGNNNNN NNNCGGGAAA A 21

(8) INFORMATION FOR SEQ ID NO:7:

(i) SEQUENCE CHARACTERISTICS:

(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:7:

UCCGNNNNNN NNCGGGAAAA NNNN 24

(9) INFORMATION FOR SEQ ID NO:8:

(i) SEQUENCE CHARACTERISTICS:

(A) LENGTH: 27 base pairs
(B) TYPE: nucleic acid
(C) STRANDEDNESS: single
(D) TOPOLOGY: linear

(xi) SEQUENCE DESCRIPTION: SEQ ID NO:8:

NNUUCCGNNN NNNNNCGGGA AAANNNN 27

(10) INFORMATION FOR SEQ ID NO:9:

(i) SEQUENCE CHARACTERISTICS:

(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO:9:

GGAUCGGAAN NAGUAGGC 18

(11) INFORMATION FOR SEQ ID NO:10:

(i) SEQUENCE CHARACTERISTICS:

(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:10:

GCGGCUUUGG GCGCCGUGCU U 21

(12) INFORMATION FOR SEQ ID NO:11:

(i) SEQUENCE CHARACTERISTICS:

(A) LENGTH: 57 base pairs
(B) TYPE: nucleic acid
(C) STRANDEDNESS: single
(D) TOPOLOGY: linear

(xi) SEQUENCE DESCRIPTION: SEQ ID NO:11:

GGUCCGAAGU GCAACGGGAA AAUGCACUAU GAAAGAAUUU UAUAUCUCUA UUGAAAC 57

(13) INFORMATION FOR SEQ ID NO:12:

(i) SEQUENCE CHARACTERISTICS:

(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:12:

**CCGGATCCGT TTCAATAGAG ATATAAAATT C 31**

(14) INFORMATION FOR SEQ ID NO:13:

(i) SEQUENCE CHARACTERISTICS:

(A) LENGTH: 55 base pairs
(B) TYPE: nucleic acid
(C) STRANDEDNESS: single
(D) TOPOLOGY: linear

(xi) SEQUENCE DESCRIPTION: SEQ ID NO:13:

**GGGCAAGCTT TAATACGACT CACTATAGGT CCGAAGTGCA ACGGGAAAAT GCACT 55**

(15) INFORMATION FOR SEQ ID NO:14:

(i) SEQUENCE CHARACTERISTICS:

(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:14:

**GTTTCAATAG AGATATAAAA TTCTTTCATA G 31**

(16) INFORMATION FOR SEQ ID NO:15:

(i) SEQUENCE CHARACTERISTICS:

(A) LENGTH: 89 base pairs
(B) TYPE: nucleic acid
(C) STRANDEDNESS: single
(D) TOPOLOGY: linear

(xi) SEQUENCE DESCRIPTION: SEQ ID NO:15:

**GTTTCAATAG AGATATAAAA TTCTTTCATA GNNNNNNNNN NNNNNNNNNN NNNNNNNNNN 60**
**NAGTGCATTT TCCCGTTGCA CTTCGGACC 89**

(17) INFORMATION FOR SEQ ID NO:16:

(i) SEQUENCE CHARACTERISTICS:

(A) LENGTH: 83 base pairs
(B) TYPE: nucleic acid
(C) STRANDEDNESS: single
(D) TOPOLOGY: linear

(xi) SEQUENCE DESCRIPTION: SEQ ID NO:16:

CCCGGATCCT CTTTACCTCT GTGTGAGATA CAGAGTCCAC AACGTGTTCT CAATGACCCG 60
GTCGGAAGGC CATCAATAGT CCC 83

(18) INFORMATION FOR SEQ ID NO:17:

(i) SEQUENCE CHARACTERISTICS:

(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:17:

CCGAAGCTTA ATACGACTCA CTATAGGGAC TATTGATGGG CCTTCCGACC 50

(19) INFORMATION FOR SEQ ID NO:18:

(i) SEQUENCE CHARACTERISTICS:

(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:18:

CCGAAGCTTA ATACGACTCA CTATAGGGAG CTCAGAATAA ACGCTCAA 48

(20) INFORMATION FOR SEQ ID NO:19:

(i) SEQUENCE CHARACTERISTICS:

(A) LENGTH: 87 base pairs
(B) TYPE: nucleic acid
(C) STRANDEDNESS: single
(D) TOPOLOGY: linear

(xi) SEQUENCE DESCRIPTION: SEQ ID NO:19:

GCCGGATCCG GGCCTCATGT CGAANNNNNN NNNNNNNNNN NNNNNNNNNN NNNNNNNNNN 60
NNNNTTGAGC GTTTATTCTG AGCTCCC 87

(21) INFORMATION FOR SEQ ID NO:20:

(i) SEQUENCE CHARACTERISTICS:

(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:20:

GCCGGATCCG GGCCTCATGT CGAA 24

(22) INFORMATION FOR SEQ ID NO:21:

(i) SEQUENCE CHARACTERISTICS:

(A) LENGTH: 77 base pairs
(B) TYPE: nucleic acid
(C) STRANDEDNESS: single
(D) TOPOLOGY: linear

(xi) SEQUENCE DESCRIPTION: SEQ ID NO:21:

GGGAGCUCAG AAUAAACGCU CAANNNNNNN NNNNNNNNNN NNNNNNNNNN NNNUUCGACA 60
UGAGGCCCGG AUCCGGC 77

(23) INFORMATION FOR SEQ ID NO:22:

(i) SEQUENCE CHARACTERISTICS:

(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:22:

CCGAAGCTTA ATACGACTCA CTATAGGGAG CTCAGAATAA ACGCTCAA 48

(24) INFORMATION FOR SEQ ID NO:23:

(i) SEQUENCE CHARACTERISTICS:

(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:23:

GCCGGATCCG GGCCTCATGT CGAA 24

(25) INFORMATION FOR SEQ ID NO:24:

(i) SEQUENCE CHARACTERISTICS:

(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:24:

GGGAGAUGCC UGUCGAGCAU GCUGNNNNNN NNNNNNNNNN NNNNNNNNNN NNNNGUAGCU 60
AAACAGCUUU GUCGACGGG 79

(26) INFORMATION FOR SEQ ID NO:25:

(i) SEQUENCE CHARACTERISTICS:

(A) LENGTH: 50 base pairs
(B) TYPE: nucleic acid
(C) STRANDEDNESS: single
(D) TOPOLOGY: linear

(xi) SEQUENCE DESCRIPTION: SEQ ID NO:25:

CCCGAAGCTT AATACGACTC ACTATAGGGA GATGCCTGTC GAGCATGCTG 50

(27) INFORMATION FOR SEQ ID NO:26:

(i) SEQUENCE CHARACTERISTICS:

(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:26:

CCCGTCGACA AAGCTGTTTA GCTAC 25

(28) INFORMATION FOR SEQ ID NO:27:

(i) SEQUENCE CHARACTERISTICS:

(A) LENGTH: 9 base pairs
(B) TYPE: nucleic acid
(C) STRANDEDNESS: single
(D) TOPOLOGY: linear

(xi) SEQUENCE DESCRIPTION: SEQ ID NO:27:

CUAACCNGG 9

(29) INFORMATION FOR SEQ ID NO:28:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:28:

UGCUAUUCGC CUAACUCGGC GCUCCUACCU 30

(30) INFORMATION FOR SEQ ID NO:29:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:29:

AUCUCCUCCC GUCGAAGCUA ACCUGGCCAC 30

(31) INFORMATION FOR SEQ ID NO:30:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:10:

UCGGCGAGCU AACCAAGACA CUCGCUGCAC 30

(32) INFORMATION FOR SEQ ID NO:31:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:31:

GUAGCACUAU CGGCCUAACC CGGUAGCUCC 30

(33) INFORMATION FOR SEQ ID NO:32:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:32:

ACCCGCGGCC UCCGAAGCUA ACCAGGACAC 30

(34) INFORMATION FOR SEQ ID NO:33:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:33:

UGGGUGCUAA CCAGGACACA CCCACGCUGU 30

(35) INFORMATION FOR SEQ ID NO:34:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:34:

CACGCACAGC UAACCAAGCC ACUGUGCCCC 30

(36) INFORMATION FOR SEQ ID NO:35:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:35:

CUGCGUGGUA UAACCACAUG CCCUGGGCGA 30

(37) INFORMATION FOR SEQ ID NO:36:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:36:

UGGGUGCUUA ACCAGGCCAC ACCCUGCUGU 30

(38) INFORMATION FOR SEQ ID NO:37:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:37:

CUAGGUGCUA UCCAGGACUC UCCCUGGUCC 30

(39) INFORMATION FOR SEQ ID NO:38:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:38:

UGCUAUUCGC CUAGCUCGGC GCUCCUACCU 30

(40) INFORMATION FOR SEQ ID NO:39:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:39:

AGCUAUUCGC CCAACCCGGC GCUCCCGACC 30

(41) INFORMATION FOR SEQ ID NO:40:

(i) SEQUENCE CHARACTERISTICS:

(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:40:

ACCAGCUGCG UGCAACCGCA CAUGCCUGG 29

(42) INFORMATION FOR SEQ ID NO:41:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:41:

CAGGCCCCGU CGUAAGCUAA CCUGGACCCU 30

(43) INFORMATION FOR SEQ ID NO:42:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:42:

UGGGUGCUAA CCACCACACA CUCACGCUGU 30

(44) INFORMATION FOR SEQ ID NO:43:

(i) SEQUENCE CHARACTERISTICS:

(A) LENGTH: 26 base pairs
(B) TYPE: nucleic acid
(C) STRANDEDNESS: single
(D) TOPOLOGY: linear

(xi) SEQUENCE DESCRIPTION: SEQ ID NO:43:

RRGGHAACGY WNNGDCAAGN NCACYY 26

(45) INFORMATION FOR SEQ ID NO:44:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:44:

GGGUAACGUU GUGACAAGUA CACCUGCGUC 30

(46) INFORMATION FOR SEQ ID NO:45:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:45:

GGGGCAACGC UACAGACAAG UGCACCCAAC 30

(47) INFORMATION FOR SEQ ID NO:46

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:46:

CGUCAGAAGG CAACGUAUAG GCAAGCACAC 30

(48) INFORMATION FOR SEQ ID NO:47:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:47:

CCUCUCGAAG ACAACGCUGU GACAAGACAC 30

(49) INFORMATION FOR SEQ ID NO:48:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:48:

AGUGGGAAAC GCUACUUGAC AAGACACCAC 30

(50) INFORMATION FOR SEQ ID NO:49:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:49:

GGCUACGCUA AUGACAAGUG CACUUGGGUG 30

(51) INFORMATION FOR SEQ ID NO:50:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:50:

CUCUGGUAAC GCAAUGUCAA GUGCACAUGA 30

(52) INFORMATION FOR SEQ ID NO:51:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:51:

AGCCGCAGGU AACGGACCGG CGAGACCAUU 30

(53) INFORMATION FOR SEQ ID NO:52:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:52:

ACGAGCUUCG UAACGCUAUC GACAAGUGCA 30

(54) INFORMATION FOR SEQ ID NO:53:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:53:

AAGGGGAAAC GUUGAGUCCG GUACACCCUG 30

(55) INFORMATION FOR SEQ ID NO:54:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:54:

AGGGUAACGU ACUGGCAAGC UCACCUCAGC 30

(56) INFORMATION FOR SEQ ID NO:55:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:55:

GAGGUAACGU ACGACAAGAC CACUCCAACU 30

(57) INFORMATION FOR SEQ ID NO:56:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:56:

AGGUAACGCU GAGUCAAGUG CACUCGACAU 30

(58) INFORMATION FOR SEQ ID NO:57:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:57:

GGGAAACGCU AUCGACGAGU GCACCCGGCA 30

(59) INFORMATION FOR SEQ ID NO:58:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:58:

CCGAGGGUAA CGUUGGGUCA AGCACACCUC 30

(60) INFORMATION FOR SEQ ID NO:59:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:59:

UCGGGGUAAC GUAUUGGCAA GGCACCCGAC 30

(61) INFORMATION FOR SEQ ID NO:60:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:60:

GGUAACGCUG UGGACAAGUG CACCAGCUGC 30

(62) INFORMATION FOR SEQ ID NO: 61:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:61:

AGGGUAACGU ACUGGCAAGC UCACCUCAGC 30

(63) INFORMATION FOR SEQ ID NO:62:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:62:

AGGGUAACGU AUAGUCAAGA CACCUCAAGU 30

(64) INFORMATION FOR SEQ ID NO:63:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:63:

GGGUAACGCA UUGGCAAGAC ACCCAGCCCC 30

(65) INFORMATION FOR SEQ ID NO:64:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:64:

GAGGAAACGU ACCGUCGAGC CACUCCAUGC 30

(66) INFORMATION FOR SEQ ID NO:65:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:65:

AGGUAACGCU GAGUCAAGUG CACUCGACAU 30

(67) INFORMATION FOR SEQ ID NO:66:

(i) SEQUENCE CHARACTERISTICS:

(A) LENGTH: 30 base pairs
(B) TYPE: nucleic acid
(C) STRANDEDNESS: single
(D) TOPOLOGY: linear
(xi) SEQUENCE DESCRIPTION: SEQ ID NO:66:

GGGUAACGUG UGACAAGAUC ACCCAGUUUG 30

(68) INFORMATION FOR SEQ ID NO:67:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:67:

CACAGGGCAA CGCUGCUGAC AAGUGCACCU 30

(69) INFORMATION FOR SEQ ID NO:68:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:68:

ACGCCAAGUG AGUCAGCAAC AGAGCGUCCG 30

(70) INFORMATION FOR SEQ ID NO:69:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:69:

CCAGUGAGUC CUGGUAAUCC GCAUCGGGCU 30

(71) INFORMATION FOR SEQ ID NO:70:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:70:

CUUCAGAACG GCAUAGUGGU CGGCCGCGCC 30

(72) INFORMATION FOR SEQ ID NO:71:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:71:

AGGUCACUGC GUCACCGUAC AUGCCUGGCC 30

(73) INFORMATION FOR SEQ ID NO:72:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:72:

UCCAACGAAC GGCCCUCGUA UUCAGCCACC 30

(74) INFORMATION FOR SEQ ID NO:73:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:73:

ACUGGAACCU GACGUAGUAC AGCGACCCUC 30

(75) INFORMATION FOR SEQ ID NO:74:

(i) SEQUENCE CHARACTERISTICS:

(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:74:

UCUCGCUGCG CCUACACGGC AUGCCGGGA 29

(76) INFORMATION FOR SEQ ID NO:75:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:75:

GAUCACUGCG CAAUGCCUGC AUACCUGGUC 30

(77) INFORMATION FOR SEQ ID NO:76:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:76:

UCUCGCUGCG CCUACACGGC AUGCCCGGGA 30

(78) INFORMATION FOR SEQ ID NO:77:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:77:

UGACCAGCUG CAUCCGACGA UAUACCCUGG 30

(79) INFORMATION FOR SEQ ID NO:78:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:78:

GGCACACUCC AACGAGGUAA CGUUACGGCG 30

(80) INFORMATION FOR SEQ ID NO:79:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:79:

AGCGGAACGC CACGUAGUAC GCCGACCCUC 30

(81) INFORMATION FOR SEQ ID NO:80:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:80:

ACCCACGCCC GACAACCGAU GAGUUCUCGG 30

(82) INFORMATION FOR SEQ ID NO:81:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:81:

UGCUUUGAAG UCCUCCCCGC CUCUCGAGGU 30

(83) INFORMATION FOR SEQ ID NO:82:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:82:

AUGCUGAGGA UAUUGUGACC ACUUCGGCGU 30

(84) INFORMATION FOR SEQ ID NO:83:

(i) SEQUENCE CHARACTERISTICS:

(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:83:

ACCCACGCCC GACAACCGAU GAGCUCGGA 29

(85) INFORMATION FOR SEQ ID NO:84:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:84:

AGUCCGGAUG CCCCACUGGG ACUACAUUGU 30

(86) INFORMATION FOR SEQ ID NO:85:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:85:

AAGUCCGAAU GCCACUGGGA CUACCACUGA 30

(87) INFORMATION FOR SEQ ID NO:86:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:86:

ACUCUCACUG CGAUUCGAAA UCAUGCCUGG 30

(88) INFORMATION FOR SEQ ID NO:87:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:87:

AGGCUGGGUC ACCGACAACU GCCCGCCAGC 30

(89) INFORMATION FOR SEQ ID NO:88:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:88:

AGCCGCAGGU AACGGACCGG CGAGACCACU 30

(90) INFORMATION FOR SEQ ID NO:89:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:89:

GCAUGAAGCG GAACUGUAGU ACGCGAUCCA 30

(91) INFORMATION FOR SEQ ID NO:90:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:90:

GGGUAACGUU GUGACAAGUA CACCUGCGUU 30

(92) INFORMATION FOR SEQ ID NO:91:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:91:

GGGUAACGUU GUGACAAGUA CACCUGCGUC 30

(93) INFORMATION FOR SEQ ID NO:92:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:92:

GGGUAACGUU GUGACAAGUA CACCUGCGUC 30

(94) INFORMATION FOR SEQ ID NO:93:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:93:

GGGUAACGUU GUGACAACUA CACCUGCGUC 30

(95) INFORMATION FOR SEQ ID NO:94:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:94:

GGGUAACGUU GUGACAACUA CACCUGCGUC 30

(96) INFORMATION FOR SEQ ID NO:95:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:95:

GGGUAACGUU GUGACAACUA CACCUGCGUC 30

(97) INFORMATION FOR SEQ ID NO:96:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:96:

CGUCAGAAGG CAACGUAUAG GCAAGCACAC 30

(98) INFORMATION FOR SEQ ID NO:97:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:97:

GUAGCACUAU CGGCCUAACC CGGUAGCUCC 30

(99) INFORMATION FOR SEQ ID NO:98:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:98:

ACCCGCGGCC UCCGAAGCUA ACCAGGACAC 30

(100) INFORMATION FOR SEQ ID NO:99:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:99:

AGGUCACUGC GUCACCGUAC AUGCCUGGCC 30

(101) INFORMATION FOR SEQ ID NO:100:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:100:

AGGUCACUGC GUCACCGUAC AUGCCUGGCC 30

(102) INFORMATION FOR SEQ ID NO:101:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:101:

GGCACACUCC AACGAGGUAA CGUUACGGCG 30

(103) INFORMATION FOR SEQ ID NO:102:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:102:

GGGGCAACGC UACAGACAAG UGCACCCAAC 30

(104) INFORMATION FOR SEQ ID NO:103:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:103:

GGGGCAACGC UACAGACAAG UGCACCCAAC 30

(105) INFORMATION FOR SEQ ID NO:104:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:104:

GGGGCAACGC UACAGACAAG UGCACCCAAC 30

(106) INFORMATION FOR SEQ ID NO:105:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:105:

UGGGUGCUAA CCAGGACACA CCCACGCUGU 30

(107) INFORMATION FOR SEQ ID NO:106:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:106:

CCGAGGGUAA CGUUGGGUCA AGCACACCUC 30

(108) INFORMATION FOR SEQ ID NO:107:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:107:

GGGAAACGCU AUCGACGAGU GCACCCGGCA 30

(109) INFORMATION FOR SEQ ID NO:108:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:108:

GGGAAACGCU AUCGACGAGU GCACCCGGCA 30

(110) INFORMATION FOR SEQ ID NO:109:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:109:

ACUCUCACUG CGAUUCGAAA UCAUGCCUGG 30

(111) INFORMATION FOR SEQ ID NO:110:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:110:

GCAUGAAGCG GAACUGUAGU ACGCGAUCCA 30

(112) INFORMATION FOR SEQ ID NO:111:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:111:

GCAUGAAGCG GAACUGUAGU ACGCGAUCCA 30

(113) INFORMATION FOR SEQ ID NO:112:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:112:

AGGGUAACGU ACUGGCAAGC UCACCUCAGC 30

(114) INFORMATION FOR SEQ ID NO:113:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:113:

AGGGUAACGU ACUGGCAAGC UCACCUCAGC 30

(115) INFORMATION FOR SEQ ID NO:114:

(i) SEQUENCE CHARACTERISTICS:

(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:114:

GGUAACGCUG UGGACAAGUG CACCAGCUGC 30

(116) INFORMATION FOR SEQ ID NO:115:

(i) SEQUENCE CHARACTERISTICS:

(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:115:

UAGCUCGUGA GGCUUUCGUG CUGUUCCGAG CU 32

(117) INFORMATION FOR SEQ ID NO:116:

(i) SEQUENCE CHARACTERISTICS:

(A) LENGTH: 31 base pairs
(B) TYPE: nucleic acid
(C) STRANDEDNESS: single
(D) TOPOLOGY: linear

(xi) SEQUENCE DESCRIPTION: SEQ ID NO:116:

UGCAUGUGAG GCGGUAACGC UGUUCCGUGC U 31

(118) INFORMATION FOR SEQ ID NO:117:

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:117:

UGGUGAGUGA GGCCGAUGCU GUUCCUCGCC GCU 33

(119) INFORMATION FOR SEQ ID NO:118:

(i) SEQUENCE CHARACTERISTICS:

(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:118:

UGACGCGCGA GGUCUUGGUA CUGUUCCGUG GCUCU 35

(120) INFORMATION FOR SEQ ID NO:119:

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:119:

UCUGGGUGAG ACUUGAAGUC GUUCCCCAGG UCU 33

(121) INFORMATION FOR SEQ ID NO:120:

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:120:

UCCCGGUGAA GCAUAAUGCU GUUCCUGGGG UCU 33

(122) INFORMATION FOR SEQ ID NO:121

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:121:

UGGGAGUGAG GUUCCCCGUU CCUCCCGCAC CCU 33

(123) INFORMATION FOR SEQ ID NO:122:

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:122:

UAGCGAUGUG AAGUGAUACU GGUCCAUCGU GCU 33

(124) INFORMATION FOR SEQ ID NO:123:

(i) SEQUENCE CHARACTERISTICS:

(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:123:

UCACAGUGAG CCUUCUGGUG GUCCUGUGUG CU 32

(125) INFORMATION FOR SEQ ID NO:124:

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:124:

UUGUUGUGAG UGGUUGAUUC CAUGGUCCAA CCU 33

(126) INFORMATION FOR SEQ ID NO:125:

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:125:

UGCCUGUGAG CUGUUUAGCG GUCCAGGUCG UCU 33

(127) INFORMATION FOR SEQ ID NO:126:

(i) SEQUENCE CHARACTERISTICS:

(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:126:

UCAAGGCGAA GACUUAGUCU GCUCCCUGUG CU 32

(128) INFORMATION FOR SEQ ID NO:127:

(i) SEQUENCE CHARACTERISTICS:

(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:127:

UUGCGUCGAA GUUAAUUCUG GUCGAUGCCA CU 32

(129) INFORMATION FOR SEQ ID NO:128:

(i) SEQUENCE CHARACTERISTICS:

(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:128:

UUUCAAUGAG GUAUGUAAUG AUGGUCGUGC GCCU 34

(130) INFORMATION FOR SEQ ID NO:129:

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:129:

UGCGGGAGAG UCUUUUGACG UUGCUCCUGC GCU 33

(131) INFORMATION FOR SEQ ID NO:130:

(i) SEQUENCE CHARACTERISTICS:

(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:130:

UCAUGGGAGC CCAUCGAUUC UGGGUGUUGC CUAUGA 36

(132) INFORMATION FOR SEQ ID NO:131:

(i) SEQUENCE CHARACTERISTICS:

(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:131:

UUGCACAGAG CCAAAUUUGG UGUUGCUGUG CU 32

(133) INFORMATION FOR SEQ ID NO:132:

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:132:

UGGCCAGAGC UUAAAUUCAA GUGUUGCUGG CCU 33

(134) INFORMATION FOR SEQ ID NO:133:

(i) SEQUENCE CHARACTERISTICS:

(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:133:

UCAUAGCAGU CCUUGAUACU AUGGAUGGUG GCUAUGA 37

(135) INFORMATION FOR SEQ ID NO:134:

(i) SEQUENCE CHARACTERISTICS:

(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:134:

UGGAUGCAAG UUAACUCUGG UGGCAUCCGU CCU 33

(136) INFORMATION FOR SEQ ID NO:135:

(i) SEQUENCE CHARACTERISTICS:

(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:135:

UCAGUGGAGA UUAAGCCUCG CUAGGGGCCG CUAU 34

(137) INFORMATION FOR SEQ ID NO:136:

(i) SEQUENCE CHARACTERISTICS:

(A) LENGTH: 28 base pairs
(B) TYPE: nucleic acid
(C) STRANDEDNESS: single
(D) TOPOLOGY: linear

(xi) SEQUENCE DESCRIPTION: SEQ ID NO:136:

GGUCCGAAGU GCAACGGGGAA AAUGCAC 27

(138) INFORMATION FOR SEQ ID NO:137:

(i) SEQUENCE CHARACTERISTICS:

(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:137:

AGAUGCCUGU CGAGCAUGCU GAGGAUCGAA GUUAGUAGGC UUUGUGUGCU 50
CGUAGCUAAA CAGCUUUGUC GACGGG 76

(139) INFORMATION FOR SEQ ID NO:138:

(i) SEQUENCE CHARACTERISTICS:

(A) LENGTH: 74 base pairs
(B) TYPE: nucleic acid
(C) STRANDEDNESS: single
(D) TOPOLOGY: linear

(xi) SEQUENCE DESCRIPTION: SEQ ID NO:138:

AGAUGCCUGU CGAGCAUGCU GUACUGGAUC GAAGGUAGUA GGCAGUCACG 50
UAGCUAAACA GCUUUGUCGA CGGG 74

(140) INFORMATION FOR SEQ ID NO:139:

(i) SEQUENCE CHARACTERISTICS:

(A) LENGTH: 74 base pairs
(B) TYPE: nucleic acid
(C) STRANDEDNESS: single
(D) TOPOLOGY: linear

(xi) SEQUENCE DESCRIPTION: SEQ ID NO:139:

AGAUGCCUGU CGAGCAUGCU GAUAUCACGG AUCGAAGGAA GUAGGCGUGG 50
UAGCUAAACA GCUUUGUCGA CGGG 74

(141) INFORMATION FOR SEQ ID NO:140:

(i) SEQUENCE CHARACTERISTICS:

(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:140:

AGAUGCCUGU CGAGCAUGCU GCCUUUCCCG GGUUCGAAGU CAGUAGGCCG 50
GGUAGCUAAA CAGUUUGUCG ACGGG 75

(142) INFORMATION FOR SEQ ID NO:141:

(i) SEQUENCE CHARACTERISTICS:

(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:141:

AGAUGCCUGU CGAGCAUGCU GCACCCGGAU CGAAGUUAGU AGGCGUGAGU 50
GUAGCUAAAC AGCUUUGUCG ACGGG 75

(143) INFORMATION FOR SEQ ID NO:142:

(i) SEQUENCE CHARACTERISTICS:

(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:142:

AGAUGCCUGU CGAGCAUGCU GUGUACGGAU CGAAGGUAGU AGGCAGGUUA 50
CGUAGCUAAA CAGCUUUGUC GACGGG 76

(144) INFORMATION FOR SEQ ID NO:143:

(i) SEQUENCE CHARACTERISTICS:

(A) LENGTH: 76 base pairs
(B) TYPE: nucleic acid

(C) STRANDEDNESS: single
(D) TOPOLOGY: linear

(xi) SEQUENCE DESCRIPTION: SEQ ID NO:143:

AGAUGCCUGU CGAGCAUGCU GCAUCCGGAU CGAAGUUAGU AGGCCGAGGU 50
GGUAGCUAAA CAGCUUUGUC GACGGG 76

(145) INFORMATION FOR SEQ ID NO:144:

(i) SEQUENCE CHARACTERISTICS:

(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:144:

AGAUGCCUGU CGAGCAUGCU GAUUGUUGCG GAUCGAAGUG AGUAGGCGCU 50
AGUAGCUAAA CAGCUUUGUC GACGGG 76

(146) INFORMATION FOR SEQ ID NO:145:

(i) SEQUENCE CHARACTERISTICS:

(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:145:

AGAUGCCUGU CGAGCAUGCU GUGUACUGGA UCGAAGGUAG UAGGCAGUCA 50
CGUAGCUAAA CAGCUUUGUC GACGGG 76

(147) INFORMATION FOR SEQ ID NO:146:

(i) SEQUENCE CHARACTERISTICS:

(A) LENGTH: 69 base pairs
(B) TYPE: nucleic acid
(C) STRANDEDNESS: single
(D) TOPOLOGY: linear

(xi) SEQUENCE DESCRIPTION: SEQ ID NO:146:

AGAUGCCUGU CGAGCAUGCU GAUCGAAGUU AGUAGGAGCG UGUGGUAGCU 50
AAACAGCUUU GUCGACGGG 69

(148) INFORMATION FOR SEQ ID NO:147:

(i) SEQUENCE CHARACTERISTICS:

(A) LENGTH: 81 base pairs
(B) TYPE: nucleic acid
(C) STRANDEDNESS: single
(D) TOPOLOGY: linear

(xi) SEQUENCE DESCRIPTION: SEQ ID NO:147:

AGAUGCCUGU CGAGCAUGCU GACGCUGGAG UCGGAUCGAA AGGUAAGUAG 50
GCGACUGUAG CUAAACAGCU UUGUCGACGG G 81

(149) INFORMATION FOR SEQ ID NO:148:

(i) SEQUENCE CHARACTERISTICS:

(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:148:

AGAUGCCUGU CGAGCAUGCU GGGGUCGGAU CGAAAGGUAA GUAGGCGACU 50
GUAGCUAAAC AGCUUUGUCG ACGGG 75

(150) INFORMATION FOR SEQ ID NO:149:

(i) SEQUENCE CHARACTERISTICS:

(A) LENGTH: 74 base pairs
(B) TYPE: nucleic acid
(C) STRANDEDNESS: single
(D) TOPOLOGY: linear

(xi) SEQUENCE DESCRIPTION: SEQ ID NO:149:

AGAUGCCUGU CGAGCAUGCU GAUAUCACGG AUCGAAAGAG AGUAGGCGUG 50
UAGCUAAACA GCUUUGUCGA CGGG 74

(151) INFORMATION FOR SEQ ID NO:150:

(i) SEQUENCE CHARACTERISTICS:

(A) LENGTH: 76 base pairs
(B) TYPE: nucleic acid
(C) STRANDEDNESS: single
(D) TOPOLOGY: linear

(xi) SEQUENCE DESCRIPTION: SEQ ID NO:150:

AGAUGCCUGU CGAGCAUGCU GUGUACUGGA UCGAAGGUAG UAGGCAGGCA 50
CGUAGCUAAA CAGCUUUGUC GACGGG 76

(152) INFORMATION FOR SEQ ID NO:151:

(i) SEQUENCE CHARACTERISTICS:

(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:151:

**AGAUGCCUGU CGDGCAUGCU GAUAUCACGG AUCGAAGGAA AGUAGGCGUG 50**
**GUAGCUAAAC AGCUUUGUCG ACGGG 75**

(153) INFORMATION FOR SEQ ID NO:152:

(i) SEQUENCE CHARACTERISTICS:

(A) LENGTH: 72 base pairs
(B) TYPE: nucleic acid
(C) STRANDEDNESS: single
(D) TOPOLOGY: linear

(xi) SEQUENCE DESCRIPTION: SEQ ID NO:152:

**AGAUGCCUGU CGAGCAUGCU GGUGCGGCUU UGGGCGCCGU GCUUGGCGUA 50**
**GCUAAACAGC UUUGUCGACG GG 72**

(154) INFORMATION FOR SEQ ID NO:153:

(i) SEQUENCE CHARACTERISTICS:

(A) LENGTH: 71 base pairs
(B) TYPE: nucleic acid
(C) STRANDEDNESS: single
(D) TOPOLOGY: linear

(xi) SEQUENCE DESCRIPTION: SEQ ID NO:153:

**AGAUGCCUGU CGAGCAUGCU GGUGCGGCUU UGGGCGCCGU GCUUACGUAG 50**
**CUAAACAGCU UUGUCGACGG G 71**

(155) INFORMATION FOR SEQ ID NO:154:

(i) SEQUENCE CHARACTERISTICS:

(A) LENGTH: 72 base pairs
(B) TYPE: nucleic acid
(C) STRANDEDNESS: single
(D) TOPOLOGY: linear

(xi) SEQUENCE DESCRIPTION: SEQ ID NO:154:

**AGAUGCCUGU CGAGCAUGCU GGUGCGGCUU UGGGCGCCGU GCUUGACGUA 50**
**GCUAAACAGC UUUGUCGACG GG 72**

(156) INFORMATION FOR SEQ ID NO:155:

(i) SEQUENCE CHARACTERISTICS:

(A) LENGTH: 72 base pairs
(B) TYPE: nucleic acid
(C) STRANDEDNESS: single
(D) TOPOLOGY: linear

(xi) SEQUENCE DESCRIPTION: SEQ ID NO:155:

```
AGAUGCCUGU CGAGCAUGCU GGGGCGGCUU UGGGCGCCGU GCUUGACGUA   50
GCUAAACAGC UUUGUCGACG GG                                 72
```

(157) INFORMATION FOR SEQ ID NO:156:

(i) SEQUENCE CHARACTERISTICS:

(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:156:

```
GGGAGAUGCC UGUCGAGCAU GCUGAGGAUC GAAGUUAGUA GGCUUUGUGU   50
GCUCGUAGCU AAACAGCUUU GUCGACGGG                          79
```

(158) INFORMATION FOR SEQ ID NO:157:

(i) SEQUENCE CHARACTERISTICS:

(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:157:

```
GGGAGAUGCC UGUCGAGCAU GCUGCAUCCG GAUCGAAGUU AGUAGGCCGA   50
GGUGGUAGCU AAACAGCUUU GUCGACGGG                          79
```

(159) INFORMATION FOR SEQ ID NO:158:

(i) SEQUENCE CHARACTERISTICS:

(A) LENGTH: 79 base pairs
(B) TYPE: nucleic acid
(C) STRANDEDNESS: single
(D) TOPOLOGY: linear

(xi) SEQUENCE DESCRIPTION: SEQ ID NO:158:

GGGAGAUGCC UGUCGAGCAU GCUGAUUGUU GCGGAUCGAA GUGAGUAGGC 50
GCUAGUAGCU AAACAGCUUU GUCGACGGG 79

(160) INFORMATION FOR SEQ ID NO:159:

(i) SEQUENCE CHARACTERISTICS:

(A) LENGTH: 75 base pairs
(B) TYPE: nucleic acid
(C) STRANDEDNESS: single
(D) TOPOLOGY: linear

(xi) SEQUENCE DESCRIPTION: SEQ ID NO:159:

GGGAGAUGCC UGUCGAGCAU GCUGGUGCGG CUUUGGGCGC CGUGCUUGAC 50
GUAGCUAAAC AGCUUUGUCG ACGGG 75

## Description

FIELD OF THE INVENTION

Described herein are methods for identifying and producing nucleic acid ligands. Nucleic acid ligands are double or single stranded DNA or RNA species that bind specifically to a desired target molecule. The basis for identifying nucleic acid ligands is a method termed SELEX, an acronym for Systematic Evolution of Ligands for EXponential enrichment.

The methods of the present invention include means for analyzing and applying the information learned from the SELEX method to create an improved nucleic acid ligand for the selected target. These methods include computer modeling, boundary determination methods and chemical modification methods. According to the methods of this invention it is possible to determine: 1) which nucleic acid residues of a nucleic acid ligand are critical in binding to the selected target; 2) which nucleic acid residues affect the structural conformation of the nucleic acid ligand; and 3) what is the three-dimensional structure of the nucleic acid ligand. This information allows for the identification and production of improved nucleic acid ligands that have superior binding capacity to the target as well as enhanced structural stability. This information may also be utilized to produce non-nucleic acid or hybridnucleic acid species that also function as ligands to the target. The methods of the present invention further provide an analysis of the target species that can be used in the preparation of therapeutic and/or diagnostic methods.

Specifically described herein are high-affinity nucleic acid ligands to the HIV-RT, HIV-1 Rev, HIV-1 tat, thrombin, and basic fibroblast growth factor (bFGF) proteins. Included within the scope of the invention are modified nucleic acid ligands and mimetic ligands that are informed by the nucleic acid ligands identified herein. Further included within the scope of the invention are nucleic acid ligands capable of modifying the biological activity of the target molecule, for example, nucleic acid ligands that inhibit the action of bFGF. Still further included in the present invention are nucleic acid ligands containing modified nucleotides.

BACKGROUND OF THE INVENTION

Most proteins or small molecules are not known to specifically bind to nucleic acids. The known protein exceptions are those regulatory proteins such as repressors, polymerases, activators and the like which function in a living cell to bring about the transfer of genetic information encoded in the nucleic acids into cellular structures and the replication of the genetic material. Furthermore, small molecules such as GTP bind to some intron RNAs.

Living matter has evolved to limit the function of nucleic acids to a largely informational role. The Central Dogma, as postulated by Crick, both originally and in expanded form, proposes that nucleic acids (either RNA or DNA) can serve as templates for the synthesis of other nucleic acids through replicative processes that "read" the information in a template nucleic acid and thus yield complementary nucleic acids. All of the experimental paradigms for genetics and gene expression depend on these properties of nucleic acids: in essence, double-stranded nucleic acids are informationally redundant because of the chemical concept of base pairs and because replicative processes are able to use that base pairing in a relatively error-free manner.

The individual components of proteins, the twenty natural amino acids, possess sufficient chemical differences and activities to provide an enormous breadth of activities for both binding and catalysis. Nucleic acids, however, are thought to have narrower chemical possibilities than proteins, but to have an informational role that allows genetic information to be passed from virus to virus, cell to cell, and organism to organism. In this context nucleic acid components, the nucleotides, must possess only pairs of surfaces that allow informational redundancy within a Watson-Crick base pair. Nucleic acid components need not possess chemical differences and activities sufficient for either a wide range of binding or catalysis.

However, some nucleic acids found in nature do participate in binding to certain target molecules and even a few instances of catalysis have been reported. The range of activities of this kind is narrow compared to proteins and more specifically antibodies. For example, where nucleic acids are known to bind to some protein targets with high affinity and specificity, the binding depends on the exact sequences of nucleotides that comprise the DNA or RNA ligand. Thus, short double-stranded DNA sequences are known to bind to target proteins that repress or activate transcription in both prokaryotes and eukaryotes. Other short double-stranded DNA sequences are known to bind to restriction endonucleases, protein targets that can be selected with high affinity and specificity. Other short DNA sequences serve as centromeres and telomeres on chromosomes, presumably by creating ligands for the binding of specific proteins that participate in chromosome mechanics. Thus, double-stranded DNA has a well-known capacity to bind within the nooks and crannies of target proteins whose functions are directed to DNA binding. Single-stranded DNA can also bind to some proteins with high affinity and specificity, although the number of examples is rather smaller. From the known examples of double-stranded DNA binding proteins, it has become possible to describe the binding interactions as involving various protein motifs projecting amino acid side chains into the major groove of B form double-stranded DNA,

providing the sequence inspection that allows specificity.

Double-stranded RNA occasionally serves as a ligand for certain proteins, for example, the endonuclease RNase III from E. coli. There are more known instances of target proteins that bind to single-stranded RNA ligands, although in these cases the single-stranded RNA often forms a complex three-dimensional shape that includes local regions of intramolecular double-strandedness. The amino-acyl tRNA synthetases bind tightly to tRNA molecules with high specificity. A short region within the genomes of RNA viruses binds tightly and with high specificity to the viral coat proteins. A short sequence of RNA binds to the bacteriophage T4-encoded DNA polymerase, again with high affinity and specificity. Thus, it is possible to find RNA and DNA ligands, either double- or single-stranded, serving as binding partners for specific protein targets. Most known DNA binding proteins bind specifically to double-stranded DNA, while most RNA binding proteins recognize single-stranded RNA. This statistical bias in the literature no doubt reflects the present biosphere's statistical predisposition to use DNA as a double-stranded genome and RNA as a single-stranded entity in the roles RNA plays beyond serving as a genome. Chemically there is no strong reason to dismiss single-stranded DNA as a fully able partner for specific protein interactions.

RNA and DNA have also been found to bind to smaller target molecules. Double-stranded DNA binds to various antibiotics, such as actinomycin D. A specific single-stranded RNA binds to the antibiotic thiostreptone; specific RNA sequences and structures probably bind to certain other antibiotics, especially those whose functions is to inactivate ribosomes in a target organism. A family of evolutionary related RNAs binds with specificity and decent affinity to nucleotides and nucleosides (Bass, B. and Cech, T. (1984) Nature 308:820) as well as to one of the twenty amino acids (Yarus, M. (1988) Science 240:1751). Catalytic RNAs are now known as well, although these molecules perform over a narrow range of chemical possibilities, which are thus far related largely to phosphodiester transfer reactions and hydrolysis of nucleic acids.

Despite these known instances, the great majority of proteins and other cellular components are thought not to bind to nucleic acids under physiological conditions and such binding as may be observed is non-specific. Either the capacity of nucleic acids to bind other compounds is limited to the relatively few instances enumerated supra, or the chemical repertoire of the nucleic acids for specific binding is avoided (selected against) in the structures that occur naturally. The present invention is premised on the inventors' fundamental insight that nucleic acids as chemical compounds can form a virtually limitless array of shapes, sizes and configurations, and are capable of a far broader repertoire of binding and catalytic functions than those displayed in biological systems.

The chemical interactions have been explored in cases of certain known instances of protein-nucleic acid binding. For example, the size and sequence of the RNA site of bacteriophage R17 coat protein binding has been identified by Uhlenbeck and coworkers. The minimal natural RNA binding site (21 bases long) for the R17 coat protein was determined by subjecting variable-sized labeled fragments of the mRNA to nitrocellulose filter binding assays in which protein-RNA fragment complexes remain bound to the filter (Carey et al. (1983) Biochemistry 22:2601). A number of sequence variants of the minimal R17 coat protein binding site were created in vitro in order to determine the contributions of individual nucleic acids to protein binding (Uhlenbeck et al. (1983) J. Biomol. Structure Dynamics 1:539 and Romaniuk et al. (1987) Biochemistry 26:1563). It was found that the maintenance of the hairpin loop structure of the binding site was essential for protein binding but, in addition, that nucleotide substitutions at most of the single-stranded residues in the binding site, including a bulged nucleotide in the hairpin stem, significantly affected binding. In similar studies, the binding of bacteriophage Qβ coat protein to its translational operator was examined (Witherell and Uhlenbeck (1989) Biochemistry 28:71). The Qβ coat protein RNA binding site was found to be similar to that of R17 in size, and in predicted secondary structure, in that it comprised about 20 bases with an 8 base pair hairpin structure which included a bulged nucleotide and a 3 base loop. In contrast to the R17 coat protein binding site, only one of the single-stranded residues of the loop is essential for binding and the presence of the bulged nucleotide is not required. The protein-RNA binding interactions involved in translational regulation display significant specificity.

Nucleic acids are known to form secondary and tertiary structures in solution. The double-stranded forms of DNA include the so-called B double-helical form, Z-DNA and superhelical twists (Rich, A. et al. (1984) Ann. Rev. Biochem. 53:791). Single-stranded RNA forms localized regions of secondary structure such as hairpin loops and pseudoknot structures (Schimmel, P. (1989) Cell 58:9). However, little is known concerning the effects of unpaired loop nucleotides on stability of loop structure, kinetics of formation and denaturation, thermodynamics, and almost nothing is known of tertiary structures and three dimensional shape, nor of the kinetics and thermodynamics of tertiary folding in nucleic acids (Tuerk et al. (1988) Proc. Natl. Acad. Sci. USA 85:1364).

A type of in vitro evolution was reported in replication of the RNA bacteriophage Qβ. Mills et al. (1967) Proc. Natl. Acad. Sci USA 58:217; Levinsohn & Spiegleman (1968) Proc. Natl. Acad. Sci. USA 60:866; Levinsohn & Spiegelman (1969) Proc. Natl. Acad. Sci. USA 63:805; Saffhill et al. (1970) J. Mol. Biol. 51:531; Kacian et al. (1972) Proc. Natl. Acad. Sci. USA 69:3038; Mills et al. (1973) Science 180:916. The phage RNA serves as a poly-cistronic messenger RNA directing translation of phage-specific proteins and also as a template for its own replication catalyzed by Qβ RNA replicase. This RNA replicase was shown to be highly specific for its own RNA templates. During the course of cycles of replication in vitro small variant RNAs were isolated which were also replicated by Qβ replicase. Minor alterations in

the conditions under which cycles of replication were performed were found to result in the accumulation of different RNAs, presumably because their replication was favored under the altered conditions. In these experiments, the selected RNA had to be bound efficiently by the replicase to initiate replication and had to serve as a kinetically favored template during elongation of RNA. Kramer et al. (1974) J. Mol. Biol. 89:719 reported the isolation of a mutant RNA template of Qβ replicase, the replication of which was more resistant to inhibition by ethidium bromide than the natural template. It was suggested that this mutant was not present in the initial RNA population but was generated by sequential mutation during cycles of in vitro replication with Qβ replicase. The only source of variation during selection was the intrinsic error rate during elongation by Qβ replicase. In these studies what was termed "selection" occurred by preferential amplification of one or more of a limited number of spontaneous variants of an initially homogenous RNA sequence. There was no selection of a desired result, only that which was intrinsic to the mode of action of Qβ replicase.

Joyce and Robertson (Joyce (1989) in RNA: Catalysis, Splicing, Evolution, Belfort and Shub (eds.), Elsevier, Amsterdam pp. 83-87; and Robertson and Joyce (1990) Nature 344:467) reported a method for identifying RNAs which specifically cleave single-stranded DNA. The selection for catalytic activity was based on the ability of the ribozyme to catalyze the cleavage of a substrate ssRNA or DNA at a specific position and transfer the 3'-end of the substrate to the 3'-end of the ribozyme. The product of the desired reaction was selected by using an oligodeoxynucleotide primer which could bind only to the completed product across the junction formed by the catalytic reaction and allowed selective reverse transcription of the ribozyme sequence. The selected catalytic sequences were amplified by attachment of the promoter of T7 RNA polymerase to the 3'-end of the cDNA, followed by transcription to RNA. The method was employed to identify from a small number of ribozyme variants the variant that was most reactive for cleavage of a selected substrate.

The prior art has not taught or suggested more than a limited range of chemical functions for nucleic acids in their interactions with other substances: as targets for proteins evolved to bind certain specific oligonucleotide sequences; and more recently, as catalysts with a limited range of activities. Prior "selection" experiments have been limited to a narrow range of variants of a previously described function. Now, for the first time, it will be understood that the nucleic acids are capable of a vastly broad range of functions and the methodology for realizing that capability is disclosed herein.

WO 91/19813 of Gold and Tuerk, entitled Nucleic Acid Ligands describes a fundamentally novel method for making a nucleic acid ligand for any desired target. This method is known as the SELEX method.

The method of the SELEX Patent referred to above is based on the unique insight that nucleic acids have sufficient capacity for forming a variety of two- and three-dimensional structures and sufficient chemical versatility available within their monomers to act as ligands (form specific binding pairs) with virtually any chemical compound, whether large or small in size.

The method involves selection from a mixture of candidates and step-wise iterations of structural improvement, using the same general selection theme, to achieve virtually any desired criterion of binding affinity and selectivity. Starting from a mixture of nucleic acids, preferably comprising a segment of randomized sequence, the method, termed SELEX herein, includes steps of contacting the mixture with the target under conditions favorable for binding, partitioning unbound nucleic acids from those nucleic acids which have bound to target molecules, dissociating the nucleic acid-target pairs, amplifying the nucleic acids dissociated from the nucleic acid-target pairs to yield a ligand-enriched mixture of nucleic acids, then reiterating the steps of binding, partitioning, dissociating and amplifying through as many cycles as desired.

While not bound by a theory of preparation, SELEX is based on the inventors' insight that within a nucleic acid mixture containing a large number of possible sequences and structures there is a wide range of binding affinities for a given target. A nucleic acid mixture comprising, for example a 20 nucleotide randomized segment can have $4^{20}$ candidate possibilities. Those which have the higher affinity constants for the target are most likely to bind. After partitioning, dissociation and amplification, a second nucleic acid mixture is generated, enriched for the higher binding affinity candidates. Additional rounds of selection progressively favor the best ligands until the resulting nucleic acid mixture is predominantly composed of only one or a few sequences. These can then be cloned, sequenced and individually tested for binding affinity as pure ligands.

Cycles of selection and amplification are repeated until a desired goal is achieved. In the most general case, selection/amplification is continued until no significant improvement in binding strength is achieved on repetition of the cycle. The method may be used to sample as many as about $10^{18}$ different nucleic acid species. The nucleic acids of the test mixture preferably include a randomized sequence portion as well as conserved sequences necessary for efficient amplification. Nucleic acid sequence variants can be produced in a number of ways including synthesis of randomized nucleic acid sequences and size selection from randomly cleaved cellular nucleic acids. The variable sequence portion may contain fully or partially random sequence; it may also contain subportions of conserved sequence incorporated with randomized sequence. Sequence variation in test nucleic acids can be introduced or increased by mutagenesis before or during the selection/amplification iterations.

In one embodiment of the method of the SELEX Patent Applications, the selection process is so efficient at isolating those nucleic acid ligands that bind most strongly to the selected target, that only one cycle of selection and amplification is required. Such an efficient selection may occur, for example, in a chromatographic-type process wherein

the ability of nucleic acids to associate with targets bound on a column operates in such a manner that the column is sufficiently able to allow separation and isolation of the highest affinity nucleic acid ligands.

In many cases, it is not necessarily desirable to perform the iterative steps of SELEX until a single nucleic acid ligand is identified. The target-specific nucleic acid ligand solution may include a family of nucleic acid structures or motifs that have a number of conserved sequences and a number of sequences which can be substituted or added without significantly effecting the affinity of the nucleic acid ligands to the target. By terminating the SELEX process prior to completion, it is possible to determine the sequence of a number of members of the nucleic acid ligand solution family.

A variety of nucleic acid primary, secondary and tertiary structures are known to exist. The structures or motifs that have been shown most commonly to be involved in non-Watson-Crick type interactions are referred to as hairpin loops, symmetric and asymmetric bulges, psuedoknots and myriad combinations of the same. Almost all known cases of such motifs suggest that they can be formed in a nucleic acid sequence of no more than 30 nucleotides. For this reason, it is often preferred that SELEX procedures with contiguous randomized segments be initiated with nucleic acid sequences containing a randomized segment of between about 20-50 nucleotides.

The SELEX Patent Applications also describe methods for obtaining nucleic acid ligands that bind to more than one site on the target molecule, and to nucleic acid ligands that include non-nucleic acid species that bind to specific sites on the target. The SELEX method provides means for isolating and identifying nucleic acid ligands which bind to any envisonable target. However, in preferred embodiments the SELEX method is applied to situations where the target is a protein, including both nucleic acid-binding proteins and proteins not known to bind nucleic acids as part of their biological function.

Little is known about RNA structure at high resolution. The basic A-form helical structure of double stranded RNA is known from fiber diffraction studies. X-ray crystallography has yielded the structure of a few tRNAs and a short poly-AU helix. The X-ray structure of a tRNA/synthetase RNA/protein complex has also been solved. The structures of two tetranucleotide hairpin loops and one model pseudoknot are know from NMR studies.

There are several reasons behind the paucity of structural data. Until the advent of in vitro RNA synthesis, it was difficult to isolate quantities of RNA sufficient for structural work. Until the discovery of catalytic RNAs, there were few RNA molecules considered worthy of structural study. Good tRNA crystals have been difficult to obtain, discouraging other crystal studies. The technology for NMR study of molecules of this size has only recently become available.

As described above, several examples of catalytic RNA structures are known, and the SELEX technology has been developed which selects RNAs that bind tightly to a variety of target molecules - and may eventually be able to select for new catalytic RNA structures as well. It has become important to know the structure of these molecules, in order to learn how exactly they work, and to use this knowledge to improve upon them.

It would be desirable to understand enough about RNA folding to be able to predict the structure of an RNA with less effort than resorting to rigorous NMR, and X-ray crystal structure determination. For both proteins and RNAs, there has always been a desire to be able to compute structures based on sequences, and with limited (or no) experimental data.

Protein structure prediction is notoriously difficult. To a first approximation, the secondary structure and tertiary structure of proteins form cooperatively; protein folding can be approximated thermodynamically by a two-state model, with completely folded and completely unfolded states. This means that the number of degrees of freedom for modeling a protein structure are very large; without predictable intermediates, one cannot break the prediction problem into smaller, manageable sub problems. In contrast, RNAs often appear to make well-defined secondary structures which provide more stability than the tertiary interactions. For example, the tertiary structure of tRNA can be disrupted without disrupting the secondary structure by chelation of magnesium or by raising the temperature. Secondary structure prediction for RNAs is well-understood, and is generally quite accurate for small RNA molecules. For RNAs, structural prediction can be broken into subproblems; first, predict the secondary structure; then, predict how the resulting helices and remaining single strands are arranged relative to each other.

For RNA, the first attempts at structural prediction were for tRNAs. The secondary structure of the canonical tRNA cloverleaf was known from comparative sequence analysis, reducing the problem to one of arranging four short A-form helices in space relative to each other. Manual CPK modeling, back-of-the-envelope energy minimization, and a few distance restraints available from crosslinking studies and phylogenetic covariations were used to generate a tRNA model - which unfortunately proved wrong when the first crystal structure of phenylalanine tRNA was solved a few years later.

Computer modeling has supplanted manual modeling, relieving the model-builder of the difficulties imposed by gravitation and mass. Computer modeling can only be used without additional experimental data for instances in which a homologous structure is known; for instance, the structure of the 3' end of the turnip yellow mosaic virus RNA genome was modeled, based on the known 3D structure of tRNA and the knowledge that the 3' end of TYMV is recognized as tRNA-like by a number of cellular tRNA modification enzymes. This model was the first 3D model of an RNA pseudoknot; the basic structure of an isolated model pseudoknot has been corroborated by NMR data.

Computer modeling protocols have been used, restrained by the manual inspection of chemical and enzymatic

protection data, to model the structures of several RNA molecules. In one isolated substructure, one model for the conformation of a GNRA tetranucleotide loop has been shown to be essentially correct by NMR study of an isolated GNRA hairpin loop.

Francois Michel ((1989) Nature 342:391) has constructed a model for the catalytic core of group I introns. Like the tRNAs, the secondary structure of group I intron cores is well-known from comparative sequence analysis, so the problem is reduced to one of properly arranging helices and the remaining single-stranded regions. Michel (1989) supra, analyzed an aligned set of 87 group I intron sequences by eye and detected seven strong pairwise and triplet covariations outside of the secondary structure, which he interpreted as tertiary contacts and manually incorporated as restraints on his model. As yet, there is no independent confirmation of the Michel model.

Others have attempted to devise an automated procedure to deal with distance restraints from crosslinking, fluorescence transfer, or phylogentic covariation. The RNA is treated as an assemblage of cylinders (A-form helices) and beads (single-stranded residues), and a mathematical technique called distance geometry is used to generate arrangements of these elements which are consistent with a set of distance restraints. Using a small set of seven distance restraints on the phenylalanine tRNA tertiary structure, this protocol generated the familiar L-form of the tRNA structure about 2/3 of the time.

The HIV-1 tat protein activates transcription in the long terminal repeat (LTR) of the viral genome of HIV-1. See, Cullen et al. (1989) Cell 58:423. The mechanism of activation is unclear or at least controversial, but requires that the transcribed RNA contain a specific hairpin structure with a trinucleotide bulge (called TAR). The natural TAR RNA and the site of tat interaction is shown in Figure 25. A small basic domain of the tat protein has been shown to interact directly with the TAR RNA sequence. See, Weeks et al. (1990) Science 249:1281; Roy et al. (1990) Genes Dev. 4:1365; Calnan et al. (1991a) Genes Dev. 5:201. Arginines within this basic domain are apparently crucial to the interaction. See, Calnan et al. (1991a) supra; Subramanian et al. (1991) EMBO 10:2311-2318; Calnan et al (1991b) Science 252:1167-1171. Arginine alone is specifically bound by the TAR RNA sequence and may compete for tat protein binding. See, Tao et al. (1992) Proc. Natl. Acad. Sci. USA 89:2723-2726; Puglisi et al. (1992) Science 257:76-80.

Tat - TAR interactions alone are insufficient to support transactivation; presumably a cellular factor - - a 68 kD loop binding protein -- is required for cooperative binding with the tat protein to TAR, and subsequent in vivo or in vitro transactivation. See, Marciniak et al. (1990a) Proc. Natl. Acad. Sci. USA 87:3624; Marciniak et al. (1990b) Cell 63:791. Overexpression of the TAR sequence in retrovirally transformed cell lines renders them highly resistant to HIV-1 infections. See, Sullenger et al. (1990) Cell 63:601.

Thrombin is a multifunctional serine protease that has important procoagulant and anticoagulant activities. As a procoagulant enzyme thrombin clots fibrinogen, activates clotting factors V, VIII, and XIII, and activates platelets. The specific cleavage of fibrinogen by thrombin initiates the polymerization of fibrin monomers, a primary event in blood clot formation. The central event in the formation of platelet thrombi is the activation of platelets from the "nonbinding" to the "binding" mode and thrombin is the most potent physiologic activator of platelet aggregation (Berndt and Phillips (1981) in Platelets in Biology and Pathology, J.L. Gordon, ed. (Amsterdam: Elsevier/North Holland Biomedical Press), pp. 43-74; Hansen and Harker (1988) Proc. Natl. Acad. Sci. USA 85:3184; Eidt et al. (1989) J. Clin. Invest. 84:18). Thus, as a procoagulant, thrombin plays a key role in the arrest of bleeding (physiologic hemostasis) and formation of vasoocclusive thrombi (pathologic thrombosis).

As an anticoagulant thrombin binds to thrombomodulin (TM), a glycoprotein expressed on the surface of vascular endothelial cells. TM alters substrate specificity from fibrinogen and platelets to protein C through a combination of an allosteric change in the active site conformation and an overlap of the TM and fibrinogen binding sites on thrombin. Activated protein C, in the presence of a phospholipid surface, $Ca^{2+}$, and a second vitamin K-dependent protein cofactor, protein S, inhibits coagulation by proteolytically degrading factors Va and VIIIa. Thus the formation of the thrombin-TM complex converts thrombin from a procoagulant to an anticoagulant enzyme, and the normal balance between these opposing activities is critical to the regulation of hemostasis.

Thrombin is also involved in biological responses that are far removed from the clotting system (reviewed in Shuman (1986) Ann. N. Y. Acad. Sci. 485:349; Marx (1992) Science 256:1278). Thrombin is chemotactic for monocytes (Bar-Shavit et al. (1983) Science 220:728), mitogenic for lymphocytes (Chen et al. (1976) Exp. Cell Res. 101:41), mesenchymal cells (Chen and Buchanan (1975) Proc. Natl. Acad. Sci. USA 72:131), and fibroblasts (Marx (1992) supra). Thrombin activates endothelial cells to express the neutrophil adhesive protein GMP-140 (PADGEM) (Hattori et al. (1989) J. Biol. Chem. 264:7768) and produce platelet-derived growth factor (Daniel et al. (1986) J. Biol. CHem. 261:9579). Recently it has been shown that thrombin causes cultured nerve cells to retract their neurites (reviewed in Marx (1992) supra.

The mechanism by which thrombin activates platelets and endothelial cells is through a functional thrombin receptor found on these cells. A putative thrombin cleavage site (LDR/S) in the receptor suggests that the thrombin receptor is activated by proteolytic cleavage of the receptor. This cleavage event "unmasks" an N-terminal domain which then acts as the ligand, activating the receptor (Vu et al. (1991) Cell 64:1054).

Vascular injury and thrombus formation represent the key events in the pathogenesis of various vascular

diseases, including atherosclerosis. The pathogenic processes of the activation of platelets and/or the clotting system leading to thrombosis in various disease states and in various sites, such as the coronary arteries, cardiac chambers, and prosthetic heart valves, appear to be different. Therefore, the use of a platelet inhibitor, an anticoagulant, or a combination of both may be required in conjunction with thrombolytics to open closed vessels and prevent reocclusion.

Controlled proteolysis by compounds of the coagulation cascade is critical for hemostasis. As a result, a variety of complex regulatory systems exist that are based, in part, on a series of highly specific protease inhibitors. In a pathological situation functional inhibitory activity can be interrupted by excessive production of active protease or inactivation of inhibitory activity. Perpetuation of inflammation in response to multiple trauma (tissue damage) or infection (sepsis) depends on proteolytic enzymes, both of plasma cascade systems, including thrombin, and lysosomal origin. Multiple organ failure (MOF) in these cases is enhanced by the concurrently arising imbalance between proteases and their inhibitory regulators. An imbalance of thrombin activity in the brain may lead to neurodegenerative diseases.

Thrombin is naturally inhibited in hemostasis by binding to antithrombin III (ATIII), in a heparin-dependent reaction. Heparin exerts its effect through its ability to accelerate the action of ATIII. In the brain, protease nexin (PN-1) may be the natural inhibitor of thrombin to regulate neurite outgrowth.

Heparin is a glycosoaminoglycan composed of chains of alternating residues of D-glucosamine and uronic acid. Heparin is currently used extensively as an anticoagulant in the treatment of unstable angina, pulmonary embolism, atherosclerosis, thrombosis, and following myocardial infarction. Its anticoagulant effect is mediated through its interaction with ATIII. When heparin binds ATIII, the conformation of ATIII is altered, and it becomes a significantly enhanced inhibitor of thrombin. Although heparin is generally considered to be effective for certain indications, it is believed that the physical size of the ATIII•heparin complex prevents access to much of the biologically active thrombin in the body, thus diminishing its ability to inhibit clot formation. Side effects of heparin include bleeding, thrombocytopenia, osteoporosis, skin necrosis, alpe, hypersensitivity and hypoaldoseronism.

Hirudin is a potent peptide inhibitor of thrombin derived from the European medicinal leech Hirudis medicinalis. Hirudin inhibits all known functions of $\alpha$-thrombin, and has been shown to bind thrombin at two separate sites kinetically; a high affinity site at or near the catalytic site for serine protease activity and a second anionic exosite. The anionic exosite also binds fibrinogen, heparin, TM and probably the receptor involved in mediating the activation of platelets and endothelial cells. A C-terminal hirudin peptidewhich has been shown by co-crystallization with thrombin to bind in the anionic exosite -- has inhibitory effects on fibrin formation, platelet and endothelial cell activation, and Protein C activation via TM binding, presumably by competing for binding at this site. This peptide does not inhibit proteolytic activity towards tripeptide chromogenic substrates, Factor V or X.

The structure of thrombin makes it a particularly desirable target for nucleic acid binding, due to the anionic exosite. Site-directed mutagenesis within this site has shown that fibrinogen-clotting and TM binding activities are separable. Conceivably, an RNA ligand could be selected that has procoagulatory and/or anticoagulatory effects depending on how it interacts with thrombin, i.e., which substrate it mimics.

A single stranded DNA ligand to thrombin has been prepared according to a procedure identical to SELEX. See, Bock et al. (1992) Nature 355:564. A consensus ligand was identified after relatively few rounds of SELEX were performed, that was shown to have some ability to prevent clot formation in vitro. The ligand is the 15mer DNA 5'GGTTGGTGTGGTTGG-3', referred to herein as G15D (SEQ ID NO:1). The symmetrical nature of the primary sequence suggests that G15D has a regular fixed tertiary structure. The kD of G15D to thrombin is about 2 x $10^{-7}$. For effective thrombin inhibition as an anticoagulant, the stronger the affinity of the ligand to thrombin the better.

Basic fibroblast growth factor (bFGF) is a multifunctional effector for many cells of mesenchymal and neuroectodermal origin (Rifkin & Moscatelli (1989) J. Cell Biol. 109:1; Baird & Bohlen (1991) in Peptide Growth Factors and Their Receptors (Sporn, M. B. & Roberts, A. B., eds.); pp. 369-418, Springer, N.Y.; Basilico & Moscatelli (1992) Adv. Cancer Res. 59:115). It is one of the most studied and best characterized members of a family of related proteins that also includes acidic FGF (Jaye et al. (1986) Science 233:541; Abraham et al. (1986) Science 233:545), int-2 (Moore et al. (1986) EMBO J. 5:919), kFGF/hst/KS3 (Delli-Bovi et al. (1987) Cell 50:729; Taira et al. (1987) Proc. Natl. Acad. Sci. USA 84:2980), FGF-5 (Zhan et al. (1988) Mol. Cell. Biol. 8:3487), FGF-6 (Marics et al. (1989) Oncogene 4:335) and keratinocyte growth factor/FGF-7 (Finch et al. (1989) Science 245:752).

In vitro, bFGF stimulates cell proliferation, migration and induction of plasminogen activator and collagenase activities (Presta et al. (1986) Mol. Cell. Biol. 6:4060; Moscatelli et al. (1986) Proc. Natl. Acad. Sci. USA 83:2091; Mignatti et al. (1989) J. Cell Biol. 108:671). In vivo, it is one of the most potent inducers of neovascularization. Its angiogenic activity in vivo suggests a role in tissue remodeling and wound healing but also in some disease states that are characterized by pathological neovascularization such as tumor proliferation, tumor metastasis, diabetic retinopathy and rheumatoid arthritis (Folkman & Klagsbrun (1987) Science 235:442; Gospodarowitz (1991) Cell Biology Reviews 25:307).

Although bFGF does not have a signal sequence for secretion, it is found on both sides of the plasma membrane, presumably being exported via exocytosis (Vlodavsky et al. (1991) Trends Biol. Sci. 16:268; Mignatti & Rifkin (1991) J. Cell. Biochem. 47:201). In the extracellular matrix, it is typically associated with a fraction that contains heparan sulfate proteoglycans. Indeed, heparin affinity chromatography has been a useful method for purification of this and other

heparin-binding growth factors. In cell culture, bFGF binds to low- and high-affinity sites. The low-affinity sites are composed of cell-associated heparan sulfate proteoglycans to which bFGF binds with approximately nanomolar affinity (Moscatelli (1987) J. Cell. Physiol. 131:123). All biological effects of bFGF are mediated through interaction with the high-affinity binding sites (10-100 pM) that represent the dimeric tyrosine kinase FGF receptor (Ueno et al. (1992) J. Biol. Chem. 267:1470). Five FGF receptor genes have been identified to date, each of which can produce several structural variants as a result of alternative mRNA splicing (Armstrong et al. (1992) Cancer Res. 52:2004; Ueno et al. (1992) supra). There is by now substantial evidence that the low- and the high-affinity binding sites act cooperatively in determining the overall affinity of bFGF. Experiments with mutant cell lines that are deficient in glycosaminoglycan synthesis (Yayon et al. (1991) Cell 64:841) or heparitinase treated cells (Rapraeger et al. (1991) Science 252:1705) have shown that binding of either cell-associated heparan sulfate or, in its absence, exogenously added heparin to bFGF is required for signaling via the tyrosine kinase receptor. Recent resolution of observed Kd into its kinetic components demonstrates that while the association rates of bFGF to the low- and the high-affinity sites are comparable, the dissociation rate of bFGF from the cell surface receptor is 23-fold slower than that for the cell-associated heparan sulfate (Nugent & Edelman (1992) Biochemistry 31:8876). The slower off-rate, however, is only observed when the receptor is bound to the cell surface suggesting that simultaneous binding to both sites contributes to the overall high-affinity binding. This is plausible in light of the observation that the heparin-binding and the receptor-binding sites are located on adjacent but separate regions of the molecule, as determined from the recently solved X-ray crystal structure of bFGF (Zhang et al. (1991) Proc. Natl. Acad. Sci. USA 88:3446; Eriksson et al. (1991) Proc. Natl. Acad. Sci. USA 88:3441; Ago et al. (1991) J. Biochem. 110:360; Zhu et al. (1991) Science 251:90).

The idea that bFGF antagonists may have useful medicinal applications is not new (reviewed in Gospodarowicz (1991) supra). bFGF is now known to play a key role in the development of smooth-muscle cell lesions following vascular injury (Reidy et al. Circulation, Suppl. III 86:III-43). Overexpression of bFGF (and other members of the FGF family) is correlated with many malignant disorders (Halaban et al. (1991) Ann. N. Y. Acad. Sci. 638:232; Takahashi et al. (1990) Proc. Natl. Acad. Sci. USA 87:5710; Fujimoto et al. (1991) Biochem. Biophys. Res. Commun. 180:386) and recently, neutralizing anti-bFGF antibodies have been found to suppress solid tumor growth in vivo by inhibiting tumor-linked angiogenesis (Hori et al. (1991) Cancer Res. 51:6180). Notable in this regard is the recent therapeutic examination of suramin, a polysulfated naphthalene derivative with known antiprotozoal activity, as an anti-tumor agent. Suramin is believed to inhibit the activity of bFGF through binding in the polyanion binding site and disrupting interaction of the growth factor with its receptor (Middaugh et al. (1992) Biochemistry 31:9016; Eriksson et al. (1992) supra). In addition to having a number of undesirable side effects and substantial toxicity, suramin is known to interact with several other heparin-binding growth factors which makes linking of its beneficial therapeutic effects to specific drug-protein interactions difficult (La Rocca et al. (1990) Cancer Cells 2:106). Anti-angiogenic properties of certain heparin preparations have also been observed (Folkman et al. (1983) Science 221:719; Crum et al. (1985) Science 250:1375) and these effects are probably based at least in part on their ability to interfere with bFGF signaling. While the specific heparin fraction that contributes to bFGF binding is now partially elucidated (Ishai-Michaeli et al. (1992) Biochemistry 31:2080; Turnbull et al. (1992) J. Biol. Chem. 267:10337), a typical heparin preparation is heterogeneous with respect to size, degree of sulfation and iduronic acid content. Additionally, heparin also affects many enzymes and growth factors. Excluding monoclonal antibodies, therefore, specific antagonists of bFGF are not known.

Tuerk & Gold, *Science* 1990 249:505-10, describe the basic SELEX method using an RNA candidate mixture and T4 DNA polymerase as a target.

Thiesen & Bach, *Nucleic Acids Res.* 1990 18:3203-9, describes a new method called Target Detection Assay (IDA) to determine DNA binding sites for putative DNA binding proteins. The TDA cycle consists of four separate steps: a randomized DNA/protein incubation step, a protein:DNA complex separation step, a DNA elution step and a polymerase chain reaction (PCR) DNA amplification step. To test the efficiency of the TDA procedure, potential DNA binding sites were selected by the DNA binding protein SP1 from a pool of oligonucleotides with random nucleotides at 12 positions. Target sites selected by recombinant SP1 closely matched the SP1 consensus site.

Famulok & Szostak, *Angew. Chem. Int. Ed. Engl*. 1992 31:979-88, is a review article describing *in vitro* selection of specific ligand binding nucleic acids. The article describes SELEX as a strategy for generating RNA molecules that bind to DNA. Also discussed are selection of DNA for protein recognition, selection for altered catalytic properties of ribozymes, and molecular recognition of small substrates by nucleic acids.

Bartel, et al., *Cell* 1991 67:529-36 describes an *in vitro* genetic selection to identify the important structural features of the viral RNA element bound by the Rev protein of HIV-1. Functional Rev-binding RNAs were selected from a pool of $10^{13}$ variants of the wild-type Rev-binding domain. Bases conserved among the binding species defined a 20 nucleotide core binding element. Covariation of some of these conserved bases indicated that the Rev-binding element was a stem-bulge-stem with a G:G base pair in the bulge.

Toole, et al., WO 92/14843 describes a SELEX method for identifying oligomer sequences which specifically bind target molecules such as serum proteins, kinins, eicosanoids and extracellular proteins. The method was used to generate aptamers that bound to serum Factor X, thrombin, bradykinin, PGF2 alpha and cell surface molecules.

Ecker, et al., WO 93/04204 describes SELEX-like methods useful for the determination of oligomers which have specific activity for a target molecule from a pool of primarily randomly assembled subunits. The disclosed methods involve repeated syntheses of increasingly simplified sets of oligomers coupled with selection procedures for determining oligomers having the highest activity."

## SUMMARY OF THE INVENTION

The present invention includes methods for identifying and producing nucleic acid ligands and the nucleic acid ligands so identified and produced. The SELEX method described above allows for the identification of a single nucleic acid ligand or a family of nucleic acid ligands to a given target. The methods of the present invention allow for the analysis of the nucleic acid ligand or family of nucleic acid ligands obtained by SELEX in order to identify and produce improved nucleic acid ligands.

Included in this invention are methods for determining the three-dimensional structure of nucleic acid ligands. Such methods include mathematical modeling and structure modifications of the SELEX derived ligands. Further included are methods for determining which nucleic acid residues in a nucleic acid ligand are necessary for maintaining the three-dimensional structure of the ligand, and which residues interact with the target to facilitate the formation of ligand-target binding pairs.

In one embodiment of the present invention, nucleic acid ligands are desired for their ability to inhibit one or more of the biological activities of the target. In such cases, methods are provided for determining whether the nucleic acid ligand effectively inhibits the desired biological activity.

Further included in this invention are methods for identifying tighter-binding RNA ligands and smaller, more stable ligands for use in pharmaceutical or diagnostic purposes.

The present invention includes improved nucleic acid ligands to the HIV-RT and HIV-1 Rev proteins. Also included are nucleic acid sequences that are substantially homologous to and that have substantially the same ability to bind HIV-RT or the HIV-1 Rev protein as the nucleic acid ligands specifically identified herein.

Also included within the scope of the invention is a method for performing sequential SELEX experiments in order to identify extended nucleic acid ligands. In particular, extended nucleic acid ligands to the HIV-RT protein are disclosed. Nucleic acid sequences that are substantially homologous to and that have substantially the same ability to bind HIV-RT as the extended HIV-RT nucleic acid ligands are also included in this invention.

Included within the scope of the invention are nucleic acid ligands to the HIV-1 tat protein. More specifically, RNA sequences have been identified that are capable of binding to the tat protein. Included within the invention are the nucleic acid ligand solutions shown in Figures 26 and 27.

Further included in this invention is a method of identifying nucleic acid ligands and ligand solutions to the HIV-1 tat protein comprising the steps of a) preparing a candidate mixture of nucleic acids; b) partitioning between members of said candidate mixture on the basis of affinity to the tat protein; and c) amplifying the selected molecules to yield a mixture of nucleic acids enriched for nucleic acid sequences with a relatively higher affinity for binding to the tat protein.

Further included in this invention are nucleic acid ligands to thrombin. More specifically, RNA sequences have been identified that are capable of binding to thrombin. Included within the invention are the nucleic acid ligand solutions shown in Figures 29 and 30.

Further included in this invention is a method of identifying nucleic acid ligands and ligand solutions to thrombin comprising the steps of a) preparing a candidate mixture of nucleic acids; b) partitioning between members of said candidate mixture on the basis of affinity to thrombin; and c) amplifying the selected molecules to yield a mixture of nucleic acids enriched for nucleic acid sequences with a relatively higher affinity for binding to thrombin.

More specifically, the present invention includes the RNA ligands to thrombin identified according to the above-described method, including those ligands listed in Figure 29 (SEQ ID NO:137-155). Also included are RNA ligands to thrombin that are substantially homologous to any of the given ligands and that have substantially the same ability to bind to thrombin. Further included in this invention are RNA ligands to thrombin that have substantially the same structural form as the ligands presented herein and that have substantially the same ability to bind thrombin.

Further included in this invention are nucleic acid ligands to bFGF. Specifically, RNA sequences are provided that are capable of binding specifically to bFGF. Included within the invention are the nucleic acid ligand sequences shown in Tables II-IV (SEQ ID NO:27-89).

Also included in this invention are nucleic acid ligands of bFGF that are inhibitors of bFGF. Specifically, RNA ligands are identified and described which inhibit the binding of bFGF to its receptors.

Further included in this invention is a method of identifying nucleic acid ligands and ligand sequences to bFGF comprising the steps of a) preparing a candidate mixture of nucleic acids; b) partitioning between members of said candidate mixture on the basis of affinity to bFGF; and c) amplifying the selected molecules to yield a mixture of nucleic acids enriched for nucleic acid sequences with a relatively higher affinity for binding to bFGF.

More specifically, the present invention includes the RNA ligands to bFGF identified according to the above-described method, including those ligands listed in Tables II-IV. Also included are RNA ligands to bFGF that are substantially homologous to any of the given ligands and that have substantially the same ability to bind and inhibit bFGF. Further included in this invention are RNA ligands to bFGF that have substantially the same structural form as the ligands presented herein and that have substantially the same ability to bind and inhibit bFGF.

The present invention also includes modified nucleotide sequences based on the nucleic acid ligand sequences identified herein and mixtures of the same. Specifically included in this invention are RNA ligands, that have been modified at the ribose and/or phosphate and/or base positions to increase in vivo stability of the RNA ligand. Other modifications to RNA ligands are encompassed by this invention, including specific alterations in base sequence, and additions of nucleic acids or non-nucleic acid moieties to the original compound.

## BRIEF DESCRIPTION OF THE FIGURES

Figure 1 depicts the consensus pseudoknot derived from primary and secondary SELEX experiments describing high affinity inhibitory ligands of HIV-1 reverse transcriptase (HIV-RT). The consensus secondary structure is a pseudoknot; the 5' helix of that pseudoknot (Stem 1) is conserved at the primary sequence level and the 3' helix or Stem 2 is not. X indicates a nucleotide position that is non-conserved; X-X' indicates a preferred base-pair. The 26 nucleotide positions are numbered as shown.

Figure 2 depicts refinement of the 5' information boundary. A set of model ligands were synthesized with T7 RNA polymerase from template oligos. Milligan et al. (1987) Nucl. Acid. Res. 15:8783). Illustrated in the upper left is the complete ligand B. On the right margin are shown the variations in the individual ligands A through E that occur in the boxed areas. In the graph are shown the individual binding curves for these model ligands.

Figure 3 depicts the effect of various nucleotide substitutions within the ligand B sequence on binding to HIV-RT. Illustrated are the various substitutions and resultant affinities to HIV-RT expressed relative to the binding of ligand B. Ligand B was a control tested in each experiment; the affinity of ligand B is normalized as 1.0 and the relative affinity (Kd of ligand B is divided by the Kd of each ligand) is shown. Also shown are the affinities of various truncations of ligand B. The value associated with the asterisked G-G which replaces U1-G16 comes from ligand C of Figure 2.

Figure 4 depicts a chemical probe of the native versus denatured conformations of ligand B. The various nucleotides of ligand B were reacted with chemicals under native and denaturing conditions, assayed for the modified positions, electrophoresed and visualized for comparison. ■ indicate highly reactive base-pairing groups of the base at that position and □ partially reactivity; Δindicates strong reactivity of purine N7 positions and Δ partial reactivity (to modification with DEPC). The question marks indicate that these positions on G(-2) and G(-1) could not be distinguished due to band crowding on the gel.

Figure 5 depicts reactivities of modifiable groups of ligand B when bound to HIV-RT. Diagrammed are those groups that show altered reactivity when bound to BIV-RT as compared to that of the native conformation.

Figure 6 depicts modification interference results for ligand B complexing with HIV-RT. Symbols for modification are as in the boxed legend. The modifications indicated are those that are strongly (filled symbols) or partially (unfilled symbols) selected against by binding to HIV-RT (reflected by decreased modification at those positions in the selected population).

Figure 7 depicts substitution of 2'-methoxy for hydroxyl on the riboses of the ligand B sequence shown in the upper right. Open circles represent hydroxyl groups at indicated positions and filled circles indicated methoxy substitution.

Figure 8 depicts selection by HIV-RT from mixed populations of 2'-methoxy ribose versus 2'-hydroxyl at positions U1 through A5 and A12 through A20. An oligonucleotide was synthesized with the following sequence (SEQ ID NO:2):

$$5'\text{-}(AAAAA)_d(UCCGA)_x(AGUGCA)_m(ACGGGAAAA)_x(UGCACU)_{m-3'}$$

where subscripted "d" indicates 2'-deoxy, subscripted "x" that those nucleotides are mixed 50-50 for phosphoramidite reagents resulting in 2'-methoxy or 2'-hydroxyl on the ribose, and subscripted "m" indicating that those nucleotides are all 2'-methoxy on the ribose.

Figure 9 shows the starting RNA and the collection of sequences (SEQ ID NO:115-135) obtained from SELEX with HIV-RT as part of a walking experiment.

Figure 10 illustrates the consensus extended HIV-RT ligand (SEQ ID NO:136) obtained from the list of sequences shown in Figure 9.

Figure 11 illustrates the revised description of the pseudoknot ligand of HIV-RT. In addition to the labeling conventions

of Figure 1, the S-S' indicates the preferred C-G or G-C base-pair at this position.

Figure 12 shows the sequence of a high-affinity RNA ligand for HIV-1 Rev protein obtained from SELEX experiments. Shown is the numbering scheme used for reference to particular bases in the RNA. This sequence was used for chemical modification with ENU. Figure 12 also shows at a) the extended RNA sequence used in chemical modification experiments with DMS, kethoxal, CMCT, and DEPC. The sequence of the oligonucleotide used for primer extension of the extended ligand sequence is shown at b).

Figure 13 depicts the results of chemical modification of the HIV-1 Rev ligand RNA under native conditions. a) lists chemical modifying agents, their specificity, and the symbols denoting partial and full modification. The RNA sequence is shown, with degree and type of modification displayed for every modified base. b) depicts the helical, bulge, and hairpin structural elements of the HIV-1 Rev RNA ligand corresponding to the modification and computer structural prediction data.

Figure 14 depicts the results of chemical modification of the ligand RNA that interferes with binding to the HIV-1 Rev protein. Listed are the modifications which interfere with protein binding, classified into categories of strong interference and slight interference. Symbols denote either base-pairing modifications, N7 modifications, or phosphate modifications.

Figure 15 depicts the modification interference values for phosphate alkylation. Data is normalized to A17 3' phosphate.

Figure 16 depicts the modification interference values for DMS modification of N3C and N1A. Data is normalized to C36; A34.

Figure 17 depicts the modification intereference values for kethoxal modification of N1G and N2G. Data is normalized to G5.

Figure 18 depicts the modification interference values for CMCT modification of N3U and N1G. Data is normalized to U38.

Figure 19 depicts the modification interference values for DEPC modification of N7A and N7G. Data normalied to G19; A34.

Figure 20 depicts the chemical modification of the RNA ligand in the presence of the HIV-1 Rev protein. Indicated are those positions that showed either reduced modification or enhanced modification in the presence of protein as compared to modification under native conditions but without protein present.

Figure 21 shows the 5' and 3' sequences which flank the "6a" biased random region used in SELEX. The template which produced the initial RNA population was constructed from the following oligonucleotides:

**5'-CCCGGATCCTCTTTACCTCTGTGTGagatacagagtccacaaacgtgttc tcaatgcacccGGTCGGAAGGCCATCAATAGTCCC-3' (template oligo) (SEQ ID NO:3)**

**5'-CCGAAGCTTAATACGACTCACTATAGGGACTATTGATGGCCTTCCGACC-3' (5' primer) (SEQ ID NO:4)**

**5'-CCCGGATCCTCTTTACCTCTGTGTG-3' (3' primer)(SEQ ID NO:5)**

where the small-case letters in the template oligo indicate that at each position that a mixture of reagents were used in synthesis by an amount of 62.5% of the small case letter, and 12.5% each of the other three nucleotides. Listed below the 6a sequence are the sequences of 38 isolates cloned after six rounds of SELEX performed with Rev protein with this population of RNA. The differences found in these isolates from the 6a sequences are indicated by bold-faced characters. Underlined are the predicted base pairings that comprise the bulge-flanking stems of the Motif I Rev ligands. Bases that are included from the 5' and 3' fixed flanking sequences are lower case.

Figure 22 shows three sets of tabulations containing:

A) The count of each nucleotide found at corresponsing positions of the Rev 6a ligand sequence in the collection of sequences found in Figure 21;

B) The fractional frequency of each nucleotide found at these positions (x ÷ 38, where x is the count from 1.); and

C) The difference between the fractional frequency of B) and the expected frequency based on the input mixture of oligonucleotides during template synthesis [for "wild type" positions, (x ÷ 38) - 0.625 and for alternative sequences (x ÷ 38) - 0.125 ].

Figure 23 shows three sets of tabulations containing:

A) The count of each base pair found at corresponsing positions of the Rev 6a ligand sequence in the collection of sequences found in Figure 21,

B) The fractional frequency of each nucleotide found at these positions (x ÷ 38, where x is the count from A),

C) The difference between the fractional frequency of B) and the expected frequency based on the input mixture of oligonucleotides during template synthesis [for "wild type" positions, (x ÷ 38) - 0.39; for base pairs that contain one alternate nucleotide and one wild type nucleotide, (x ÷ 38) - 0.078; and for base pairings of two alternate nucleotides (x + 38) - 0.016]. Values are shown for purine pyrimidine pairings only, the other eight pyrimidine and purine pairings are collectively counted and shown as "other" and are computed for section C) as (x ÷ 38) - 0.252.

Figure 24 shows the previously determined Rev protein ligand Motif I consensus (U.S. patent application serial number 07/714,131 filed June 10, 1991; WO 91/19813). The 6a sequence from the same application, and the preferred consensus derived from the biased randomization SELEX as interpreted from the data presented in Figures 22 and 23. Absolutely conserved positions in the prefered consensus are shown in bold face characters, and S-S' indicates either a C-G or G-C base pair.

Figure 25 depicts the natural RNA sequence (or TAR RNA) from HIV-1 with which the tat protein interacts. The boxed region of the sequence identifies those nucleotides that have been found to be important in the tat-TAR interaction.

Figure 26 lists the sequences of ligands isolated by the present invention as nucleic acid ligands to the HIV-1 tat protein. The sequences are grouped according to common secondary structures and primary sequence in three motifs (I, II, and III). Inverted repeat sequences that predict RNA helices are shown with arrows. The regions of primary sequence homology within each motif are outlined with dashed boxes. The boundaries of the sequence information essential for high affinity binding is indicated by a solid-lined box. Sequences 1 and 17 do not fit into any of the three identified motifs.

Figure 27 depicts a schematic diagram of the consensus secondary structure and primary sequence of each of the ligand motifs given in Figure 26. X indicates non-conserved nucleotide positions. X' indicates a base-pairing complement to X at that position in a helix, R indicates purine and Y pyrimidine. The dashed line in motif III indicates a variable number of nucleotides at that portion of the loop.

Figure 28 is the tat protein concentration-dependent binding of selected ligand RNAs from Figure 26, and the 40n RNA candidate mixture, to nitrocellulose filters.

FIGURE 29 depicts nucleotide sequences (SEQ ID NO:137-155) of RNA ligands isolated by SELEX for the human thrombin protein (Sigma). Each sequence is divided into 3 blocks from left to right: 1) the 5' fixed region, 2) the 30N variable region, and 3) the 3' fixed region. Individual sequences are grouped into class I and class II by conserved sequence motifs within the 30N variable region as indicated by bold, underlined characters.

FIGURE 30 shows proposed secondary structures of RNA ligands (SEQ ID NO: 156-159). (A) shows the sequence of the 76 nucleotide class I RNA clones 6, 16, and 18, and the class II 72 nucleotide clone 27. The boundary determinations where [ denotes a 5' boundary and ] denotes a 3' boundary are also shown. The possible secondary structures of each RNA are shown in (B) as determined from boundary experiments. Boundaries are underlined. In (A) and (B) the 5' and 3' fixed regions are depicted by small case lettering, the 30N random region by caps and the conserved region by bold caps. The hairpin structures that were synthesized are boxed with the total number of nucleotides indicated.

FIGURE 31 depicts binding curves for thrombin ligands. In (A), RNAs with unique 30N sequence motifs (see Fig. 29) were chosen for binding analysis with human thrombin (Sigma), including the three from Class I: RNA 6, RNA 16, and RNA 18, and one from Class II: RNA 27. Binding of bulk RNA sequences of the 30N3 candidate mixture is also shown. In (B), binding of class I RNA clones 6, 16, 18 and class II RNA clone 27 is shown, but with human thrombin from Enzyme Research Laboratories. In (C), binding of the 15mer ssDNA 5'-GGTTGGTGTGGTTGG-3' (G15D) (SEQ ID NO:1), the class I clone 16 hairpin structures (24R, 39D) and the class II clone 27 hairpin structure (33R) (see Fig. 30B) are shown under identical conditions as in (B). In the case of the RNA hairpin structures, R denotes RNA synthesis and D denotes transcription from a DNA template.

FIGURE 32 depicts a binding comparison of RNA ligands between unmodified RNA and RNA with pyrimidines modified to contain the 2'-$NH_2$ ribose nucleotide. Binding comparisons of (A) bulk RNA 30N candidate mixture and 2-$NH_2$, modified 30N candidate mixture, (B) class I RNA 16 and 2-$NH_2$ modified RNA 16, and (C) class II RNA 27 and 2-$NH_2$ modified RNA 27 are shown.

FIGURE 33 depicts the competition experiments between the 15mer ssDNA G15D and RNA hairpin ligands of this invention for binding to human thrombin. In A) concentration of the tracer G15D is equal to the concentration of protein at 1 μM. The competitors for binding include G15D itself, the 24 and 39 nucleotide RNA hairpin structures from class I RNA 16, and the 33 nucleotide RNA hairpin structure from class II RNA 27 (see Figure 30). Binding is expressed as the relative fraction G15D bound, which is the ratio of G15D binding with competitor to G15D binding without competitor. In B) the RNA 33 is the tracer and the concentration of the tracer is equal to the concentration of protein at 300 $^{n}$M. The competitors for binding include th ssDNA G15D and RNA 24.

FIGURE 34 shows the results of functional assays of thrombin in the presence and absence of the RNA ligand inhibitors described. In A) the hydrolysis by thrombin of the chromogenic substrate S-2238 (H-D-Phe-Pip-Arg-pNitroaniline) at the indicated thrombin and RNA concentration was measured by the change in OD 405. In B) the conversion of fibrinogen to fibrin and resulting clot formation was measured by the tilt test in the presence and absence of the RNA ligand inhibitors described.

FIGURE 35 shows specificity of binding for thrombin ligands. Class I RNA 16, class II RNA 27, and bulk 30N3 RNA were chosen for binding analysis with A) human antithrombin III (Sigma), and B) human prothrombin (Sigma).

FIGURE 36 shows binding curves for family 1 ligand 7A (Δ), family 2 ligand 12A (□), random RNA, SELEX experiment A(+) and random RNA, SELEX experiment B (x). The fraction of RNA bound to nitrocellulose filters is plotted as a function of free protein concentration and data points were fitted to eq. 2. The following concentrations of RNA were used: < 100 pM for 7A and 12A, and 10 nM for random RNAs. Binding reactions were done at 37°C in phosphate buffered saline containing 0.01% human serum albumin.

FIGURE 37 shows the effect of RNA ligands 5A (○), 7A (Δ), 12A (□), 26A (◇), random RNA, SELEX experiment A (+) and random RNA, SELEX experiment B (x) on binding of $^{125}$I-bFGF to the low-affinity (panel A) and the high-affinity (panel B) cell-surface receptors. Experiments were done essentially as described in Roghani & Moscatelli (1992) J. Biol. Chem. 267:22156.

FIGURE 38 shows the competitive displacement of 32P-labeled RNA ligands 5A (○), 7A (Δ), 12A (□), and 26A (◇) by heparin (average molecular weight 5,000 Da). Percent of total input RNA bound to nitrocellulose filters is plotted as a function of heparin concentration. Experiments were done at 37°C in phosphate buffered saline containing 0.01% human serum albumin, 0.3 uM RNA, and 30 nM bFGF.

FIGURE 39 shows the proposed secondary structures for Family 1 ligands that bind to bFGF with high affinity. Arrows indicate double stranded (stem) regions that flank the conserved loop. Lower case symbols indicate nucleotides in the constant region.

FIGURE 40 shows the proposed secondary structures for Family 1 ligands.

FIGURE 41 shows the consensus structures for Family 1 and Family 2 ligands. Y = C or U; R = A or G; W = A or U; H = A, U, or C; D = A, G, or U; N = any base. Complementary bases are primed. Symbols in parenthesis indicate a variable number of bases or base pairs at that position ranging within limits given in the subscript.

## DETAILED DESCRIPTION OF THE PREFERRED EMBODIMENTS

This application is an extension and improvement of the method for identifying nucleic acid ligands referred to as SELEX. The SELEX method is described in detail in PCT Patent Publication WO 91/19813. published December 26, 1991 entitled Nucleic Acid Ligands.

This application includes methods for identifying and producing improved nucleic acid ligands based on the basic SELEX process. The application includes separate sections covering the following embodiments of the invention: I. The SELEX Process; II. Techniques for Identifying Improved Nucleic Acid Ligands Subsequent to Performing SELEX; III. Sequential SELEX Experiments - Walking; IV. Elucidation of Structure of Ligands Via Covariance Analysis; V. Elucidation of an Improved Nucleic Acid Ligand for HIV-RT (Example I); VI. Performance of Walking Experiment With HIV-RT Nucleic Acid Ligand to Identify Extended Nucleic Acid Ligands; and VII. Elucidation of an Improved Nucleic Acid Ligand for HIV-1 Rev Protein (Example II); Nucleic Acid Ligands to the HIV-1 tat Protein (Example III); Nucleic Acid Ligands to Thrombin (Example IV); and Nucleic Acid Ligands to bFGF (Example V).

Improved nucleic acid ligands to the HIV-RT and HIV-1 Rev proteins are disclosed and claimed herein. This invention includes the specific nucleic acid ligands identified herein. The scope of the ligands covered by the invention extends to all ligands of the HIV-RT and Rev proteins identified according to the procedures described herein. More specifically, this invention includes nucleic acid sequences that are substantially homologous to and that have substantially the same ability to bind the HIV-RT or Rev proteins, under physiological conditions, as the nucleic acid ligands identified herein. By substantially homologous, it is meant, a degree of homology in excess of 70%, most preferably in

excess of 80%. Substantially homologous also includes base pair flips in those areas of the nucleic acid ligands that include base pairing regions. Substantially the same ability to bind the HIV-RT or Rev protein means that the affinity is within two orders of magnitude of the affinity of the nucleic acid ligands described herein and preferably within one order of magnitude. It is well within the skill of those of ordinary skill in the art to determine whether a given sequence is substantially homologous to and has substantially the same ability to bind the BIV-RT or HIV-1 Rev protein as the sequences identified herein.

I. The SELEX Process.

In its most basic form, the SELEX process may be defined by the following series of steps:

1) A candidate mixture of nucleic acids of differing sequence is prepared. The candidate mixture generally includes regions of fixed sequences (i.e., each of the members of the candidate mixture contains the same sequences in the same location) and regions of randomized sequences. The fixed sequence regions are selected either: a) to assist in the amplification steps described below; b) to facilitate mimicry of a sequence known to bind to the target; or c) to enhance the concentration of a given structural arrangement of the nucleic acids in the candidate mixture. The randomized sequences can be totally randomized (i.e., the probability of finding a base at any position being one in four) or only partially randomized (e.g., the probability of finding a base at any location can be selected at any level between 0 and 100 percent).
2) The candidate mixture is contacted with the selected target under conditions favorable for binding between the target and members of the candidate mixture. Under these circumstances, the interaction between the target and the nucleic acids of the candidate mixture can be considered as forming nucleic acid-target pairs between the target and the nucleic acids having the strongest affinity for the target.
3) The nucleic acids with the highest affinity for the target are partitioned from those nucleic acids with lesser affinity to the target. Because only an extremely small number of sequences (and possibly only one molecule of nucleic acid) corresponding to the highest affinity nucleic acids exist in the candidate mixture, it is generally desirable to set the partitioning criteria so that a significant amount of the nucleic acids in the candidate mixture (approximately 5-50%) are retained during partitioning.
4) Those nucleic acids selected during partitioning as having the relatively higher affinity to the target are then amplified to create a new candidate mixture that is enriched in nucleic acids having a relatively higher affinity for the target.
5) By repeating the partitioning and amplifying steps above, the newly formed candidate mixture contains fewer and fewer unique sequences, and the average degree of affinity of the nucleic acids to the target will generally increase. Taken to its extreme, the SELEX process will yield a candidate mixture containing one or a small number of unique nucleic acids representing those nucleic acids from the original candidate mixture having the highest affinity to the target molecule.

The SELEX Patent Applications describe and elaborate on this process in great detail. Included are targets that can be used in the process; methods for the preparation of the initial candidate mixture; methods for partitioning nucleic acids within a candidate mixture; and methods for amplifying partitioned nucleic acids to generate enriched candidate mixtures. The SELEX Patent Applications also describe ligand solutions obtained to a number of target species, including both protein targets wherein the protein is and is not a nucleic acid binding protein.

SELEX delivers high affinity ligands of a target molecule. This represents a singular achievement that is unprecedented in the field of nucleic acids research. The present invention is directed at methods for taking the SELEX derived ligand solution in order to develop novel nucleic acid ligands having the desired characteristics. The desired characteristics for a given nucleic acid ligand may vary. All nucleic acid ligands are capable of forming a complex with the target species. In some cases, it is desired that the nucleic acid ligand will serve to inhibit one or more of the biological activities of the target. In other cases, it is desired that the nucleic acid ligand serves to modify one or more of the biological activities of the target. In other cases, the nucleic acid ligand serves to identify the presence of the target, and its effect on the biological activity of the target is irrelevant.

II. Techniques for Identifying Improved Nucleic Acid Ligands Subsequent to Performing SELEX.

In order to produce nucleic acids desirable for use as a pharmaceutical, it is preferred that the nucleic acid ligand 1) binds to the target in a manner capable of achieving the desired effect on the target; 2) be as small as possible to obtain the desired effect; 3) be as stable as possible; and 4) be a specific ligand to the chosen target. In most, if not all, situations it is preferred that the nucleic acid ligand have the highest possible affinity to the target. Modifications or derivatizations of the ligand that confer resistance to degradation and clearance in situ during therapy, the capability to

cross various tissue or cell membrane barriers, or any other accessory properties that do not significantly interfere with affinity for the target molecule may also be provided as improvements. The present invention includes the methods for obtaining improved nucleic acid ligands after SELEX has been performed.

Assays of ligand effects on target molecule function. One of the uses of nucleic acid ligands derived by SELEX is to find ligands that alter target molecule function. Because ligand analysis requires much more work than is encountered during SELEX enrichments, it is a good procedure to first assay for inhibition or enhancement of function of the target protein. One could even perform such functional tests of the combined ligand pool prior to cloning and sequencing. Assays for the biological function of the chosen target are generally available and known to those skilled in the art, and can be easily performed in the presence of the nucleic acid ligand to determine if inhibition occurs.

Affinity assays of the ligands. SELEX enrichment will supply a number of cloned ligands of probable variable affinity for the target molecule. Sequence comparisons may yield consensus secondary structures and primary sequences that allow grouping of the ligand sequences into motifs. Although a single ligand sequence (with some mutations) can be found frequently in the total population of cloned sequences, the degree of representation of a single ligand sequence in the cloned population of ligand sequences may not absolutely correlate with affinity for the target molecule. Therefore mere abundance is not the sole criterion for judging "winners" after SELEX and binding assays for various ligand sequences (adequately defining each motif that is discovered by sequence analysis) are required to weigh the significance of the consensus arrived at by sequence comparisons. The combination of sequence comparison and affinity assays should guide the selection of candidates for more extensive ligand characterization.

Information boundaries determination. An important avenue for narrowing down what amount of sequence is relevant to specific affinity is to establish the boundaries of that information within a ligand sequence. This is conveniently accomplished by selecting end-labeled fragments from hydrolyzed pools of the ligand of interest so that 5' and 3' boundaries of the information can be discovered. To determine a 3' boundary, one performs a large-scale in vitro transcription of the PCR ligand, gel purifies the RNA using UV shadowing on an intensifying screen, phosphatases the purified RNA, phenol extracts extensively, labels by kinasing with $^{32}P$, and gel purifies the labeled product (using a film of the gel as a guide). The resultant product may then be subjected to pilot partial digestions with RNase T1 (varying enzyme concentration and time, at 50°C in a buffer of 7 M urea, 50 mM NaCitrate pH 5.2) and alkaline hydrolysis (at 50 mM $NaCO_3$, adjusted to pH 9.0 by prior mixing of 1M bicarbonate and carbonate solutions; test over ranges of 20 to 60 minutes at 95°C). Once optimal conditions for alkaline hydrolysis are established (so that there is an even distribution of small to larger fragments) one can scale up to provide enough material for selection by the target (usually on nitrocellulose filters). One then sets up binding assays, varying target protein concentration from the lowest saturating protein concentration to that protein concentration at which approximately 10% of RNA is bound as determined by the binding assays for the ligand. One should vary target concentration (if target supplies allow) by increasing volume rather than decreasing absolute amount of target; this provides a good signal to noise ratio as the amount of RNA bound to the filter is limited by the absolute amount of target. The RNA is eluted as in SELEX and then run on a denaturing gel with T1 partial digests so that the positions of hydrolysis bands can be related to the ligand sequence.

The 5' boundary can be similarly determined. Large-scale in vitro transcriptions are purified as described above. There are two methods for labeling the 3' end of the RNA. One method is to kinase Cp with $^{32}P$ (or purchase $^{32}P$-Cp) and ligate to the purified RNA with RNA ligase. The labeled RNA is then purified as above and subjected to very identical protocols. An alternative is to subject unlabeled RNAs to partial alkaline hydrolyses and extend an annealed, labeled primer with reverse transcriptase as the assay for band positions. One of the advantages over pCp labeling is the ease of the procedure, the more complete sequencing ladder (by dideoxy chain termination sequencing) with which one can correlate the boundary, and increased yield of assayable product. A disadvantage is that the extension on eluted RNA sometimes contains artifactual stops, so it may be important to control by spotting and eluting starting material on nitrocellulose filters without washes and assaying as the input RNA.

The result is that it is possible to find the boundaries of the sequence information required for high affinity binding to the target.

An instructive example is the determination of the boundaries of the information found in the nucleic acid ligand for HIV-RT. (See, U.S. patent application serial number 07/714,131 filed June 10, 1991 and PCT Patent Application Publication WO 91/19813, published December 26, 1991 entitled Nucleic Acid Ligands). These experiments are described in detail below. The original pool of enriched RNAs yielded a few specific ligands for HIV-RT (one ligand, 1.1, represented 1/4 of the total population, nitrocellulose affinity sequences represented 1/2 and some RNAs had no affinity for either). Two high-affinity RT ligands shared the sequence ...UUCCGNNNNNNNNCGGGAAAA.... (SEQ ID NO:6) Boundary experiments of both ligands established a clear 3' boundary and a less clear 5' boundary. It can be surmised from the boundary experiments and secondary SELEX experiments that the highest affinity ligands contained the essential information UCCGNNNNNNNNCGGGAAAAN'N'N'N' (SEQ ID NO:7)(where N's base pair to Ns in the 8 base loop sequence of the hairpin formed by the pairing of UCCG to CGGG) and that the 5' U would be dispensable with some small loss in affinity. In this application, the construction of model compounds confirmed that there was no difference in the affinity of sequences with only one 5' U compared to 2 5' U's (as is shared by the two compared ligands), that

removal of both U's caused a 5-fold decrease in affinity and of the next C a more drastic loss in affinity. The 3' boundary which appeared to be clear in the boundary experiments was less precipitous. This new information can be used to deduce that what is critical at the 3' end is to have at least three base-paired nucleotides (to sequences that loop between the two strands of Stem 1). Only two base-paired nucleotides result in a 12-fold reduction in affinity. Having no 3' base-paired nucleotides (truncation at the end of Loop 2) results in an approximately 70-fold reduction in affinity.

Quantitative and qualitative assessment of individual nucleotide contributions to affinity.

SECONDARY SELEX. Once the minimal high affinity ligand sequence is identified, it may be useful to identify the nucleotides within the boundaries that are crucial to the interaction with the target molecule. One method is to create a new random template in which all of the nucleotides of a high affinity ligand sequence are partially randomized or blocks of randomness are interspersed with blocks of complete randomness. Such "secondary" SELEXes produce a pool of ligand sequences in which crucial nucleotides or structures are absolutely conserved, less crucial features preferred, and unimportant positions unbiased. Secondary SELEXes can thus help to further elaborate a consensus that is based on relatively few ligand sequences. In addition, even higher-affinity ligands may be provided whose sequences were unexplored in the original SELEX.

In this application we show such a biased randomization for ligands of the BIV-1 Rev protein. In PCT Patent Application Publication HO 91/19813, published December 26, 1991 entitled Nucleic Acid Ligands, nucleic acid ligands to the HIV-1 Rev protein were described. One of these ligand sequences bound with higher affinity than all of the other ligand sequences (Rev ligand sequence 6a, shown in Figure 12) but existed as only two copies in the 53 isolates that were cloned and sequenced. In this application, this sequence was incorporated in a secondary SELEX experiment in which each of the nucleotides of the 6a sequence (confined to that part of the sequence which comprises a Rev protein binding site defined by homology to others of Rev ligand motif I) was mixed during oligonucleotide synthesis with the other three nucleotides in the the ratio 62.5:12.5:12.5:12.5. For example, when the sequence at position G1 is incorporated during oligo synthesis, the reagents for G,A,T, and C are mixed in the ratios 62.5:12.5:12.5:12.5. After six rounds of SELEX using the Rev protein, ligands were cloned from this mixture so that a more comprehensive consensus description could be derived.

NUCLEOTIDE SUBSTITUTION. Another method is to test oligo-transcribed variants where the SELEX consensus may be confusing. As shown above, this has helped us to understand the nature of the 5' and 3' boundaries of the information required to bind HIV-RT. As is shown in the attached example this has helped to quantitate the consensus of nucleotides within Stem 1 of the HIV-RT pseudoknot.

CHEMICAL MODIFICATION. Another useful set of techniques are inclusively described as chemical modification experiments. Such experiments may be used to probe the native structure of RNAs, by comparing modification patterns of denatured and non-denatured states. The chemical modification pattern of an RNA ligand that is subsequently bound by target molecule may be different from the native pattern, indicating potential changes in structure upon binding or protection of groups by the target molecule. In addition, RNA ligands will fail to be bound by the target molecule when modified at positions crucial to either the bound structure of the ligand or crucial to interaction with the target molecule. Such experiments in which these positions are identified are described as "chemical modification interference" experiments.

There are a variety of available reagents to conduct such experiments that are known to those skilled in the art (see, Ehresmann et al. (1987) Nuc. Acids. Res. 15:9109). Chemicals that modify bases can be used to modify ligand RNAs. A pool is bound to the target at varying concentrations and the bound RNAs recovered (much as in the boundary experiments) and the eluted RNAs analyzed for the modification. Assay can be by subsequent modification-dependent base removal and aniline scission at the baseless position or by reverse transcription assay of sensitive (modified) positions. In such assays bands (indicating modified bases) in unselected RNAs appear that disappear relative to other bands in target protein-selected RNAs. Similar chemical modifications with ethylnitrosourea, or via mixed chemical or enzymatic synthesis with, for example, 2'-methoxys on ribose or phosphorothioates can be used to identify essential atomic groups on the backbone. In experiments with 2'-methoxy vs. 2'-OH mixtures, the presence of an essential OH group results in enhanced hydrolysis relative to other positions in molecules that have been stringently selected by the target.

An example of how chemical modification can be used to yield useful information about a ligand and help efforts to improve its functional stability is given below for HIV-RT. Ethylnitrosourea modification interference identified 5 positions at which modification interfered with binding and 2 of those positions at which it interfered drastically. Modification of various atomic groups on the bases of the ligand were also identified as crucial to the interaction with HIV-RT. Those positions were primarily in the 5' helix and bridging loop sequence that was highly conserved in the SELEX phylogeny (Stem I and Loop 2, Figure 1). These experiments not only confirmed the validity of that phylogeny, but informed ongoing attempts to make more stable RNAs. An RT ligand was synthesized in which all positions had 2'-methoxy at the ribose portions of the backbone. This molecule bound with drastically reduced affinity for HIV-RT. Based on the early modification

interference experiments and the SELEX phylogeny comparisons, it could be determined that the 3' helix (Stem II Fig. 1) was essentially a structural component of the molecule. A ligand in which the 12 ribose residues of that helix were 2'-methoxy was then synthesized and it bound with high affinity to HIV-RT. In order to determine if any specific 2'-OHs of the remaining 14 residues were specifically required for binding, a molecule in which all of the riboses of the pseudoknot were synthesized with mixed equimolar (empirically determined to be optimal) reagents for 2'-OH and 2'-methoxy formation. Selection by HIV-RT from this mixture followed by alkaline hydrolysis reveals bands of enhanced hydrolysis indicative of predominating 2' hydroxyls at those positions. Analysis of this experiment lead to the conclusion that residues (G4, A5, C13 and G14) must have 2'-OH for high affinity binding to HIV-RT.

Comparisons of the intensity of bands for bound and unbound ligands may reveal not only modifications that interfere with binding, but also modifications that enhance binding. A ligand may be made with precisely that modification and tested for the enhanced affinity. Thus chemical modification experiments can be a method for exploring additional local contacts with the target molecule, just as "walking" (see below) is for additional nucleotide level contacts with adjacent domains.

One of the products of the SELEX procedure is a consensus of primary and secondary structures that enables the chemical or enzymatic synthesis of oligonucleotide ligands whose design is based on that consensus. Because the replication machinery of SELEX requires that rather limited variation at the subunit level (ribonucleotides, for example), such ligands imperfectly fill the available atomic space of a target molecule's binding surface. However, these ligands can be thought of as high-affinity scaffolds that can be derivatized to make additional contacts with the target molecule. In addition, the consensus contains atomic group descriptors that are pertinent to binding and atomic group descriptors that are coincidental to the pertinent atomic group interactions. For example, each ribonucleotide of the pseudoknot ligand of HIV-RT contains a 2' hydroxyl group on the ribose, but only two of the riboses of the pseudoknot ligand cannot be substituted at this position with 2'-methoxy. A similar experiment with deoxyribonucleotide mixtures with ribonucleotide mixtures (as we have done with 2'-methoxy and 2' hydroxy mixtures) would reveal which riboses or how many riboses are dispensable for binding HIV-RT. A similar experiment with more radical substitutions at the 2' position would again reveal the allowable substitutions at 2' positions. One may expect by this method to find derivatives of the pseudoknot ligand that confer higher affinity association with HIV-RT. Such derivatization does not exclude incorporation of cross-linking agents that will give specifically directly covalent linkages to the target protein. Such derivatization analyses are not limited to the 2' position of the ribose, but could include derivatization at any position in the base or backbone of the nucleotide ligand.

A logical extension of this analysis is a situation in which one or a few nucleotides of the polymeric ligand is used as a site for chemical derivative exploration. The rest of the ligand serves to anchor in place this monomer (or monomers) on which a variety of derivatives are tested for non-interference with binding and for enhanced affinity. Such explorations may result in small molecules that mimic the structure of the initial ligand framework, and have significant and specific affinity for the target molecule independent of that nucleic acid framework. Such derivatized subunits, which may have advantages with respect to mass production, therapeutic routes of administration, delivery, clearance or degradation than the initial SELEX ligand, may become the therapeutic and may retain very little of the original ligand. This approach is thus an additional utility of SELEX. SELEX ligands can allow directed chemical exploration of a defined site on the target molecule known to be important for the target function.

Structure determination. These efforts have helped to confirm and evaluate the sequence and structure dependent association of ligands to HIV-RT. Additional techniques may be performed to provide atomic level resolution of ligand/target molecule complexes. These are NMR spectroscopy and X-ray crystallography. With such structures in hand, one can then perform rational design as improvements on the evolved ligands supplied by SELEX. The computer modeling of nucleic acid structures is described below.

Chemical Modification. This invention includes nucleic acid ligands wherein certain chemical modifications have been made in order to increase the in vivo stability of the ligand or to enhance or mediate the delivery of the ligand. Examples of such modifications include chemical substitutions at the ribose and/or phosphate positions of a given RNA sequence. See, e.g., Cook, et al. PCT Application WO 9203568; U.S. Patent No. 5,118,672 of Schinazi et al.; Hobbs et al. (1973) Biochem. 12:5138; Guschlbauer et al. (1977) Nucleic Acids Res. 4:1933; Shibaharu et al. Nucl. Acids. Res. (1987) 15:4403; Pieken et al. (1991) Science 253:314.

## III. Sequential SELEX Experiments - Walking.

In one embodiment of this invention, after a minimal consensus ligand sequence has been determined for a given target, it is possible to add random sequence to the minimal consensus ligand sequence and evolve additional contacts with the target, perhaps to separate but adjacent domains. This procedure is referred to as "walking" in the SELEX Patent Applications. The successful application of the walking protocol is presented below to develop an enhanced binding ligand to HIV-RT.

The walking experiment involves two SELEX experiments performed sequentially. A new candidate mixture is

produced in which each of the members of the candidate mixture has a fixed nucleic acid region that corresponds to a SELEX-derived nucleic acid ligand. Each member of the candidate mixture also contains a randomized region of sequences. According to this method it is possible to identify what are referred to as "extended" nucleic acid ligands, that contain regions that may bind to more than one binding domain of a target.

IV. Elucidation of Structure of Ligands Via Covariance Analysis.

In conjunction with the empirical methods for determining the three dimensional structure of nucleic acids, the present invention includes computer modeling methods for determining structure of nucleic acid ligands.

Secondary structure prediction is a useful guide to correct sequence alignment. It is also a highly useful stepping-stone to correct 3D structure prediction, by constraining a number of bases into A-form helical geometry.

Tables of energy parameters for calculating the stability of secondary structures exist. Although early secondary structure prediction programs attempted to simply maximize the number of base-pairs formed by a sequence, most current programs seek to find structures with minimal free energy as calculated by these thermodynamic parameters. There are two problems in this approach. First, the thermodynamic rules are inherently inaccurate, typically to 10% or so, and there are many different possible structures lying within 10% of the global energy minimum. Second, the actual secondary structure need not lie at a global energy minimum, depending on the kinetics of folding and synthesis of the sequence. Nonetheless, for short sequences, these caveats are of minor importance because there are so few possible structures that can form.

The brute force predictive method is a dot-plot: make an N by N plot of the sequence against itself, and mark an X everywhere a basepair is possible. Diagonal runs of X's mark the location of possible helices. Exhaustive tree-searching methods can then search for all possible arrangements of compatible (i.e., nonoverlapping) helices of length L or more; energy calculations may be done for these structures to rank them as more or less likely. The advantages of this method are that all possible topologies, including pseudoknotted conformations, may be examined, and that a number of suboptimal structures are automatically generated as well. The disadvantages of the method are that it can run in the worst cases in time proportional to an exponential factor of the sequence size, and may not (depending on the size of the sequence and the actual tree search method employed) look deep enough to find a global minimum.

The elegant predictive method, and currently the most used, is the Zuker program (Zuker (1989) Science 244:48). Originally based on an algorithm developed by Ruth Nussinov, the Zuker program makes a major simplifying assumption that no pseudoknotted conformations will be allowed. This permits the use of a dynamic programming approach which runs in time proportional to only $N^3$ to $N^4$, where N is the length of the sequence. The Zuker program is the only program capable of rigorously dealing with sequences of than a few hundred nucleotides, so it has come to be the most commonly used by biologists. However, the inability of the Zuker program to predict pseudoknotted conformations is a fatal flaw, in that several different SELEX experiments so far have yielded pseudoknotted RNA structures, which were recognized by eye. A brute-force method capable of predicting pseudoknotted conformations must be used.

The central element of the comparative sequence analysis of the present invention is sequence covariations. A covariation is when the identity of one position depends on the identity of another position; for instance, a required Watson-Crick base pair shows strong covariation in that knowledge of one of the two positions gives absolute knowledge of the identity at the other position. Covariation analysis has been used previously to predict the secondary structure of RNAs for which a number of related sequences sharing a common structure exist, such as tRNA, rRNAs, and group I introns. It is now apparent that covariation analysis can be used to detect tertiary contacts as well.

Stormo and Gutell have designed and implemented an algorithm that precisely measures the amount of covariations between two positions in an aligned sequence set. The program is called "MIXY" - Mutual Information at position X and Y.

Consider an aligned sequence set. In each column or position, the frequency of occurrence of A, C, G, U, and gaps is calculated. Call this frequency $f(b_x)$, the frequency of base b in column x. Now consider two columns at once. The frequency that a given base b appears in column x is $f(b_x)$ and the frequency that a given base b appears in column y is $f(b_y)$. If position x and position y do not care about each other's identity - that is, the positions are independent; there is no covariation - the frequency of observing bases $b_x$ and $b_y$ at position x and y in any given sequence should be just $f(b_xb_y) = f(b_x)f(b_y)$. If there are substantial deviations of the observed frequencies of pairs from their expected frequencies, the positions are said to covary. The amount of deviation from expectation may be quantified with an information measure M(x,y), the mutual information of x and y:

$$M(x,y) = \sum_{b_x b_y} f(b_x b_y) \ln \frac{f(b_x b_y)}{f(b_x) f(b_y)}$$

M(x,y) can be described as the number of bits of information one learns about the identity of position y from knowing just the identity of position y from knowing just the identity of position x. If there is no covariation, M(x,y) is zero; larger values of M(x,y) indicate strong covariation.

These numbers correlated extremely well to a probability for close physical contact in the tertiary structure, when this procedure was applied to the tRNA sequence data set. The secondary structure is extremely obvious as peaks in the M(x,y) values, and most of the tertiary contacts known from the crystal structure appear as peaks as well.

These covariation values may be used to develop three-dimensional structural predictions.

In some ways, the problem is similar to that of structure determination by NMR. Unlike crystallography, which in the end yields an actual electron density map, NMR yields a set of interatomic distances. Depending on the number of interatomic distances one can get, there may be one, few, or many 3D structures with which they are consistent. Mathematical techniques had to be developed to transform a matrix of interatomic distances into a structure in 3D space. The two main techniques in use are distance geometry and restrained molecular dynamics.

Distance geometry is the more formal and purely mathematical technique. The interatomic distances are considered to be coordinates in an N-dimensional space, where N is the number of atoms. In other words, the "position" of an atom is specified by N distances to all the other atoms, instead of the three (x,y,z) that we are used to thinking about. Interatomic distances between every atom are recorded in an N by N distance matrix. A complete and precise distance matrix is easily transformed into a 3 by N Cartesian coordinates, using matrix algebra operations. The trick of distance geometry as applied to NMR is dealing with incomplete (only some of the interatomic distances are known) and imprecise data (distances are known to a precision of only a few angstroms at best). Much of the time of distance geometry-based structure calculation is thus spent in pre-processing the distance matrix, calculating bounds for the unknown distance values based on the known ones, and narrowing the bounds on the known ones. Usually, multiple structures are extracted from the distance matrix which are consistent with a set of NMR data; if they all overlap nicely, the data were sufficient to determine a unique structure. Unlike NMR structure determination, covariance gives only imprecise distance values, but also only probabilistic rather than absolute knowledge about whether a given distance constraint should be applied.

Restrained molecular dynamics is a more ad hoc procedure. Given an empirical force field that attempts to describe the forces that all the atoms feel (van der Waals, covalent bonding lengths and angles, electrostatics), one can simulate a number of femtosecond time steps of a molecule's motion, by assigning every atom at a random velocity (from the Boltzmann distribution at a given temperature) and calculating each atom's motion for a femtosecond using Newtonian dynamical equations; that is "molecular dynamics". In restrained molecular dynamics, one assigns extra ad hoc forces to the atoms when they violate specified distance bounds.

In the present case, it is fairly easy to deal with the probabilistic nature of data with restrained molecular dynamics. The covariation values may be transformed into artificial restraining forces between certain atoms for certain distance bounds; varying the magnitude of the force according to the magnitude of the covariance.

NMR and covariance analysis generates distance restraints between atoms or positions, which are readily transformed into structures through distance geometry or restrained molecular dynamics. Another source of experimental data which may be utilized to determine the three dimensional structures of nucleic acids is chemical and enzymatic protection experiments, which generate solvent accessibility restraints for individual atoms or positions.

V. ELUCIDATION OF AN IMPROVED NUCLEIC ACID LIGAND FOR HIV-RT.

An example of the methods of the present invention are presented herein for the nucleic acid ligand for HIV-1 reverse transcriptase (HIV-RT).

PCT Patent Publication WO 91/19813, published December 26, 1991 entitled Nucleic Acid Ligands describes the results obtained when SELEX was performed with the HIV-RT target. Inspection of the nucleic acid sequences that were found to have a high affinity to HIV-RT, it was concluded that the nucleic acid ligand solution was configured as a pseudoknot.

Described herein are experiments which establish the minimum number of sequences necessary to represent the nucleic acid ligand solution via boundary studies. Also described are the construction of variants of the ligand solution which are used to evaluate the contributions of individual nucleotides in the solution to the binding of the ligand solution to HIV-RT. Also described is the chemical modification of the ligand solution; 1) to corroborate its predicted pseudoknot structure; 2) to determine which modifiable groups are protected from chemical attack when bound to HIV-RT (or become unprotected during binding); and 3) to determine what modifications interfere with binding to HIV-RT (presumably by modification of the three dimensional structure of the ligand solution) and, therefore, which are presumably involved in the proximal contacts with the target.

The nucleic acid ligand solution previously determined is shown in Figure 1. Depicted is an RNA pseudoknot in which Stem 1 (as labeled) is conserved and Stem 2 is relatively non-conserved; X indicates no conservation and X' base-pairs to X. In the original SELEX consensus U1 was preferred (existing at this relative position in 11 of the 18 sequences that contributed to the consensus), but A1 was also found frequently (in 6 of the 18). There were two sequences

in which C-G was substituted for the base-pair of G4-C13 and one A-U substitution. The preferred number of nucleotides connecting the two strands of Stem 1 was eight (in 8 of 18). The number and pattern of base-paired nucleotides comprising Stem 2 and the preference for A5 and A12 were derived from the consensus of a secondary SELEX in which the random region was constructed as follows NNUUCCGNNNNNNNNCGGGAAAANNNN (SEQ ID NO:8)(Ns are randomized). One of the ligands was found to significantly inhibit HIV-RT and failed to inhibit AMV or MMLV reverse transcriptases.

Refinement of the information boundaries. The first two SELEX experiments in which 32 nucleotide positions were randomized provided high affinity ligands in which there was variable length for Stem 1 at its 5' end; that is, some ligands had the sequence UUCCG which could base pair to CGGGA, UCCG to CGGG or CCG to CGG. Determination of the boundaries of the sequences donating high-affinity to the interaction with HIV-RT was accomplished by selection from partial alkaline hydrolysates of end-labeled clonal RNAs, a rapid but qualitative analysis which suggested that the highest affinity ligands contained the essential information UCCGNNNNNNNNCGGGAAAANN'N'N'(SEQ ID NO:7)(where N's base pair to Ns in the 8 base loop sequence of the hairpin formed by the pairing of UCCG to CGGG) and that the 5' U would be dispensable with some small loss in affinity. In order to more stringently test the 5' sequences in a homogeneous context, the binding experiments depicted in Figure 2 were performed. The RNA's transcribed from oligonucleotide templates were all the same as the complete sequence shown in the upper right hand corner of the figure, except for the varying 5' ends as shown in the boxes A-E lining the left margin. The result is that one 5' U is sufficient for the highest-affinity binding to HIV-RT (boxes A and B), that with no U there is reduced binding (box C), and that any further removal of 5' sequences reduces binding to that of non-specific sequences (box D). The design (hereafter referred to as ligand B) with only one 5' U (U1) was used for the rest of the experiments described here.

Dependence on the length of Stem 2 was also examined by making various 3' truncations at the 3' end of ligand B. Deletion of as many as 3 nucleotides from the 3' end (A24-U26) made no difference in affinity of the molecule for HIV-RT. Deletion of the 3'-terminal 4 nucleotides (C23-U26) resulted in 7-fold reduced binding, of 5 (G22-U26) resulted in approximately 12-fold reduction and of 6 nucleotides (U21-U26, or no 3' helix) an approximately 70-fold reduction in affinity. Such reductions were less drastic than reductions found for single-base substitutions reported below, suggesting (with other data reported below) that this helix serves primarily a structural role that aids the positioning of crucial groups in Loop 2.

Testing the SELEX consensus for Stem 1. Various nucleotide substitutions in the conserved Stem 1 were prepared and their affinity to HIV-RT determined. As shown in Figure 3, substitution of an A for U1 in model RNAs made little difference in affinity for HIV-RT. C (which would increase the stability of Stem1) or G (represented by the U deletion experiment above) at this position resulted in approximately 20-fold lowering in affinity. Substitution of A for G16 (which would base-pair to U1) abolished specific binding. A G-C pair was substituted for C2-G15 which also abolished binding and for C3-G14 which reduced binding about 10-fold. These two positions were highly conserved in the phylogeny of SELEX ligands. Various combinations were substituted for the G4-C13 base pair. The order of affect of these on affinity were G4-C13=C-G>U-A>A-U>>>>A-C where A-U is about 20-fold reduced in affinity compared to G4-C13 and A-C is at least 100- fold reduced. These results are consistent with the SELEX consensus determined previously.

Chemical probing of the pseudoknot structure. A number of chemical modification experiments were conducted to probe the native structure of ligand B, to identify chemical modifications that significantly reduced affinity of ligand B for HIV-RT, and to discover changes in structure that may accompany binding by HIV-RT. The chemicals used were ethylnitrosourea (ENU) which modifies phosphates, dimethyl sulfate (DMS) which modifies the base-pairing faces of C (at N3) and A (at N1), carbodiimide (CMCT) which modifies the base-pairing face of U (at N3) and to some extent G (at N1), diethylpyrocarbonate (DEPC) which modifies N7 of A and to a lesser extent the N7 of G, and kethoxal which modifies the base-pairing N1 and N2 of G. Most of the assays of chemical modification were done on a ligand B sequence which was lengthened to include sequences to which a labeled primer could be annealed and extended with AMV reverse transcriptase. Assay of ENU or DEPC modified positions were done on ligand B by respective modification-dependent hydrolysis, or modified base removal followed by aniline scission of the backbone at these sites.

The results of probing the native structure as compared to modification of denatured ligand B are summarized in Figure 4. The pattern of ENU modification was not different between denatured native states of the ligand suggesting that there is no stable involvement of the phosphates or N7 positions of purines in the solution structure of the pseudoknot. The other modification data suggest that Stem 2 forms rather stably and is resistant to any chemical modifications affecting the base-pairs shown, although the terminal A6-U26 is somewhat sensitive to modification indicating equilibration between base-paired and denatured states at this position. The single-stranded As (A5, A17, A18, A19, and A20) are fully reactive with DMS although A5, A19, and A20 are diminished in reactivity to DEPC. The base-pairs of Stem 1 seem to exhibit a gradation of resistance to modification such that G4-C13>C3-G14>C2-G15>U1-G16 where G4-C13 is completely resistant to chemical modification and U1-G16 is highly reactive. This suggests that this small helix of the pseudoknot undergoes transient and directional denaturation or "fraying".

Protection of ligand B from chemical modification by HIV-RT. Binding of protein changes the fraying character of Helix I as shown in Figure 5 either by stabilizing or protecting it. The natively reactive U1 is also protected upon binding. Binding of protein increases the sensitivity of the base-pair A6-U26 suggesting that this is unpaired in the bound state.

This may be an indication of insufficient length of a single nucleotide Loop I during binding, either because it cannot bridge the bound Stem 1 to the end of Stem 2 in the native pseudoknot recognized by RT or because binding increases the length requirement of Loop I by changing the conformation from the native state. A17 and A19 of Loop II are also protected by binding to HIV-RT. In addition, the single base bridge A12 is protected upon binding.

Modification interference studies of the RT ligand B. The RNA ligand B was partially modified (with all of the chemicals mentioned above for structure determination). This modified population was bound with varying concentrations of the protein, and the bound species were assayed for the modified positions. From this, it can be determined where modification interferes with binding, and where there is no or little effect. A schematic diagram summarizing these modification interference results is shown in Figure 6. As shown, most of the significant interference with binding is clustered on the left hand side of the pseudoknot which contains the Stem 1 and Loop 2. This is also the part of the molecule that was highly conserved (primary sequence) in the collection of sequences isolated by SELEX and where substitution experiments produced the most drastic reduction in binding affinity to HIV-RT.

Substitution of 2'-methoxy for 2'-hydroxyl on riboses of ligand B. "RNA" molecules in which there is a 2'-methoxy bonded to the 2' carbon of the ribose instead of the normal hydroxyl group are resistant to enzymatic and chemical degradation. In order to test how extensively 2'-methoxys can be substituted for 2'-OH's in RT ligands, four oligos were prepared as shown in Figure 7. Because fully substituted 2'-methoxy ligand binds poorly (ligand D), and because we had found that most of the modification interference sites were clustered at one end of the pseudoknot, subsequent attempts to substitute were confined to the non-specific 3' helix as shown in boxes B and C. Both of these ligands bind with high affinity to HIV-RT. Oligonucleotides were then prepared in which the allowed substitutions at the ribose of Stem 2 were all 2'-methoxy as in C of Figure 7 and at the remaining 14 positions mixed synthesis were done with 2'-methoxy and 2'-OH phosphoramidite reagents. These oligos were subjected to selection by HIV-RT followed by alkaline hydrolysis of selected RNAs and gel separation (2'-methoxys do not participate in alkaline hydrolysis as do 2'-hydroxyls). As judged by visual inspection of films (see Figure 8) and quantitative determination of relative intensities using an Ambis detection system (see Example below for method of comparison), the ligands selected by HIV-RT from the mixed incorporation populations showed significantly increased hydrolysis at positions C13 and G14 indicating interference by 2'-methoxys at these positions. In a related experiment where mixtures at all positions were analyzed in this way, G4, A5, C13 and G14 showed 2' 0-methyl interference.

The results of substitution experiments, quantitative boundary experiments and chemical probing experiments are highly informative about the nature of the pseudoknot inhibitor of HIV-RT and highlight crucial regions of contact on this RNA. These results are provided on a nucleotide by nucleotide basis below.

U1 can be replaced with A with little loss in affinity but not by C or G. Although U1 probably makes transient base-pairing to G16, modification of U1-N3 with CMCT does not interfere with binding to HIV-RT. However, binding by HIV-RT protects the N3 of U1 perhaps by steric or electrostatic shielding of this position. Substitution with C which forms a more stable base-pair with G16 reduces affinity. Replacement of G16 with A which forms a stable U1-A16 pair abolishes specific affinity for HIV-RT and modification of G16-N1 strongly interferes with binding to HIV-RT. This modification of G16-N1 must prevent a crucial contact with the protein. Why G substitutions for U1 reduce affinity and A substitutions do not is not clear. Admittedly the G substitution is in a context in which the 5' end of the RNA is one nucleotide shorter, however synthetic RNAs in which U1 is the 5' terminal nucleotide bind with unchanged affinity from those in vitro transcripts with two extra Gs at the 5' end (Figure 7). Perhaps A at U1 replaces a potential U interaction with a similar or different interaction with HIV-RT a replacement that cannot be performed by C or G at this position.

The next base-pair of Stem 1 (C2-G15) cannot be replaced by a G-C base-pair without complete loss of specific affinity for HIV-RT. Modification of the base-pairing faces of either nucleotide strongly interferes with binding to HIV-RT and binding with HIV-RT protects from these modifications. Substitution of the next base-pair, C3-G14, with a G-C pair shows less drastic reduction of affinity, but modification is strongly interfering at this position. Substitution of a C-G pair for G4-C13 has no effect on binding, and substitution of the less stable A-U and U-A pairs allow some specific affinity. Substitution of the non-pairing A-C for these positions abolishes specific binding. This correlates with the appearance of C-G substitutions and one A-U substitution in the original SELEX phylogeny at this position, the non-reactivity of this base-pair in the native state, and the high degree of modification interference found for these bases.

The chemical modification data of Loop 2 corroborate well the phylogenetic conservation seen in the original SELEX experiments. Strong modification interference is seen at positions A17 and A19. Weak modification interference occurs at A20 which correlates with the finding of some Loop 2's of the original SELEX that are deleted at this relative position (although the chemical interference experiments conducted do not exhaustively test all potential contacts that a base may make with HIV-RT). A18 is unconserved in the original SELEX and modification at this position does not interfere, nor is this position protected from modification by binding to HIV-RT.

Taken together the above data suggest that the essential components of Stem 1 are a single-stranded 5' nucleotide (U or A) which may make sequence specific contact with the protein and a three base-pair helix (C2-G15, C3-G14, G4-C13) where there are sequence-specific interactions with the HIV-RT at the first two base-pairs and a preference for a strong base-pair (i.e. either C-G or G-C) at the third loop closing position of G4-C13. Loop 2 should be

more broadly described as GAXAA (16-20) due to the single-stranded character of G16 which probably interacts with HIV-RT in a sequence-specific manner, as likely do A17 and A19. Stem 2 varies considerably in the pattern and number of base-pairing nucleotides, but from 3' deletion experiments reported here one could hypothesize that a minimum of 3 base-pairs in Stem 2 are required for maximal affinity. Within the context of eight nucleotides connecting the two strands comprising the helix of Stem 1, at least 2 nucleotides are required in Loop 1 of the bound ligand.

The revised ligand description for BIV-RT obtained based on the methods of this invention is shown in Figure 11. The major differences between that shown in Figure 1 (which is based on the original and secondary SELEX consensuses) is the length of Stem 2, the more degenerate specification of the base-pair G4-C13, the size of Loop 1 (which is directly related to the size of Stem 2) and the single-stranded character of U1 and G16.

How can these differences be reconciled? Although not limited by theory, the SELEX strategy requires 5' and 3' fixed sequences for replication. In any RNA sequence, such additional sequences increase the potential for other conformations that compete with that of the high-affinity ligand. As a result, additional structural elements that do not directly contribute to affinity, such as a lengthened Stem 2, may be selected. Given that the first two base pairs of Stem 1 must be C-G because of sequence-specific contacts the most stable closing base-pair would be G4-C13 (Freier et al. (1986) Proc. Natl. Acad. Sci. USA. 83:9373) again selected to avoid conformational ambiguity. The sequence-specific selection of U1 and G16 may be coincidental to their ability to base-pair; in other nucleic acid ligand-protein complexes such as Klenow fragment/primer-template junction and tRNA/tRNA synthetase there is significant local denaturation of base-paired nucleotides (Freemont et al. (1988) Proc. Natl. Acad. Sci. USA 85:8924; Ronald et al. (1989) Science 246:1135) which may also occur in this case.

VI. Performance of Walking Experiment with HIV-RT Nucleic Acid Ligand to Identify Extended Nucleic Acid Ligands.

It had previously been found that fixed sequences (of 28 nucleotides) placed 5' to the pseudoknot consensus ligand reduced the affinity to HIV-RT and that sequences (of 31 nucleotides) added 3' to the ligand increased that affinity. A SELEX experiment was therefore performed in which a 30 nucleotide variable region was added 3' to the ligand B sequence to see if a consensus of higher affinity ligands against HIV-RT could be obtained. Individual isolates were cloned and sequenced after the sixteenth round. The sequences are listed in Figure 9 grouped in two motifs (SEQ ID NO:115-135). A schematic diagram of the secondary structure and primary sequence conservation of each motif is shown in Figure 10. The distance between the RNase H and polymerase catalytic domains of HIV-RT has recently been determined to be on the order of 18 base-pairs of an A-form RNA-DNA hybrid docked (by computer) in the pocket of a 3.5 Å resolution structure derived from X-ray crystallography (Kohlstaedt et al. (1992) Science 256:1783). The distance from the cluster of bases determined to be crucial to this interaction in the pseudoknot and the conserved bases in the extended ligand sequence is approximately 18 base-pairs as well. Accordingly, it is concluded that the pseudoknot interacts with the polymerase catalytic site -- in that the ligand has been shown to bind HIV-RT deleted for the RNAse H domain -- and that the evolved extension to the pseudoknot may interact with the RNAse H domain. In general the ligands tested from each of these motifs increase affinity of the ligand B sequence to BIV-RT by at least 10-fold.

VII. ELUCIDATION OF AN IMPROVED NUCLEIC ACID LIGAND FOR HIV-1 REV PROTEIN.

An example of the methods of the present invention are presented herein for the nucleic acid ligand for HIV-1 Rev protein.

PCT Patent Publication WO91/19813 describes the results obtained when SELEX was performed with the Rev target. Inspection of the nucleic acid sequences that were found to have a high affinity to Rev revealed a grouping of these sequences into three Motifs (I,II, and III). Ligands of Motif I seemed to be a composite of the individual motifs described by Motifs II and III, and in general bound with higher affinity to Rev. One of the Motif I ligand sequences (Rev ligand sequence 6a) bound with significantly higher affinity than all of the ligands that were cloned and sequenced. As shown in Figure 12, the 6a sequence is hypothesized to form a bulge between two helices with some base-pairing across this bulge.

Described herein are chemical modification experiments performed on ligand 6a designed to confirm the proposed secondary structure, find where binding of the Rev protein protects the ligand from chemical attack, and detect the nucleotides essential for Rev interaction. In addition, a secondary SELEX experiment was conducted with biased randomization of the 6a ligand sequence so as to more comprehensively describe a consensus for the highest affinity binding to the HIV-1 Rev protein.

Chemical modification of the Rev ligand. Chemical modification studies of the Rev ligand 6a were undertaken to determine its possible secondary structural elements, to find which modifications interfere with the binding of the ligand by Rev, to identify which positions are protected from modification upon protein binding, and to detect possible changes in ligand structure that occur upon binding.

The modifying chemicals include ethylnitrosourea (ENU) which modifies phosphates, dimethyl sulfate (DMS)

which modifies the base-pairing positions N3 of C and N1 of adenine, kethoxal which modifies base-pairing positions N1 and N2 of guanine, carbodiimide (CMCT) which modifies base-paring position N3 of uracil and to a smaller extent the N1 position of guanine, and diethylpyrocarbonate (DEPC) which modifies the N7 position of adenine and to some extent also the N7 of guanine. ENU modification was assayed by modification-dependent hydrolysis of a labeled RNA chain, while all other modifying agents were used on an extended RNA ligand, with modified positions revealed by primer extension of an annealed oligonucleotide.

The chemical probing of the Rev ligand native structure is summarized in Figure 13. The computer predicted secondary structure (Zuker (1989) supra; Jaeger et al. (1989), Proc. Natl. Acad. Sci. USA 86:7706) and native modification data are in general agreement; the ligand is composed of three helical regions, one four-base hairpin loop, and three "bulge" regions (see Figure 13 for a definition of these structural "elements").

ENU modification of phosphates was unchanged for ligands under native and denaturing conditions, indicating no involvement of phosphate groups in the secondary or tertiary structure of the RNA. In general, all computer-predicted base-pairing regions are protected from modification. One exception is the slight modifications of N7 ($G^{10}$, $A^{11}$, $G^{12}$) in the central helix (normally a protected position in helices). These modifications are possibly a result of helical breathing; the absence of base-pairing face modifications in the central helix suggest that the N7 accessibility is due to small helical distortions rather than a complete, local unfolding of the RNA. The G19-U22 hairpin loop is fully modified, except for somewhat partial modification of G19.

The most interesting regions in the native structure are the three "bulge" regions, U8-U9, A13-A14-A15, and G26-A27. U8-U9 are fully modified by CMCT, possibly indicating base orientations into solvent. A13, A14, and A15 are all modified by DMS and DEPC with the strongest modifications occurring on the central A14. The bulge opposite to the A13-A15 region shows complete protection of G26 and very slight modification of A27 by DMS. One other investigation of Rev-binding RNAs (Bartel et al. (1991) Cell 67:529) has argued for the existence of A:A and A:G non canonical base pairing, corresponding in the present ligand to A13:A27 and A15:G26. These possibilities are not ruled out by this modification data, although the isosteric A:A base pair suggested by Bartel et al. would use the N1A positions for base-pairing and would thus be resistant to DMS treatment. Also, an A:G pair would likely use either a N1A or N7A for pairing, leaving the A resistant to DMS or DEPC.

Modification interference of Rev binding. The results of the modification interference studies is summarized in Figure 14 (quantitative data on individual modifying agents is presented in Figures 15 through 19). In general, phosphate and base modification binding interference is clustered into two regions of the RNA ligand. To a first approximation, these regions correspond to two separate motifs present in the SELEX experiments that preceded this present study. Phosphate modification interference is probably the most suggestive of actual sites for ligand-protein contacts, and constitutes an additional criterion for the grouping of the modification interference data into regions.

The first region is centered on U24-G25-G26, and includes interference due to phosphate, base-pairing face, and N7 modifications. These same three nucleotides, conserved in the wild-type RRE, were also found to be critical for Rev binding in a modification interference study using short RNAs containing the RRE IIB stem loop (Kjems et al. (1992) EMBO J. 11:1119). The second region centers around G10-A11-G12 with interference again from phosphate, base-pairing face, and N7 modifications. Additionally, there is a smaller "mini-region" encompassing the stretch C6-A7-U8, with phosphate and base-pairing face modifications interfering with binding.

Throughout the ligand, many base-pairing face modifications showed binding interference, most likely because of perturbations in the ligand's secondary structure. Two of the "bulge" bases, U9 and A14, did not exhibit modification interference, indicating that both have neither a role in specific base-pairing interactions/stacking nor in contacting the protein.

Chemical modification protection when RNA is bound to Rev. The "footprinting" chemical modification data is summarized in Figure 20. Four positions, U8, A13, A15, and A27, showed at least two-fold reduction in modification of base-pairing faces (and a like reduction in N7 modification for the A positions) while bound to Rev protein. The slight N7 modifications of G10-All-G12 under native conditions were not detected when the ligand was modified in the presence of Rev. G32, unmodified in chemical probing of the RNA native structure, shows strong modification of its base-pairing face and the N7 position when complexed with Rev. U31 and U33, 5' and 3' of G32, show slight CMCT modification when the ligand is bound to protein.

Secondary SELEX using biased randomization of template. A template was synthesized as shown in Figure 21 in which the Rev ligand 6a sequence was mixed with the other three nucleotides at each position in the ratio of 62.5 (for the 6a sequence) to 12.5 for each of the other three nucleotides. This biased template gave rise to RNAs with background affinity for Rev protein (Kd = $10^{-7}$). Six rounds of SELEX yielded the list of sequences shown in Figure 21. The frequency distribution of the nucleotides and base pairs found at each position as it differs from that expected from the input distribution during template synthesis is shown in Figures 22 and 23. A new consensus based on these data is shown in Figure 24. The most significant differences from the sequence of Rev ligand 6a are replacement of the relatively weak base pair A7-U31 with a G-C pair and allowed or prefered substitution of U9 with C, A14 with U, U22 with G. Absolutely conserved positions are at sites G10, A11, G12; A15, C16, A17; U24, G25; and C28, U29, C30. No

bases were found substituted for G26 and A25, although there was one and three deletions found at those positions respectively. Two labeled transcripts were synthesized, one with a simple ligand 6a-like sequence, and one with substitutions by the significant preferences found in Figure 24. These RNAs bound identically to Rev protein.

Most of the substitutions in the stem region increase its stability. There does not seem to be significant selection of stems of length longer than 5 base-pairs although this could be a selection for replicability (for ease of replication during the reverse transcription step of SELEX, for example). There is some scattered substitution of other nucleotides for U9 in the original SELEX reported in PCT Patent Publication WO 91/19813 published December 26, 1991, but this experiment shows prefered substitution with C. Deletions of A27 also appeared in that original SELEX. A surprising result is the appearance of C18-A pairings in place of C18-G23 at a high frequency.

The reason there may be preferences found in this experiment that do not improve measured binding affinity may lie in the differences in the binding reactions of SELEX and these binding assays. In SELEX a relatively concentrated pool of heterogeneous RNA sequences (flanked by the requisite fixed sequences) are bound to the protein. In binding assays low concentrations of homogeneous RNA sequence are bound. In SELEX there may be selection for more discriminating conformational certainty due to the increased probability of intermolecular and intramolecular contacts with other RNA sequences. In the therapeutic delivery of concentrated doses of RNA ligands and their modified homologs, these preferences found in secondary SELEXes may be relevant.

Nucleic Acid Ligands to the HIV-1 tat Protein.

The present invention applies the SELEX procedure to a specific target, the HIV-1 tat protein. In Example III below, the experimental parameters used to isolate and identify the nucleic acid ligand solution to the HIV-1 tat protein are described. Figure 26 lists the nucleic acids that were sequenced after 10 iterations of the SELEX process.

Figure 25 shows the naturally occurring TAR sequence that has been found to be a natural ligand to the tat protein. The specific site of interaction between the tat protein and the TAR sequence has been determined, and is also identified in Figure 25.

The sequences presented in Figure 26 are grouped into three "motifs". Each of these motifs represents a nucleic acid ligand solution to the HIV-1 tat protein. Regions of primary sequence conservation within each motif are boxed with dashed lines. Motifs I and II contain a common structure that places conserved sequences (those sequences found in all or most all of the nucleic acid sequences that make up the given motif) in a bulge flanked by helical elements. The primary sequence conservation -- which is mainly in the single stranded domains of each bulge -- are also similar between motifs I and II. The third motif (III) is characterized by a large loop. The three motifs are depicted schematically in Figure 27. There is no apparent similarity between the nucleic acid ligands identified herein and the TAR sequence given in Figure 25.

A boundary analysis determination was performed on one of the ligand sequences in motif III. The boundaries of recognition are indicated by a solid-lined box in Figure 26. The boundary determination was performed according to previously described techniques. See, Tuerk et al. (1990) J. Mol. Biol. 213:749; Tuerk & Gold (1990) Science 249:505.

In Figure 28, the binding affinities of sequences 7 (motif I), 24 (motif II), 29 (motif II), 31 (motif III) and the original candidate mixture are depicted. As can be seen, members from each of the nucleic acid ligand solution motifs have increased affinity to the tat protein relative to the candidate mixture of nucleic acids. Each of the ligands exhibits a significantly greater affinity to the tat protein relative to the TAR sequence.

In order to produce nucleic acids desirable for use as a pharmaceutical, it is preferred that the nucleic acid ligand 1) binds to the target in a manner capable of achieving the desired effect on the target; 2) be as small as possible to obtain the desired effect; 3) be as stable as possible; and 4) be a specific ligand to the chosen target. In most, if not all, situations it is preferred that the nucleic acid ligand have the highest possible affinity to the target.

This invention includes the specific nucleic acid ligands shown in Figure 26 and the nucleic acid ligand solutions as depicted schematically in Figure 27. The scope of the ligands covered by this invention extends to all ligands to the tat protein identified according to the SELEX procedure. More specifically, this invention includes nucleic acid sequences that are 1) substantially homologous to and that have substantially the same ability to bind the tat protein as the specific nucleic acid ligands shown in Figure 26 or that are 2) substantially homologous and that have substantially the same ability to bind the tat protein as the nucleic acid ligand solutions shown in Figure 27. By substantially homologous, it is meant, a degree of primary sequence homology in excess of 70%, most preferably in excess of 80%. Substantially the same ability to bind the tat protein means that the affinity is within two orders of magnitude of the affinity of the substantially homologous sequence described herein. It is well within the skill of those of ordinary skill in the art to determine whether a given sequencesubstantially homologous to those specifically described herein -- has substantially the same ability to bind the tat protein.

A review of motifs I, II and III, and the binding curves shown in Figure 28, show that sequences that have little or no primary sequence homology may still have substantially the same ability to bind the tat protein. If one assumes that each of these motifs of ligands binds the same binding site of the tat protein, it is clear that binding is controlled by

the secondary or tertiary structure of the nucleic acid ligand. Certain primary structures -- represented by motifs I, II and III herein -- are apparently able to assume structures that appear very similar to the binding site of the tat protein. For these reasons, the present application also includes nucleic acid ligands that have substantially the same structural form as the ligands presented herein and that have substantially the same ability to bind the tat protein as the nucleic acid ligands shown in Figure 26 or Figure 27. Wherein substantially the same structure includes all nucleic acid ligands having the common structural elements of motifs I, II and III that lead to the affinity to the tat protein.

This invention also includes the ligands as described above, wherein certain chemical modifications have been made in order to increase the in vivo stability of the ligand or to enhance or mediate the delivery of the ligand.

The nucleic acid ligands and nucleic acid ligand solutions to the HIV-1 tat protein described herein are useful as pharmaceuticals and as part of gene therapy treatments. According to methods known to those skilled in the art, the nucleic acid ligands may be introduced intracellularly into cells infected with the HIV virus, where the nucleic acid ligand will compete with the TAR sequence for the tat protein. As such, transcription of HIV genes can be prevented.

Nucleic Acid Ligands to Thrombin. This invention includes the specific nucleic acid ligands shown in Figure 29 (SEQ ID NO:137-155). The scope of the ligands covered by this invention extends to all ligands to thrombin identified according to the SELEX procedure. More specifically, this invention includes nucleic acid sequences that are substantially homologous to and that have substantially the same ability to bind thrombin as the specific nucleic acid ligands shown in Figure 29 (SEQ ID NO:137-155).

A review of the proposed structural formations shown in Figure 30 for the group I and group II ligands shows that sequences that have little or no primary sequence homology may still have substantially the same ability to bind thrombin. For these reasons, the present invention also includes RNA ligands that have substantially the same structure as the ligands presented herein and that have substantially the same ability to bind thrombin as the RNA ligands shown in Figure 30 (SEQ ID NO:156-159). "Substantially the same structure" includes all RNA ligands having the common structural elements of the sequences given in Figure 30 (SEQ ID NO:156-159).

This invention also includes the ligands as described above, wherein certain chemical modifications have been made in order to increase the in vivo stability of the ligand or to enhance or mediate the delivery of the ligand. Specifically included within the scope of this invention are RNA ligands of thrombin that contain 2'-$NH_2$ modifications of certain riboses of the RNA ligand.

The nucleic acid ligands and nucleic acid ligand solutions to thrombin described herein are useful as pharmaceuticals and as part of gene therapy treatments.

The concepts of vascular injury and thrombosis are important in the understanding of the pathogenesis of various vascular diseases, including the initiation and progression of atherosclerosis, the acute coronary syndromes, vein graft disease, and restenosis following coronary angioplasty.

The high-affinity thrombin binding RNA ligands of this invention may be expected to have various properties. These characteristics can be thought about within the context of the hirudin peptide inhibitors and the current understanding of thrombin structure and binding. Within this context and not being limited by theory, it is most likely that the RNA ligands are binding the highly basic anionic exosite. It is also likely that the RNA is not binding the catalytic site which has high specificity for the cationic arginine residue. One would expect the RNA ligands to behave in the same manner as the C-terminal Hirudin peptides. As such, they would not strongly inhibit small peptidyl substrates, but would inhibit fibrinogen-clotting, protein C activation, platelet activation, and endothelial cell activation. Given that within the anionic exosite the fibrinogen-clotting and TM-binding activities are separable, it is possible that different high-affinity RNA ligands may inhibit these activities differentially. Moreover, one may select for one activity over another in order to generate a more potent anticoagulant than procoagulant.

The SELEX process for identifying ligands to a target was performed using human thrombin as the target, and a candidate mixture containing 76 nucleotide RNAs with a 30 nucleotide region of randomized sequences (Example IV). Following twelve rounds of SELEX, a number of the selected ligands were sequenced, to reveal the existence of two groups of sequences that had common elements of primary sequence.

A dramatic shift in binding of the RNA population was observed after 12 rounds of SELEX, when compared to the bulk 30N RNA. Sequencing of bulk RNA after 12 rounds also showed a non-random sequence profile. The RNA was reverse transcribed, amplified, cloned and the sequences of 28 individual molecules were determined (Figure 29). Based on primary sequence homology, 22 of the RNAs were grouped as class I and 6 RNAs were grouped as class II. Of the 22 sequences in class I, 16 (8 of which were identical) contained an identical sequence motif GGAUCGAAG$(N)_2$AGUAGGC (SEQ ID NO:9), whereas the remaining 6 contained 1 or 2 nucleotide changes in the defined region or some variation in N=2 to N=5. This conserved motif varied in its position within the 30N region. In class II, 3 of the 6 RNAs were identical and all of them contained the conserved motif GCGGCUUUGGGCGCCGUGCUU (SEQ ID NO:10), beginning at the 3rd nucleotide from the end of the 5' fixed region.

Three sequence variant RNA ligands from class I (6, 16, and 18) and one (27) from class II, identified by the order they were sequenced, were used for individual binding analysis. Class I RNAs were exemplified by clone 16 with a kD of approximately 30 $^{n}$M and the kD for the class II RNA clone 27 was approximately 60 $n_M$.

In order to identify the minimal sequence requirements for specific high affinity binding of the 76 nucleotide RNA which includes the variable 30N region flanked by 5' and 3' fixed sequence, 5' and 3' boundary experiments were performed. For 5' boundary experiments the RNAs were 3' end labeled and hydrolyzed to give a pool of RNAs with varying 5' ends. For the 3' boundary experiments, the RNAs were 5' end-labeled and hydrolyzed to give a pool of RNAs with varying 3' ends. Minimal RNA sequence requirements were determined following RNA protein binding to nitrocellulose filters and identification of labeled RNA by gel electrophoresis.

3' boundary experiments gave the boundaries for each of the 4 sequences shown in Figure 30A (SEQ ID NO:156-159). These boundaries were consistent at all protein concentrations. 5' boundary experiments gave the boundaries shown in Figure 31 plus or minus 1 nucleotide, except for RNA 16 which gave a greater boundary with lower protein concentrations. Based on these boundary experiments, possible secondary structures of the thrombin ligands are shown in Figure 30B.

RNAs corresponding to the smallest and largest hairpin of class I clone 16 (24 and 39 nucleotides) and the hairpin of class II clone 27 (33 nucleotides) were synthesized or transcribed for binding analysis (see Figure 30B). Results show that the RNA 27 hairpin binds with affinity (kD of about 60 $^{n}$M) equal to that of the entire 72 nucleotide transcript with fixed and variable region (compare RNA 27 in Fig. 31A with RNA 33R in Fig. 31C). The kDs for class I clone 16 RNA hairpins on the other hand increased an order of magnitude from 30 $^{n}$M to 200 $^{n}$M.

Modifications in the $2NH_2$-ribose of pyrimidine residues of RNA molecules has been shown to increase stability of RNA (resistant to degradation by RNase) in serum by at least 1000 fold. Binding experiments with the $2NH_2$-CTP/UTP modified RNAs of class I and class II showed a significant drop in binding when compared to the unmodified RNA (Figure 32). Binding by the bulk 30N RNA, however, showed a slight increase in affinity when it was modified.

A ssDNA molecule with a 15 nucleotide consensus 5'-GGTTGGTGTGGTTGG-3' (G15D) (SEQ ID NO:1) has been shown to bind human thrombin and inhibit fibrin-clot formation in vitro (Bock et al. (1992) supra). The results of competition experiments for binding thrombin between G15D and the RNA hairpin ligands of this invention are shown in Figure 33. In the first of these experiments A), $^{32}$P-labeled G15D used as the tracer with increasing concentrations of unlabeled RNA or unlabeled G15D. As expected, when the G15D was used to compete for its own binding, binding of labeled DNA was reduced to 50% at equimolar concentrations (1 $\mu$M) of labeled and unlabeled competitor DNA. Both the class I clone 16 synthetic RNAs 24 and 39, and the class II clone 27 synthetic RNA 33 were able to compete for binding of G15D at this concentration. In B) the higher affinity class II hairpin RNA 33 (kD = 60 $^{n}$M) was $^{32}$P-labelled and used as the tracer with increasing concentrations of unlabelled RNA or unlabelled G15D DNA (kD = 200 $^{n}$M). In these experiments, the G15D was able to compete effectively with RNA 33 at higher concentrations than the RNA 33 competes itself (shift of binding to the right), which is what is expected when competing with a ligand with 3-4 fold higher affinity. The class II hairpin RNA 33 (kD = 60 $^{n}$M) was competed only weakly by the class I hairpin RNA 24 (kD = 200 $^{n}$M), suggesting that while there may be some overlap, the RNAs of these two classes bind with high affinity to different yet adjacent or overlapping sites. Because both of these RNAs can compete for G15D binding, this DNA 15mer probably binds in the region of overlap between the class I and class II hairpins.

Cleavage of Chromogenic Substrate S2238. The ability of thrombin to cleave the peptidyl chromogenic substrate S2238 (H-D-Phe-Pip-Arg-pNitroaniline) (H-D-Phe-Pip-Arg-pNA) (Kabi Pharmacia) was measured in the presence and absence of the RNA ligands of this invention. There was no inhibitory effect of RNA on this cleavage reaction at $10^{-8}$ M thrombin and $10^{-8}$ M RNA, $10^{-9}$ M thrombin and $10^{-8}$ M RNA or at $10^{-8}$ M thrombin and $10^{-7}$ M RNA (Figure 34A). These results suggest that the RNA ligands do not bind in the catalytic site of the enzyme.

Cleavage of Fibrinogen to Fibrin and Clot

Formation. The ability of thrombin to catalyze clot formation by cleavage of fibrinogen to fibrin was measured in the presence and absence of RNA. When RNA was present at a concentration equal to the Kd (30 $^{n}$M for class I RNAs and 60 $^{n}$M for class II RNAs), which was in 5 to 10-fold excess of thrombin, clotting time was increased by 1.5-fold (Figure 34B).

Specificity of thrombin binding. Representative ligands from class I and class II showed that these ligands had low affinity for ATIII at concentrations as high as 1 $\mu$M (Figure 35A). These ligands showed reduced affinity when compared with the bulk 30N3 RNA suggesting that there has been selection against non-specific binding. This is of particula-r importance because ATIII is an abundant plasma protein with high affinity for heparin, a polyanionic macromolecule. These results show that the evolution of a discreet structure present in the class I and class II RNAs is specific for thrombin binding and, despite its polyanionic composition, does not bind to a high affinity heparin binding protein. It is also important to note that these thrombin specific RNA ligands have no affinity for prothrombin (Figure 35B), the inactive biochemical precursor to active thrombin, which circulates at high levels in the plasma (= 1 $\mu$M).

Nucleic Acid Ligands to Basic Fibroblast Growth Factor (bFGF). The present invention applies the SELEX procedure to a specific target, bFGF. In the Example section below, the experimental parameters used to isolate and identify the nucleic acid ligand solutions to bFGF are described.

This invention includes the specific nucleic acid ligands shown in Tables II-IV. The scope of the ligands covered by this invention extends to all ligands to bFGF identified according to the SELEX procedure. More specifically, this invention includes nucleic acid sequences that are substantially homologous to and that have substantially the same ability to bind bFGF as the specific nucleic acid ligands shown in Tables II-IV.

A review of the proposed structural formations shown in Figure 41 for the family 1 and 2 ligands shows that sequences that have little or no primary sequence homology may still have substantially the same ability to bind bFGF. The present invention also includes RNA ligands that have substantially the same structure as the ligands presented herein and that have substantially the same ability to bind bFGF as the RNA ligands shown in Tables II and III. "Substantially the same structure" includes all RNA ligands having the common structural elements of the sequences given in Tables II and III (SEQ ID NO:27-67).

This invention also includes the ligands described above, wherein certain chemical modifications have been made in order to increase the in vivo stability of the ligand, enhance or mediate the delivery of the ligand, or reduce the clearance rate from the body. Specifically included within the scope of this invention are RNA ligands of bFGF that contain 2'-$NH_2$ modifications of certain riboses of the RNA ligand.

The nucleic acid ligands and nucleic acid ligand solutions to bFGF described herein are useful as pharmaceuticals, and as part of gene therapy treatments. Further, the nucleic acid ligands to bFGF described herein may be used beneficially for diagnostic purposes.

The high-affinity nucleic acid ligands of the present invention may also have various properties, including the ability to inhibit the biological activity of bFGF. Representative ligands from sequence family 1 and 2 were found to inhibit binding of bFGF to both low- and high-affinity cell-surface receptors. These nucleic acid ligands may be useful as specific and potent neutralizers of bFGF activity in vivo.

EXAMPLE I: ELUCIDATION OF IMPROVED NUCLEIC ACID LIGAND SOLUTION FOR HIV-RT

RNA synthesis. In vitro transcription with oligonucleotide templates was conducted as described by Milligan et al. (1987) supra. All synthetic nucleic acids were made on an Applied Biosystems model 394-08 DNA/RNA synthesizer using standard protocols. Deoxyribonucleotide phosphoramidites and DNA synthesis solvents and reagents were purchased from Applied Biosystems. Ribonucleotide and 2'-methoxy-ribonucleotide phosphoramidites were purchased from Glen Research Corporation. For mixed base positions, 0.1 M phosphoramidite solutions were mixed by volume to the proportions indicated. Base deprotection was carried out at 55°C for 6 hours in 3:1 ammonium hydroxide:ethanol. t-butyl-dimethylsilyl protecting groups were removed from the 2'-OH groups of synthetic RNAs by overnight treatment in tetrabutylammonium fluoride. The deprotected RNAs were then phenol extracted, ethanol precipitated and purified by gel electrophoresis.

Affinity assays with labeled RNA and HIV-RT. Model RNAs for refinement of the 5' and 3' boundaries and for determination of the effect of substitutions were labeled during transcription with T7 RNA polymerase as described in Tuerk & Gold (1990) supra except that a-$^{32}$P-ATP was used, in reactions of 0.5 mM C,G, and UTP with 0.05 mM ATP. Synthetic oligonucleotides and phosphatased transcripts (as in Tuerk & Gold (1990) supra were kinased as described in Gauss et al. (1987) Mol. Gen. Genet. 206:24. All RNA-protein binding reactions were done in a "binding buffer" of 200 mM KOAc, 50 mM Tris-HCl pH 7.7, 10 mM dithiothreitol with exceptions noted for chemical protection experiments below. RNA and protein dilutions were mixed and stored on ice for 30 minutes then transferred to 37°C for 5 minutes. In binding assays the reaction volume was 60 ul of which 50 ul was assayed. Each reaction was suctioned through a pre-wet (with binding buffer) nitrocellulose filter and rinsed with 3 mls of binding buffer after which it was dried and counted for assays or subjected to elution and assayed for chemical modification. In comparisons of binding affinity, results were plotted and the protein concentration at which half-maximal binding occurred (the approximate Kd in conditions of protein excess) was determined graphically.

Selection of modified RNAs by HIV-RT. Binding reactions were as above except that rather than to vary the amount of HIV-RT added to a reaction, the volume of reaction was increased in order to lower concentration. RNAs that were modified under denaturing conditions were selected at concentrations of 20, 4 and 0.8 nanomolar HIV-RT (in volumes of 1, 5 and 25 mls of binding buffer.) The amount of RNA added to each reaction was equivalent for each experiment (approximately 1-5 picomoles). RNA was eluted from filters as described in Tuerk & Gold (1990) supra) and assayed for modified positions. In each experiment a control was included in which unselected RNA was spotted on a filter, eluted and assayed for modified positions in parallel with the selected RNAs. Determinations of variation in chemical modification for selected versus unselected RNAs were made by visual inspection of exposed films of electrophoresed assay products with the following exceptions. The extent of modification interference by ENU was determined by densitometric scanning of films using an LKB laser densitomer. An index of modification interference (M.I.) at each position was calculated as follows:

M.I. = (O.D.unselected/ O.D.unselected A20)/(O.D.selected/O.D.selected A20)

where the value at each position assayed for selected modified RNA (O.D.selected) is divided by that value for position A20 (O.D.selected A20) and divided into likewise normalized values for the unselected lane. All values of M.I. greater than 2.0 are reported as interfering and greater than 4.0 as strongly interfering. In determination of the effects of mixed substitution of 2'-methoxys for 2' hydroxyls (on the ribose at each nucleotide position) gels of electrophoresed hydrolysis products were counted on an Ambis detection system directly. The counts associated with each band within a lane were normalized as shown above but for position A17. In addition, determinations were done by laser densitometry as described below.

Chemical modification of RNA. A useful review of the types of chemical modifications of RNA and their specificities and methods of assay was done by Ehresmann et al. (1987) supra. Modification of RNA under native conditions was done at 200 mM KOAc, 50 mM Tris-HCl pH 7.7 at 37°C with ethylnitrosourea (ENU) (1/5 dilution v/v of room temperature ENU-saturated ethanol) for 1-3 hours, dimethyl sulfate (DMS) (1/750-fold dilution v/v) for eight minutes, kethoxal (0.5 mg/ml) for eight minutes, carbodiimide (CMCT) (8 mg/ml) for 20 minutes, and diethyl pyrocarbonate (DEPC) (1/10 dilution v/v for native conditions or 1/100 dilution for denaturing conditions) for 45 minutes, and under the same conditions bound to HIV-RT with the addition of 1 mM DTT. The concentrations of modifying chemical reagent were identical for denaturing conditions (except where noted for DEPC); those conditions were 7M urea, 50 mM Tris-HCl pH 7.7, 1 mM EDTA at 90°C for 1-5 minutes except during modification with ENU which was done in the absence of 7M urea.

Assay of chemical modification. Positions of chemical modification were assayed by reverse transcription for DMS, kethoxal and CMCT on the lengthened ligand B RNA, 5'-GGUCCGAAGUGCAACGGGAAAAUGCACUAUGAAAGAAU-UUUAUAUCUCUAU UGAAAC-3' (SEQ ID NO:11) (the ligand B sequence is underlined), to which is annealed the oligonucleotide primer 5'-CCGGATCCGTTTCAATAGAG-ATATAAAATTC-3' (SEQ ID NO:12); reverse transcription products (obtained as in Gauss et al., 1987 supra) were separated by electrophoresis on 10% polyacrylamide gels. Positions of ENU and DEPC modification were assayed as in Vlassov et al. (1980) FEBS Lett. 120:12 and Peattie and Gilbert (1980) Proc. Natl. Acad. Sci. USA 77:4679, respectively (separated by electrophoresis on 20% polyacrylamide gels). Assay of 2'-methoxy ribose versus ribose at various positions was assayed by alkaline hydrolysis for 45 minutes at 90°C in 50 mM sodium carbonate pH 9.0.

Modification of RNA in the presence of HIV-RT. Conditions were as for modification of native RNA. Concentrations of HIV-RT were approximately 10-fold excess over RNA concentration. In general protein concentrations ranged from 50 nM to 1 uM.

SELEX isolation of accessory contacts with HIV-RT. The starting RNA was transcribed from PCRd templates synthesized from the following oligonucleotides:

5'-GGGCAAGCTTTAATACGACTCACTATAGGTCCGAAGTGCAACGGGAAAATG-CACT-3' (5' primer) (SEQ ID NO:13),

5'-GTTTCAATAGAGATATAAAATTCTTTCATAG-3' (3' primer) (SEQ ID NO:14),

5'-GTTTCAATAGAGATATAAAATTCTTTCATAG-[30N]AGTGCATTTTCCCGTTGC-ACTTCGGACC-3' (variable template)(SEQ ID NO:15).

SELEX was performed as described previously with HIV-RT with the following exceptions. The concentration of HIV-RT in the binding reaction of the first SELEX round was 13 nanomolar, RNA at 10 micromolar, in 4 mls of binding buffer, in the rounds 2 through 9 selection was done with 2.6 nanomolar HIV-RT, 1.8 micromolar RNA in 20 mls of buffer, in rounds 10-14 we used 1 nanomolar HIV-RT, 0.7 micromolar RNA in 50 mls, and for rounds 15 and 16 we used 0.5 nanomolar HIV-RT, 0.7 micromolar RNA in 50 mls of binding buffer.

REFERENCES TO EXAMPLE I:

Ehresman, C., Baudin, F., Mougel, M., Romby, P., Ebel, J-P. Ehresman, B. (1987) Probing the structure of RNAs in solution. Nuc. Acids. Res. 15:9109-9128.
Freemont, P.S., Friedman, J.M., Beese, M.R., Sanderson, M.R. and Steitz, T.A. (1988) Proc. Natl. Acad. Sci. USA 85:8924.
Freier, S.M., Kierzed, R., Jaeger, J.A., Suigimoto, N., Caruthers, M.H., Neilson, T., and Turner, D.H. (1986) Proc. Natl. Acad. Sci. USA 83:9373-9377.
Gauss, P., Gayle, M., Winter, R.B., Gold, L. (1987) Mol. Gen. Genet. 206:24.
Kohlstaedt, L.A., Wang, J., Friedman, J.M., Rice, P.A. and Steitz, T.A. (1992) Crystal structure at 3.5 Å resolution of HIV-1 reverse transcriptase complexed with an inhibitor. Science 256:1783-1790.
Milligan, J.F., Groebe, D.R., Witherell, G.W. and Uhlenbeck, O.C. (1987) Oligoribonucleotide synthesis using T7 RNA polymerase and synthetic DNA templates. Nucleic Acids Res. 15:8783-8798.
Moazed, D., Stern, S. and Noller, H. (1986) Rapid chemical probing of conformation in 16S ribosomal RNA and 30S ribosomal subunits using primer extension. J. Mol. Biol. 187:399-416.
Peattie, D. and Gilbert, W. (1980) Chemical probes for higher order structure in RNA. Proc. Natl. Acad. Sci. USA 77:4679-4682.
Peattie, D. and Herr, W. (1981) Chemical probing of the tRNA-ribosome complex. Proc. Natl. Acad. Sci. USA 78:2273-2277.
Roald, M.A., Perona, J., Söll, D. and Steitz, T.A. (1989) Science 246:1135.
Tuerk, C., Eddy, S., Parma, D. and Gold, L. (1990) The translational operator of bacteriophage T4 DNA polymerase. J. Mol. Biol. 213:749.
Tuerk, C. and Gold, L. (1990) Systematic evolution of ligands by exponential enrichment: RNA ligands to bacteriophage T4 DNA polymerase. Science 249:505-510.
Tuerk, C., MacDougal, S. and Gold, L. (1992a) RNA pseudoknots that inhibit HIV-1 reverse transcriptase. Proc. Natl. Acad. Sci. USA 89:6988-6992.
Vlassov, V., Giege, R. and Ebel, J.-P. (1980) The tertiary structure of yeast tRNAPhe in solution studied by phosphodiester bond modification with ethylnitrosourea. FEBS 120:12-16.

EXAMPLE II: ELUCIDATION OF IMPROVED NUCLEIC ACID LIGAND SOLUTIONS FOR HIV-1 REV PROTEIN

The Rev ligand sequence used for chemical modification is shown in Figure 12 (the numbering scheme shown will be used hereinafter). RNA for modification was obtained from T7 RNA polymerase transcription of synthetic oligonucleotide templates. ENU modification was carried out on the ligand sequence as shown in Figure 12. DMS, kethoxal, CMCT, and DEPC modifications were carried out on a extended ligand sequence, and analyzed by reverse transcription with the synthetic oligonucleotide primer shown in Figure 12.

Chemical Modification of RNA. Chemical modification techniques for nucleic acids are described in general in Ehresmann et al. (1987) supra. Modification of RNA under native conditions was performed in 200mM KOAc, 50mM Tris-HCl pH 7.7, 1 mM EDTA at 37°C. Modification under denaturing conditions was done in 7M urea, 50mM Tris-HCl pH 7.7 at 90°C. Concentration of modifying agents and incubation times are as follows: ethylnitrosourea (ENU)- 1/5 dilution v/v of ethanol saturated with ENU, native 1-3 hours, denaturing 5 minutes; dimethyl sulfate (DMS)- 1/750-fold dilution v/v, native 8 minutes, denaturing 1 minute; kethoxal- 0.5 mg/ml, native 5 minutes, denaturing 2 minutes; carbodiimide (CMCT)- 10 mg/ml, native 30 minutes, denaturing 3 minutes; diethyl pyrocarbonate (DEPC)- 1/10 dilution v/v, native 10 minutes, denaturing 1 minute.

Modification interference of Rev binding. RNAs chemically modified under denaturing conditions were selected for Rev binding through filter partitioning. Selections were carried out at Rev concentrations of 30, 6, and 1.2 nanomolar (in respective volumes of 1, 5, and 25 mls of binding buffer; 200 mM KOAc, 50 mM Tris-HCl pH 7.7, and 10 mM dithiothreitol). Approximately 3 picomoles of modified RNA were added to each protein solution, mixed and stored on ice for 15 minutes, and then transferred to 37°C for 10 minutes. Binding solutions were passed through pre-wet nitrocellulose filters, and rinsed with 5 mls of binding buffer. RNA was eluted from the filters as described in Tuerk & Gold (1990) supra and assayed for modified positions that remained. Modified RNA was also spotted on filters and eluted to check for uniform recovery of modified RNA.

The extent of modification interference was determined by densitometric scanning of autoradiographs using LKB (ENU) and Molecular Dynamics (DMS, kethoxal, CMCT, and DEPC) laser densitometers. Values for modified phosphates and bases were normalized to a chosen modified position for both selected and unselected lanes; the values for the modified positions in the selected lane were then divided by the corresponding positions in the unselected lane

(for specific normalizing positions see Figures 15-19). Values above 4.0 for modified bases and phosphates are designated as strongly interfering, and values above 2.0 are termed slightly interfering.

Modification of RNA in the presence of Rev. "Footprinting" of the Rev ligand, modification of the RNA ligand in the presence of Rev protein, was performed in 200mM KOAc, 50mM Tris-Cl pH 7.7, 1mM DTT, and 5mM MgCl. Concentration of protein was 500 nanomolar, and approximately in 3-fold molar excess over RNA concentration. Modification with protein present was attempted with all modifying agents listed above except ethylnitrosourea (ENU).

Assay of chemically modified RNA. Positions of ENU modification were detected as in Vlassov et al. (1980) supra and separated by electrophoresis on 20% denaturing acrylamide gels. DMS, kethoxal, CMCT, and DEPC were assayed by reverse transcription of the extended Rev ligand with a radiolabelled oligonucleotide primer (Figure 12) and separated by electrophoresis on 8% denaturing acrylamide gels.

SELEX with biased randomization. The templates for in vitro transcription were prepared by PCR from the following oligonucleotides:

5'-CCCGGATCCTCTTTACCTCTGTGTGagatascagagtccacaacgtg ttctcaatgacccGGTCGGAAGGCCATCAATAGTCCC-3' (template oligo) (SEQ ID NO:16),

5'-CCGAAGCTTAATACGACTCACTATAGGGACTATTGATGGGCCTTCCGACC-3' (5' primer) (SEQ ID NO:17),

5'-CCCGGATCCTCTTTACCTCTGTGTG-3' (3' primer) (SEQ ID NO:18)

where the small case letters in the template oligo indicates that at each position that a mixture of reagents were used in synthesis by an amount of 62.5% of the small case letter, and 12.5% each of the other three nucleotides.

SELEX was conducted as described previously with the following exceptions. The concentration of HIV-1 Rev protein in the binding reactions of the first and second rounds was 7.2 nanomolar and the RNA 4 micromolar in a volume of 10 mls (of 200 mM potassium acetage, 50 mM Tris-HCl pH 7.7, 10 mM DTT). For rounds three through six the concentration of Rev protein was 1 nanomolar and the RNA 1 micromolar in 40 mls volume. HIV-1 Rev protein was purchased from American Biotechnologies, Inc.

EXAMPLE III: NUCLEIC ACID LIGANDS TO THE HIV-1 tat PROTEIN.

SELEX on HIV-1 tat Protein. tat protein was purchased from American Bio-Technologies, Inc. Templates for in vitro transcription were produced by PCR using the following oligonucleotides:

5'-CCGAAGCTTAATACGACTCACTATAGGGAGCTCAGAATAAACGCTCAA-3' (5' primer) (SEQ ID NO:18),

5'-GCCGGATCCGGGCCTCATGTCGAA-[40n]-TTGAGCGTTTATTCTGAGC TCCC-3' (variable template) (SEQ ID NO:19),

5'-GCCGGATCCGGGCCTCATGTCGAA-3' (3' primer)(SEQ ID NO:20),

SELEX rounds were conducted as described in Tuerk et al. (1992a) supra, and in the SELEX Applications, under the

following conditions: Binding reactions were done with 13 nanomolar tat protein and 1.3 micromolar RNA in a volume of 2 mls for rounds 1 and 2, and 6.5 nanomolar tat protein and 0.65 micromolar RNA in 4 mls for rounds 3-10.

RNA synthesis. In vitro transcription with oligonucleotide templates was conducted as described by Milligan et al. (1987) supra. All synthetic nucleic acids were made on the Applied Biosystems model 394-08 DNA/RNA synthesizer using standard protocols. Deoxyribonucleotide phosphoramidites and DNA synthesis solvents and reagents were purchased from Applied Biosystems.

Affinity assays with labeled RNA and RIV-1 tat protein. Model RNAs for refinement of the 5' and 3' boundaries and for determination of the effect of substitutions were labeled during transcription with T7 RNA polymerase as described in the SELEX Applications, except that $\alpha$-$^{32}$P-ATP was used, in reactions of 0.5 mM C, G, and UTP with 0.05 mM ATP. All RNA-protein binding reactions were done in a "binding buffer" of 200 mM KOAc, 50 mM Tris-Hcl pH 7.7, 10 mM dithiothreitol. RNA and protein dilutions were mixed and stored on ice for 30 minutes then transferred to 37°C for 5 minutes. In binding assays the reaction volume was 60 ul of which 50 ul was assayed. Each reaction was suctioned through a pre-wet (with binding buffer) nitrocellulose filter and rinsed with 3 mls of binding buffer after which it was dried and counted for assays or subjected to elution and assayed for chemical modification. In comparisons of binding affinity, results were plotted and the protein concentration at which half-maximal binding occurred (the approximate Kd in conditions of protein excess) was determined graphically. The results of the binding assays are given in Figure 27.

EXAMPLE IV: NUCLEIC ACID LIGANDS TO THROMBIN

High affinity RNA ligands for thrombin were isolated by SELEX. Random RNA molecules used for the initial candidate mixture were generated by in vitro transcription from a 102 nucleotide double-stranded DNA template containing a random cassette 30 nucleotides (30N) long. A population of $10^{13}$ 30N DNA templates were created by PCR, using a 5' primer containing the T7 promoter for in vitro transcription, and restriction sites in both the 5' and 3' primers for cloning.

The RNA concentration for each round of SELEX was approximately 2-4 X $10^{-7}$ M and concentrations of thrombin (Sigma, 1000 units) went from 1.0 X $10^{-6}$ in the 1st round to 4.8 X $10^{-7}$ in rounds 2 and 3 and 2.4 X $10^{-7}$ in rounds 4-12. The binding buffer for the RNA and protein was 100 mM NaCl, 50 mM Tris/Cl, pH7.7, 1 mM DTT, and 1 mM $MgCl_2$. Binding was for 5 minutes at 37°C in a total volume of 100$\mu$l in rounds 1-7 and 200$\mu$l in rounds 8-12. Each binding reaction was filtered through a pre-wetted (with 50 mM Tris/Cl, pH7.7) nitrocellulose filter (2.5 cm Millipore, 0.45 $\mu$M) in a Millipore filter binding apparatus, and immediately rinsed with 5 ml of the same buffer. The RNA was eluted from the filters in 400 $\mu$l phenol (equilibrated with 0.1 M NaoAc pH5.2), 200 $\mu$l freshly prepared 7 M urea as described (Tuerk et al. (1990) supra. The RNA was precipitated with 20 $\mu$g tRNA, and was used as a template for cDNA synthesis, followed by PCR and in vitro transcription to prepare RNA for the subsequent round. The RNA was radio-labeled with $^{32}$P-ATP in rounds 1-8 so that binding could be monitored. In order to expedite the time for each round of SELEX, the RNA was not labeled for rounds 9-12. RNA was prefiltered through nitrocellulose filters (1.3 cm Millipore, 0.45 $\mu$M) before the 3rd, 4th, 5th, 8th, 11th, and 12th rounds to eliminate selection for any nonspecific nitrocellulose binding.

Binding curves were performed after the 5th, 8th, and 12th rounds to estimate changes in kD of the bulk RNA. These experiments were done in protein excess at concentrations from 1.2 X $10^{-5}$ to 2.4 X $10^{-9}$ at a final RNA concentration of 2 X $10^{-9}$ M. The RNA for these binding curves was labeled to high specific activity with $^{32}$P-ATP or $^{32}$P-UTP. Binding to nitrocellulose filters was as described for the rounds of SELEX, except that the filter bound RNA was dried and counted directly on the filters.

RNA Sequencing. Following the 12th round of SELEX, the RNA was sequenced with reverse transcriptase (AMV, Life Sciences, Inc.) using the $^{32}$P 5' end-labeled 3' complementary PCR primer.

Cloning and Sequencing individual RNAs. RNA from the 12th round was reverse transcribed to DNA and amplified by PCR. Digestion at restriction enzyme sites in the 5' and 3' fixed regions were used to remove the 30N region which was subsequently ligated into the complementary sites in the E. coli cloning vector pUC18. Ligated plasmid DNA was transformed into JM103 cells and screened by blue/white colony formation. Colonies containing unique sequences were grown up and miniprep DNA was prepared. Double-stranded plasmid DNA was used for dideoxy sequencing with the Sequenase kit version 2.0 and $^{35}$S-dATP (Amersham).

End-labeling RNA. For end-labeling, RNA transcribed with T7 polymerase was gel purified by UV shadowing. RNA was 5' end-labeled by dephosphorylating the 5' end with alkaline phosphatase 1 unit, for 30 minutes at 37°C. Alkaline phosphatase activity was destroyed by phenol:chloroform extraction. RNA was subsequently end-labeled with $\gamma^{32}$P-ATP in a reaction with polynucleotide kinase for 30 minutes at 37°C.

RNA was 3' end-labeled with (5'-$^{32}$P)pCp and RNA ligase, for 30 minutes at 37°C. 5' and 3' end-labeled RNAs were gel band purified on an 8%, 8 M urea, polyacrylamide gel.

Determination of 5' and 3' boundaries. 2 pmole RNA 3' or 5' end-labeled for the 5' or 3' boundary experiments, respectively were hydrolyzed in 50 mM $Na_2CO_3$ (pH 9.0) and 1 mM EDTA in a 10 $\mu$l reaction for 10 minutes at 90°C. The reaction was stopped by adding 1/5 volume 3 M NaOAc (pH5.2), and freezing at -20°C. Binding reactions were

done at 3 protein concentrations, 40 nM, 10 nM and 2.5 nM, in 3 volumes (100 $\mu$l, 400 $\mu$l, and 1600 $\mu$l, such that the amount of protein was kept constant) containing 1X binding buffer and 2 pmoles RNA. Reactions were incubated for 10 minutes at 37°C, filtered through a pre-wet nitrocellulose membrane, and rinsed with 5 ml wash buffer. The RNA was eluted from the filters by dicing the filter and shaking it in 200 $\mu$l 7 M urea and 400 $\mu$l phenol (pH 8.0) for 15 minutes at 20°C. After adding 200 $\mu$l $H_2O$, the phases were separated and the aqueous phase extracted once with chloroform. The RNA was precipitated with 1/5 volume 3 M NaOAc, 20 $\mu$g carrier tRNA, and 2.5 volumes ethanol. The pellet was washed once with 70% ethanol, dried, and resuspended in 5 $\mu$l $H_2O$ and 5 $\mu$l formamide loading dye. The remainder of the alkaline hydrolysis reaction was diluted 1:10 and an equal volume of loading dye was added. To locate where on the sequence ladder the boundary existed, an RNase T1 digest of the ligand was electrophoresed alongside the alkaline hydrolysis reaction and binding reactions. The digest was done in a 10 $\mu$l reaction containing 500 fmoles end-labeled RNA and 10 units RNase T1 in 7 M urea, 20 mM Na-citrate (pH 5.0) and 1 mM EDTA. The RNA was incubated 10 minutes at 50°C without enzyme and then another 10 minutes after adding enzyme. The reaction was slowed by adding 10 $\mu$l loading dyes and incubating at 4°C. Immediately after digestion, 5 $\mu$l of each of the digest, hydrolysis, and 3 binding reactions were electrophoresed on a 12% sequencing gel.

In vitro transcription of RNA 2-$NH_2$ ribose derivatives of UTP and CTP. RNA was transcribed directly from the pUC18 plasmid miniprep dsDNA template with T7 RNA polymerase in a reaction containing ATP, GTP, 2$NH_2$-UTP and 2$NH_2$-CTP. For $^{32}$P-labeled RNA, $^{32}$P-ATP was included in the reaction. Unmodified RNAs were transcribed in a mixture containing ATP, GTP, UTP, and CTP.

Synthesis of RNA. RNA molecules corresponding to lower limits of nucleotide sequence required for high affinity binding to thrombin as determined by the boundary experiments (Figure 30B) were synthesized on an Applied Biosystems 394 DNA/RNA Synthesizer. These RNA molecules include the class I clone 16 hairpin structures of 24 nucleotides (24R) and 39 nucleotides (39R) and the class II clone 27 hairpin of 33 nucleotides (33R).

Binding of individual RNA molecules. Four DNA plasmids with unique 30N sequences were chosen for in vitro transcription. $^{32}$P-labelled RNA was transcribed with conventional nucleotides as well as with the 2-$NH_2$ derivatives of CTP and UTP. Binding curves with these individual RNAs could be established using the binding buffer and thrombin (1000 units, Sigma) concentrations from 1.0 x $10^{-5}$ to 1.0 x $10^{-10}$ M. Human $\alpha$ thrombin (Enzyme Research Laboratories, ERL) was also used to determine binding affinities of RNA at concentrations from 1.0 X $10^{-6}$ to 1.0 X $10^{-10}$ M.

Binding of the 5' end-labeled single stranded 15mer DNA 5'-GGTTGGTGTGGTTGG-3' (G15D) (SEQ ID NO:1) described by Bock et al. (1992) supra, was determined under the binding conditions described herein with ERL thrombin and compared to binding by the radiolabelled RNA hairpin structures described above.

Competition Experiments. To determine whether the RNA ligands described can compete for binding of the DNA 15mer G15D to thrombin, equimolar concentrations (1 $\mu$M) of thrombin and the 5' end labeled DNA 15mer G15D were incubated under filter binding conditions (kD of approximately 200 $^{n}$M) in the presence and absence of 'cold' unlabeled RNA or DNA ligand at varying concentrations from 10 nM to 1 uM. In the absence of competition, RNA binding was 30%. The protein was added last so competition for binding could occur. The RNA ligands tested for competition were the class I clone 16 synthetic RNAs 24mer (24R) and 39mer hairpins (39R) and the class II 27 synthetic RNA 33mer (33R). Results are expressed as the relative fraction of G15D bound (G15 with competitor/G15 without competitor) vs. the concentration of cold competitor.

To determine whether class I RNAs can compete for binding with class II RNAs and to confirm the competition with the G15D DNA, equimolar concentrations (300 $^{n}$M) of thrombin and the 5' end-labelled class II RNA 33 hairpin were incubated under filter binding conditions in the presence or absence of 'cold' unlabelled RNA 24 or DNA G15D at varying concentrations from 100 $^{n}$M to 32 $\mu$M. Results are expressed as the relative fraction of RNA 33 bound (RNA 33 with competitor/RNA 33 without competitor) versus the concentration of cold competitor (Figure 33).

Chromogenic assay for thrombin activity and inhibition by RNA ligands. The hydrolysis by thrombin of the chromogenic substrate S-2238 (H-D-Phe-Pip-Arg-pNitroaniline [H-D-Phe-Pip-Arg-pNA]) (Kabi Pharmacia) was measured photometrically at 405 nm due to the release of p-nitroaniline (pNA) from the substrate.

$$\text{H-D-Phe-Pip-Arg-pNA} + H_2O \xrightarrow{\text{Thrombin}} \text{H-D-Phe-Pip-Arg-OH} + \text{pNA}$$

Thrombin was added to a final concentration of $10^{-8}$ or $10^{-9}$ M to a reaction buffer (50 mM Na citrate, pH 6.5, 150 mM NaCl, 0.1% PEG), containing 250 $\mu$M S2238 substrate at 37°C. For inhibition assays, thrombin plus RNA (equimolar or at 10-fold excess) were preincubated 30 secs at 37°C before adding to the reaction mixture (Figure 34A).

Fibrinogen clotting. Thrombin was added for a final concentration of 2.5 nM to 400 $\mu$l incubation buffer (20 mM Tris-acetate, pH 7.4, 140 mM NaCl, 5 mM KCl, 1 mM $CaCl_2$, 1 mM $MgCl_2$) containing 0.25 mg/ml fibrinogen and 1 u/$\lambda$ RNAse inhibitor (RNAasin, Promega) with or without 30 nM RNA class I or 60 $^{n}$M RNA class II at 37°C. Time in seconds

from addition of thrombin to clot formation was measured by the tilt test (Figure 34B).

Specificity of Thrombin Binding. The binding affinity of the full-length class I RNA 16, class II RNA 27 and bulk 30N3 RNA for the serum proteins Antithrombin III (ATIII) and Prothrombin was determined by filter binding, as described above for the evolution of high affinity RNA ligands. These experiments were done in protein excess at concentrations from 1 x $10^{-5}$ to 5 x $10^{-10}$ M at a final RNA concentration of 2 x $10^{-9}$ M (Figure 35).

EXAMPLE V. NUCLEIC ACID LIGANDS TO bFGF.

Materials. bFGF was obtained from Bachem California (molecular weight 18,000 Da, 154 amino acids). Tissue culture grade heparin (average molecular weight 16,000 Da) was purchased form Sigma. Low molecular weight heparin (5,000 Da) was from Calbiochem. All other chemicals were at least reagent grade and were purchased from commercial sources.

SELEX. Essential features of the SELEX protocol have been described in detail in previous papers (Tuerk & Gold (1990) supra; Tuerk et al. (1992a) supra; Tuerk et al. (1992b) in Polymerase Chain Reaction (Ferre, F, Mullis, K., Gibbs, R. & Ross, A., eds.) Birkhauser, NY). Briefly, DNA templates for in vitro transcription (that contain a region of thirty random positions flanked by constant sequence regions) and the corresponding PCR primers were synthesized chemically (Operon). The random region was generated by utilizing an equimolar mixture of the four nucleotides during oligonucleotide synthesis. The two constant regions were designed to contain PCR primer annealing sites, a primer annealing site for cDNA synthesis, T7 RNA polymerase promoter region, and restriction enzyme sites that allow cloning into vectors (See Table I).

An initial pool of RNA molecules was prepared by in vitro transcription of about 200 picomoles (pmol) ($10^{14}$ molecules) of the double stranded DNA template utilizing T7 RNA polymerase (New England Biolabs). Transcription mixtures consisted of 100-300 nM template, 5 units/ul T7 RNA polymerase, 40 mM Tris-Cl buffer (pH 8.0) containing 12 mM $MgCl_2$, 5 mM DTT, 1 mM spermidine, 0.002% Triton X-100, and 4% PEG. Transcription mixtures were incubated at 37°C for 2-3 hours. These conditions typically resulted in transcriptional amplification of 10- to 100-fold.

Selections for high affinity RNA ligands were done by incubating bFGF (10-100 pmol) with RNA (90-300 pmol) for 10 minutes at 37°C in 50 ul of phosphate buffered saline (PBS)(10.1 mM $Na_2HPO_4$, 1.8 mM $KH_2PO_4$, 137 mM NaCl, 2.7 mM KCl, pH 7.4), then separating the protein-RNA complexes from the unbound species by nitrocellulose filter partitioning (Tuerk & Gold (1990) supra). The selected RNA (which typically amount to 0.3-8% of the total input RNA) was then extracted from the filters and reverse transcribed into cDNA by avian myeloblastosis virus reverse transcriptase (AMV RT, Life Sciences). Reverse transcriptions were done at 48°C (30 minutes) in 50 mM Tris buffer (pH 8.3), 60 mM NaCl, 6 mM $Mg(OAc)_2$, 10 mM DTT, and 1 unit/ul AMV RT. Amplification of the cDNA by PCR under standard conditions yielded sufficient amounts of double-stranded DNA for the next round of in vitro transcription.

Nitrocellulose Filter Binding Assay.

Oligonucleotides bound to proteins can be effectively separated from the unbound species by filtration through nitrocellulose membrane filters (Yarus & Berg (1970) Anal. Biochem. 35:450; Lowary & Uhlenbeck (1987) Nucleic Acids Res. 15:10483; Tuerk & Gold (1990) supra). Nitrocellulose filters (Millipore, 0.45 um pore size, type HA) were secured on a filter manifold and washed with 4-10 ml of buffer. Following incubations of $^{32}$P-labeled RNA with serial dilutions of the protein (5-10 min) at 37°C in buffer (PBS) containing 0.01% human serum albumin (HSA), the solutions were applied to the filters under gentle vacuum in 45 ul aliquots and washed with 5 ml of PBS. The filters were then dried under an infrared lamp and counted in a scintillation counter.

Cloning and Sequencing. Individual members of the enriched pools were cloned into pUC18 vector and sequenced as described (Schneider et al. (1992) supra; Tuerk & Gold (1990) supra).

SELEX Experiments Targeting bFGF. Following the procedures described above, two SELEX experiments (Experiments A and B) targeting bFGF were initiated with separate pools of randomized RNA, each pool consisting of approximately $10^{14}$ molecules. The constant sequence regions that flank the randomized region, along with the corresponding primers, were different in each experiment. The two template/primer combinations used are shown in Table I.

Selections were conducted in PBS at 37°C. The selection conducted in Experiment B was done in the presence of heparin (Sigma, molecular weight 5,000-32,000 Da, average molecular weight 16,000 Da) in the selection buffer at the molar ration of 1/100 (heparin/bFGF). Heparin competes for binding of randomized RNA to bFGF and the amount of heparin. The amount of heparin used significantly reduced but did not eliminate RNA binding to bFGF (data not shown). The rationale for using heparin was two-fold. First, heparin is known to induce a small conformational change in the protein and also stabilizes bFGF against thermal denaturation. Second, the apparent competitive nature of binding of heparin with randomized RNA to bFGF was expected to either increase the stringency of selection for the heparin binding site or direct the binding of RNA ligands to alternative site(s).

Significant improvement in affinity of RNA ligands to bFGF was observed in Experiment A after ten rounds,

and in Experiment B after thirteen rounds. Sequencing of these enriched pools of RNA ligands revealed a definite departure from randomness which indicated that the number of molecules remaining in the pool was substantially reduced. Individual members of the enriched pools were then cloned into pUC18 vector and sequenced as described above.

49 clones were sequenced from Experiment A, and 37 clones from Experiment B. From the total of 86 sequences, 71 were unique. Two distinct families could be identified based on overlapping regions of sequence homology (Tables II and III). A number of sequences with no obvious homology to members of either of the two families were also present, as expected (Irvine et al.(1991) J. Mol. Biol. 222:739), and are shown in Table IV.

The consensus sequence from family 1 ligands (Table II) is defined by a contiguous stretch of 9 bases, CUAACCAGG (SEQ ID NO:27). This suggests a minimal structure consisting of a 4-5 nucleotide loop that includes the strongly conserved AACC sequence and a bulged stem (Figure 41 and Table VI). The consensus sequence for family 2 ligands (Table III) is more extended and contains less conserved regions, RRGGHAACGYWNNGDCAAGNNCACYY (SEQ ID NO:43). Here, most of the strongly conserved positions are accommodated in a larger (19-21 nucleotide) loop (Figure 41 and Table VII). Additional structure within the loop is possible.

The existence of two distinct sequence families in the enriched pools of RNA suggest that there are two convergent solutions for high-affinity binding to bFGF. SELEX experiment A contributed members to both sequence families (Table II). All of the sequences from the SELEX experiment B (selected in the presence of heparin), on the other hand, belong either to family 2 (Table III) or to the "other sequences" family (Table IV) family, but none were found in family 1. This is surprising in view of the fact that bFGF was present in a formal molar excess of 100-fold over heparin during selections. The effective molar excess of bFGF over heparin, however, was probably much smaller. Average molecular weight of heparin used in selections was 16,000 Da. Since each sugar unit weighs 320 Da and at least eight sugar units are required for high-affinity binding to bFGF, six molecules of bFGF, on average, can bind to a molecule of heparin. This reduces the molar ratio of heparin to bFGF to 1:16. In practice, this amount of heparin is sufficient to reduce the observed affinity of the unselected RNA pool for bFGF by a factor of five (data not shown). The observed exclusion of an entire ligand family by presence of a relatively small amount of heparin in the selection buffer may be a consequence of a conformational change in the protein induced by heparin. Because of the relative amounts of heparin and bFGF that were used in selections, this model requires that the heparin-induced conformation persist after the protein-heparin complex has dissociated, and that the lifetime of this conformer is long enough to permit equilibration with the RNA ligands.

Family 2 sequences are comprised of clones derived from both SELEX experiments. This suggests that the flanking constant regions typically play a relatively minor role in determining the affinity of these ligands and supports the premise that the consensus sequence in this family is the principal determinant of high-affinity binding to bFGF.

Determination of Binding Affinities for bFGF.

Equilibrium Dissociation Constants. In the simplest case, equilibrium binding of RNA to bFGF can be described by equation 1:

$$\text{RNA}\bullet\text{bFGF} \rightleftharpoons \text{RNA} + \text{bFGF} \qquad (1)$$

The fraction of bound RNA (q) is related to the concentration of free protein, [P] (equation 2):

$$q = f[P]/([P] + K_d) \qquad (2)$$

where $K_d$ is the equilibrium dissociation constant and f reflects the efficiency of retention of the protein-RNA complexes on nitrocellulose filters. Mean value of f for bFGF was 0.82.

In order to eliminate higher order structures, all RNA solutions were heated to 90°C in PBS for 2-3 minutes and cooled on ice prior to incubation with protein. Only single bands for all RNA clones were detected on non-denaturing polyacrylamide gels following this treatment.

Relative binding affinity of individual ligands to bFGF cannot be predicted from sequence information. Unique sequence clones were therefore screened for their ability to bind to bFGF by measuring the fraction of radiolabeled RNA bound to nitrocellulose filters following incubation with 4 and 40 nM protein. This screening method was sufficiently accurate to allow several clones to be identified that had dissociation constants in the nanomolar range. Binding of these select clones was then analyzed in more detail.

High-affinity RNA ligands for bFGF were found in both sequence families (Tables VI and VII). The affinity of

clones that did not belong to either family was generally lower (data not shown).

The original, unselected RNA pools bound to bFGF with 300 nM (set A) and 560 nM (set B) affinities (Figure 36). SELEX therefore allowed the isolation of ligands with at least 2 orders of magnitude better affinity for bFGF.

In order to address the question of specificity, a representative set of high-affinity ligands for bFGF (5A and 7A from family 1; 12A and 26A from family 2) was tested for binding to four other heparin-binding proteins. It was found that the affinity of these ligands for acidic FGF, thrombin, antithrombin III, and vascular endothelial growth factor was relatively weak ($K_d$ > 0.3 uM)(data not shown).

RNA Ligand Inhibition of bFGF Receptor Binding. The same four high-affinity RNA ligands were also tested for their ability to inhibit binding of bFGF to the low- and the high-affinity cell-surface receptors.

Receptor Binding Studies. bFGF was labeled with $^{125}I$ by the Iodo-Gen (Pierce) procedure as described by Moscatelli (1987) supra. Confluent baby hamster kidney (BHK) cells were washed extensively with PBS and then incubated for 2 hours at 4°C with αMEM medium containing 10 ng/ml $^{125}I$-bFGF in PBS, 0.1% HSA, 1 unit/ml RNasein, and serial dilutions of high-affinity RNA. In a separate experiment it was established that the RNA is not significantly degraded under these conditions. The amount of $^{125}I$-bFGF bound to the low- and the high-affinity receptor sites was determined as described by Moscatelli (1987) supra.

All four ligands competed for the low-affinity receptor sites while the unselected (random) RNAs did not (Figure 37A). The concentration of RNA required to effect half-displacement of bFGF from the low-affinity receptor was 5-20 nM for ligands 5A, 7A and 26A, and >100 nM for ligand 12A. Half-displacement from the high-affinity sites is observed at the concentration of RNA near 1 uM for ligands 5A, 7A and 26A, and > 1 uM for ligand 12A. Again, random RNAs did not compete for the high-affinity receptor. The observed difference in concentration of RNA required to displace bFGF from the low- and high-affinity receptors is expected as a reflection of the difference in affinity of the two receptor classes for bFGF (2-10 nM for the low-affinity sites and 10-100 pM for the high-affinity sites).

Heparin competitively displaced RNA ligands from both sequence families (Figure 38), although higher concentrations of heparin were required to displace members of family 2 from bFGF.

The selective advantage obtained through the SELEX procedure is based on affinity to bFGF. RNA ligands can in principle bind to any site on the protein, and it is therefore important to examine the activity of the ligands in an appropriate functional assay. The relevant functional experiment for the selected high-affinity ligands is testing their ability to inhibit binding of bFGF to its cell-surface receptors since this is how bFGF exerts its biological activity. The fact that several representative high-affinity RNA ligands inhibited binding of bFGF to both receptor classes (in accord with their relative binding affinities) suggests that these ligands bind at or near the receptor binding site(s). Further support for this notion comes from the observation that heparin competes for binding of these ligands to bFGF. High affinity ligands from family 1 and family 2 may bind to different sites on bFGF. This invention includes covalently connecting components from the two ligand families into a single, more potent inhibitor of bFGF.

TABLE I. OLIGONUCLEOTIDES USED IN SELEX EXPERIMENTS A AND B.

| EXPERIMENT A | SEQUENCE 5'-3' | SEQ ID NUMBER |
|---|---|---|
| Starting RNA | GGGAGCUCAGAAUAAACGCUCAANNNNNNN NNNNNNNNNNNNNNNNNNNNNNNNNUUCGACA UGAGGCCCGGAUCCGGC | SEQ ID NO:21 |
| PCR Primer 1 | HindIII<br>CCGAAGCTTAATACGACTCACTATAGGGAG<br>T7 Promoter<br>CTCAGAATAAACGCTCAA | SEQ ID NO:22 |
| PCR Primer 2 | BamH1<br>GCCGGATCCGGGCCTCATGTCGAA | SEQ ID NO:23 |
| **EXPERIMENT B** | | |
| Starting RNA | GGGAGAUGCCUGUCGAGCAUGCUGNNNNNNN NNNNNNNNNNNNNNNNNNNNNNNNNGUAGCUAA ACAGCUUUGUCGACGGG | SEQ ID NO:24 |
| PCR Primer 1 | HindIII<br>CCCGAAGCTTAATACGACTCACTATAGGGAG<br>T7 Promoter<br>ATGCCTGTCGAGCATGCTG | SEQ ID NO:25 |
| PCR Primer 2 | Sal1<br>CCCGTCGACAAAGCTGTTTAGCTAC | SEQ ID NO:26 |

TABLE II. FAMILY 1 SEQUENCES OF THE RANDOM REGION FROM SELEX EXPERIMENT A AND B.

| FAMILY 1 | CONSENSUS SEQUENCE CUAACCNGG (SEQ ID NO:27) | SEQ ID NUMBER |
|---|---|---|
| 4A | UGCUAUUCGCCUAACUCGGCGCUCCUACCU | SEQ ID NO:28 |
| 5A | AUCUCCUCCCGUCGAAGCUAACCUGGCCAC | SEQ ID NO:29 |
| 7A | UCGGCGAGCUAACCAAGACACUCGCUGCAC | SEQ ID NO:30 |
| 10A | GUAGCACUAUCGGCCUAACCCGGUAGCUCC | SEQ ID NO:31 |
| 13A | ACCCGCGGCCUCCGAAGCUAACCAGGACAC | SEQ ID NO:32 |
| 14A | UGGGUGCUAACCAGGACACACCCACGCUGU | SEQ ID NO:33 |
| 16A | CACGCACAGCUAACCAAGCCACUGUGCCCC | SEQ ID NO:34 |
| 18A | CUGCGUGGUAUAACCACAUGCCCUGGGCGA | SEQ ID NO:35 |
| 21A | UGGGUGCUUAACCAGGCCACACCCUGCUGU | SEQ ID NO:36 |
| 25A | CUAGGUGCUAUCCAGGACUCUCCCUGGUCC | SEQ ID NO:37 |
| 29A | UGCUAUUCGCCUAGCUCGGCGCUCCUACCU | SEQ ID NO:38 |
| 38A | AGCUAUUCGCCCAACCCGGCGCUCCCGACC | SEQ ID NO:39 |
| 39A | ACCAGCUGCGUGCAACCGCACAUGCCUGG | SEQ ID NO:40 |
| 56A | CAGGCCCCGUCGUAAGCUAACCUGGACCCU | SEQ ID NO:41 |
| 61A | UGGGUGCUAACCACCACACACUCACGCUGU | SEQ ID NO:42 |

TABLE III. FAMILY 2 SEQUENCES OF THE RANDOM REGION FROM SELEX EXPERIMENTS A AND B.

| FAMILY 2 | CONSENSUS SEQUENCE: RRGGHAACGYWNHGDCAAGNNCACYY (SEQ ID NO:43) | SEQ ID NUMBER |
|---|---|---|
| 11A | GGGUAACGUUGU GACAAGUACACCUGCGUC | SEQ ID NO:44 |
| 12A | GGGGCAACGCUACA GACAAGUGCACCCAAC | SEQ ID NO:45 |
| 26A | CGUCAGAAGGCAACGUAUA GGCAAGCACAC | SEQ ID NO:46 |
| 27A | CCUCUCGAAGACAACGCUGU GACAAG ACAC | SEQ ID NO:47 |
| 47A | AGUGGGAAACGCUACUUGACAAG ACACCAC | SEQ ID NO:48 |
| 65A | GGCUACGCUAAU GACAAGUGCACUUGGGUG | SEQ ID NO:49 |
| 1B | CUCUGGUAACGCAAU GUCAAGUGCACAUGA | SEQ ID NO:50 |
| 2B | AGCCGCAGGUAACGGACC GGCGAGACCAUU | SEQ ID NO:51 |
| 6B | ACGAGCUUCGUAACGCUAUC GACAAGUGCA | SEQ ID NO:52 |
| 8B | AAGGGGAAACGUUGA GUCCGGUACACCCUG | SEQ ID NO:53 |
| 9B | AGGGUAACGUACU GGCAAGCUCACCUCAGC | SEQ ID NO:54 |
| 11B | GAGGUAACGUAC GACAAGACCACUCCAACU | SEQ ID NO:55 |
| 12B | AGGUAACGCUGA GUCAAGUGCACUCGACAU | SEQ ID NO:56 |
| 13B | GGGAAACGCUAUC GACGAGUGCACCCGGCA | SEQ ID NO:57 |
| 14B | CCGAGGGUAACGUUGG GUCAAGCACACCUC | SEQ ID NO:58 |
| 15B | UCGGGGUAACGUAUU GGCAAGGC ACCCGAC | SEQ ID NO:59 |
| 19B | GGUAACGCUGUG GACAAGUGCACCAGCUGC | SEQ ID NO:60 |
| 22B | AGGGUAACGUACU GGCAAGCUCACCUCAGC | SEQ ID NO:61 |
| 28B | AGGGUAACGUAUA GUCAAGAC ACCUCAAGU | SEQ ID NO:62 |
| 29B | GGGUAACGCAUU GGCAAGAC ACCCAGCCCC | SEQ ID NO:63 |
| 36B | GAGGAAACGUACC GUCGAGCC ACUCCAUGC | SEQ ID NO:64 |
| 38B | AGGUAACGCUGA GUCAAGUGCACUCGACAU | SEQ ID NO:65 |
| 48B | GGGUAACGUGU GACAAGAUCACCCAGUUUG | SEQ ID NO:66 |
| 49B | CACAGGGCAACGCUGCU GACAAGUGCACCU | SEQ ID NO:67 |

TABLE IV. OTHER SEQUENCES OF THE RANDOM REGION FROM SELEX EXPERIMENTS A AND B.

| NUMBER | SEQUENCE | SEQ ID NUMBER |
|---|---|---|
| 8A | ACGCCAAGUGAGUCAGCAACAGAGCGUCCG | SEQ ID NO:68 |
| 9A | CCAGUGAGUCCUGGUAAUCCGCAUCGGGCU | SEQ ID NO:69 |
| 24A | CUUCAGAACGGCAUAGUGGUCGGCCGCGCC | SEQ ID NO:70 |
| 33A | AGGUCACUGCGUCACCGUACAUGCCUGGCC | SEQ ID NO:71 |
| 34A | UCCAACGAACGGCCCUCGUAUUCAGCCACC | SEQ ID NO:72 |
| 36A | ACUGGAACCUGACGUAGUACAGCGACCCUC | SEQ ID NO:73 |
| 37A | UCUCGCUGCGCCUACACGGCAUGCCGGGA | SEQ ID NO:74 |
| 40A | GAUCACUGCGCAAUGCCUGCAUACCUGGUC | SEQ ID NO:75 |
| 43A | UCUCGCUGCGCCUACACGGCAUGCCCGGGA | SEQ ID NO:76 |
| 44A | UGACCAGCUGCAUCCGACGAUAUACCCUGG | SEQ ID NO:77 |
| 45A | GGCACACUCCAACGAGGUAACGUUACGGCG | SEQ ID NO:78 |
| 55A | AGCGGAACGCCACGUAGUACGCCGACCCUC | SEQ ID NO:79 |
| 4B | ACCCACGCCCGACAACCGAUGAGUUCUCGG | SEQ ID NO:80 |
| 5B | UGCUUUGAAGUCCUCCCCGCCUCUCGAGGU | SEQ ID NO:81 |
| 7B | AUGCUGAGGAUAUUGUGACCACUUCGGCGU | SEQ ID NO:82 |
| 16B | ACCCACGCCCGACAACCGAUGAGCUCGGA | SEQ ID NO:83 |
| 20B | AGUCCGGAUGCCCCACUGGGACUACAUUGU | SEQ ID NO:84 |
| 21B | AAGUCCGAAUGCCACUGGGACUACCACUGA | SEQ ID NO:85 |
| 23B | ACUCUCACUGCGAUUCGAAAUCAUGCCUGG | SEQ ID NO:86 |
| 40B | AGGCUGGGUCACCGACAACUGCCCGCCAGC | SEQ ID NO:87 |
| 42B | AGCCGCAGGUAACGGACCGGCGAGACCACU | SEQ ID NO:88 |
| 26B | GCAUGAAGCGGAACUGUAGUACGCGAUCCA | SEQ ID NO:89 |

TABLE V. REPEAT SEQUENCES OF THE RANDOM REGION FROM SELEX EXPERIMENTS A AND B.

| NUMBER | SEQUENCE | SEQUENCE ID NUMBER | CLONE REPEATED |
|---|---|---|---|
| 3A | GGGUAACGUUGUGACAAGUACACCUGCGUU | SEQ ID NO:90 | 11A |
| 15A | GGGUAACGUUGUGACAAGUACACCUGCGUC | SEQ ID NO:91 | 11A |
| 20A | GGGUAACGUUGUGACAAGUACACCUGCGUC | SEQ ID NO:92 | 11A |
| 48A | GGGUAACGUUGUGACAACUACACCUGCGUC | SEQ ID NO:93 | 11A |
| 58A | GGGUAACGUUGUGACAACUACACCUGCGUC | SEQ ID NO:94 | 11A |
| 64A | GGGUAACGUUGUGACAACUACACCUGCGUC | SEQ ID NO:95 | 11A |
| 28A | CGUCAGAAGGCAACGUAUAGGCAAGCACAC | SEQ ID NO:96 | 26A |
| 30A | GUAGCACUAUCGGCCUAACCCGGUAGCUCC | SEQ ID NO:97 | 10A |
| 23A | ACCCGCGGCCUCCGAAGCUAACCAGGACAC | SEQ ID NO:98 | 13A |
| 46A | AGGUCACUGCGUCACCGUACAUGCCUGGCC | SEQ ID NO:99 | 33A |
| 49A | AGGUCACUGCGUCACCGUACAUGCCUGGCC | SEQ ID NO:100 | 33A |
| 50A | GGCACACUCCAACGAGGUAACGUUACGGCG | SEQ ID NO:101 | 45A |
| 41A | GGGGCAACGCUACAGACAAGUGCACCCAAC | SEQ ID NO:102 | 12A |
| 51A | GGGGCAACGCUACAGACAAGUGCACCCAAC | SEQ ID NO:103 | 12A |
| 54A | GGGGCAACGCUACAGACAAGUGCACCCAAC | SEQ ID NO:104 | 12A |
| 35A | UGGGUGCUAACCAGGACACACCCACGCUGU | SEQ ID NO:105 | 14A |
| 18B | CCGAGGGUAACGUUGGGUCAAGCACACCUC | SEQ ID NO:106 | 14B |
| 24B | GGGAAACGCUAUCGACGAGUGCACCCGGCA | SEQ ID NO:107 | 13B |
| 39B | GGGAAACGCUAUCGACGAGUGCACCCGGCA | SEQ ID NO:108 | 13B |
| 37B | ACUCUCACUGCGAUUCGAAAUCAUGCCUGG | SEQ ID NO:109 | 23B |
| 43B | GCAUGAAGCGGAACUGUAGUACGCGAUCCA | SEQ ID NO:110 | 26B |
| 46B | GCAUGAAGCGGAACUGUAGUACGCGAUCCA | SEQ ID NO:111 | 26B |
| 25B | AGGGUAACGUACUGGCAAGCUCACCUCAGC | SEQ ID NO:112 | 9B |
| 33B | AGGGUAACGUACUGGCAAGCUCACCUCAGC | SEQ ID NO:113 | 9B |
| 31B | GGUAACGCUGUGGACAAGUGCACCAGCUGC | SEQ ID NO:114 | 19B |

TABLE VI. SECONDARY STRUCTURES AND DISSOCIATION CONSTANTS ($K_d$'s) FOR A REPRESENTATIVE SET OF HIGH-AFFINITY LIGANDS FROM FAMILY 1.

| LIGAND | STRUCTURE[a] | Kd, nM |
|---|---|---|
| 5A | CC AA<br>CCUC GUCGAA---GCU C<br>ggag cagcuu CGG C<br>ua CAC U | 23 ± 3 |
| 7A | AA<br>CGGCGAG---CU C<br>GUCGCUC GA C<br>ACA A | 5.0 ± 0.5 |
| 13A | C A<br>CCG GGCCUC----CGAAG----CU A<br>ggc-ccggag gcuuC GA C<br>uaca ACAG C | 3.2 ± 0.5 |
| 14A | cucaa A<br>aaacg UGGGUG----CU A<br>uuUGU- -ACCCAC GA C<br>CGC ACAG C | 3.0 ± 0.5 |
| 21A | A<br>aaU----GGGU---GCUU A<br>uUG CCCA CGGA C<br>UCGU CAC C | 8.1 ± 0.8 |
| 25A | A<br>CUA-GGUG----CU U<br>GGU CCUC GA C<br>C UCAG C | 5.9 ± 1.4 |
| 39A | CU A<br>AACCAG GC--GUGC A<br>uuGGUC--CG CACG C<br>UA C | 8.5 ± 1.2 |

[a]Strongly conserved positions are shown in boldface symbols. Nucleotides in the constant region are in lowercase type.

TABLE VII. SECONDARY STRUCTURES AND DISSOCIATION CONSTANTS ($K_d$'s) FOR A REPRESENTATIVE SET OF HIGH-AFFINITY LIGANDS FROM FAMILY 2.

| LIGAND | STRUCTURE[a] | Kd, nM |
|---|---|---|
| 12A | CAACGCU<br>G A<br>C<br>uc-aa---GGG A<br>ag uu CCC G<br>c CAA A A<br>CGUGAAC | 0.9 ± 0.2 |
| 26A | CAACGUA<br>A G U<br>GUC GAAG A<br>cag-cuuC G<br>A G<br>CACGAAC | 0.4 ± 0.1 |
| 65A | CUACGUA<br>G A<br>A<br>aacgcucaaG U<br>uuGUGGGUUC G<br>A A<br>CGUGAAC | 0.6 ± 0.04 |
| 22B | UAACGUA<br>G C<br>agc-augcugAGG U<br>ucg ugCGACUCC G<br>a A G<br>CUCGAAC | 1 ± 0.6 |
| 28B | UAACGUA<br>G U<br>augc-ugAGG<br>ugUG ACUCC A<br>A A G<br>CAGAACU | 2 ± 1 |
| 38B | UAACGCU<br>c G G<br>gcaug ugAG A<br>ugUAC GCUC G<br>A A U<br>CGUGAAC | 4 ± 1 |
| 2B | UAACGCA<br>C G C<br>AGC GCAG C<br>ucg ugUU G<br>a A G<br>CCAGAGC | 170 ± 80 |

[a]Strongly conserved positions are shown in boldface symbols. Nucleotides in the constant region are in lowercase type.

## Claims

**1.** A method for producing an improved nucleic acid ligand for a given target, wherein the ligand to be improved is identified by a process comprising:

(1) contacting a candidate mixture of nucleic acids with the target molecule, wherein nucleic acids having an increased affinity to the target relative to the candidate mixture may be partitioned from the remainder of the candidate mixture;
(2) partitioning the increased affinity nucleic acids from the remainder of the candidate mixture;
(3) amplifying the increased affinity nucleic acids to yield a ligand-enriched mixture of nucleic acids;
(4) repeating steps (1)-(3), as necessary, to identify a nucleic acid ligand,

wherein the ligand-enriched mixture of nucleic acids produced in step (3) is used as the candidate mixture in step (1), the improvement comprising modifying the ligand so that it has at least one of the following improved properties:
decreased size;
improved stability;
improved binding to the target;
modification of biological activity of the target;
capability to cross tissue or cell membrane barriers;
resistance to clearance;
wherein the ligand modification takes the form of specific alterations in the base sequence or chemical modification or derivatisation of the ligand based on:

(a) determining which nucleotides are crucial to the interaction with the target molecule;
(b) chemical modification experiments; or
(c) determining the structural conformation of said nucleic acid ligand.

**2.** A method according to claim 1 wherein (a), (b) or (c) includes performing covariance analysis.

**3.** A method according to claim 1 wherein (a), (b) or (c) comprises determining which nucleotide residues are necessary for maintaining the three dimensional structure of the ligand.

**4.** A method according to claim 1 wherein (a), (b) or (c) comprises determining the positions of the ligand which are crucial to the bound structure of the ligand with the target.

**5.** A method according to claim 1 wherein (a), (b) or (c) comprises determining which nucleotide residues interact with

the target.

6. A method according to claim 5 wherein said determining step comprises determining which nucleotide residues interact with the target to facilitate the formation of ligand-target binding pairs.

7. A method according to claim 5 wherein said determining step comprises determining which nucleotide residues are involved in proximal contacts with the target.

8. A method according to claim 1 wherein (a), (b) or (c) comprises determining the binding affinity of a modified ligand relative to the ligand before modification, where the modification is provided by nucleotide substitution in the ligand.

9. A method according to claim 1 wherein (a), (b) or (c) comprises determining the binding affinity of a modified ligand relative to the ligand before modification, where the modification is provided by the absence of one or more nucleotides of the ligand.

10. A method according to claim 1 wherein (a), (b) or (c) comprises determining the binding affinity of a chemically modified form of the ligand relative to the ligand before chemical modification.

11. A method according to claim 1 wherein (a), (b) or (c) comprises chemically modifying the ligand in the presence of the target, and determining which nucleotides of the ligand are not chemically modified.

12. A method according to claim 1 wherein (a), (b) or (c) comprises denaturing the ligand and chemically modifying both the denatured and non-denatured forms of the ligand, and determining which nucleic acid residues are modified in the denatured ligand that are not modified in the non-denatured ligand.

13. A method according to claim 1 wherein said ligand modification comprises addition, substitution or deletion of a nucleotide.

14. A method according to claim 1 wherein said ligand modification comprises truncation of the ligand.

15. A method according to claim 1 wherein said ligand modification includes incorporation of a cross-linking agent to covalently link the ligand to the target.

16. A method according to claim 1 wherein said ligand modification produces a small molecule that mimics the structure of the initial ligand framework.

17. A method according to claim 1 wherein said ligand modification has the effect of increasing the in vivo stability of the ligand.

18. A method according to claim 1 wherein said ligand modification has the effect of enhancing or mediating the delivery of the ligand.

19. A method according to claim 1 wherein said ligand modification provides resistance to enzymatic or chemical degradation.

20. A method according to claim 1 wherein said ligand modification reduces the rate of clearance of the modified ligand from the body during therapy.

21. A method according to claim 1 wherein said ligand modification includes chemical modification or derivatisation of the ligand at the ribose and/or phosphate and/or base positions.

22. A method according to claim 1 wherein the chemical modification experiments (b) are performed using a chemical selected from:

ethylnitrosourea;
dimethyl sulfate;
carbodiimide;
diethylpyrocarbonate; and

kethoxal.

**23.** A method according to claim 1 wherein the target is selected from:

HIV-RT protein;
HIV-1 Rev protein;
HIV-1 tat protein;
Thrombin; or
Basic fibroblast growth factor.

**24.** A method according to claim 1 wherein the modified ligand is a single stranded nucleic acid.

**25.** A method according to claim 1 wherein the modified ligand is an RNA or DNA.

**26.** A method according to claim 1 wherein the modified ligand is an RNA and said ligand modification includes a substitution on ribose of 2 -methoxy for 2 - hydroxyl.

**27.** A method according to claim 1 wherein the modified ligand is an RNA and said ligand modification includes modification of certain riboses to contain 2 -$NH_2$.

**28.** A nucleic acid ligand to HIV-RT protein that

(a) has the sequence:

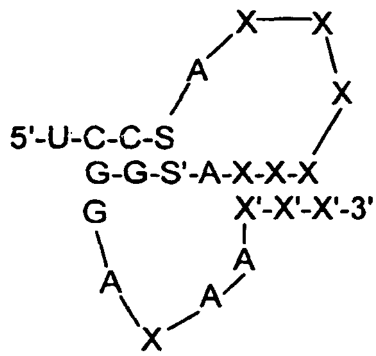


wherein X-X' indicates a preferred base-pair or
(b) has a sequence that has a degree of homology in excess of 70% of the sequence in (a), which may include base pair flips in those areas of the nucleic acid ligands that include base pairing regions, and which has an affinity for HIV-RT protein within two orders of magnitude of the affinity of a ligand having said sequence.

**29.** A nucleic acid ligand to HIV-RT protein that

(a) has the sequence:

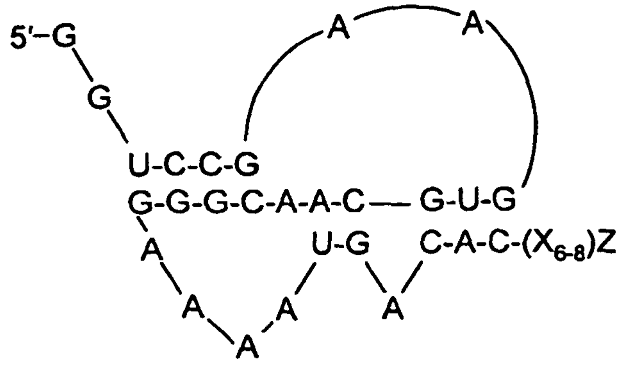


wherein X indicates a nucleotide position that is non-conserved, and wherein Z is selected from the group consisting of the sequence set forth in Figure 9 (SEQ ID NO:115-135) or

(b) has a sequence that has a degree of homology in excess of 70% of the sequence in (a), which may include base pair flips in those areas of the nucleic acid ligands that include base pairing regions, and which has an affinity for HIV-RT protein within two orders of magnitude of the affinity of a ligand having said sequence.

**30.** A nucleic acid ligand to HIV-1 Rev protein that

(a) has the sequence:

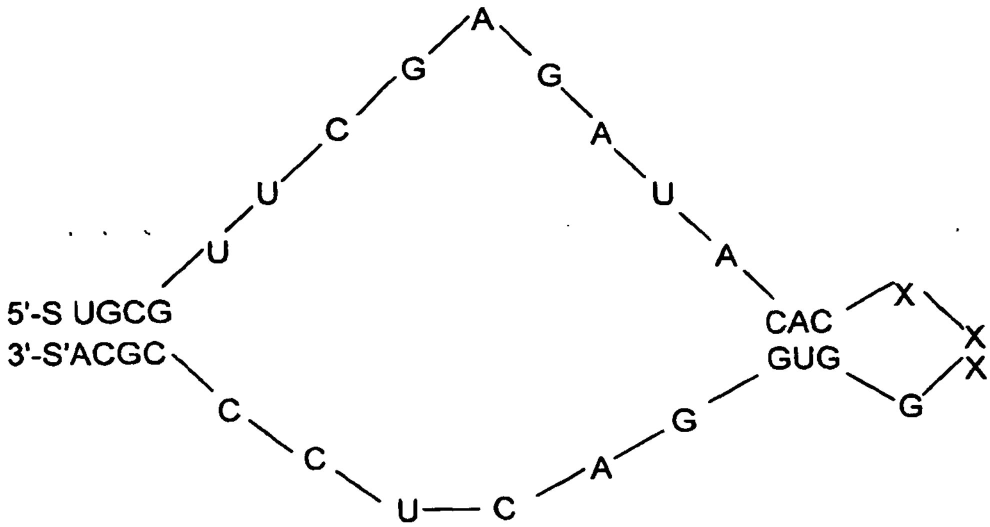


(b) has a sequence that has a degree of homology in excess of 70% of the sequence in (a), which may include base pair flips in those areas of the nucleic acid ligands that include base pairing regions, and which has an affinity for HIV-1 Rev protein within two orders of magnitude of the affinity of a ligand having said sequence.

**31.** The nucleic acid ligand of any of claims 28 to 30 chemically modified or derivatised at the ribose and/or phosphate and/or base positions.

**Patentansprüche**

**1.** Verfahren zur Herstellung eines verbesserten Nucleinsäureliganden für ein vorgegebenes Ziel, worin der zu verbessernde Ligand durch ein Verfahren identifiziert wird, das Folgendes umfasst:

(1) das Kontaktieren eines Kandidatengemischs aus Nucleinsäuren mit dem Zielmolekül, worin Nucleinsäuren,

die im Vergleich zum Kandidatengemisch eine verstärkte Affinität zum Ziel aufweisen, vom Rest des Kandidatengemischs abgetrennt werden können;
(2) das Abtrennen der Nucleinsäuren mit verstärkter Affinität vom Rest des Kandidatengemischs;
(3) das Amplifizieren der Nucleinsäuren mit verstärkter Affinität, um ein ligandenangereichertes Gemisch aus Nucleinsäuren zu erhalten;
(4) das Wiederholen der Schritte (1)-(3), falls erforderlich, um einen Nucleinsäureliganden zu identifizieren, worin das ligandenangereicherte Gemisch aus Nucleinsäuren, das in Schritt (3) hergestellt wird, als Kandidatengemisch in Schritt (1) verwendet wird,
wobei die Verbesserung eine Modifikation des Liganden umfasst, sodass er zumindest einer der folgenden verbesserten Eigenschaften aufweist:

verringerte Größe;
verbesserte Stabilität;
verbesserte Bindung an das Ziel;
Modifikation der biologischen Aktivität des Ziels;
Fähigkeit, Gewebe- oder Zellmembranbarrieren zu überwinden;
Resistenz gegenüber Clearance;

worin die Ligandenmodifikation die Form von spezifischen Veränderungen in der Basensequenz oder einer chemischen Modifikation oder Derivatisierung der Liganden annimmt, basierend auf:

(a) der Bestimmung, welche Nucleotide für die Wechselwirkung mit dem Zielmolekül entscheidend sind;
(b) chemischen Modifikationsexperimenten; oder
(c) der Bestimmung der strukturellen Konformation des Nucleinsäureliganden.

**2.** Verfahren nach Anspruch 1, worin (a), (b) oder (c) die Durchführung einer Kovarianzanalyse umfasst.

**3.** Verfahren nach Anspruch 1, worin (a), (b) oder (c) das Bestimmen, welche Nucleotidreste zur Beibehaltung der dreidimensionalen Struktur des Liganden erforderlich sind, umfasst.

**4.** Verfahren nach Anspruch 1, worin (a), (b) oder (c) das Bestimmen der Positionen des Liganden umfasst, die für die Bindungsstruktur des Liganden mit dem Ziel entscheidend sind.

**5.** Verfahren nach Anspruch 1, worin (a), (b) oder (c) das Bestimmen, welche Nucleotidreste mit dem Ziel wechselwirken, umfasst.

**6.** Verfahren nach Anspruch 5, worin der Bestimmungsschritt das Bestimmen, welche Nucleotidreste mit dem Ziel wechselwirken, umfasst, um die Bildung von Ligand-Ziel-Bindungspaaren zu erleichtern.

**7.** Verfahren nach Anspruch 5, worin der Bestimmungsschritt das Bestimmen, welche Nucleotidreste an proximalen Kontakten mit dem Ziel beteiligt sind, umfasst.

**8.** Verfahren nach Anspruch 1, worin (a), (b) oder (c) das Bestimmen der Bindungsaffinität eines modifizierten Liganden in Bezug auf den Liganden vor der Modifikation umfasst, worin die Modifikation durch Nucleotidsubstitution im Liganden bereitgestellt ist.

**9.** Verfahren nach Anspruch 1, worin (a), (b) oder (c) das Bestimmen der Bindungsaffinität eines modifizierten Liganden in Bezug auf den Liganden vor der Modifikation umfasst, worin die Modifikation durch die Abwesenheit eines oder mehrerer Nucleotide des Liganden bereitgestellt ist.

**10.** Verfahren nach Anspruch 1, worin (a), (b) oder (c) das Bestimmen der Bindungsaffinität einer chemisch modifizierten Form des Liganden in Bezug auf den Liganden vor der chemischen Modifikation umfasst.

**11.** Verfahren nach Anspruch 1, worin (a), (b) oder (c) das chemische Modifizieren des Liganden in Gegenwart eines Ziels und das Bestimmen, welche Nucleotide des Liganden nicht chemisch modifiziert sind, umfasst.

**12.** Verfahren nach Anspruch 1, worin (a), (b) oder (c) das Denaturieren des Liganden und das chemische Modifizieren sowohl der denaturierten als auch der nicht denaturierten Formen des Liganden sowie das Bestimmen, welche

Nucleinsäurereste im denaturierten Liganden modifiziert sind, die im nicht denaturierten Liganden nicht modifiziert sind, umfasst.

**13.** Verfahren nach Anspruch 1, worin die Ligandenmodifikation die Addition, Substitution oder Deletion eines Nucleotids umfasst.

**14.** Verfahren nach Anspruch 1, worin die Ligandenmodifikation die Trunkierung des Liganden umfasst.

**15.** Verfahren nach Anspruch 1, worin die Ligandenmodifikation die Inkorporation eines Vernetzungsmittels umfasst, um den Liganden kovalent an das Ziel zu binden.

**16.** Verfahren nach Anspruch 1, worin die Ligandenmodifikation ein kleines Molekül ergibt, das die Struktur des anfänglichen Ligandengerüsts nachahmt.

**17.** Verfahren nach Anspruch 1, worin die Ligandenmodifikation eine Steigerung der In-vivo-Stabilität des Liganden bewirkt.

**18.** Verfahren nach Anspruch 1, worin die Ligandenmodifikation eine Steigerung oder Vermittlung der Ligandenanlieferung bewirkt.

**19.** Verfahren nach Anspruch 1, worin die Ligandenmodifikation Resistenz gegenüber enzymatischem oder chemischem Abbau bereitstellt.

**20.** Verfahren nach Anspruch 1, worin die Ligandenmodifikation die Clearance-Geschwindigkeit des modifizierten Liganden aus dem Körper während einer Therapie verringert.

**21.** Verfahren nach Anspruch 1, worin die Ligandenmodifikation eine chemische Modifikation oder Derivatisierung des Liganden an Ribose- und/oder Phosphat- und/oder Basenpositionen umfasst.

**22.** Verfahren nach Anspruch 1, worin die chemischen Modifikationsexperimente (b) unter Verwendung einer Chemikalie durchgeführt werden, die ausgewählt ist aus:

Ethylnitrosoharnstoff;
Dimethylsulfat;
Carbodiimid;
Diethylpyrocarbonat; und
Kethoxal.

**23.** Verfahren nach Anspruch 1, worin das Ziel ausgewählt ist aus:

HIV-RT-Proteinen;
HIV-1-Rev-Proteinen;
HIV-1-tat-Proteinen;
Thrombin; oder
basischen Fibroblastenwachstumsfaktoren.

**24.** Verfahren nach Anspruch 1, worin der modifizierte Ligand eine einsträngige Nucleinsäure ist.

**25.** Verfahren nach Anspruch 1, worin der modifizierte Ligand eine RNA oder DNA ist.

**26.** Verfahren nach Anspruch 1, worin der modifizierte Ligand eine RNA ist und die Ligandenmodifikation die Substitution auf Ribose von 2-Hydroxyl durch 2-Methoxy umfasst.

**27.** Verfahren nach Anspruch 1, worin der modifizierte Ligand eine RNA ist und die Ligandenmodifikation die Modifikation bestimmter Ribosen umfasst, sodass sie 2-$NH_2$ umfassen.

**28.** Nucleinsäureligand für das HIV-RT-Protein, der

(a) die Sequenz

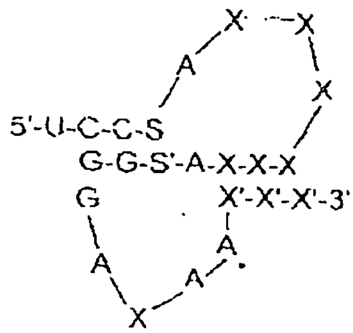

aufweist, worin X-X' ein bevorzugtes Basenpaar bezeichnet, oder
(b) eine Sequenz mit einem Homologiegrad von über 70 % mit der Sequenz aus (a) aufweist, die Basenpaar-Flips in jenen Bereichen der Nucleinsäureliganden enthalten kann, die Basenpaarungsregionen umfassen, und eine Affinität für ein HIV-RT-Protein innerhalb von zwei Größenordnungen der Affinität eines Liganden mit der Sequenz aufweist.

**29.** Nucleinsäureligand für das HIV-RT-Protein, der

(a) die Sequenz

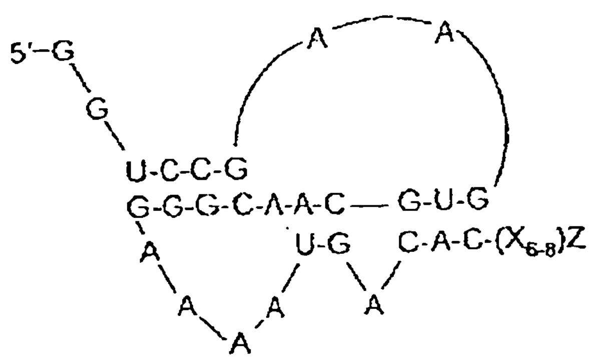

aufweist, worin X eine Nucleotidposition bezeichnet, die nicht konserviert ist, und worin Z aus der aus den in Fig. 9 dargelegten Sequenzen (Seq.-ID Nr. 115-135) bestehenden Gruppe ausgewählt ist, oder
(b) eine Sequenz mit einem Homologiegrad von über 70 % mit der Sequenz aus (a) aufweist, die Basenpaar-Flips in jenen Bereichen der Nucleinsäureliganden enthalten kann, die Basenpaarungsregionen umfassen, und eine Affinität für ein HIV-RT-Protein innerhalb von zwei Größenordnungen der Affinität eines Liganden mit der Sequenz aufweist.

**30.** Nucleinsäureligand für ein HIV-1-Rev-Protein, der

(a) die Sequenz

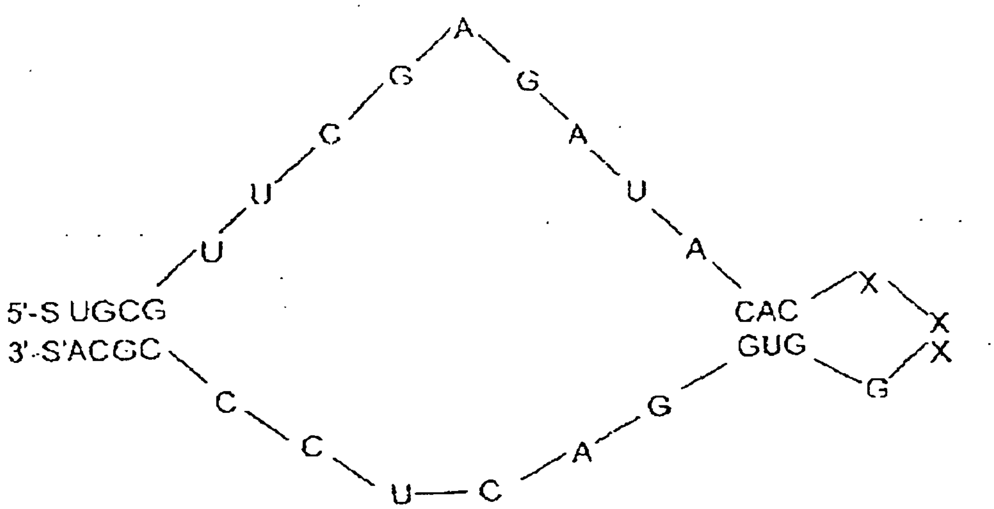


aufweist,

(b) eine Sequenz mit einem Homologiegrad von über 70 % mit der Sequenz aus (a) aufweist, die Basenpaar-Flips in jenen Bereichen der Nucleinsäureliganden enthalten kann, die Basenpaarungsregionen umfassen, und eine Affinität für ein HIV-1-Rev-Protein innerhalb von zwei Größenordnungen der Affinität eines Liganden mit der Sequenz aufweist.

**31.** Nucleinsäureligand nach einem der Ansprüche 28 bis 30, der an den Ribose- und/oder Phosphat- und/oder Basenpositionen chemisch modifiziert oder derivatisiert ist.

## Revendications

**1.** Un procédé de production d'un ligand amélioré d'acide nucléique pour une cible déterminée, dans lequel le ligand à améliorer est identifié par un processus comprenant les étapes consistant à :

(1) mettre en contact un mélange candidat d'acides nucléiques avec la molécule cible où les acides nucléiques ayant une affinité augmentée à la cible par rapport au mélange candidat peuvent être séparés du reste du mélange candidat ;
(2) séparer du reste du mélange candidat, les acides nucléiques d'affinité augmentée ;
(3) amplifier les acides nucléiques d'affinité augmentée pour donner un mélange d'acides nucléiques enrichi en ligand ;
(4) répéter les étapes (1)-(3), tel que nécessaire, pour identifier un ligand d'acide nucléique, où le mélange d'acides nucléiques enrichi en ligand produit dans l'étape (3) est utilisé en tant que mélange candidat dans l'étape (1),

le perfectionnement comprenant le fait de modifier le ligand de manière qu'il ait au moins une des propriétés améliorées suivante :

taille diminuée ;
stabilité améliorée ;
liaison améliorée à la cible ;
modification de l'activité biologique de la cible ;
capacité à des barrières de tissu croisé ou de
membrane cellulaire ;
résistance à la clairance ;

où la modification du ligand prend la forme d'altérations spécifiques dans la séquence de base ou de modification chimique ou de dérivatisation du ligand sur la base de :

(a) détermination de quels nucléotides sont cruciaux à l'interaction avec la molécule cible ;
(b) expérimentation de modification chimique ; ou
(c) détermination de la conformation structurelle du ligand d'acide nucléique.

**2.** Un procédé selon la revendication 1 dans lequel (a), (b) ou (c) comprend l'étape consistant à effectuer une analyse de covariance.

**3.** Un procédé selon la revendication 1 dans lequel (a), (b) ou (c) comprend l'étape consistant à déterminer quels résidus de nucléotides sont nécessaires pour maintenir la structure tridimensionnelle du ligand.

**4.** Un procédé selon la revendication 1 dans lequel (a), (b) ou (c) comprend l'étape consistant à déterminer les positions du ligand qui sont cruciales à la structure de liaison du ligand avec la cible.

**5.** Un procédé selon la revendication 1 dans lequel (a), (b) ou (c) comprend l'étape consistant à déterminer quels résidus de nucléotides réagissent avec la cible.

**6.** Un procédé selon la revendication 5 dans lequel ladite étape de détermination comprend le fait de déterminer quels résidus de nucléotides réagissent avec la cible afin de faciliter la formation de paires de liaison ligand-cible.

**7.** Un procédé selon la revendication 5 dans lequel ladite étape de détermination comprend le fait de déterminer quels résidus de nucléotides sont impliqués dans des contacts proximaux avec la cible.

**8.** Un procédé selon la revendication 1 dans lequel (a), (b) ou (c) comprend l'étape consistant à déterminer l'affinité de liaison d'un ligand modifié par rapport au ligand avant modification, où la modification est créée par substitution de nucléotides dans le ligand.

**9.** Un procédé selon la revendication 1 dans lequel (a), (b) ou (c) comprend l'étape consistant à déterminer l'affinité de liaison d'un ligand modifié par rapport au ligand avant modification, où la modification est créée par l'absence d'un ou de plusieurs nucléotides du ligand.

**10.** Un procédé selon la revendication 1 dans lequel (a), (b) ou (c) comprend l'étape consistant à déterminer l'affinité de liaison d'un forme chimiquement modifiée du ligand par rapport au ligand avant modification chimique.

**11.** Un procédé selon la revendication 1 dans lequel (a), (b) ou (c) comprend les étapes consistant à modifier chimiquement le ligand en présence de la cible, et de déterminer quels nucléotides du ligand ne sont pas modifiés chimiquement.

**12.** Un procédé selon la revendication 1 dans lequel (a), (b) ou (c) comprend les étapes consistant à dénaturer le ligand et à modifier chimiquement à la fois la forme dénaturée et la forme non-dénaturée du ligand, et à déterminer quels résidus d'acides nucléiques sont modifiés dans le ligand dénaturé qui ne sont pas modifiés dans le ligand non-dénaturé.

**13.** Un procédé selon la revendication 1 dans lequel ladite modification du ligand comprend l'addition, la substitution ou la suppression d'un nucléotide.

**14.** Un procédé selon la revendication 1 dans lequel ladite modification du ligand comprend une troncature du ligand.

**15.** Un procédé selon la revendication 1 dans lequel ladite modification du ligand comprend l'incorporation d'un agent de réticulation pour relier de manière covalente le ligand à la cible.

**16.** Un procédé selon la revendication 1 dans lequel ladite modification du ligand produit une petite molécule qui imite la structure de l'ossature du ligand initial.

**17.** Un procédé selon la revendication 1 dans lequel ladite modification du ligand a l'effet d'augmenter la stabilité in vivo du ligand.

**18.** Un procédé selon la revendication 1 dans lequel ladite modification du ligand a l'effet d'augmenter ou d'assurer la médiation de la fourniture du ligand.

**19.** Un procédé selon la revendication 1 dans lequel ladite modification du ligand crée une résistance à une dégradation enzymatique ou chimique.

**20.** Un procédé selon la revendication 1 dans lequel ladite modification du ligand réduit le taux de clairance du ligand modifié issu du corps pendant une thérapie.

**21.** Un procédé selon la revendication 1 dans lequel ladite modification du ligand comprend une modification chimique ou une dérivatisation du ligand au niveau des positions ribose et/ou phosphate et/ou base.

**22.** Un procédé selon la revendication 1 dans lequel les expérimentations de modification chimique (b) sont effectuées en utilisant un produit chimique sélectionné parmi :

l'éthylnitrourée ;
le sulfate de diméthyle ;
le carbodiimide ;
le diéthylpyrocarbonate ; et
le kethoxal.

**23.** Un procédé selon la revendication 1 dans lequel la cible est sélectionnée parmi :

une protéine HIV-RT
une protéine HIV-1 ReV ;
une protéine HIV-1 tat ;
une thrombine ; ou
un facteur de croissance de fibroblast de base.

**24.** Un procédé selon la revendication 1 dans lequel le ligand modifié est un acide nucléique à un seul brin.

**25.** Un procédé selon la revendication 1 dans lequel le ligand modifié est un ARN ou un ADN.

**26.** Un procédé selon la revendication 1 dans lequel le ligand modifié est un ARN et ladite modification du ligand comprend une substitution sur ribose de 2 -méthoxy pour 2-hydroxyle.

**27.** Un procédé selon la revendication 1 dans lequel le ligand modifié est un ARN et ladite modification du ligand comprend une modification de certaines riboses pour contenir 2-$NH_2$.

**28.** Un ligand d'acide nucléique à une protéine HIV-RT qui

(a) a la séquence :

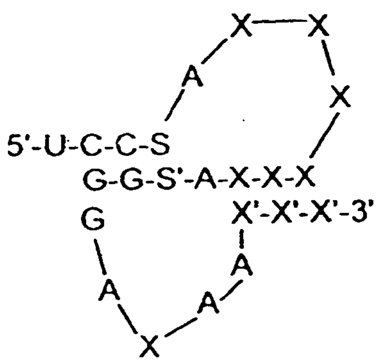


où X-X' indique une paire de base préférée ou
(b) a une séquence qui a un degré d'homologie supérieur à 70% de la séquence de (a), laquelle peut comprendre des flips de paires de base dans celles des zones des ligands d'acide nucléique qui comprennent des régions d'appariement de base, et laquelle a une affinité pour la protéine HIV-RT à l'intérieur de deux ordres de grandeur

de l'affinité d'un ligand ayant ladite séquence.

**29.** Un ligand d'acide nucléique à la protéine HIV-RT qui

(a) a la séquence :

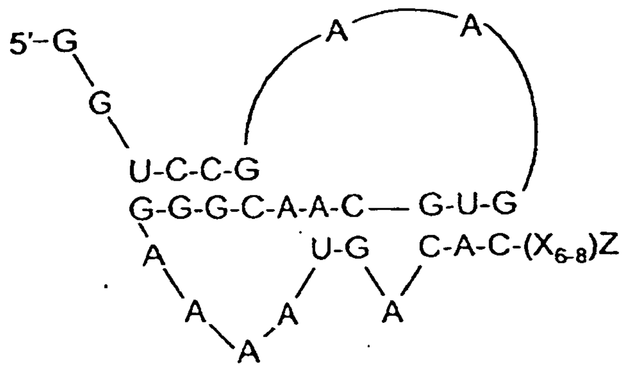


où X indique une position de nucléotide qui n'est pas conservée, et où Z est sélectionné dans le groupe se composant de la séquence énoncée à la Figure 9 (ID SEQ NO:115-135) ou

(b) a une séquence qui a un degré d'homologie supérieur à 70% de la séquence de (a), laquelle peut comprendre des flips de paires de base dans celles des zones des ligands d'acide nucléique qui comprennent des régions d'appariement de base, et laquelle a une affinité pour la protéine HIV-RT à l'intérieur de deux ordres de grandeur de l'affinité d'un ligand ayant ladite séquence.

**30.** Un ligand d'acide nucléique à la protéine HIV-1 Rev qui

(a) a la séquence :

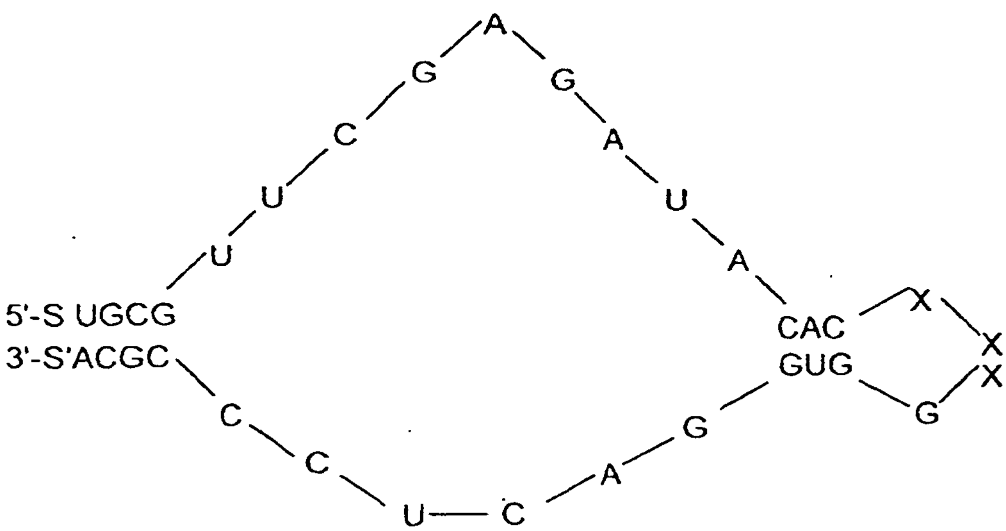


(b) a une séquence qui a un degrés d'homologie supérieur à 70% de la séquence (a), laquelle peut comprendre des flips de paires de base dans celles des zones des ligands d'acide nucléique qui comprennent des régions d'appariement de base, et laquelle a une affinité pour la protéine HIV-1 Rev à l'intérieur de deux ordres de grandeur de l'affinité d'un ligand ayant ladite séquence.

**31.** Le ligand d'acide nucléique de l'une quelconque des revendications 28 à 30 modifié chimiquement ou dérivatisé

au niveau des positions ribose et/ou phosphate et/ou base.

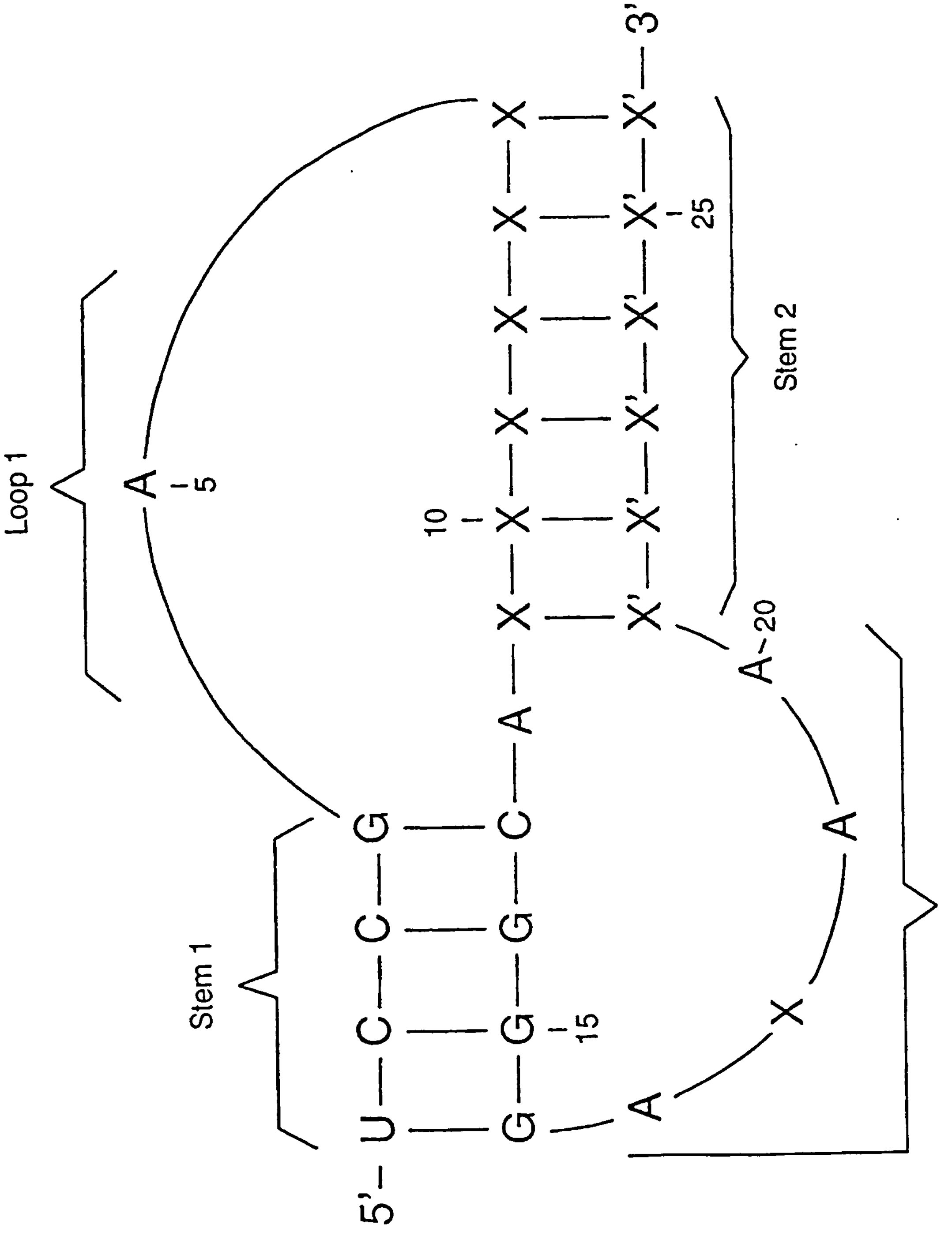


Fig. 1

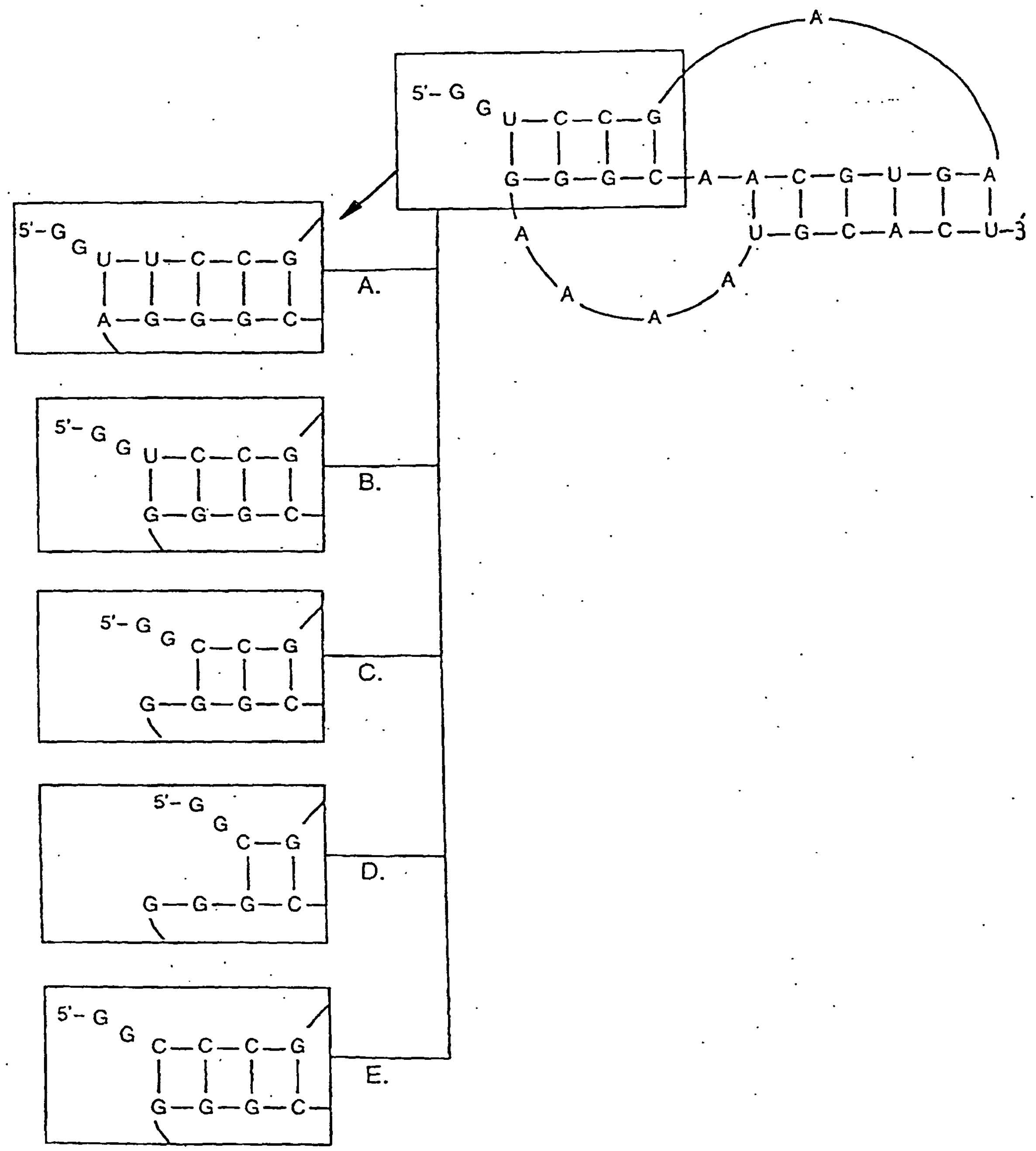

5'- G G U—C—C—G
G—G—G—C—A—A—C—G—U—G—A
U—G—C—A—C—U—3'
A
A A A A
5'- G G U—U—C—C—G
A—G—G—G—C
A.
5'- G G U—C—C—G
G—G—G—C
B.
5'- G G C—C—G
G—G—G—C
C.
5'- G G C—G
G—G—G—C
D.
5'- G G C—C—C—G
G—G—G—C
E.


FIG.2A

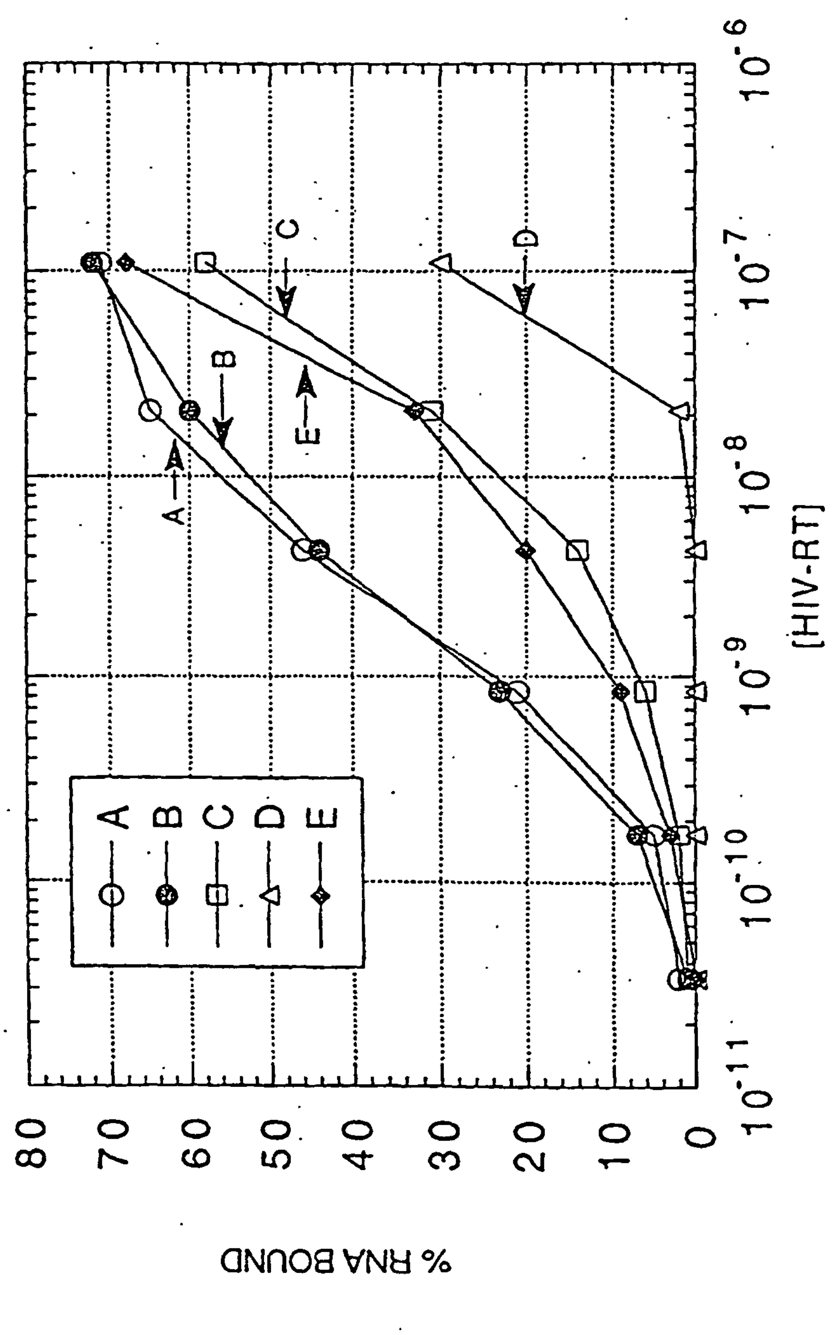
% RNA BOUND
0
10
20
30
40
50
60
70
80
[HIV-RT]
$10^{-11}$
$10^{-10}$
$10^{-9}$
$10^{-8}$
$10^{-7}$
$10^{-6}$
A
B
C
D
E

FIG.2B

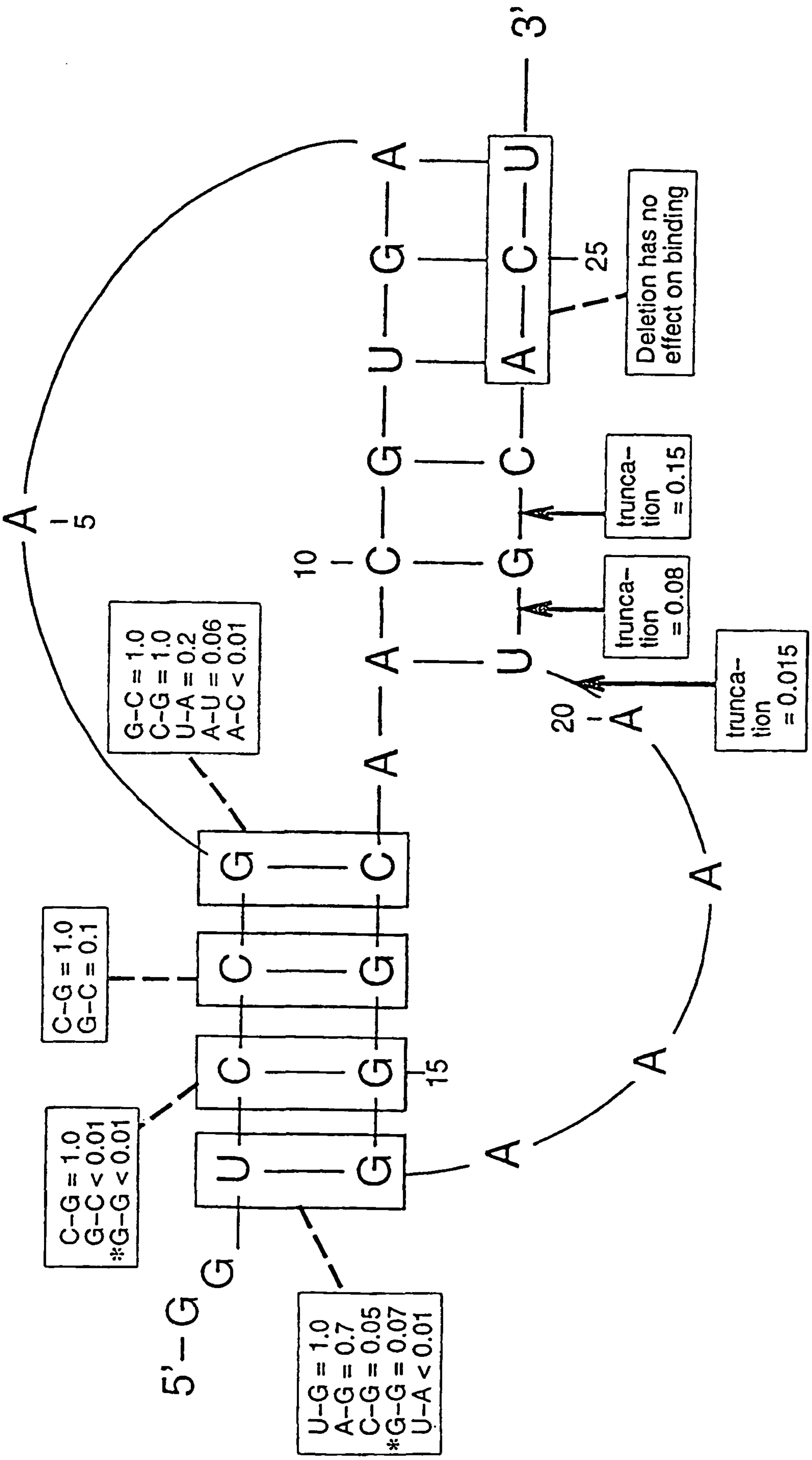
5'
3'
G
G
U
C
C
G
A
G
G
G
C
A
A
C
G
U
G
A
U
G
G
C
A
C
U
A
A
A
A
5
10
15
20
25
U-G = 1.0
A-G = 0.7
C-G = 0.05
*G-G = 0.07
U-A < 0.01
C-G = 1.0
G-C < 0.01
*G-G < 0.01
C-G = 1.0
G-C = 0.1
G-C = 1.0
C-G = 1.0
U-A = 0.2
A-U = 0.06
A-C < 0.01
truncation = 0.015
truncation = 0.08
truncation = 0.15
Deletion has no effect on binding

Fig. 3

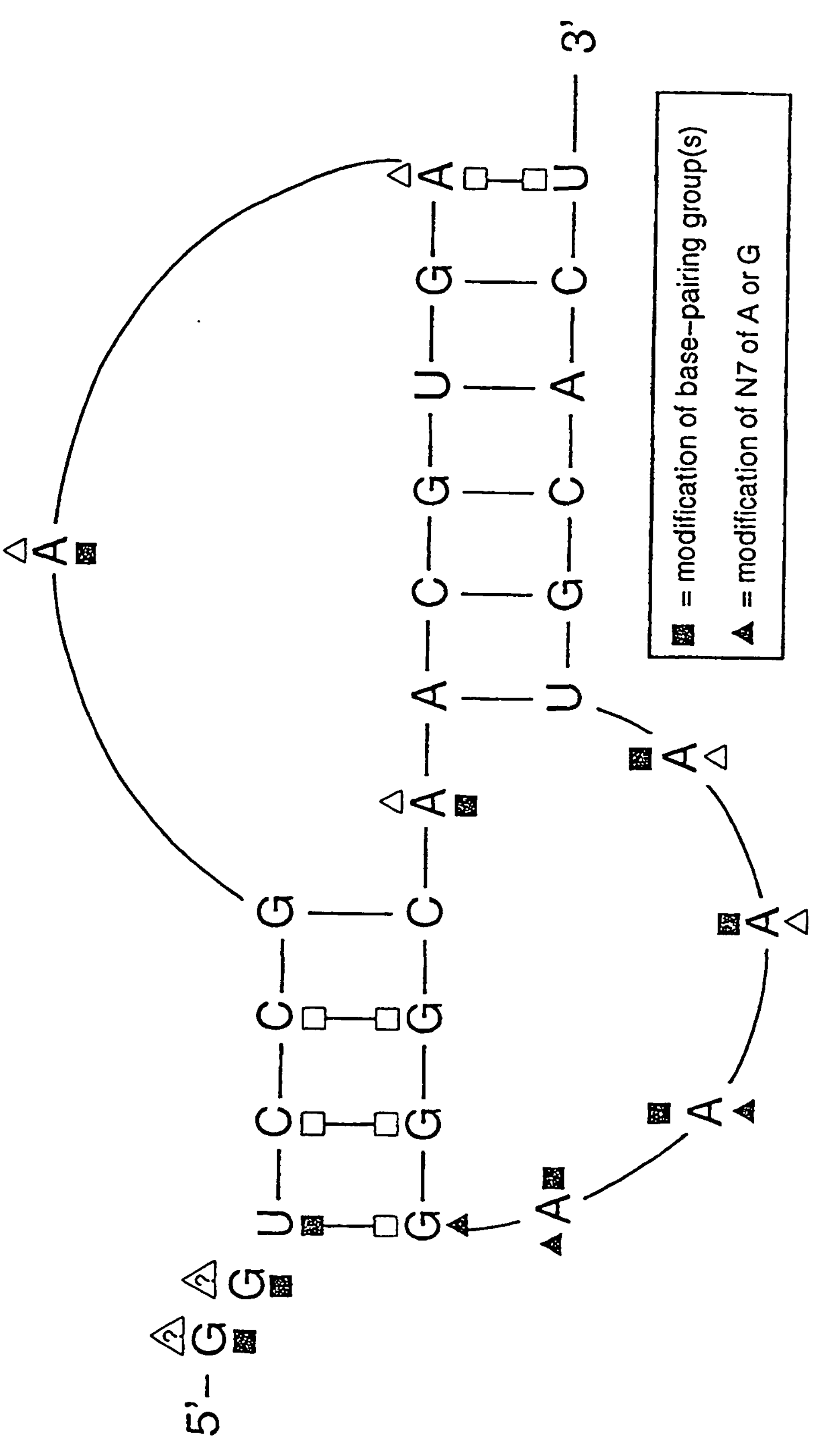
5'
3'
= modification of base-pairing group(s)
= modification of N7 of A or G

Fig. 4

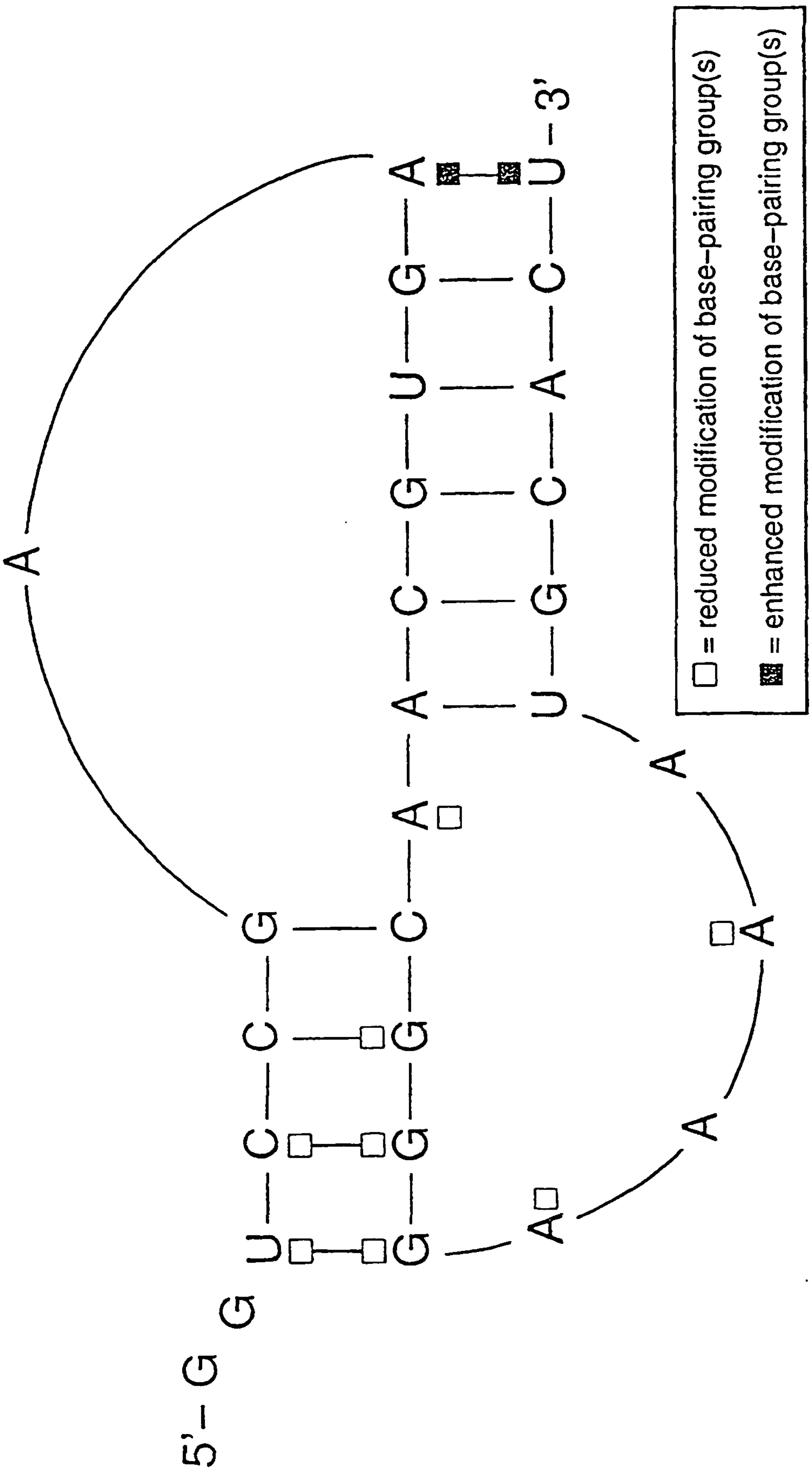
5'– G G
3'
□ = reduced modification of base-pairing group(s)
▩ = enhanced modification of base-pairing group(s)

Fig. 5

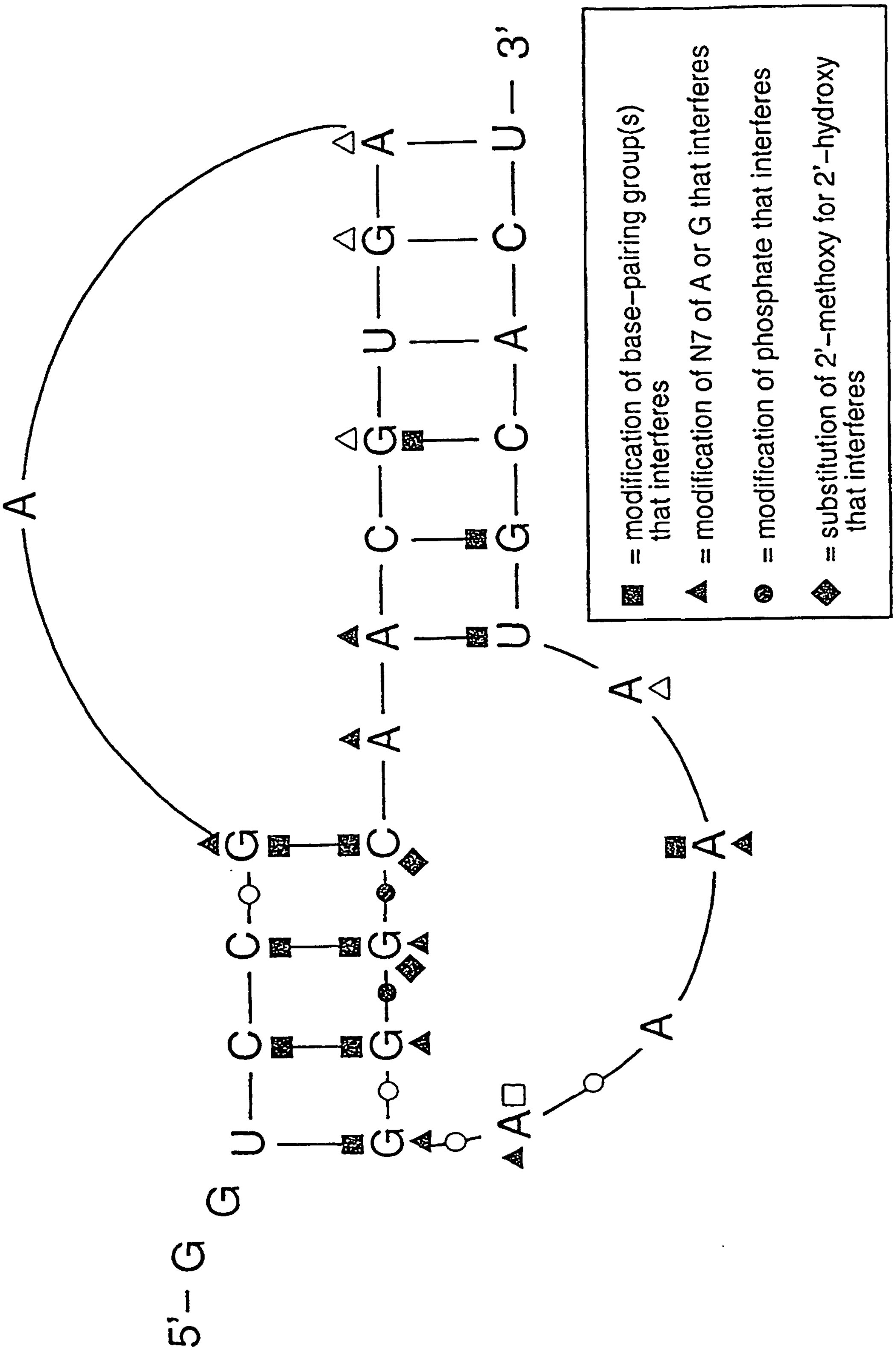
5'— G G U C C G G G C A A C G U G A
3' U C A C G U A A A A
= modification of base-pairing group(s) that interferes
= modification of N7 of A or G that interferes
= modification of phosphate that interferes
= substitution of 2'-methoxy for 2'-hydroxy that interferes

Fig. 6

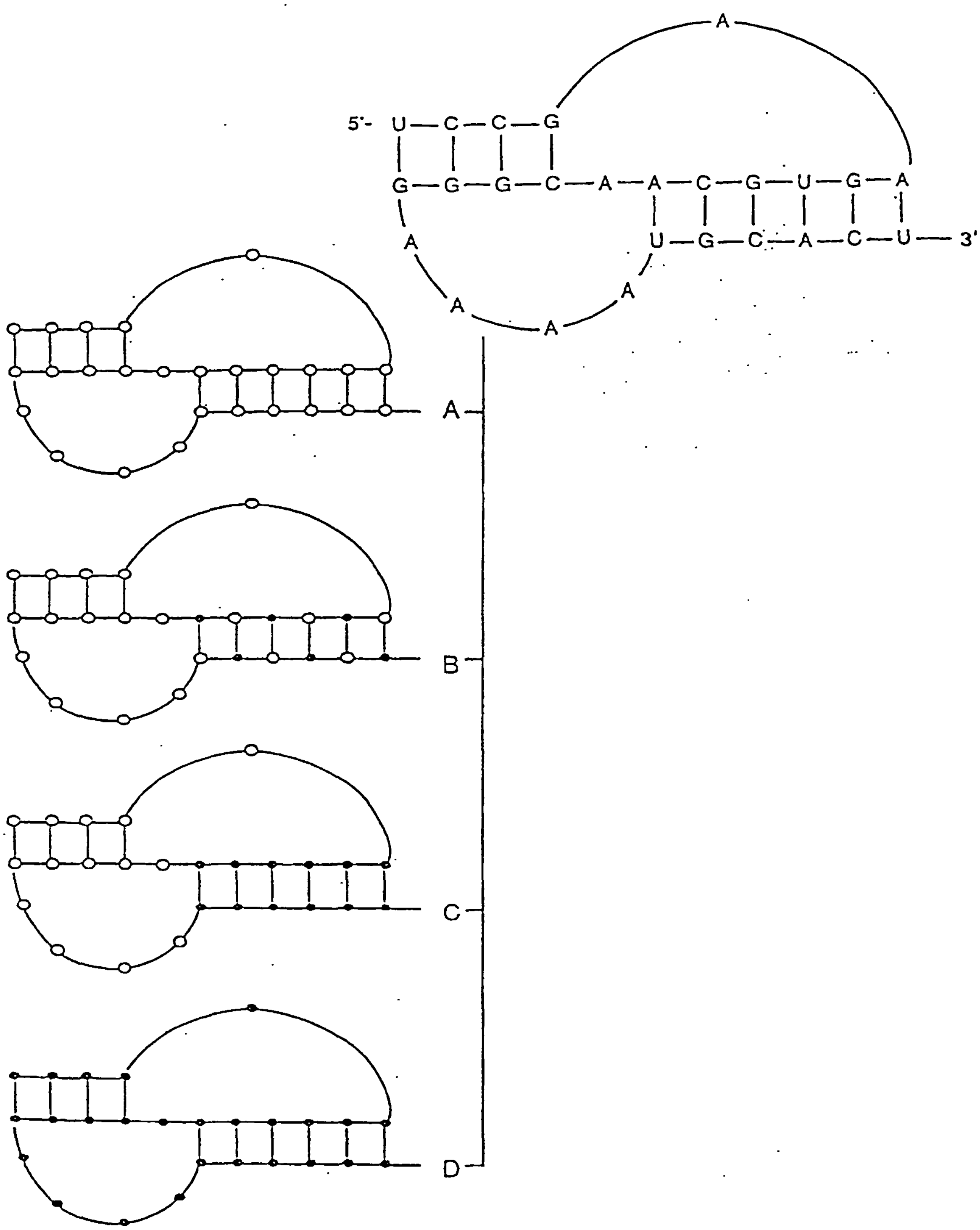


: denotes a 2' O-methyl instead of an OH, denoted by (O), at this position on the ribose

FIG.7A

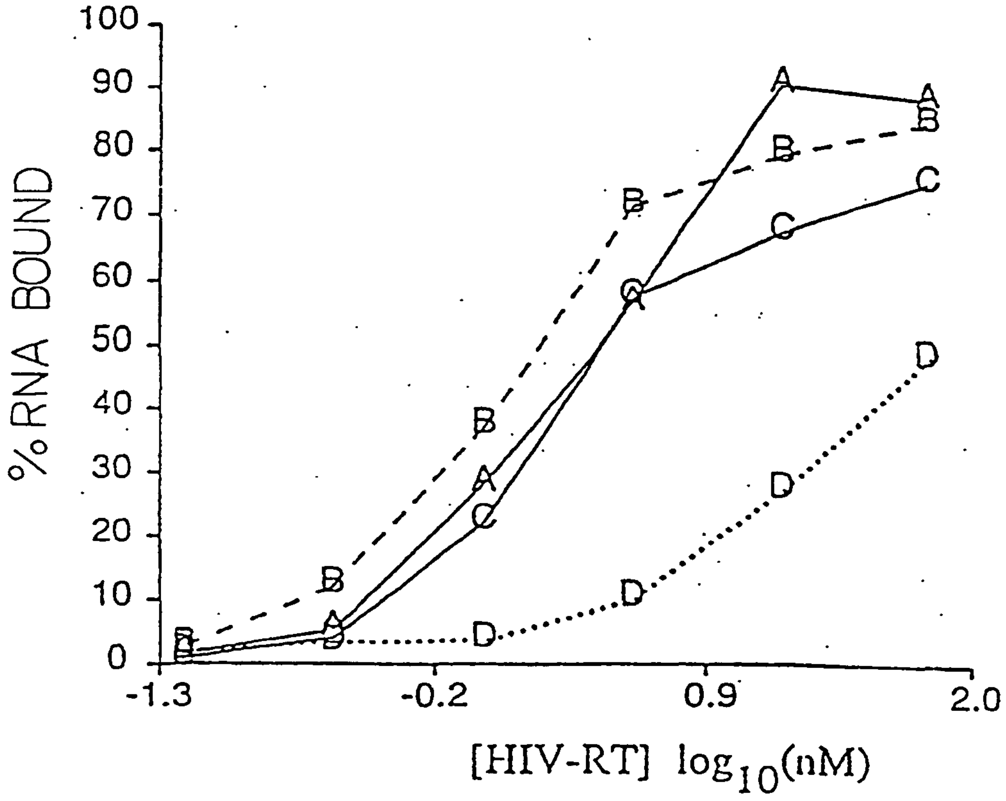
100
90
80
70
60
50
40
30
20
10
0
% RNA BOUND
-1.3
-0.2
0.9
2.0
[HIV-RT] log10(nM)
A
B
C
D

FIG.7B

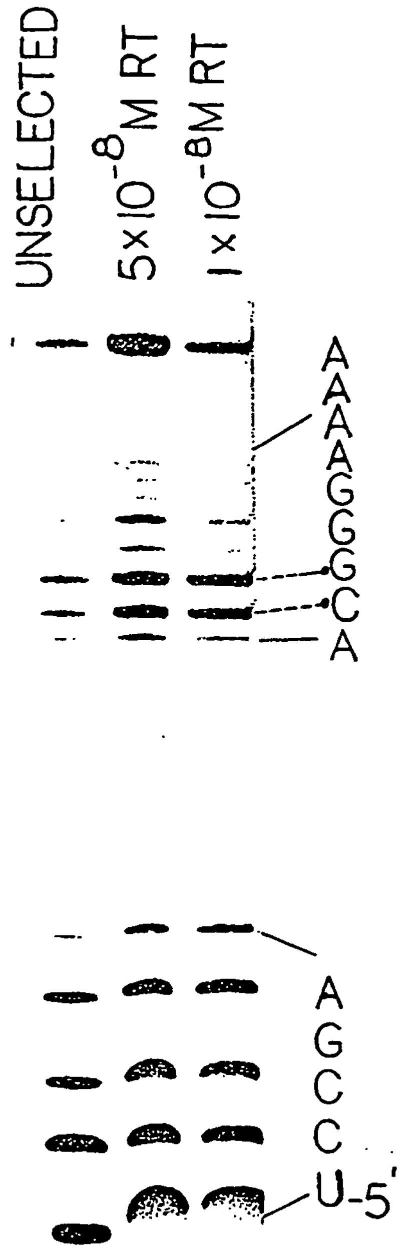


FIG.8

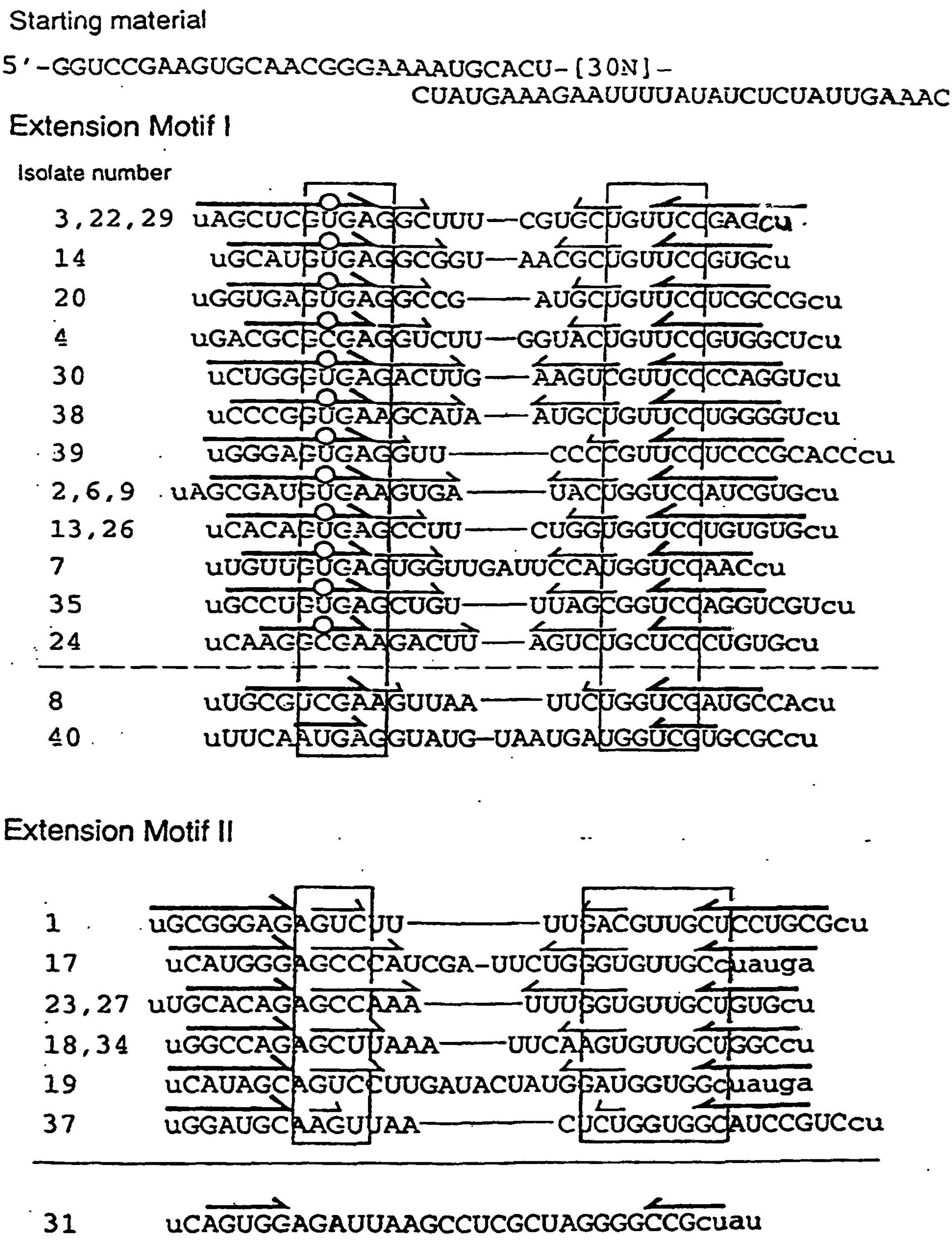
Starting material
5'-GGUCCGAAGUGCAACGGGAAAAUGCACU-[30N]-
CUAUGAAAGAAUUUUAUAUCUCUAUUGAAAC-3'
Extension Motif I
Isolate number
3,22,29 uAGCUCGUGAGGCUUU—CGUGCUGUUCCGAGcu
14 uGCAUGUGAGGCGGU—AACGCUGUUCCGUGcu
20 uGGUGAGUGAGGCCG——AUGCUGUUCCUCGCCGcu
4 uGACGCCCGAGGUCUU—GGUACUGUUCCGUGGCUcu
30 uCUGGCUGAGACUUG——AAGUCGUUCCCCAGGUcu
38 uCCCGGUGAAGCAUA——AUGCUGUUCCUGGGGUcu
39 uGGGAGUGAGGUU————CCCCGUUCCUCCCGCACCcu
2,6,9 uAGCGAUGUGAAGUGA————UACUGGUCCAUCGUGcu
13,26 uCACAGUGAGCCUU——CUGGUGGUCCUGUGUGcu
7 uUGUUGUGAGUGGUUGAUUCCAUGGUCCAACcu
35 uGCCUGUGAGCUGU——UUAGCGGUCCAGGUCGUcu
24 uCAAGCCGAAGACUU——AGUCUGCUCCCUGUGcu
8 uUGCGUCGAAGUUAA——UUCUGGUCGAUGCCAcu
40 uUUCAAUGAGGUAUG–UAAUGAUGGUCGUGCGCcu
Extension Motif II
1 uGCGGGAGAGUCUU—————UUGACGUUGCUCCUGCGcu
17 uCAUGGGAGCCCAUCGA-UUCUGGGUGUUGCCuauga
23,27 uUGCACAGAGCCAAA————UUUGGUGUUGCUGUGcu
18,34 uGGCCAGAGCUUAAA——UUCAAGUGUUGCUGGCcu
19 uCAUAGCAGUCCUUGAUACUAUGGAUGGUGGCuauga
37 uGGAUGCAAGUUAA—————CUCUGGUGGCAUCCGUCcu
31 uCAGUGGAGAUUAAGCCUCGCUAGGGGCCGcuau

Fig. 9

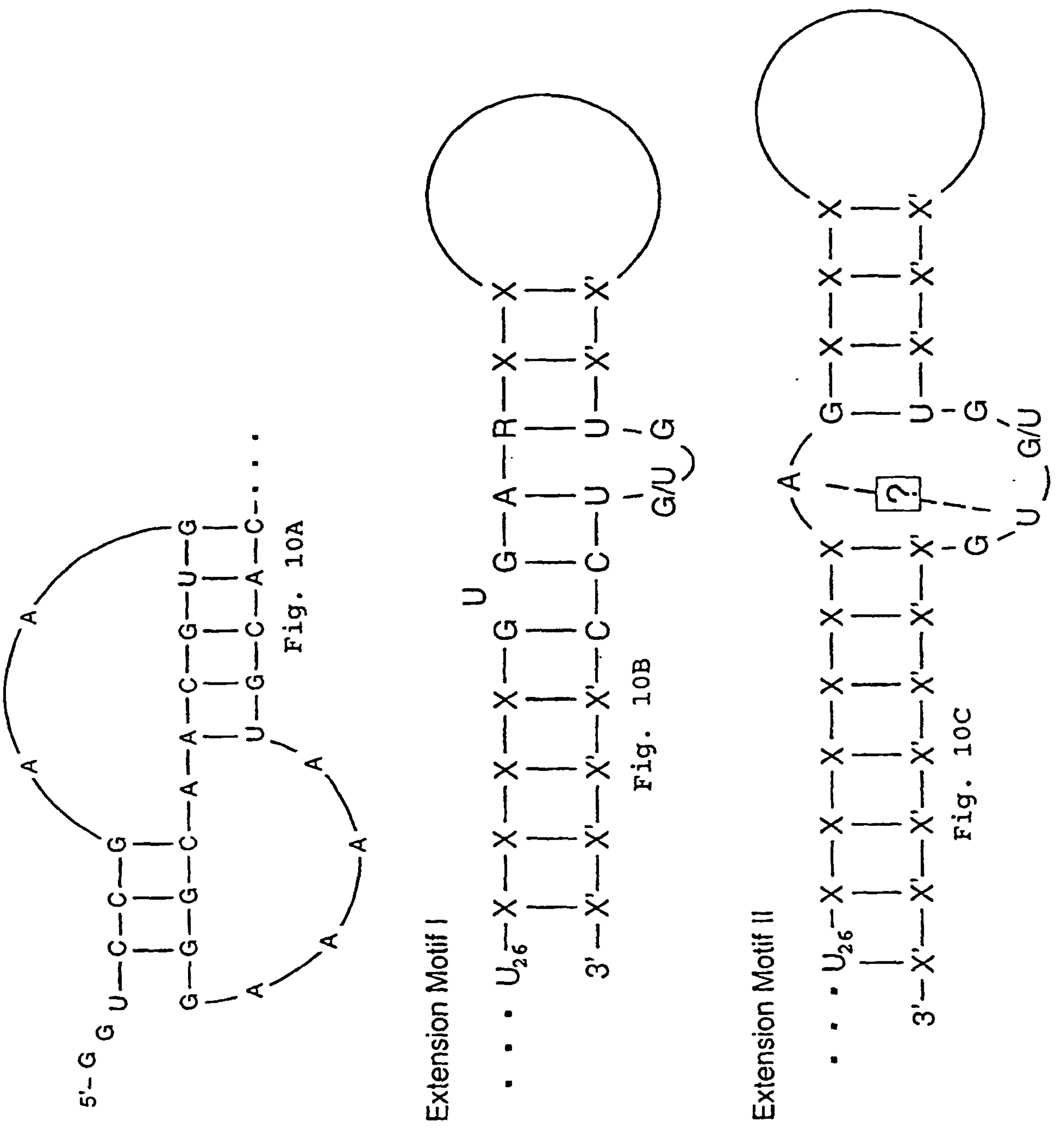


Fig. 10A

Extension Motif I

Fig. 10B

Extension Motif II

Fig. 10C

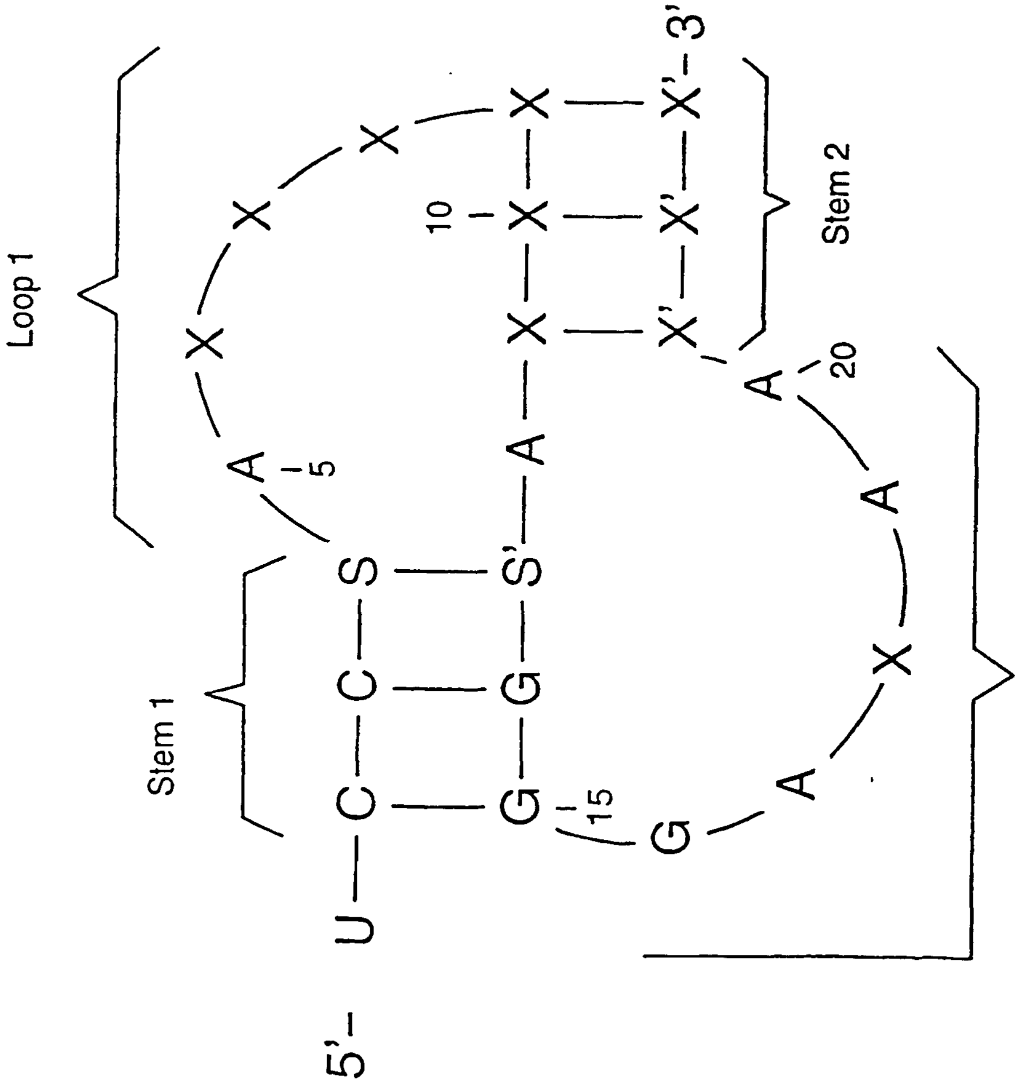


Fig. 11

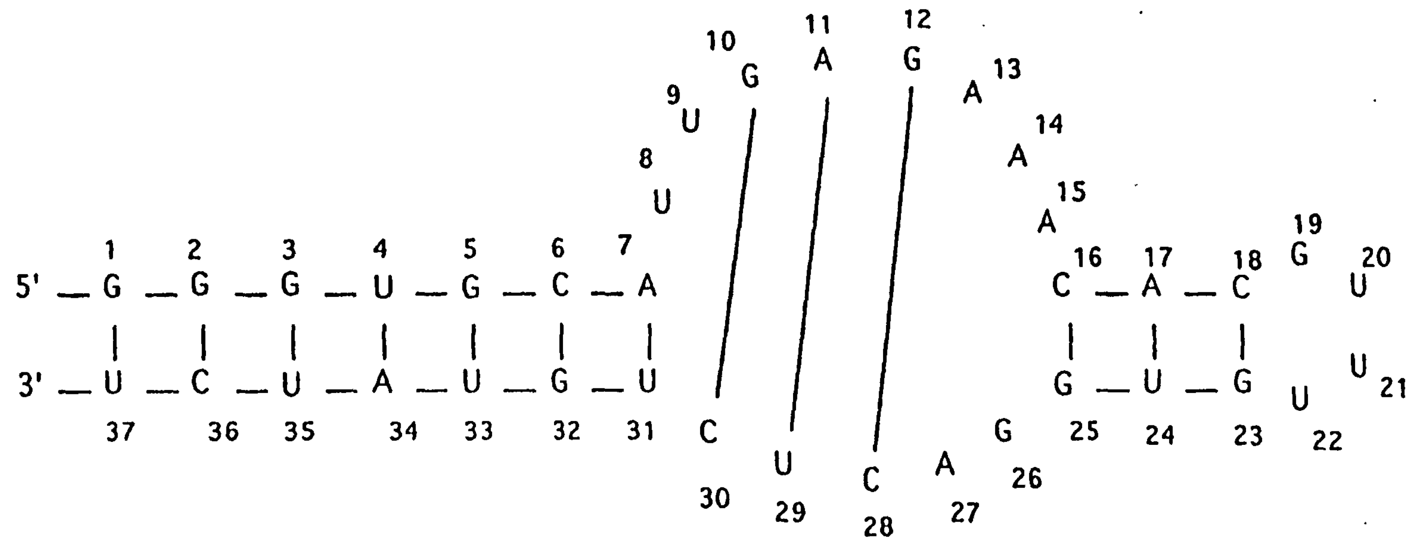

Fig. 12A

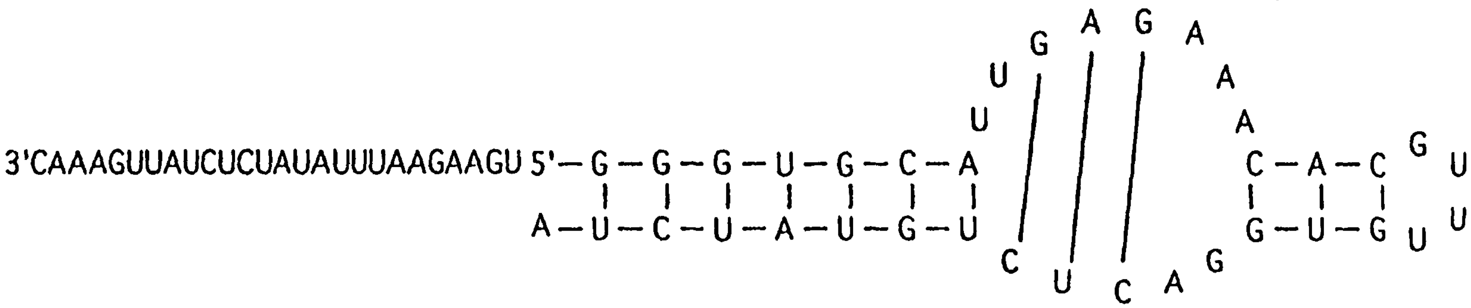


Fig. 12B

5' GTTTCAATAGAGATATAAAATTC 3'

Fig. 12C

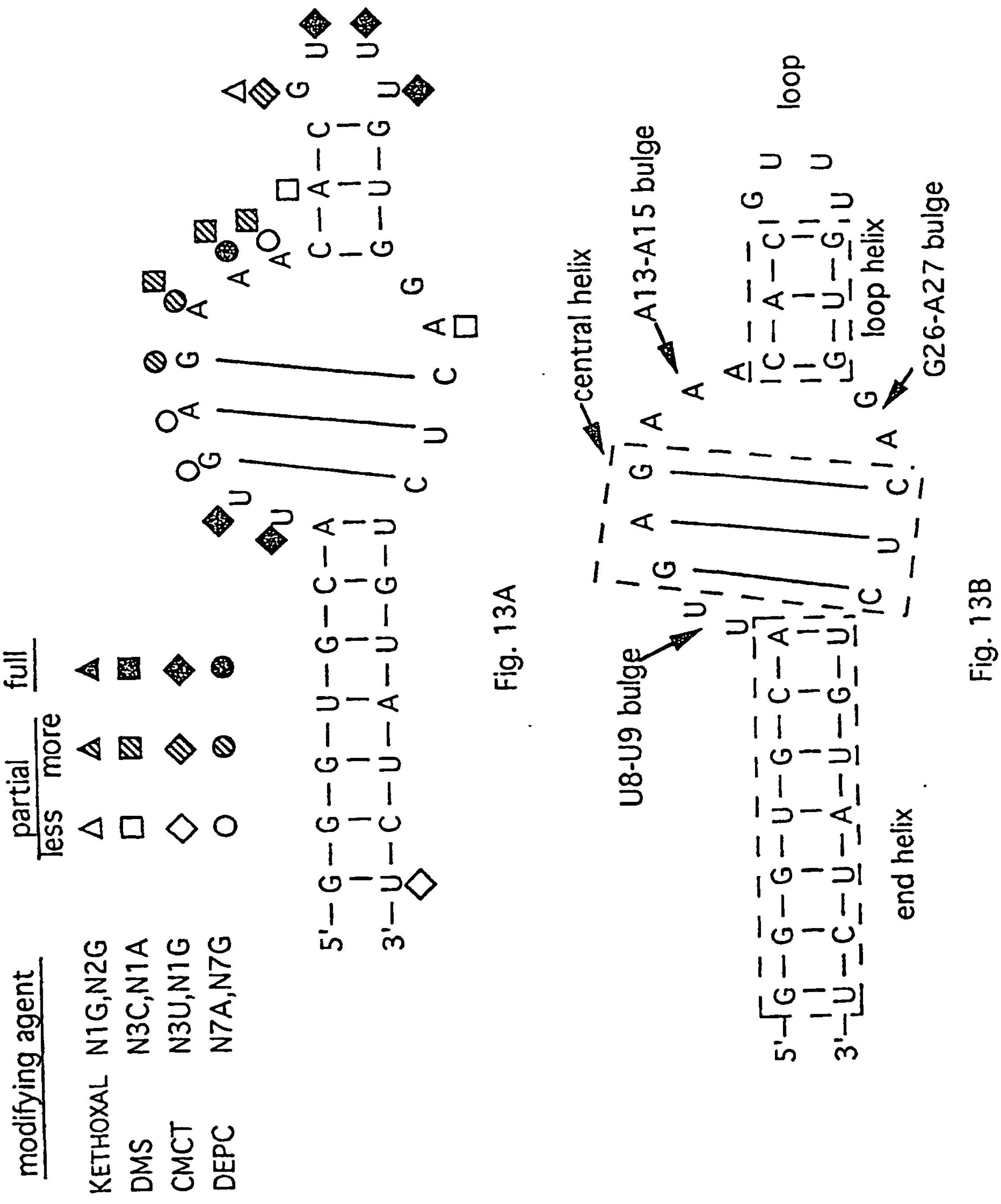


Fig. 13A

Fig. 13B

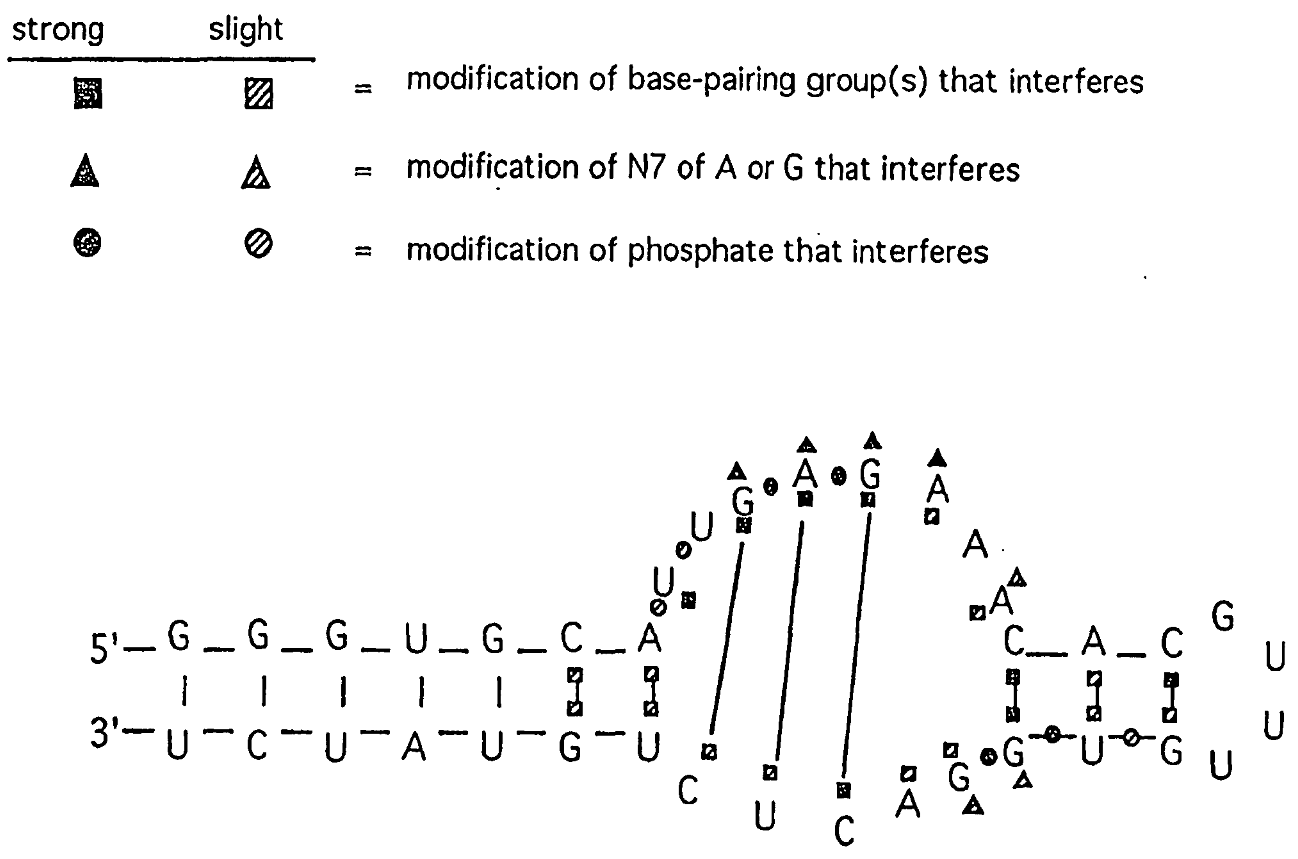
strong
slight
= modification of base-pairing group(s) that interferes
= modification of N7 of A or G that interferes
= modification of phosphate that interferes
5'
3'

Fig. 14

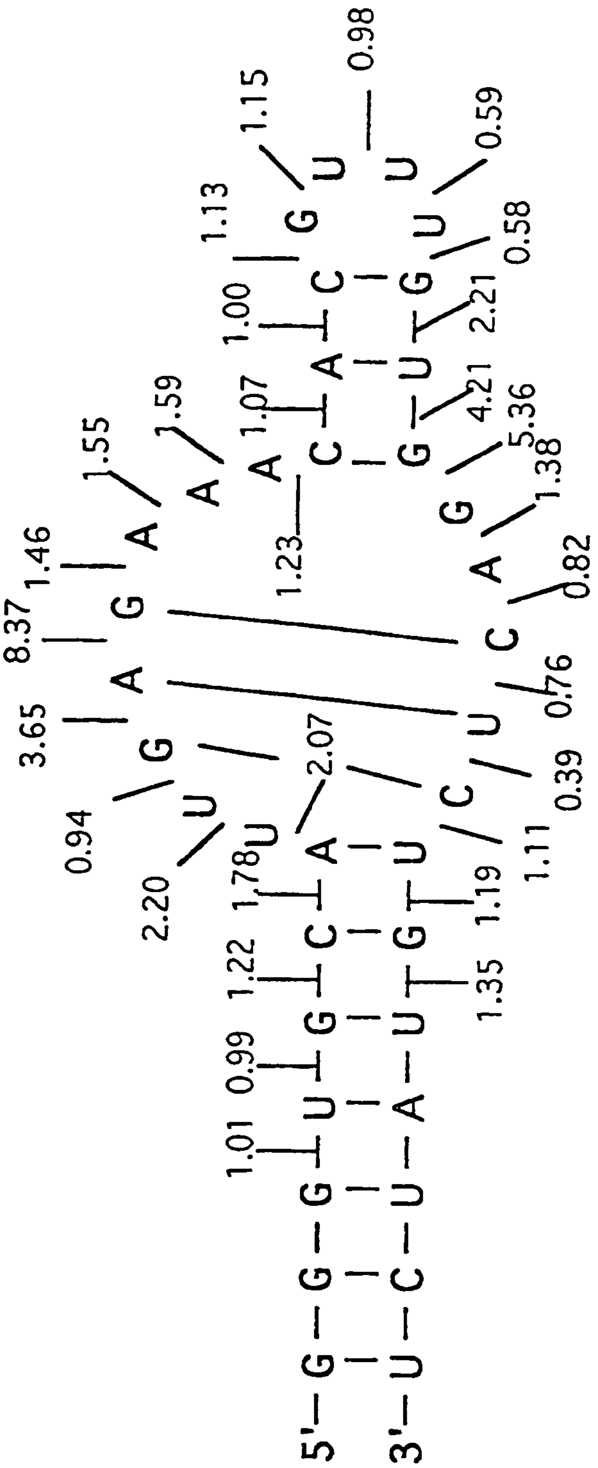
5'
3'
0.98
1.15
0.59
1.13
0.58
1.00
2.21
1.59
1.07
4.21
5.36
1.55
1.38
1.46
1.23
0.82
8.37
0.76
3.65
2.07
0.39
0.94
1.11
2.20
1.78
1.19
1.22
1.35
0.99
1.01

Fig. 15

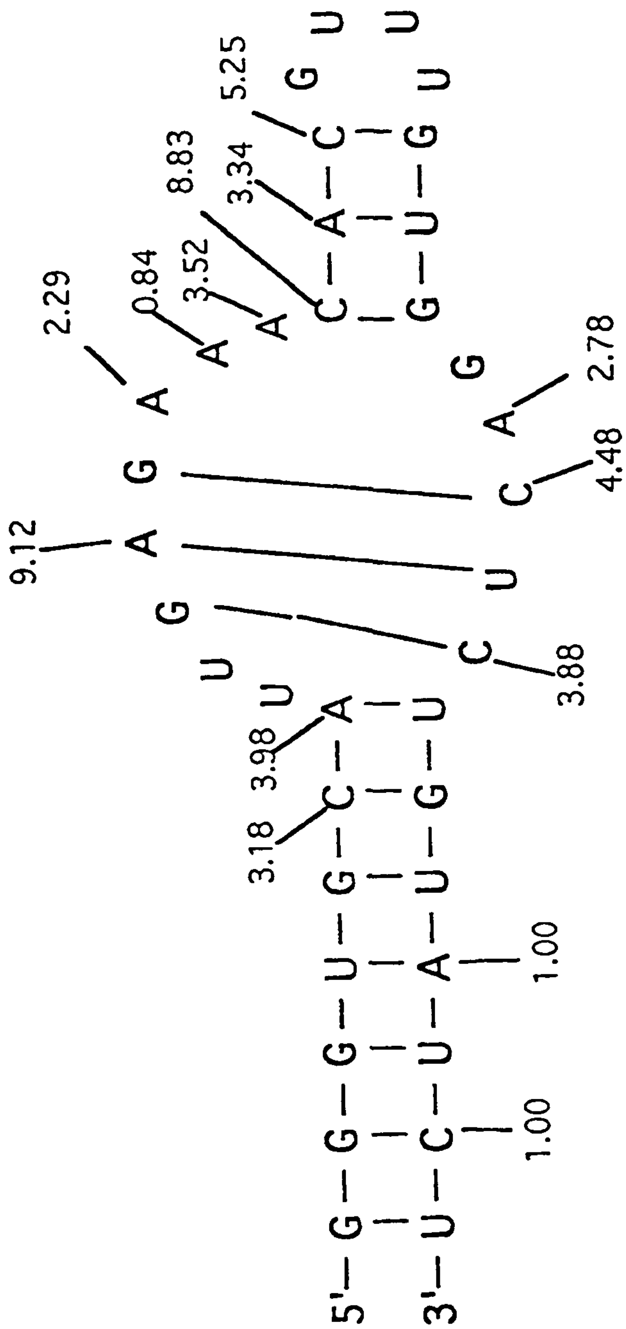


Fig. 16

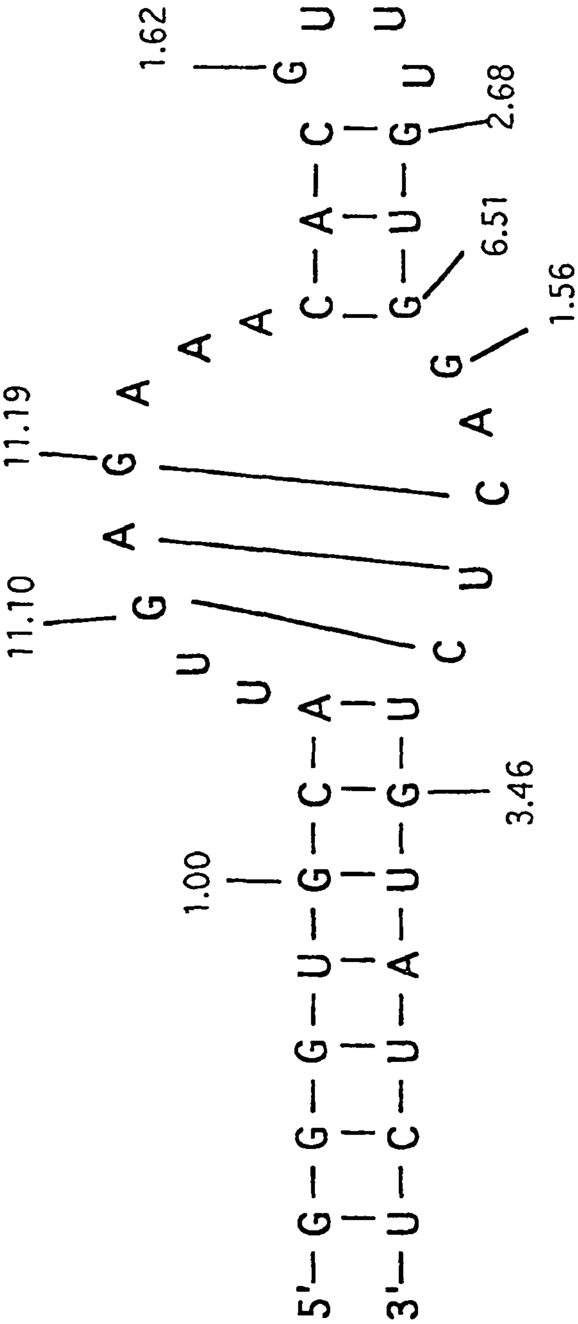


Fig. 17

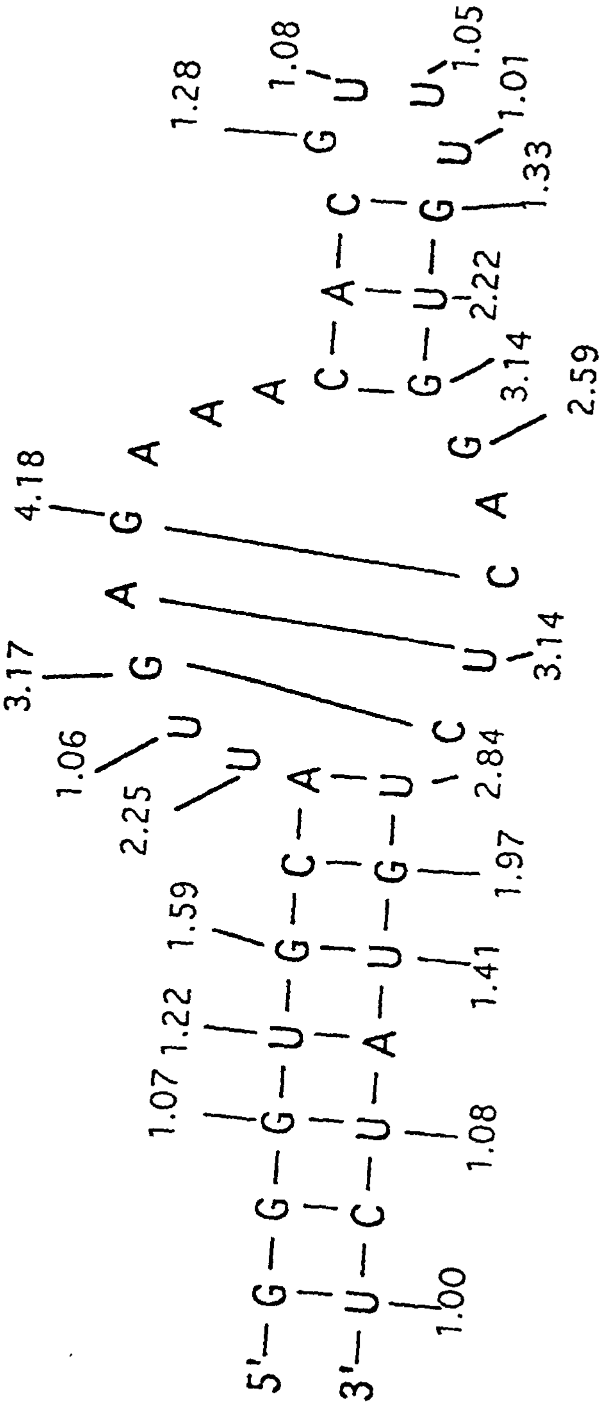
5'
3'
1.00
1.08
1.07
1.22
1.59
1.41
1.97
2.25
1.06
2.84
3.17
3.14
4.18
1.28
1.08
1.05
1.01
1.33
2.22
3.14
2.59

Fig. 18

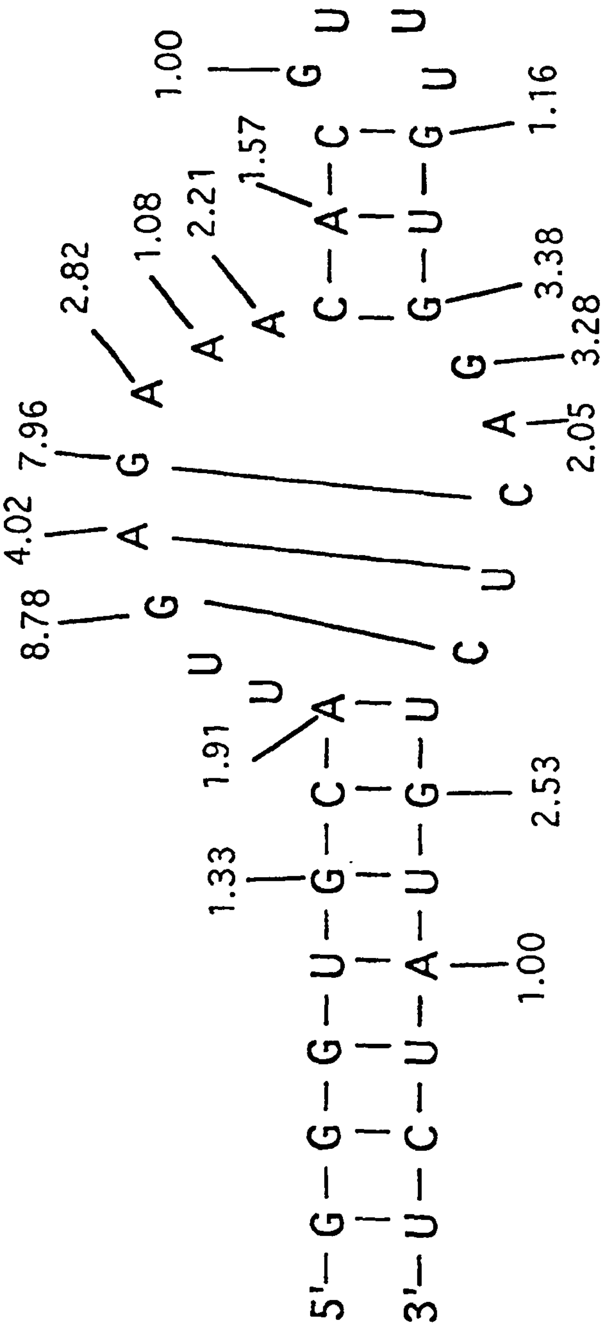
5'—G—G—G—U—G—C—A
3'—U—C—U—A—U—G—U
8.78
4.02
7.96
2.82
1.08
2.21
1.57
1.00
1.16
3.38
3.28
2.05
1.91
1.33
2.53
1.00

Fig. 19

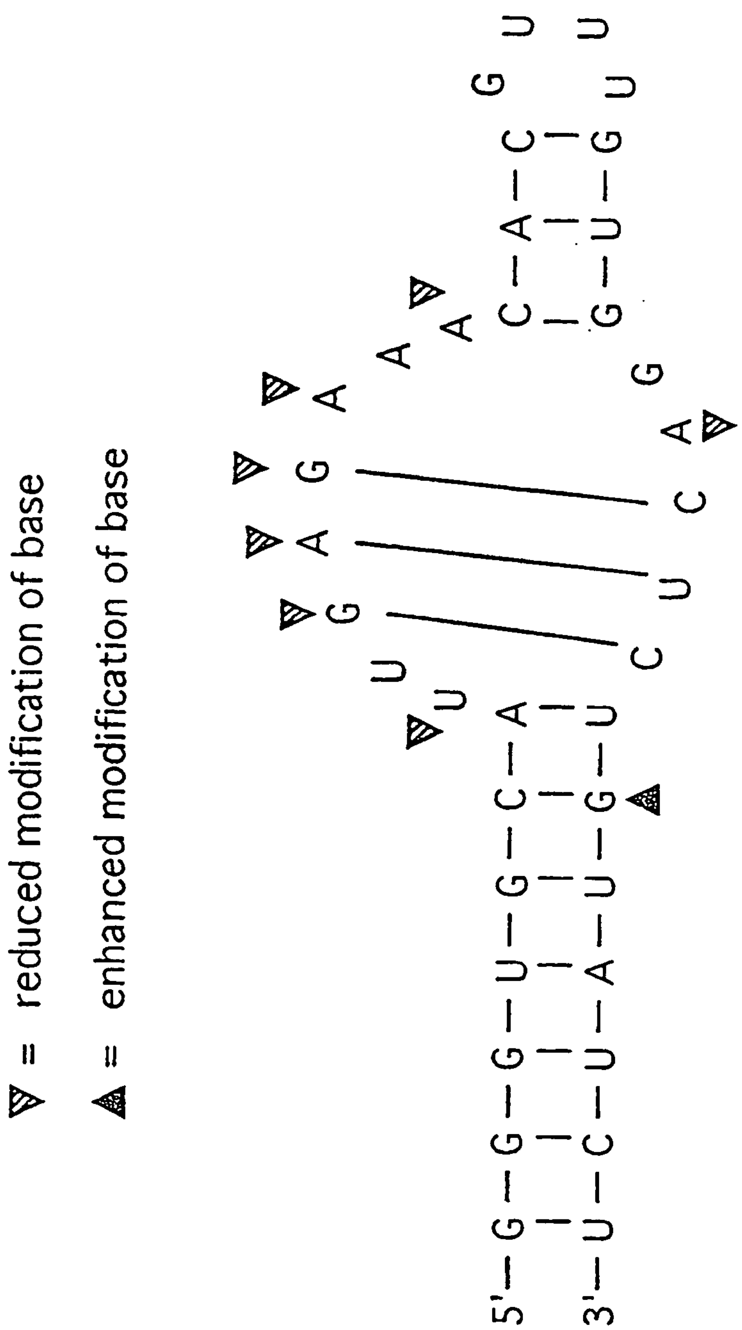
= reduced modification of base
= enhanced modification of base
5'
3'

Fig. 20

5'-GGGACUAUUGAUGGCCUUCCGACC-6a-CACACAGAGGUAAAGAGGAUCCGGG-3'

Biased ligand sequences

| | |
|---|---|
| 6a | GGGUGCAUUGAGAAACACGU-UUGUGGACUCUGUAUCU |
| 1 | UGGUGCGUUGAGAAACAGGU-UUUUGGACUCCGUACCa |
| 2 | GUAUGCAUUGAGAGACACAC-UUGUGGACUCUGCAUCC |
| 3 | AGAUGGAUUGAGAAACACUA-UUAUGGACUCUCCAUCG |
| 4 | AGCUUCGUCGAGAUACACGU-UGAUGGACUCCGAAGCA |
| 5 | UCGUACGUUGAGAAACAAGU-UUAUGGACUCCGUACCU |
| 6 | UCGAUCGUUGAGAUACACGC-UAGUGGACUCCGAAACU |
| 8 | UACUGCAUCGAGAUACACGU-UUGUGGACUCUGCACAU |
| 9 | UGAUACGUUGAGAAACACAA-UGCUGGACUCCGCAUCC |
| 10 | GCCUGCAUUGAGAAACAGGA-UUCUGGACUCUGCCACU |
| 12 | CGCUAUGUUGAGAAACACUU-UGCUGGACUCCGUAGCU |
| 13 | UACUGCAUCGAGAAACACGU-AAGUG-ACUCUGCAUCC |
| 15 | CGGUACGUCGAGAUACACGA-AGAUGGACUCCGUAUCG |

Fig. 21A

17 AACUCCAUCGAGAAACACGA-UAGUGGACUCUGGAGCU

18 GGAGACGUCGAGAAACACGU-UUGUGGACUCCGUCUCU

21 AGCUACAUCGAGAAACAAGA-UUUUGGACUCUGUAGCG

23 AAGUGCAUUGAGAUACAAAU-GAUUGGACUCUGCAcac

24 UGCUACGUUGAGAUACACGU-UGAUGCACUCCGUAGCU

25 AGCUACGUUGAGAUACACGUUACGUGG-CUCCGUAGCC

27 GAGUGGCUCGAGAAACAGGU-UGCUGGACUCGCCACAU

28 UCGUGCGUCGAGCAACACGU-UGAUGGACUCCGCACAG

29 GGCACCGUUGAGAAACACAU-GCGUGGACUCCGUGCCC

30 UCCUGCAUUGAGAAACAGUG-AUCUGGACUCUGCAACU

31 cUGUGGAUUGAGCAACACGU-GAGUGGACUCUCCACAU

32 CCGUGCGUUGAGACACACAC-CGAUGGACUCCGCAUGU

33 AGCUGCAUCGAGAUACACGA-UUGUGGACUCUGCAGCC

Fig. 21B

| | |
|---|---|
| 35 | AGAUUCGUUGAGAAACACAU-GGGUGGACUCUCCCGCUA |
| 36 | AGAUGGAUUGAGAAACACGU-UCGUGGACUCUCCAACU |
| 37 | GACUGCAUCGAGAAACACUG-AUGUGGCCUCCGCACGG |
| 38 | AGCUACGUUGAGAAACAGUA-UAAUGGACUCCGUAGCU |
| 40 | GAGUGCGUCGAGAAACACAU-UUGUGGACUCCGCACAC |
| 42 | UCGUACGUUGAGAAACACGC-UAGUGGACUCCGUAUGU |
| 43 | AGAUACGUUGAGAGACACGC-ACGUGGACUCCGUAUCU |
| 44 | AGGAUCACAGAGAAACACCGUGGGUGG-CUCCCUCUAU |
| 45 | GUGCGCAUCGAGAAACACGU-UGAUGGACUCUGCAUGCAC |
| 47 | GAGAGGAUCGAGAAACACGU-AUGUGGACUCUCCAUCU |
| 48 | GGAUGGAUUGAGACACACGU-AUGUGGACUCUCCAUCA |
| 49 | UCGGGCAUUGAGAUACACGU-AGAUGGACUCUGUCUCA |
| 50 | UGGACCGUAGAGAAACACGUUUGAUGG-CUCCCUCUGU |

Fig. 21C

| 6a | G | G | G | U | G | C | A | U | U | G | A | G | A | A | A | C | A | C |
|---|---|---|---|---|---|---|---|---|---|---|---|---|---|---|---|---|---|---|
| wt | 10 | 19 | 16 | 30 | 20 | 31 | 18 | 37 | 22 | 38 | 38 | 38 | 36 | 25 | 38 | 38 | 38 | 30 |
| Δ | 0 | 1 | 0 | 0 | 0 | 0 | 0 | 0 | 0 | 0 | 0 | 0 | 0 | 0 | 0 | 0 | 0 | 0 |
| A | 12 | 8 | 8 | 5 | 11 | 0 | 18 | 0 | 2 | 0 | 38 | 0 | 36 | 25 | 38 | 0 | 38 | 3 |
| C | 3 | 8 | 14 | 1 | 3 | 31 | 1 | 1 | 14 | 0 | 0 | 0 | 2 | 2 | 0 | 38 | 0 | 30 |
| G | 10 | 19 | 16 | 2 | 20 | 4 | 19 | 0 | 0 | 38 | 0 | 38 | 0 | 2 | 0 | 0 | 0 | 5 |
| U | 19 | 2 | 0 | 30 | 4 | 1 | 0 | 37 | 22 | 0 | 0 | 0 | 0 | 9 | 0 | 0 | 0 | 0 |
| bp | 16 | 20 | 26 | 31 | 33 | 35 | 35 | | | 38 | 38 | 38 | | | | 38 | 38 | 26 |

Fig. 22A

| 6a | G | G | G | U | G | C | A | U | U | G | A | G | A | A | A | C | A | C |
|---|---|---|---|---|---|---|---|---|---|---|---|---|---|---|---|---|---|---|
| wt | .26 | .50 | .42 | .79 | .53 | .82 | .47 | .97 | .58 | 1.00 | 1.00 | 1.00 | .95 | .66 | 1.00 | 1.00 | 1.00 | .79 |
| D | .00 | .03 | .00 | .00 | .00 | .00 | .00 | .00 | .00 | .00 | .00 | .00 | .00 | .00 | .00 | .00 | .00 | .00 |
| A | .32 | .21 | .21 | .13 | .29 | .00 | .47 | .00 | .05 | .00 | 1.00 | .00 | .95 | .66 | 1.00 | .00 | 1.00 | .08 |
| C | .08 | .21 | .37 | .03 | .08 | .82 | .03 | .03 | .37 | .00 | .00 | .00 | .05 | .05 | .00 | 1.00 | .00 | .79 |
| G | .26 | .50 | .42 | .05 | .53 | .11 | .50 | .00 | .00 | 1.00 | .00 | 1.00 | .00 | .05 | .00 | .00 | .00 | .13 |
| U | .50 | .05 | .00 | .79 | .11 | .03 | .00 | .97 | .58 | .00 | .00 | .00 | .00 | .24 | .00 | .00 | .00 | .00 |
| bp | .42 | .53 | .68 | .82 | .87 | .92 | .92 | .00 | .00 | 1.00 | 1.00 | 1.00 | .00 | .00 | .00 | 1.00 | 1.00 | .68 |

Fig. 22B

| 6a | G | G | G | U | G | C | A | U | U | G | A | G | A | A | A | C | A | C |
|---|---|---|---|---|---|---|---|---|---|---|---|---|---|---|---|---|---|---|
| wt | -.37 | -.13 | -.21 | .16 | -.10 | .19 | -.16 | .34 | -.05 | .37 | .37 | .37 | .32 | .03 | .37 | .37 | .37 | .16 |
| A | .19 | .09 | .09 | .01 | .16 | -.13 | -.16 | -.13 | -.07 | -.13 | .37 | -.13 | .32 | .03 | .37 | -.13 | .37 | -.05 |
| C | -.05 | .09 | .24 | -.09 | -.05 | .19 | -.10 | -.10 | .24 | -.13 | -.13 | -.13 | -.07 | -.07 | -.13 | .37 | -.13 | .16 |
| G | -.37 | -.13 | -.21 | -.07 | -.10 | -.02 | .38 | -.13 | -.13 | .37 | -.13 | .37 | -.13 | -.07 | -.13 | -.13 | -.13 | .01 |
| U | .38 | -.07 | -.13 | .16 | -.02 | -.10 | -.13 | .34 | -.05 | -.13 | -.13 | -.13 | -.13 | .11 | -.13 | -.13 | -.13 | -.13 |

Fig. 22C

| G | U | space | U | U | G | U | G | G | A | C | U | C | U | G | U | A | U | C | U |
|---|---|---|---|---|---|---|---|---|---|---|---|---|---|---|---|---|---|---|---|
| 25 | 22 | 35 | 23 | 14 | 19 | 38 | 38 | 37 | 36 | 38 | 38 | 38 | 17 | 28 | 15 | 31 | 15 | 26 | 19 |
| 0 | 0 | 35 | 0 | 0 | 0 | 0 | 0 | 1 | 3 | 0 | 0 | 0 | 0 | 0 | 0 | 0 | 0 | 0 | 0 |
| 7 | 8 | 0 | 9 | 7 | 11 | 0 | 0 | 0 | 36 | 0 | 0 | 0 | 0 | 0 | 2 | 31 | 4 | 7 | 3 |
| 1 | 5 | 0 | 1 | 4 | 5 | 0 | 0 | 0 | 0 | 38 | 0 | 38 | 20 | 9 | 20 | 6 | 10 | 26 | 11 |
| 25 | 3 | 0 | 5 | 13 | 19 | 0 | 38 | 37 | 0 | 0 | 0 | 0 | 1 | 28 | 1 | 1 | 9 | 5 | 5 |
| 5 | 22 | 3 | 23 | 14 | 3 | 38 | 0 | 0 | 0 | 0 | 38 | 0 | 17 | 0 | 15 | 0 | 15 | 0 | 19 |
| 18 | | | | 18 | 26 | 38 | 38 | | | 38 | 38 | 38 | 35 | 35 | 33 | 31 | 26 | 20 | 16 |

Fig. 22D

| G | U | space | U | U | G | U | G | G | A | C | U | C | U | G | U | A | U | C | U |
|---|---|---|---|---|---|---|---|---|---|---|---|---|---|---|---|---|---|---|---|
| .66 | .58 | .92 | .61 | .37 | .50 | 1.00 | 1.00 | .97 | .95 | 1.00 | 1.00 | 1.00 | .45 | .74 | .39 | .82 | .39 | .68 | .50 |
| .00 | .00 | .92 | .00 | .00 | .00 | .00 | .00 | .03 | .08 | .00 | .00 | .00 | .00 | .00 | .00 | .00 | .00 | .00 | .00 |
| .18 | .21 | .00 | .24 | .18 | .29 | .00 | .00 | .00 | .95 | .00 | .00 | .00 | .00 | .00 | .05 | .82 | .11 | .18 | .08 |
| .03 | .13 | .00 | .03 | .11 | .13 | .00 | .00 | .00 | .00 | 1.00 | .00 | 1.00 | .53 | .24 | .53 | .16 | .26 | .68 | .29 |
| .66 | .08 | .00 | .13 | .34 | .50 | .00 | 1.00 | .97 | .00 | .00 | .00 | .00 | .03 | .74 | .03 | .03 | .24 | .13 | .13 |
| .13 | .58 | .08 | .61 | .37 | .08 | 1.00 | .00 | .00 | .00 | .00 | 1.00 | .00 | .45 | .00 | .39 | .00 | .39 | .00 | .50 |
| .47 | .00 | .00 | .00 | .47 | .68 | 1.00 | 1.00 | .00 | .00 | 1.00 | 1.00 | 1.00 | .92 | .92 | .87 | .82 | .68 | .53 | .42 |

Fig. 22E

| G | U | space | U | U | G | U | G | G | A | C | U | C | U | G | U | A | U | C | U |
|---|---|---|---|---|---|---|---|---|---|---|---|---|---|---|---|---|---|---|---|
| .03 | -.05 | | -.02 | -.26 | -.13 | .37 | .37 | .34 | .32 | .37 | .37 | .37 | -.18 | .11 | -.24 | .19 | -.24 | .05 | -.13 |
| .06 | .09 | | .11 | .06 | .16 | -.13 | -.13 | -.13 | .32 | -.13 | -.13 | -.13 | -.13 | -.13 | -.07 | .19 | -.02 | .06 | -.05 |
| -.10 | .01 | | -.10 | -.02 | .01 | -.13 | -.13 | -.13 | -.13 | .37 | -.13 | .37 | .40 | .11 | .40 | .03 | .14 | .05 | .16 |
| .03 | -.05 | | .01 | .22 | -.13 | -.13 | .37 | .34 | -.13 | -.13 | -.13 | -.13 | -.10 | .11 | -.10 | -.10 | .11 | .01 | .01 |
| .01 | -.05 | | -.02 | .26 | -.05 | .37 | -.13 | -.13 | -.13 | -.13 | .37 | -.13 | .11 | -.13 | -.24 | -.13 | -.24 | -.13 | -.13 |

Fig. 22F

| 6a | G1/U37 | G2/C36 | G3/U35 | U4/A34 | G5/U33 | C6/G32 | A7/U31 | | C16/G25 | A17/U24 | C18/G23 | G19/U22 |
|---|---|---|---|---|---|---|---|---|---|---|---|---|
| WT | 2 | 16 | 8 | 28 | 2 | 28 | 16 | | 38 | 38 | 19 | 9 |
| AU | 6 | 0 | 6 | 0 | 10 | 0 | 16 | | 0 | 38 | 0 | 2 |
| UA | 2 | 1 | 0 | 28 | 2 | 0 | 0 | | 0 | 0 | 2 | 1 |
| GC | 5 | 16 | 7 | 2 | 18 | 6 | 18 | | 0 | 0 | 3 | 3 |
| CG | 1 | 2 | 8 | 0 | 1 | 28 | 1 | | 38 | 0 | 19 | 1 |
| GU | 1 | 1 | 8 | 0 | 2 | 0 | 1 | | 0 | 0 | 1 | 9 |
| UG | 4 | 1 | 0 | 0 | 0 | 1 | 0 | | 0 | 0 | 0 | 0 |
| AC | 3 | 3 | 0 | 2 | 1 | 0 | 2 | | 0 | 0 | 0 | 1 |
| CA | 1 | 1 | 2 | 0 | 0 | 0 | 0 | | 0 | 0 | 9 | 0 |
| other | 16 | 13 | 7 | 8 | 2 | 3 | 0 | | 0 | 0 | 4 | 14 |

Fig. 23A

| 6a | G1/U37 | G2/C36 | G3/U35 | U4/A34 | G5/U33 | C6/G32 | A7/U31 | | C16/G25 | A17/U24 | C18/G23 | G19/U22 |
|---|---|---|---|---|---|---|---|---|---|---|---|---|
| WT | .05 | .42 | .21 | .74 | .05 | .74 | .42 | | 1.00 | 1.00 | .50 | .24 |
| AU | .16 | .00 | .16 | .00 | .26 | .00 | .42 | | .00 | 1.00 | .00 | .05 |
| UA | .05 | .03 | .00 | .74 | .05 | .00 | .00 | | .00 | .00 | .05 | .03 |
| GC | .13 | .42 | .18 | .05 | .47 | .16 | .47 | | .00 | .00 | .08 | .08 |
| CG | .03 | .05 | .21 | .00 | .03 | .74 | .03 | | 1.00 | .00 | .50 | .03 |
| GU | .03 | .03 | .21 | .00 | .05 | .00 | .03 | | .00 | .00 | .03 | .24 |
| UG | .11 | .03 | .00 | .00 | .00 | .03 | .00 | | .00 | .00 | .00 | .00 |
| AC | .08 | .08 | .00 | .05 | .03 | .00 | .05 | | .00 | .00 | .00 | .03 |
| CA | .03 | .03 | .05 | .00 | .00 | .00 | .00 | | .00 | .00 | .24 | .00 |
| other | .42 | .34 | .18 | .21 | .05 | .08 | .00 | | .00 | .00 | .11 | .37 |

Fig. 23B

| 6a | G1/U37 | G2/C36 | G3/U35 | U4/A34 | G5/U33 | C6/G32 | A7/U31 | | C16/G25 | A17/U24 | C18/G23 | G19/U22 |
|---|---|---|---|---|---|---|---|---|---|---|---|---|
| WT | -.34 | .03 | -.18 | .35 | -.34 | .35 | .03 | | .61 | .61 | .11 | -.15 |
| AU | .08 | -.02 | .08 | -.02 | .19 | -.02 | .03 | | -.02 | .61 | -.02 | -.03 |
| UA | .04 | .01 | -.02 | .35 | .04 | -.02 | -.02 | | -.02 | -.02 | .04 | .01 |
| GC | .05 | .03 | .11 | .04 | .40 | .14 | .46 | | -.02 | -.02 | .06 | .00 |
| CG | .01 | .04 | .19 | -.02 | .01 | .35 | .01 | | .61 | -.02 | .11 | .01 |
| GU | -.34 | -.05 | -.18 | -.02 | -.34 | -.02 | -.05 | | -.02 | -.08 | .01 | -.15 |
| UG | .09 | .01 | -.02 | -.08 | -.02 | -.05 | -.02 | | -.08 | -.02 | -.08 | -.02 |
| AC | .06 | .00 | -.02 | .04 | .01 | -.02 | -.03 | | -.02 | -.08 | -.02 | .01 |
| CA | .01 | .01 | .04 | -.08 | -.02 | -.08 | -.02 | | -.08 | -.02 | .16 | -.02 |
| other | .17 | .09 | -.07 | -.04 | -.20 | -.17 | -.25 | | -.25 | -.25 | -.15 | .12 |

Fig. 23C

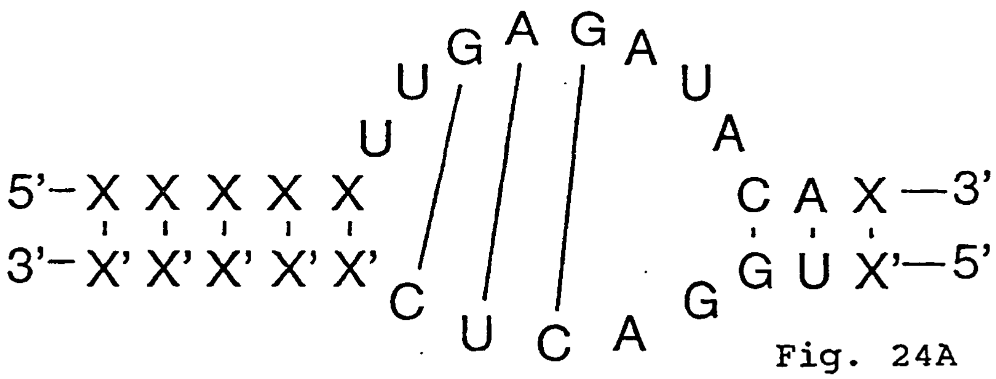


Fig. 24A

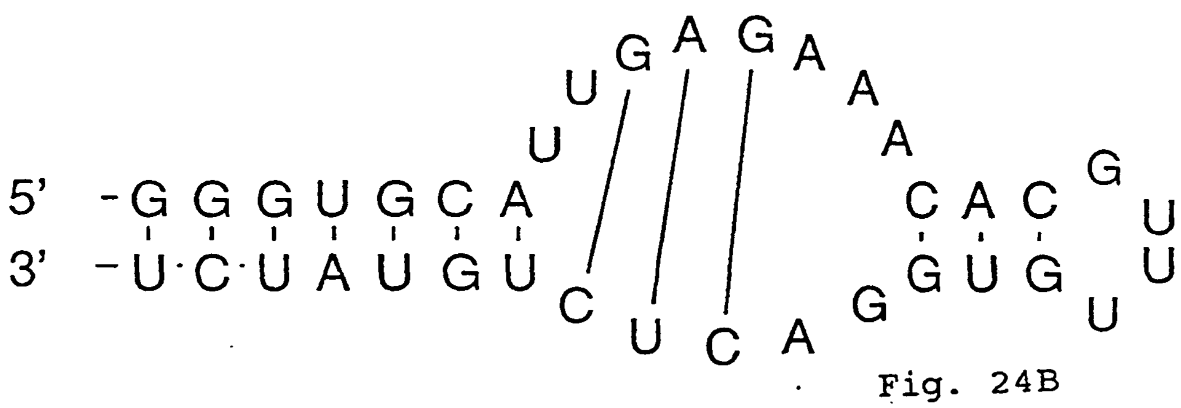


Fig. 24B

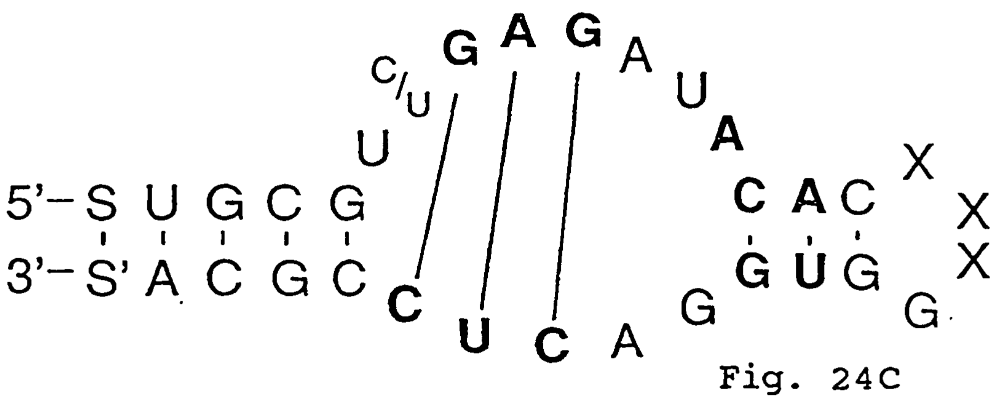


Fig. 24C

# TAR RNA

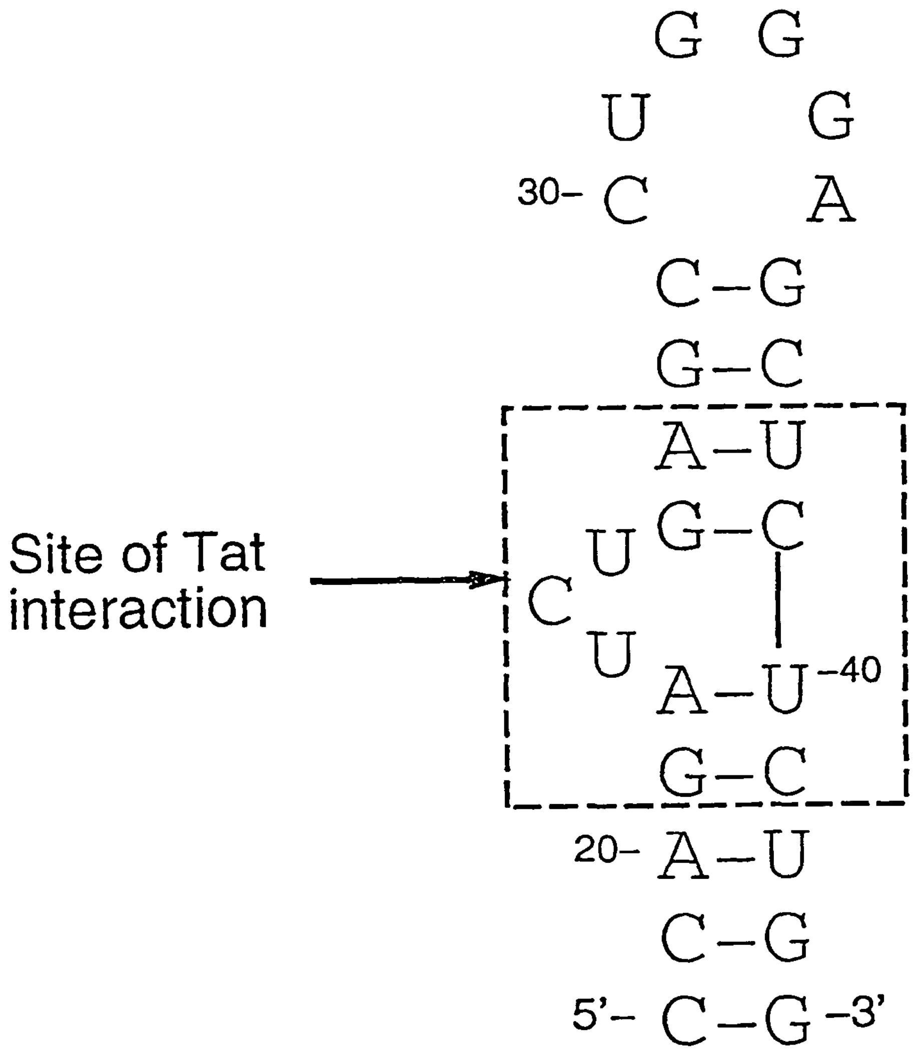


Fig. 25

Tat Ligand Sequences

| Isolate Number | | Tat ligand motif |
|---|---|---|
| 4,12,16 | aaGCCUCAGUAAGGCAACGGAAUCCGCA———AGAGGAUGGACCACuucgacaug | I |
| 7,9 | gcucaaCACGAAGGAAACGGAGGGAAUCUU–GAAGAACCCGGACCACuucgacaug | I |
| 5 | cucaaGCGGAAUGGAAACGGAGCCAUCAAC—AAGCUGGCGGACCACAuucgacau | I |
| 26 | ucaaCAAACGAAGGAAACGGAUGACCCAA——GCAGGUCAGGACCACuucgacaug | I |
| 19 | cgcucaaACGAAGGAAACGGAUGCGGACAUAUGUGCCGCAGGACCACuucgacaug | I |
| 21 | aaACACAUCGAAGGUAACGGAGCGAAAA———GAACGCGGACCACuucgacaug | I |
| 30 | aacgcucaaGCGGGCAACGGAGUCCUGA———ACGGACGGACCACCGCAAGAAu | I |

FIG. 26A

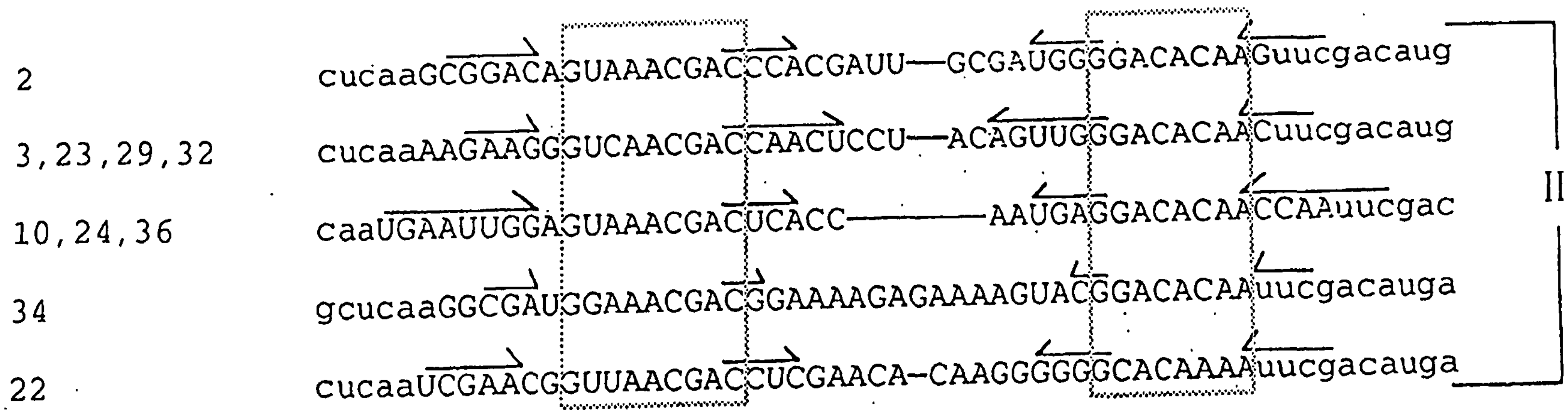
2 cucaaGCGGACAGUAAACGACCCACGAUU—GCGAUGGGGACACAAGuucgacaug
3,23,29,32 cucaaAAGAAGGGUCAACGACCAACUCCU—ACAGUUGGGACACAACuucgacaug
10,24,36 caaUGAAUUGGAGUAAACGACUCACC——AAUGAGGACACAACCAAuucgac
34 gcucaaGGCGAUGGAAACGACGGAAAAGAGAAAAGUACGGACACAAuucgacauga
22 cucaaUCGAACGGUUAACGACCUCGAACA-CAAGGGGGGCACAAAAuucgacauga
II

FIG. 26B

15 aaGAGAAUCUAACGAGACGUAAGCCGUCGGAGAAUGAGACGAuucgacaugaggc

14,25,28 aGUGAGGACUAAUGAGGCGUACGA—CCGGAGAUUGAGACGUCuucgacaugagg
31,33

III

17 cgcucaaGGCGACGGGACUGCAAGCAUGGAGCUAACGAGAAAAUUGCuucgaca

1 cgcucaaCCUGGAGAGGAUCGCUGGCGGGCUUGAUCCCCAGAUCAAAuucgaca

FIGURE.26C

Tat motif I

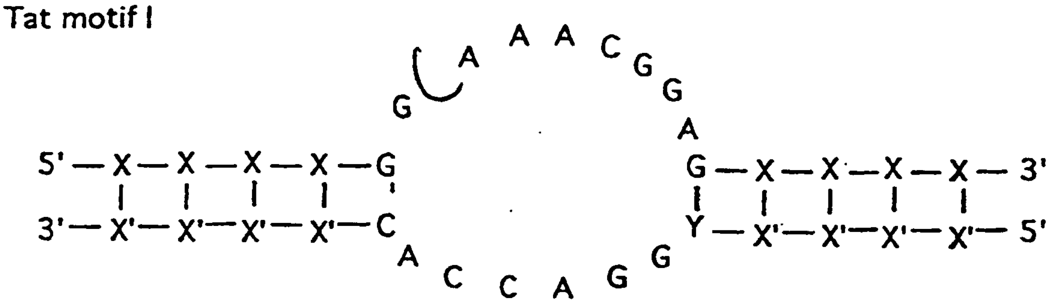

Fig. 27A

Tat motif II

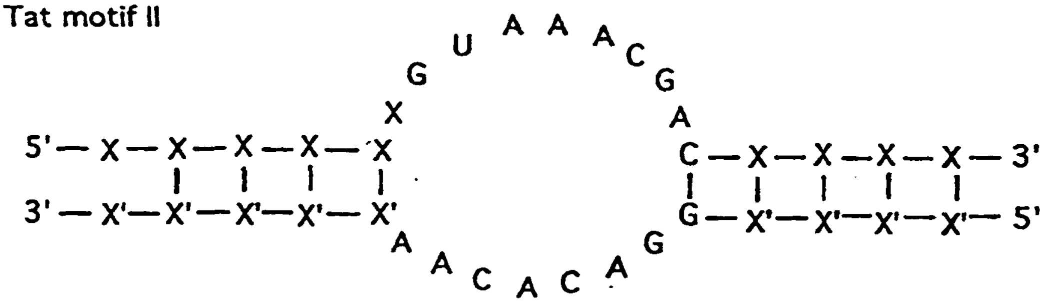

Fig. 27B

Tat motif III

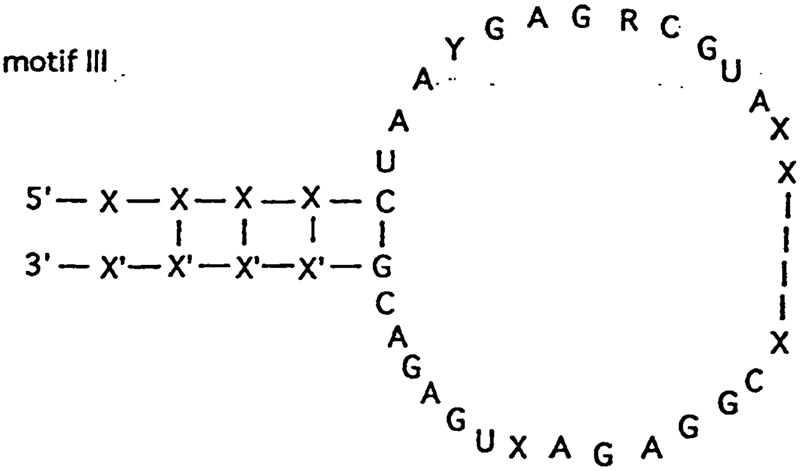

Fig. 27C

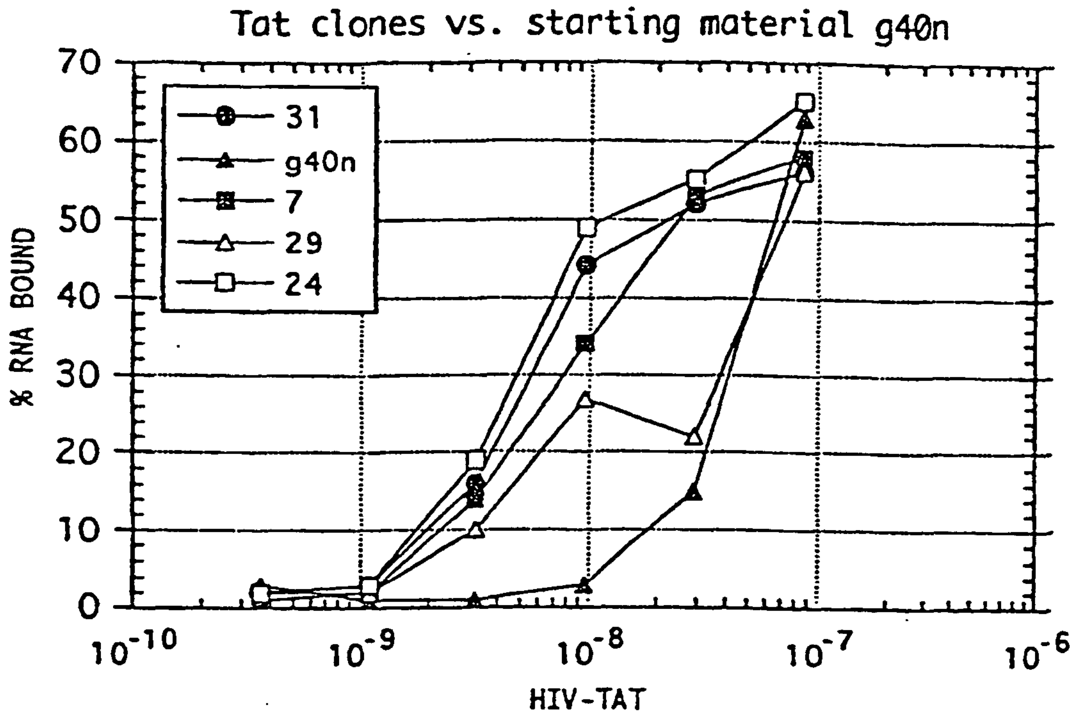
Tat clones vs. starting material g40n
70
60
50
40
30
20
10
0
% RNA BOUND
31
g40n
7
29
24
10-10
10-9
10-8
10-7
10-6
HIV-TAT

Fig. 28

| CLASS I | 1 | 2 | 3 | SEQ ID NO: |
|---|---|---|---|---|
| #1 | AGAUGCCUGUCGAGCAUGCUG | AGGAUCGAAGUUAGUAGGCUUUGUGUGCUC | GUAGCUAAACAGCUUUGUCGACGGG | 137 |
| #6 | AGAUGCCUGUCGAGCAUGCUG | AGGAUCGAAGUUAGUAGGCUUUGUGUGCUC | GUAGCUAAACAGCUUUGUCGACGGG | 137 |
| #13 | AGAUGCCUGUCGAGCAUGCUG | AGGAUCGAAGUUAGUAGGCUUUGUGUGCUC | GUAGCUAAACAGCUUUGUCGACGGG | 137 |
| #19 | AGAUGCCUGUCGAGCAUGCUG | AGGAUCGAAGUUAGUAGGCUUUGUGUGCUC | GUAGCUAAACAGCUUUGUCGACGGG | 137 |
| #23 | AGAUGCCUGUCGAGCAUGCUG | AGGAUCGAAGUUAGUAGGCUUUGUGUGCUC | GUAGCUAAACAGCUUUGUCGACGGG | 137 |
| #24 | AGAUGCCUGUCGAGCAUGCUG | AGGAUCGAAGUUAGUAGGCUUUGUGUGCUC | GUAGCUAAACAGCUUUGUCGACGGG | 137 |
| #25 | AGAUGCCUGUCGAGCAUGCUG | AGGAUCGAAGUUAGUAGGCUUUGUGUGCUC | GUAGCUAAACAGCUUUGUCGACGGG | 137 |
| #30 | AGAUGCCUGUCGAGCAUGCUG | AGGAUCGAAGUUAGUAGGCUUUGUGUGCUC | GUAGCUAAACAGCUUUGUCGACGGG | 137 |
| #2 | AGAUGCCUGUCGAGCAUGCUG | UACUGGAUCGAAGGUAGUAGGCAGUCAC | GUAGCUAAACAGCUUUGUCGACGGG | 138 |
| #5 | AGAUGCCUGUCGAGCAUGCUG | AUAUCACGGAUCGAAGGAAGUAGGCGUG | GUAGCUAAACAGCUUUGUCGACGGG | 139 |
| #9 | AGAUGCCUGUCGAGCAUGCUG | CCUUUCCCGGGUUCGAAGUCAGUAGGCCGG | GUAGCUAAACAGCUUUGUCGACGGG | 140 |
| #10 | AGAUGCCUGUCGAGCAUGCUG | CACCCGGAUCGAAGUUAGUAGGCGUGAGU | GUAGCUAAACAGCUUUGUCGACGGG | 141 |
| #15 | AGAUGCCUGUCGAGCAUGCUG | UGUACGGAUCGAAGGUAGUAGGCAGGUUAC | GUAGCUAAACAGCUUUGUCGACGGG | 142 |
| #16 | AGAUGCCUGUCGAGCAUGCUG | CAUCCGGAUCGAAGUUAGUAGGC[redacted] | GUAGCUAAACAGCUUUGUCGACGGG | 143 |
| #18 | AGAUGCCUGUCGAGCAUGCUG | AUUGUUGCGGAUCGAAGU[redacted] | GUAGCUAAACAGCUUUGUCGACGGG | 144 |
| #21 | AGAUGCCUGUCGAGCAUGCUG | UGUACUGGAUCGAAGGUAGUAGGCAGUCAC | GUAGCUAAACAGCUUUGUCGACGGG | 145 |
| #22 | AGAUGCCUGUCGAGCAUGCUG | AUCGAAGUUAGUAGGAGCGUGUG | GUAGCUAAACAGCUUUGUCGACGGG | 146 |
| #26 | AGAUGCCUGUCGAGCAUGCUG | ACGCUGGAGUCGGAUCGAAAGGUAAGUAGGCGACU | GUAGCUAAACAGCUUUGUCGACGGG | 147 |
| #31 | AGAUGCCUGUCGAGCAUGCUG | GGGUCGGAUCGAAAGGUAAGUAGGCGACU | GUAGCUAAACAGCUUUGUCGACGGG | 148 |
| #33 | AGAUGCCUGUCGAGCAUGCUG | AUAUCACGGAUCGAAAGAGAGUAGGCGU | GUAGCUAAACAGCUUUGUCGACGGG | 149 |
| #34 | AGAUGCCUGUCGAGCAUGCUG | UGUACUGGAUCGAAGGUAGUAGGCAGGCAC | GUAGCUAAACAGCUUUGUCGACGGG | 150 |
| #37 | AGAUGCCUGUCGDGCAUGCUG | AUAUCACGGAUCGAAGGAAAGUAGGCGUG | GUAGCUAAACAGCUUUGUCGACGGG | 151 |

Fig. 29A

CLASS II

| | 1 | 2 | 3 | SEQ ID |
|---|---|---|---|---|
| #3 | AGAUGCCUGUCGAGCAUGCUG | GU<u>GCGGCUUUGGGCGCCGUGCUU</u>GGC | GUAGCUAAACAGCUUUGUCGACGGG | 152 |
| #20 | AGAUGCCUGUCGAGCAUGCUG | GU<u>GCGGCUUUGGGCGCCGUGCUU</u>AC | GUAGCUAAACAGCUUUGUCGACGGG | 153 |
| #27 | AGAUGCCUGUCGAGCAUGCUG | GU<u>GCGGCUUUGGGCGCCGUGCUU</u>GAC | GUAGCUAAACAGCUUUGUCGACGGG | 154 |
| #35 | AGAUGCCUGUCGAGCAUGCUG | GG<u>GCGGCUUUGGGCGCCGUGCUU</u>GAC | GUAGCUAAACAGCUUUGUCGACGGG | 155 |
| #38 | AGAUGCCUGUCGAGCAUGCUG | GU<u>GCGGCUUUGGGCGCCGUGCUU</u>GAC | GUAGCUAAACAGCUUUGUCGACGGG | 154 |
| #39 | AGAUGCCUGUCGAGCAUGCUG | GU<u>GCGGCUUUGGGCGCCGUGCUU</u>GAC | GUAGCUAAACAGCUUUGUCGACGGG | 154 |

Fig. 29B

| CLONE | RANDOM REGION | SEQ ID NO: |
|---|---|---|
| 6 | gggagaugccuguc[g[agcaugcug AGGAUCGAAGUUAGUAGGCUUUGUGUGCU]C guagcuaaacagcuuugucgacggg | 23 |
| 16 | gggagaugccugucgagcau[gcug C[AU[CCGGAUCGAAGUUAGUAGGCCGAG]GUG guagcuaaacagcuuugucgacggg | 24 |
| 18 | gggagaugccugucgagcaugcug AUUGU[UGCGGAUCGAAGUGAGUAGGCGCUA] guagcuaaacagcuuugucgacggg | 25 |
| 27 | gggagaugccuguc[g[agcaugcug GUGCGGCUUUGGGCGCCGUGCUU]GAC guagcuaaacagcuuugucgacggg | 26 |

Fig. 30A

```
Clone 6                             Clone 16

          AGUUA                                   AGUUA
         A     G                                 A     G
        G       U                               G       U
        C  15   A                               C  15   A
        U       G                               U       G
         A     G                                 A     G
          G - C                                   G - C
          G - U                                   G - C
          A - U                                   C - G
          g - U                                        A
         u                                        C - G
          c - G                                   U - G
          g - U                                   A - U
          u - G                                        G
          a - U                                   C - g
          c - G                                   g - u
          g - C                                   u - a
          a - U                                   c - g
          g - C                                   g - c
         c                       5'-gggagaugccugucgagcau    uaaacagcuuugucgacggg-3'
          u - g
          g - u
          u - a
          c - g
5' gggagaugc     cuaaacagcuuugucgacggg-3'

acgu = fixed regions
ACGU = random region
ACGU = conserved region
---  = boundaries
```

Fig. 30B

**Clone 18**

```
               AGUGA
              A     G
             G       U
             C   16  A
             U       G
             A      G
              G
               G - C
               C - G
               G - C
                    U
               U - A
               U - g
               G - u
               U - a
               U - g
5'-gggagaugccugucgagcaugcugA   cuaaacagcuuugucgacggg-3'
```

acgu = fixed regions
ACGU = random region
**ACGU** = conserved region
--- = boundaries

Fig. 30C

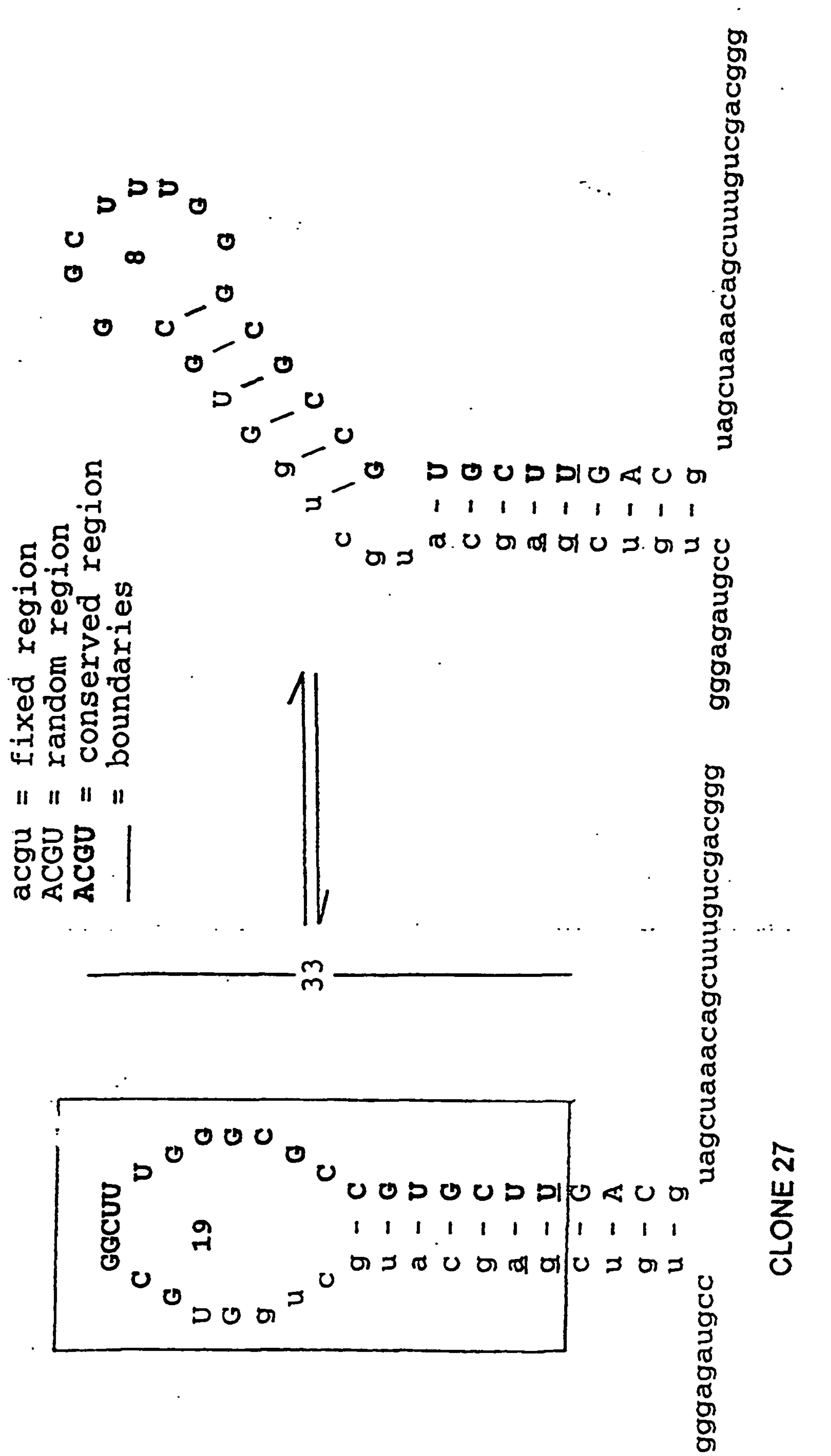
acgu = fixed region
ACGU = random region
ACGU = conserved region
—— = boundaries
33
gggagaugcc
uagcuaaacagcuuugucgacggg
CLONE 27

Fig. 30D

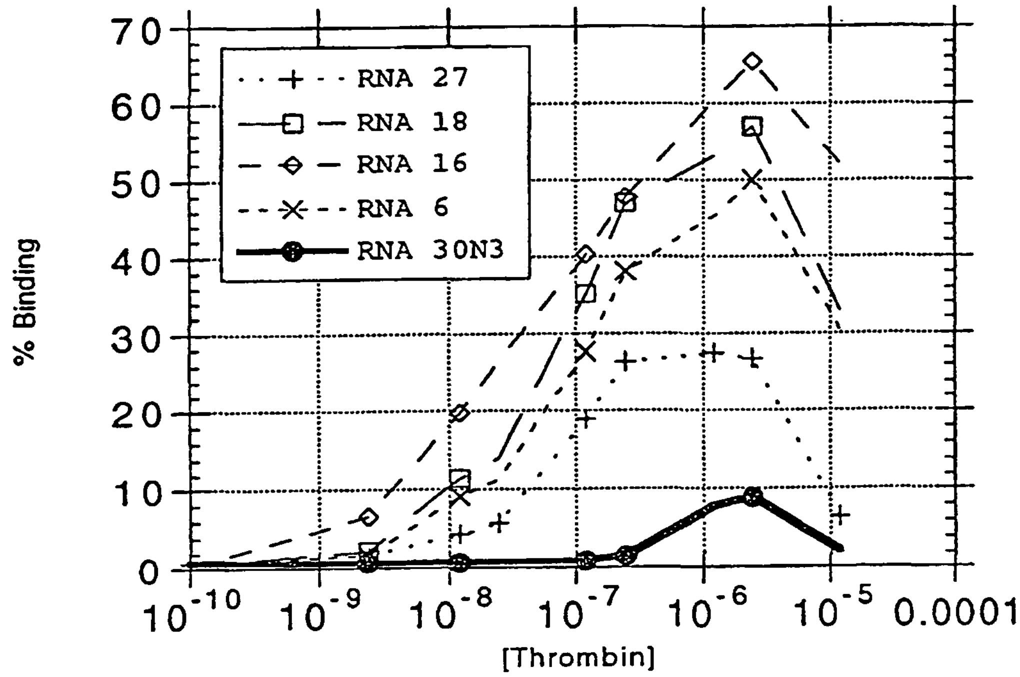


Fig. 31A

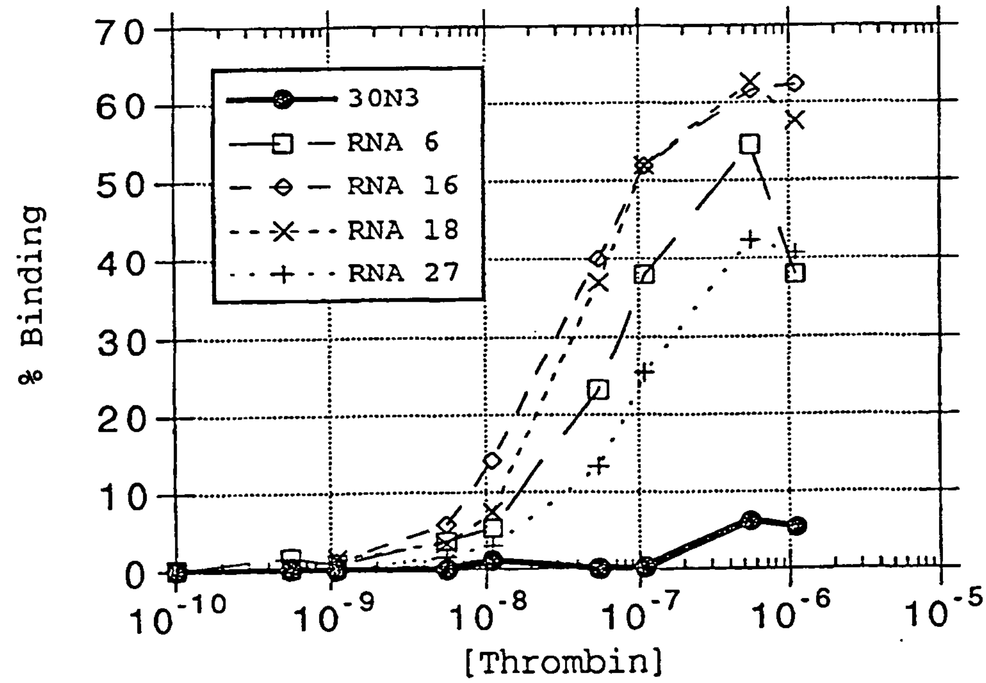


Fig. 31B

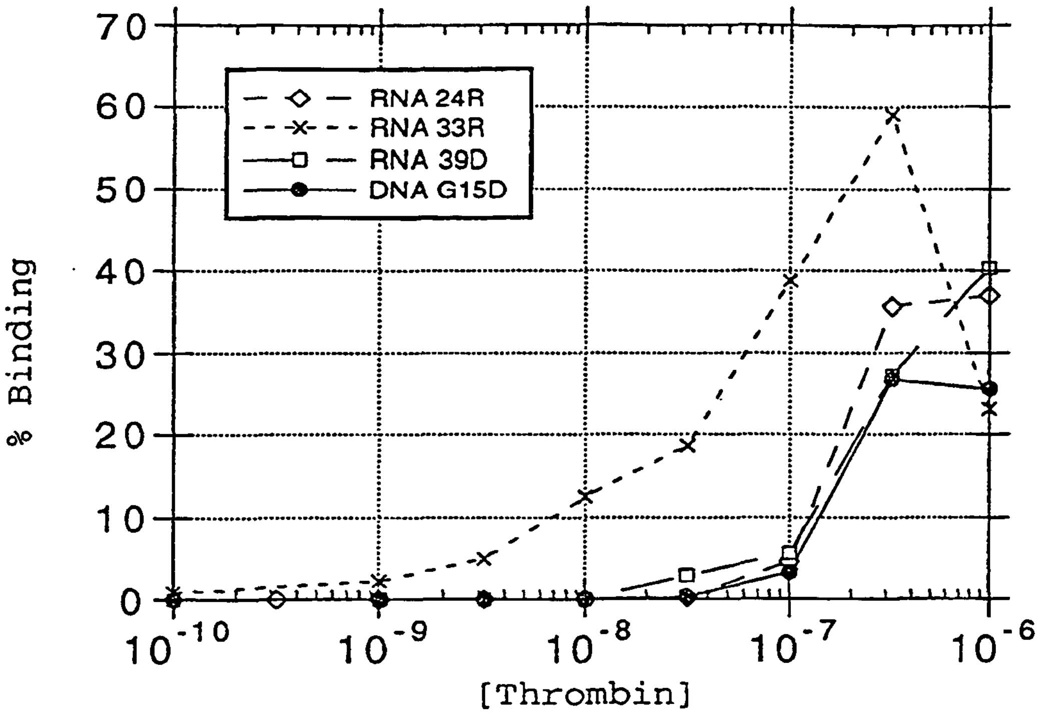
70
60
50
40
30
20
10
0
% Binding
RNA 24R
RNA 33R
RNA 39D
DNA G15D
$10^{-10}$
$10^{-9}$
$10^{-8}$
$10^{-7}$
$10^{-6}$
[Thrombin]

Fig. 31C

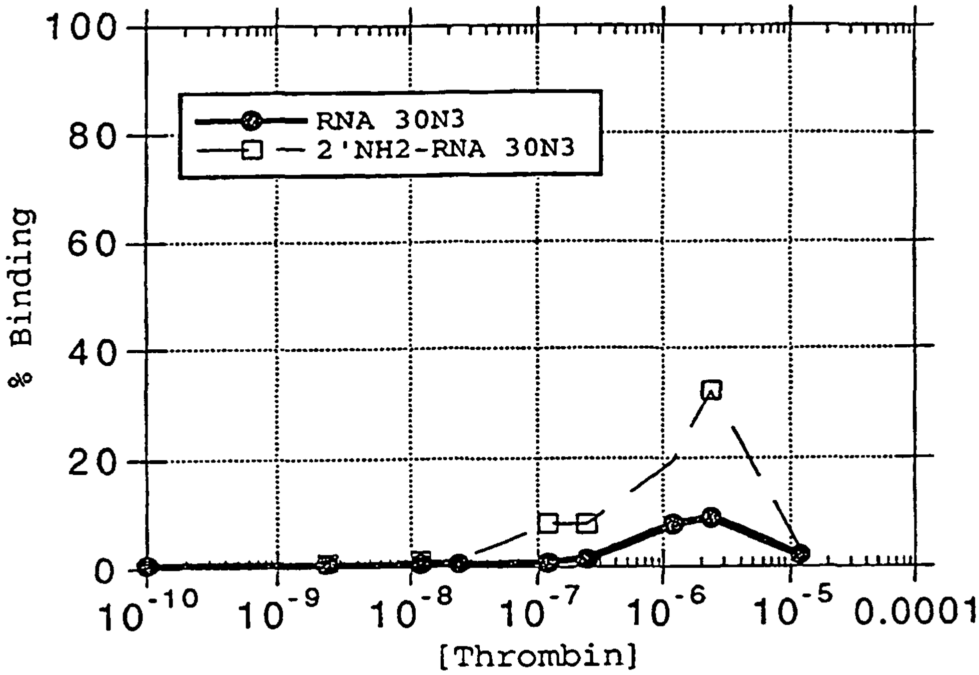


Fig. 32A

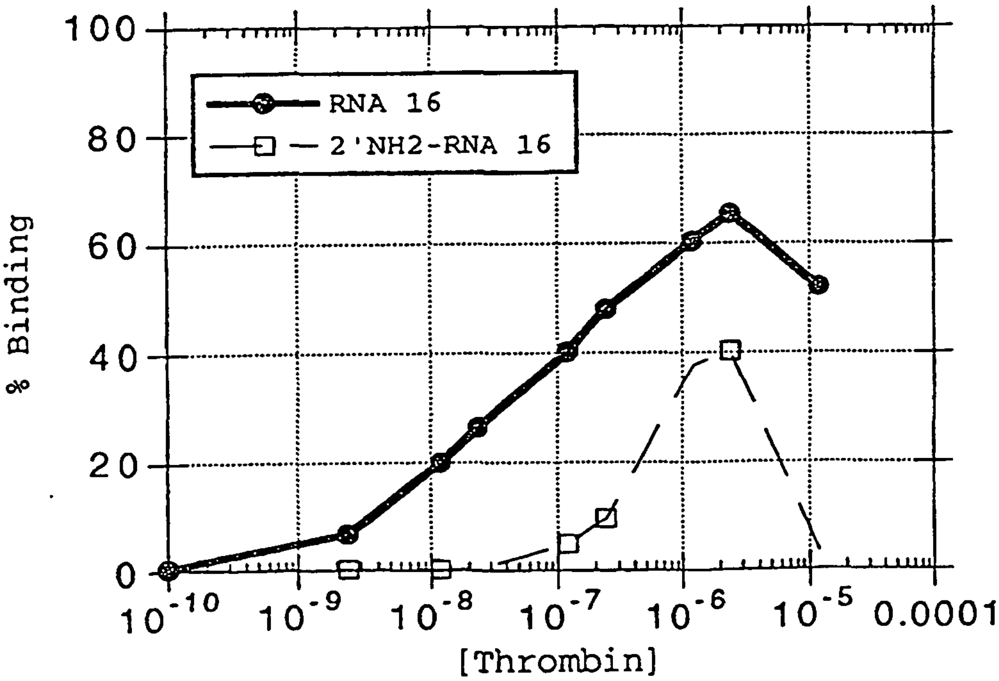


Fig. 32B

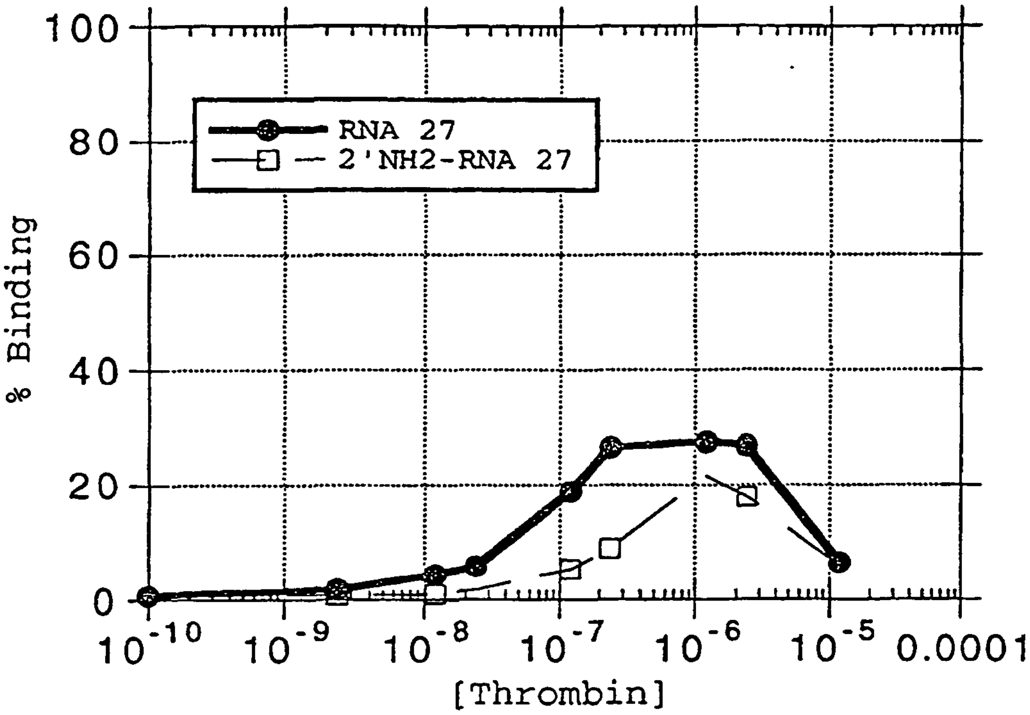
100
80
60
40
20
0
% Binding
RNA 27
2'NH2-RNA 27
10-10
10-9
10-8
10-7
10-6
10-5
0.0001
[Thrombin]

Fig. 32C

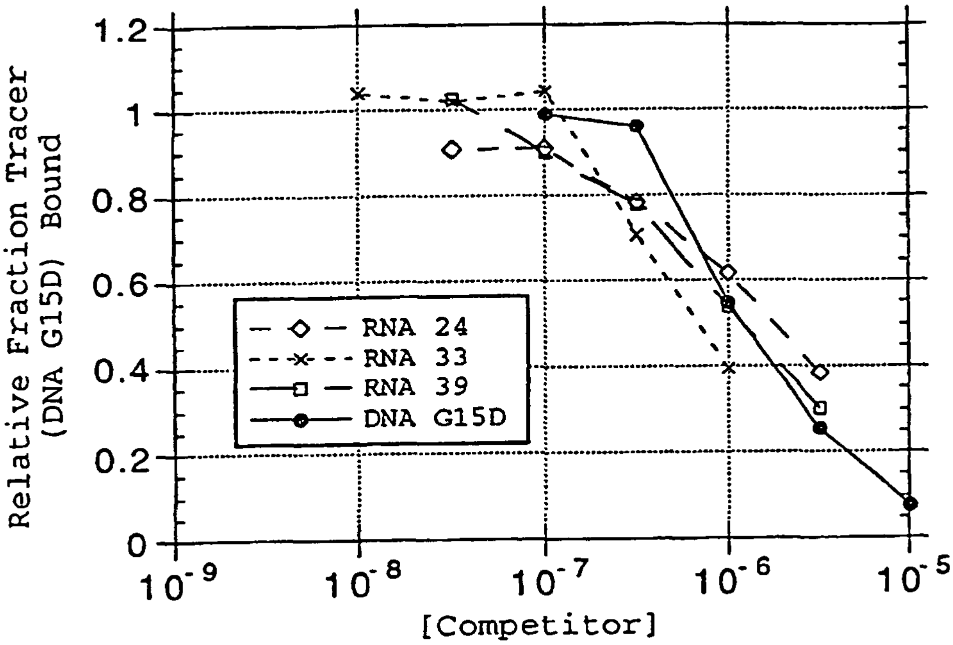


Fig. 33A

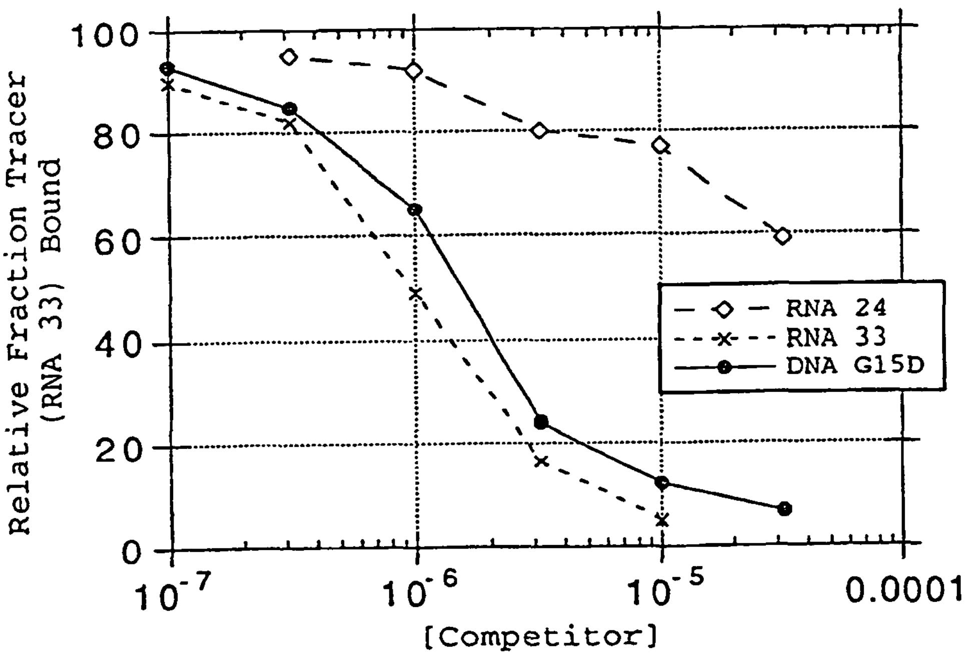


Fig. 33B

Thrombin
H-D-Phe-Pip-Arg-p-Nitroaniline + H2O ------> H-D-Phe-Pip-Arg-OH + p-Nitroaniline
(OD 405 indicates release of p-Nitroaniline)

| | [Thrombin] | [RNA] | Inhibition |
|---|---|---|---|
| Class I RNA 16 | $10^{-8}$M | $10^{-8}$M | - |
| | $10^{-8}$M | $10^{-7}$M | - |
| | $10^{-9}$M | $10^{-8}$M | - |
| Class II RNA 27 | 10-8M | 10-8M | - |
| | 10-8M | 10-7M | - |
| | 10-9M | 10-8M | - |

Fig. 34A

| Ligand plus purified human thrombin (2.5 nM) | Clotting time (sec) for purified fibrinogen (.25 mg/ml) |
|---|---|
| No RNA/DNA | 65 |
| Class I RNA 16 (30nM) | 117 |
| Class II RNA 27 (60nM) | 115 |
| DNA 15mer G15D | 270-330 |

Fig. 34B

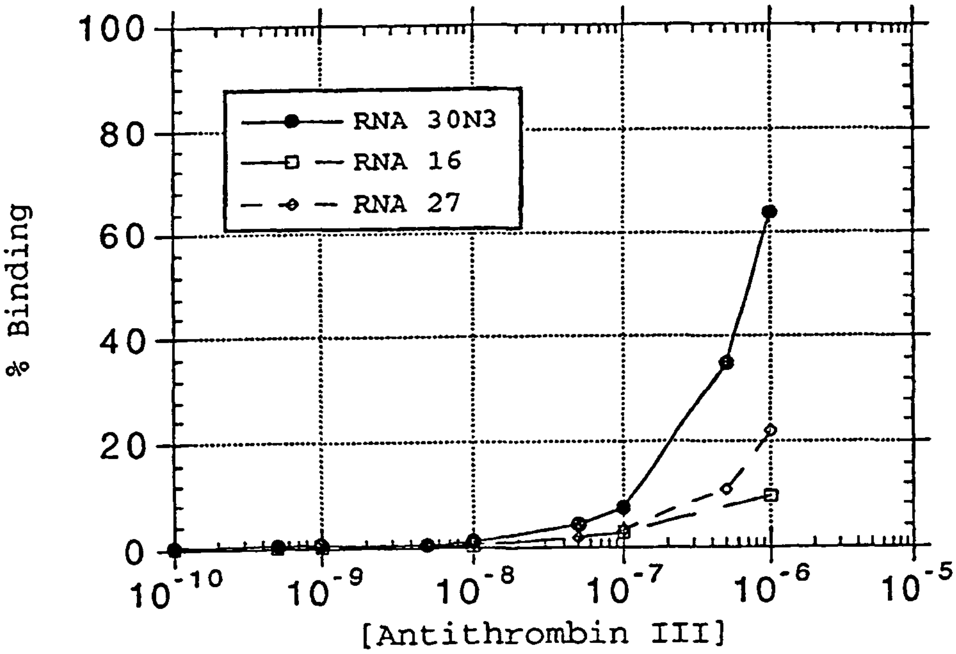


Fig. 35A

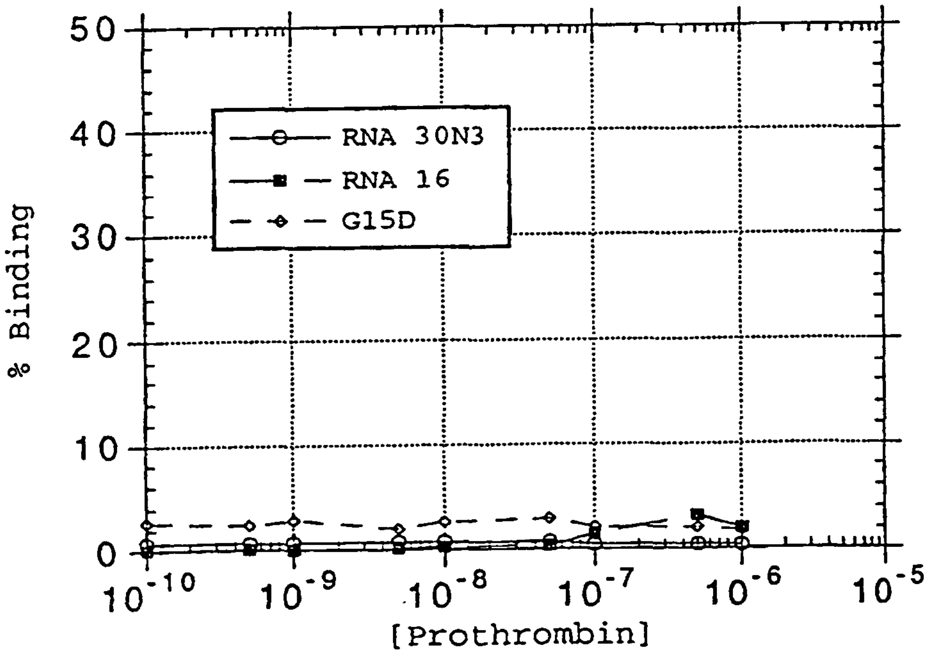


Fig. 35B

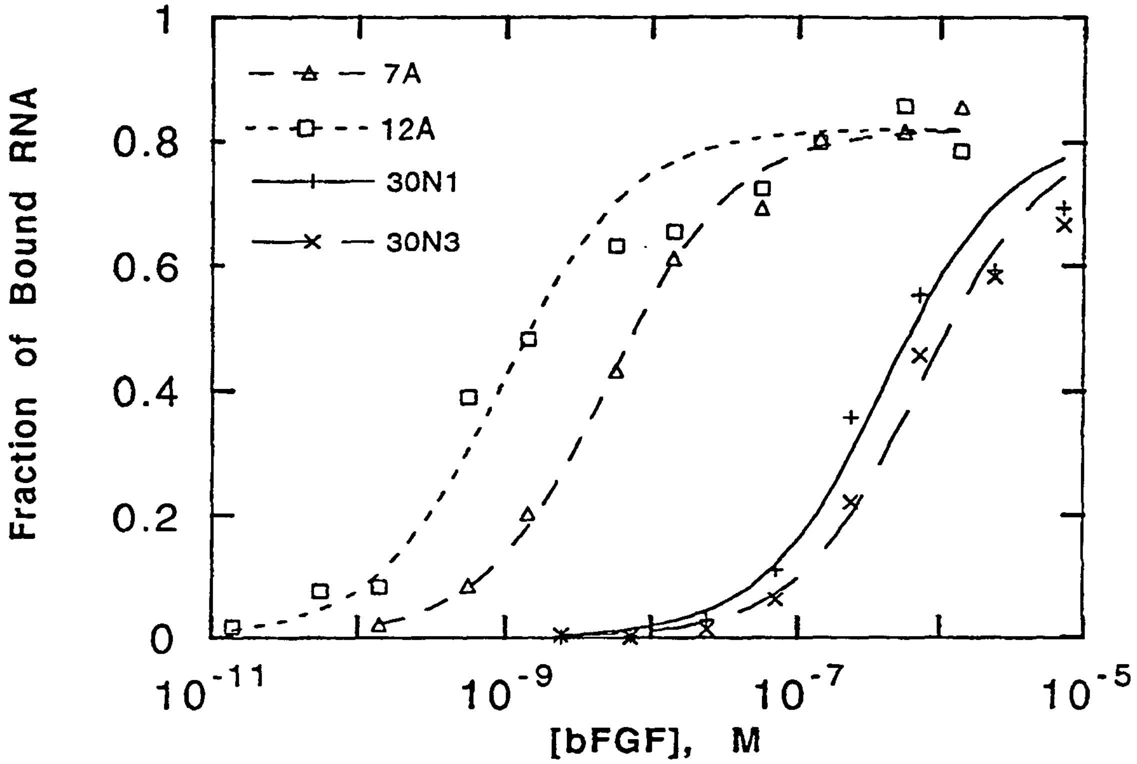

Fraction of Bound RNA
1
0.8
0.6
0.4
0.2
0
7A
12A
30N1
30N3
$10^{-11}$
$10^{-9}$
$10^{-7}$
$10^{-5}$
[bFGF], M


**Fig. 36**

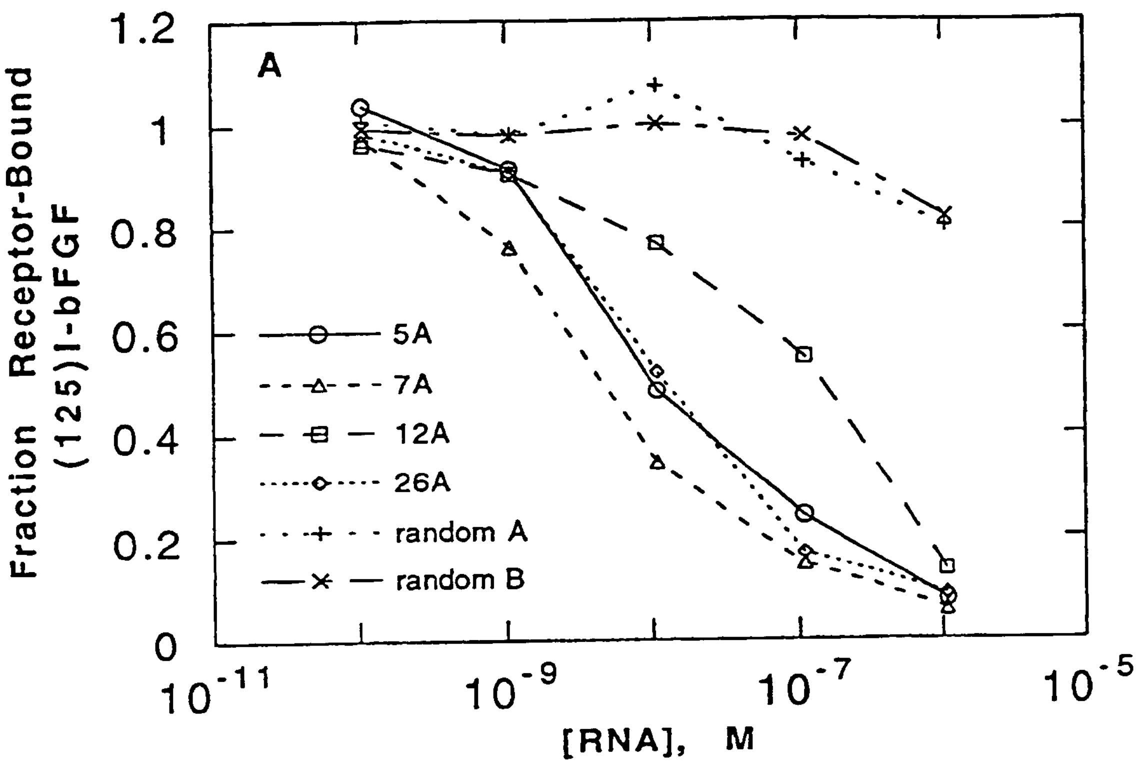
A
1.2
1
0.8
0.6
0.4
0.2
0
Fraction Receptor-Bound
(125)I-bFGF
$10^{-11}$
$10^{-9}$
$10^{-7}$
$10^{-5}$
[RNA], M
5A
7A
12A
26A
random A
random B

Fig. 37A

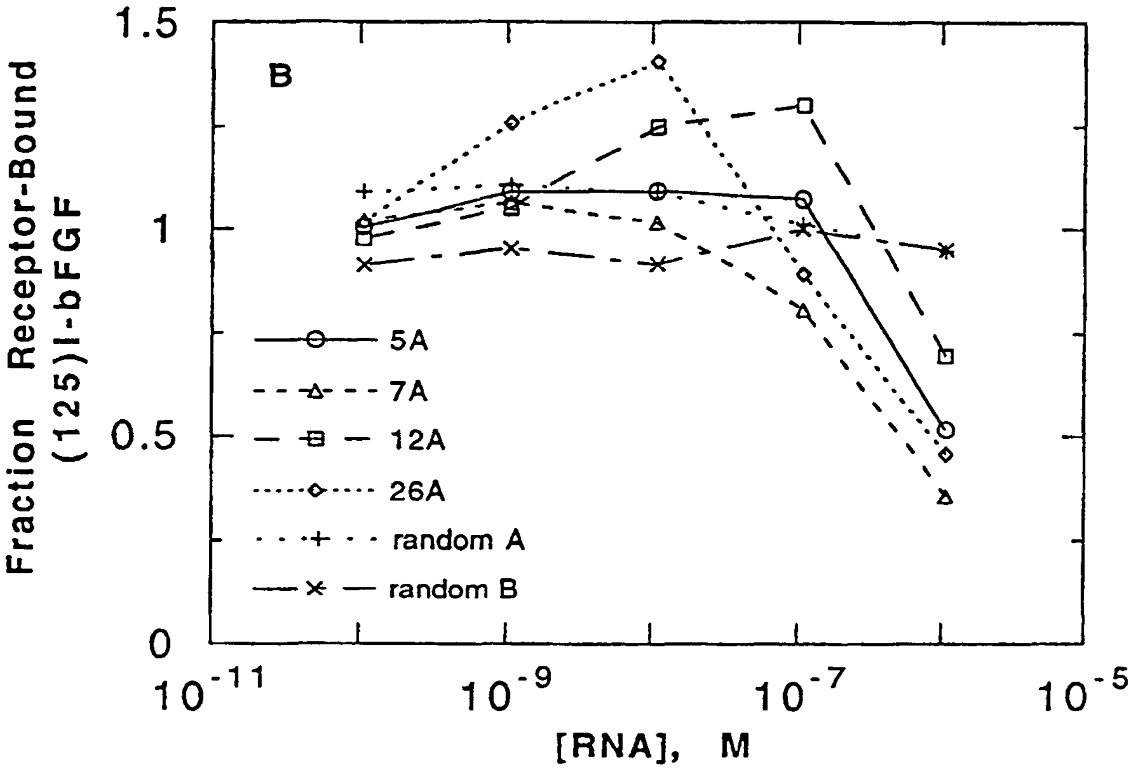
B
Fraction Receptor-Bound (125)I-bFGF
1.5
1
0.5
0
$10^{-11}$
$10^{-9}$
$10^{-7}$
$10^{-5}$
[RNA], M
5A
7A
12A
26A
random A
random B

Fig. 37B

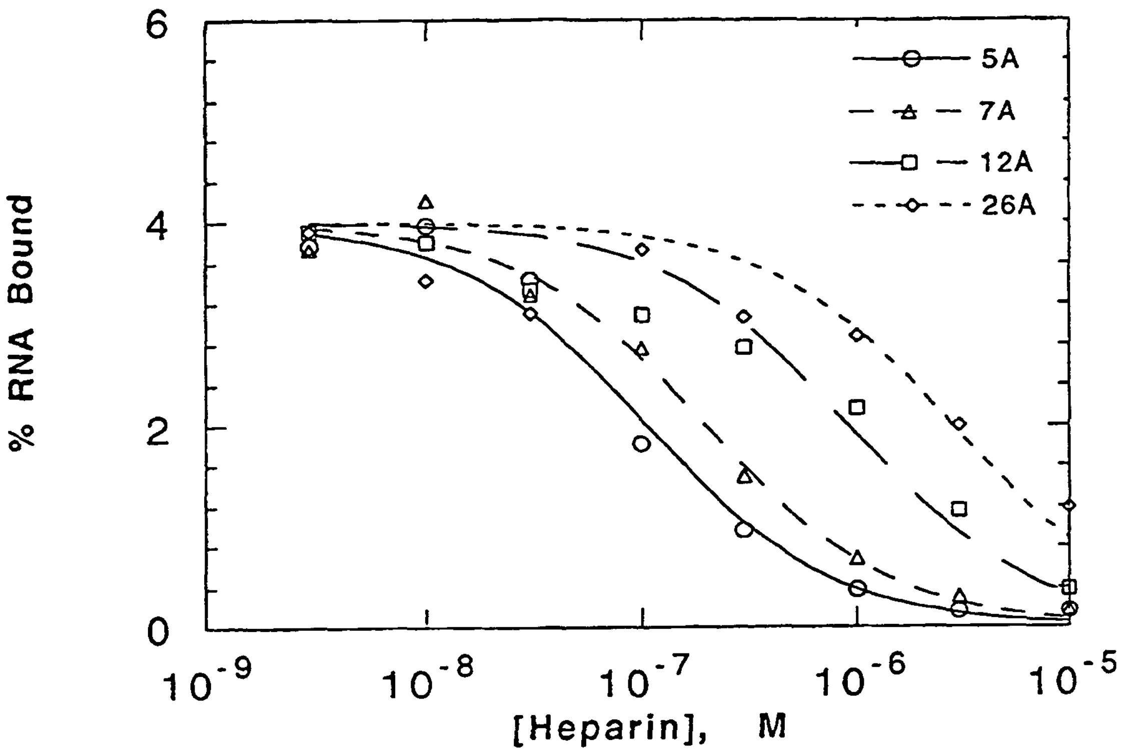


Fig. 38

| | |
|---|---|
| 4A | gggagcucagaauaaacgcucaaUGCUAUUCGCCUAACUCGGCGCUCCUACCUuucgacaugaggcccggauccggc |
| 5A | gggagcucagaauaaacgcucaaAUCUCCUCCCGUCGAAGCUAACCUGGCCACuucgacaugaggcccggauccggc |
| 7A | gggagcucagaauaaacgcucaaUCGGCGAGCUAACCAAGACACUCGCUGCACuucgacaugaggcccggauccggc |
| 10A | gggagcucagaauaaacgcucaaGUAGCACUAUCGGCCUAACCCGGUAGCUCCuucgacaugaggcccggauccggc |
| 13A | gggagcucagaauaaacgcucaaACCCGCGGCCUCCGAAGCUAACCAGGACACuucgacaugaggcccggauccggc |
| 14A | gggagcucagaauaaacgcucaaUGGGUGCUAACCAGGACACACCCACGCUGUuucgacaugaggcccggauccggc |
| 16A | gggagcucagaauaaacgcucaaCACGCACAGCUAACCAAGCCACUGUGCCCCuucgacaugaggcccggauccggc |
| 18A | gggagcucagaauaaacgcucaaCUGCGUGGUAUAACCACAUGCCCUGGGCGAuucgacaugaggcccggauccggc |
| 21A | gggagcucagaauaaacgcucaaUGGGUGCUUAACCAGGCCACACCCUGCUGUuucgacaugaggcccggauccggc |
| 25A | gggagcucagaauaaacgcucaaCUAGGUGCUAUCCAGGACUCUCCCUGGUCCuucgacaugaggcccggauccggc |
| 29A | gggagcucagaauaaacgcucaaUGCUAUUCGCCUAGCUCGGCGCUCCUACCUuucgacaugaggcccggauccggc |
| 38A | gggagcucagaauaaacgcucaaAGCUAUUCGCCCAACCCGGCGCUCCCGACCuucgacaugaggcccggauccggc |
| 39A | gggagcucagaauaaacgcucaaACCAGCUGCGUGCAACCGCACAUGCCUGGuucgacaugaggcccggauccggc |
| 56A | gggagcucagaauaaacgcucaaCAGGCCCCGUCGUAAGCUAACCUGGACCCUuucgacaugaggcccggauccggc |

Fig. 39

| | |
|---|---|
| 11A | gggagcucagaauaaacgcucaaGGGUAACGUUGU--GACAAGUACACCUGCGUCuucgacaugaggcccggauccggc |
| 12A | gggagcucagaauaaacgcucaaGGGGCAACGCUACA-GACAAGUGCACCCAACuucgacaugaggcccggauccggc |
| 26A | gggagcucagaauaaacgcucaaCGUCAGAAGGCAACGUAUA--GGCAAGCACACuucgacaugaggcccggauccggc |
| 27A | gggagcucagaauaaacgcucaaCCUCUCGAAGACAACGCUGU--GACAAGA-CACuucgacaugaggcccggauccggc |
| 47A | gggagcucagaauaaacgcucaaAGUGGGAAACGCUACUUGACAAGA-CACCACuucgacaugaggcccggauccggc |
| 65A | gggagcucagaauaaacgcucaaGGCUACGCUAAU-GACAAGUGCACUUGGGUGuucgacaugaggcccggauccggc |
| 1B | gggagaugccugucgagcaugcugCUCUGGUAACGCAAU--GUCAAGUGCACAUGAguagcuaaacagcuuugucgacggg |
| 2B | gggagaugccugucgagcaugcugAGCCGCAGGUAACGGACC--GGCGAGACCAUUguagcuaaacagcuuugucgacggg |
| 6B | gggagaugccugucgagcaugcugACGAGCUUCGUAACGCUAUC-GACAAGUGCAguagcuaaacagcuuugucgacggg |
| 8B | gggagaugccugucgagcaugcugAAGGGGAAACGUUGA--GUCCGGUACACCCUGGuagcuaaacagcuuugucgacggg |
| 9B | gggagaugccugucgagcaugcugAGGGUAACGUACU--GGCAAGCUCACCUCAGCguagcuaaacagcuuugucgacggg |
| 11B | gggagaugccugucgagcaugcugGAGGUAACGUAC---GACAAGACCACUCCAACUguagcuaaacagcuuugucgacggg |
| 12B | gggagaugccugucgagcaugcugAGGUAACGCUGA--GUCAAGUGCACUCGACAUguagcuaaacagcuuugucgacggg |
| 13B | gggagaugccugucgagcaugcugGGAAACGCUAUC-GACGAGUGCACCCGGCAguagcuaaacagcuuugucgacggg |
| 14B | gggagaugccugucgagcaugcugCCGAGGGUAACGUUGG--GUCAAGCACACCUCguagcuaaacagcuuugucgacggg |
| 15B | gggagaugccugucgagcaugcugUCGGGGUAACGUAUU--GGCAAGG-CACCCGACguagcuaaacagcuuugucgacggg |
| 19B | gggagaugccugucgagcaugcugGGUAACGCUGUG-GACAAGUGCACCAGCUGCguagcuaaacagcuuugucgacggg |
| 22B | gggagaugccugucgagcaugcugAGGGUAACGUACU--GGCAAGCUCACCUCAGCguagcuaaacagcuuugucgacggg |
| 28B | gggagaugccugucgagcaugcugAGGGUAACGUAUA--GUCAAGA-CACCUCAAGUguagcuaaacagcuuugucgacggg |
| 29B | gggagaugccugucgagcaugcugGGGUAACGCAUU--GGCAAGA-CACCCAGCCCguagcuaaacagcuuugucgacggg |
| 36B | gggagaugccugucgagcaugcugGAGGAAACGUACC--GUCGAGC-CACUCCAUGCguagcuaaacagcuuugucgacggg |
| 38B | gggagaugccugucgagcaugcugAGGUAACGCUGA--GUCAAGUGCACUCGACAUguagcuaaacagcuuugucgacggg |
| 48B | gggagaugccugucgagcaugcugGGGUAACGUGU---GACAAGAUCACCCAGUUUGguagcuaaacagcuuugucgacggg |
| 49B | gggagaugccugucgagcaugcugCACAGGGCAACGCUGCU-GACAAGUGCACCUguagcuaaacagcuuugucgacggg |

Fig. 40

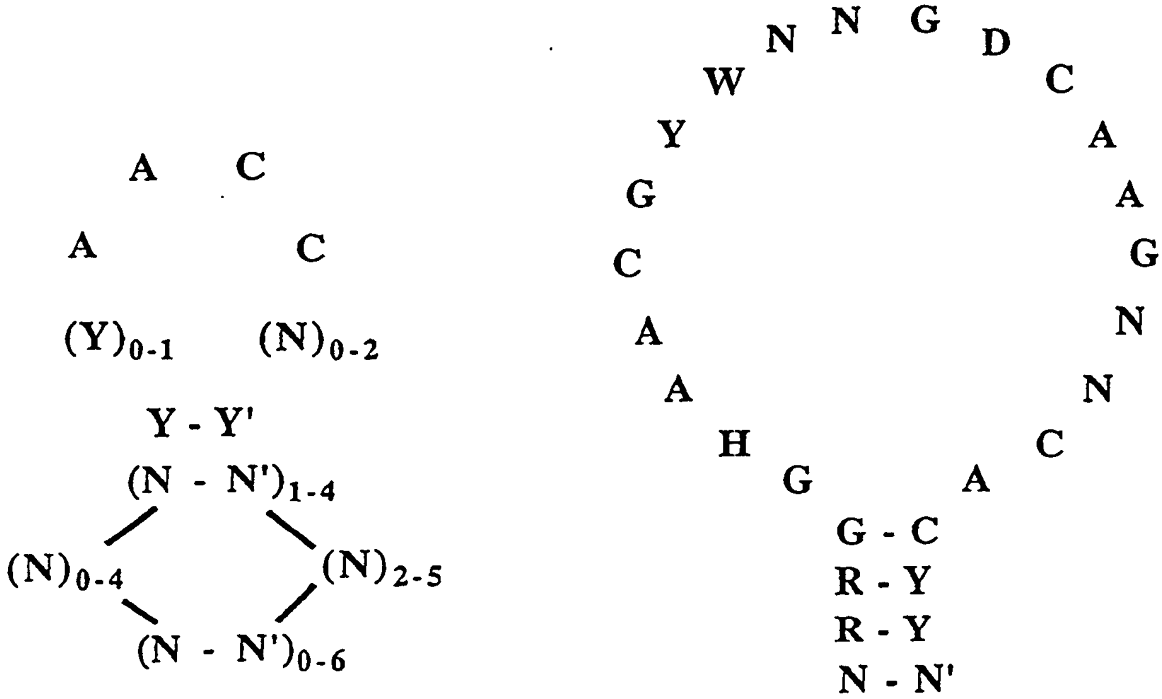


FAMILY 1 FAMILY 2

Fig. 41